# EUROPEAN PATENT APPLICATION

(11) **EP 4 837 910 A1**
(43) Date of publication of application: **11.11.2026**
(21) Application number: 24915110.1
(22) Date of filing: 18.12.2024
(51) Int. Cl.: C07D 413/10, C07D 413/14, C07C 233/08, C07C 327/20, A01N 43/00, A61P 13/00

(54) **ISOXAZOLINE COMPOUND AND PREPARATION METHOD THEREFOR, HERBICIDAL COMPOSITION, AND USE**

(30) Priority: 02.01.2024 CN 202410003452; 08.07.2024 CN 202410910361
(71) Applicant: Qingdao KingAgroot Chemical Compound Co., Ltd., Qingdao, Shandong 266000 (CN)
(72) Inventor: LIAN, Lei, Qingdao, Shandong 266000 (CN); HUA, Rongbao, Qingdao, Shandong 266000 (CN); PENG, Xuegang, Qingdao, Shandong 266000 (CN); GUO, Xiang, Qingdao, Shandong 266000 (CN)
(74) Representative: Cabinet Becker et Associés
(86) International application number: PCT/CN2024/140225
(87) International publication number: WO 2025/145895

(57) **Abstract**

The present invention belongs to the technical field of pesticides, and specifically relates to an isoxazoline compound and a preparation method therefor, an herbicidal composition, and the use. The compound is as represented by general formula (I), wherein X is an aryl or heterocyclic group; Y is a halogen, a cyano group, an alkyl, etc.; Z is hydrogen, a halogen, an alkyl, etc.; R₁ is hydrogen, an alkyl, etc.; R₂ is hydrogen, an alkyl, etc.; R₃ is O, NH, or an N-alkyl; and Q₁ and Q₂ are respectively independently O or S. The compound has excellent herbicidal activity and crop safety.

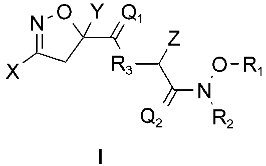

## Description

### Technical Field

The present invention belongs to the technical field of pesticides, particularly relating to an isoxazoline compound, as well as a preparation method therefor, a herbicidal composition and an application thereof.

### Background Art

Weed control is one of the most important links in the course of achieving high-efficiency agriculture. Although various herbicides are available in the market, the herbicidal properties against harmful plants and the selectivity to crops of these known compounds are not completely satisfactory. In addition, due to the continuous expansion of the market, issues such as resistance of weeds and service life and economy of drugs, and people's increasing attention to the environment, scientists are expected to continuously study and further develop new efficient, safe and economical herbicide varieties with different modes of action.

### Summary of the Invention

The present invention provides an isoxazoline compound, as well as a preparation method therefor, a herbicidal composition and an application thereof. The compound has excellent herbicidal activity and crop safety.

The technical solution adopted in the present invention is as follows:
An isoxazoline compound or its salt is as shown in Formula I: 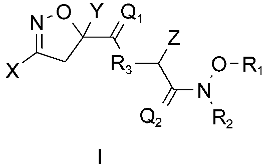
wherein, X is aryl or heterocyclyl;
Y is halogen, cyano, alkyl, alkenyl, alkynyl, cycloalkyl, cycloalkylalkyl, cycloalkenyl, cycloalkenylalkyl, haloalkyl, haloalkenyl, haloalkynyl, -OR, -SR, -alkylene-OR, -alkylene-O(CO)R, -alkylene-SR, -(CO)OR, or -(CO)R;
Z is hydrogen, halogen, alkyl, alkenyl, alkynyl, cycloalkyl, cycloalkylalkyl, cycloalkenyl, cycloalkenylalkyl, haloalkyl, haloalkenyl, haloalkynyl, cyanoalkyl, -OR, -SR, -N(R)₂, -alkylene-OR, -alkylene-SR, -alkylene-N(R)₂, -(CO)OR, -alkylene-(CO)OR, -alkylene-(CO)N(R)₂, -(CO)N(R)₂, aryl, heterocyclyl, arylalkyl, or heterocyclylalkyl;
R₁ is hydrogen, alkyl, alkenyl, alkynyl, cycloalkyl, cycloalkenyl, -alkylene-OR, -alkylene-SR, -alkylene-(SO)R, -alkylene-(SO)₂R, -alkylene-(CO)OR, -alkylene-(CO)N(R)₂, -alkylene-(CO)NR(OR), -alkylene-O-N=C(R)₂, -alkylene-(CO)O-N=C(R)₂, aryl, heterocyclyl; or alkyl, alkenyl, or alkynyl that are substituted by at least one group selected from halogen, cycloalkyl, cycloalkenyl, heterocyclyl, and aryl;
R₂ is hydrogen, alkyl, alkenyl, alkynyl, cycloalkyl, cycloalkylalkyl, cycloalkenyl, cycloalkenylalkyl, haloalkyl, haloalkenyl, haloalkynyl, aryl, heterocyclyl, arylalkyl, heterocyclylalkyl, -alkylene-OR, -alkylene-SR, cyanoalkyl, -(CO)OR, -SO₂R, or -SO₂N(R)₂;
or R₁ and R₂ together form -(CH₂)ₙ- that is unsubstituted or substituted by at least one group selected from alkyl, halogen, and oxo;
n is 3, 4, or 5;
R₃ is O, NH, or N-alkyl;
Q₁ and Q₂ each independently represent O or S;
the aforementioned cycloalkyl, cycloalkenyl, aryl, or heterocyclyl are optionally substituted by at least one group selected from oxo, halogen, cyano, nitro, alkyl, alkenyl, alkynyl, cycloalkyl, cycloalkylalkyl, haloalkyl, haloalkenyl, haloalkynyl, -OR, -SR, -alkylene-OR, -alkylene-SR, -O(CO)R, -(CO)R, -(CO)OR, -(CO)N(R)₂, -SO₂R, -OSO₂R, -N(R)₂, and -NR(OR);
R each independently represents hydrogen, alkyl, alkenyl, alkynyl; alkyl, alkenyl or alkynyl that are substituted by at least one group selected from halogen, hydroxy, alkoxy, cyano, trialkylsilyl, phenyl, and alkoxycarbonyl; cycloalkyl, cycloalkylalkyl, cycloalkenyl, cycloalkenylalkyl, phenyl, heterocyclyl; phenyl or heterocyclyl that are substituted by at least one group selected from halogen, cyano, nitro, alkyl, haloalkyl, alkoxycarbonyl, alkylsulfanyl, alkylsulfonyl, alkoxy, haloalkoxy, phenyl, and halophenyl; or N(R)₂ together forms

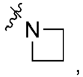

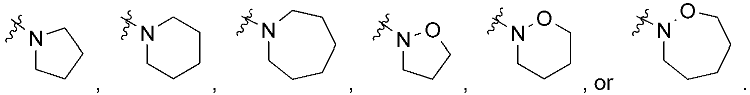

In a specific embodiment, Y is halogen, cyano, C1-C8 alkyl, C2-C8 alkenyl, C2-C8 alkynyl, C3-C8 cycloalkyl, C3-C8 cycloalkyl C1-C8 alkyl, C3-C8 cycloalkenyl, C3-C8 cycloalkenyl C1-C8 alkyl, halo C1-C8 alkyl, halo C2-C8 alkenyl, halo C2-C8 alkynyl, -OR, -SR, -(C1-C8 alkylene)-OR, -(C1-C8 alkylene)-O(CO)R, -(C1-C8 alkylene)-SR, -(CO)OR, or -(CO)R;
Z is hydrogen, halogen, C1-C8 alkyl, C2-C8 alkenyl, C2-C8 alkynyl, C3-C8 cycloalkyl, C3-C8 cycloalkyl C1-C8 alkyl, C3-C8 cycloalkenyl, C3-C8 cycloalkenyl C1-C8 alkyl, halo C1-C8 alkyl, halo C2-C8 alkenyl, halo C2-C8 alkynyl, cyano C1-C8 alkyl, -OR, -SR, -N(R)₂, -(C1-C8 alkylene)-OR, -(C1-C8 alkylene)-SR, -(C1-C8 alkylene)-N(R)₂, -(CO)OR, -(C1-C8 alkylene)-(CO)OR, -(C1-C8 alkylene)-(CO)N(R)₂, -(CO)N(R)₂, aryl, heterocyclyl, aryl C1-C8 alkyl, or heterocyclyl C1-C8 alkyl;
R₁ is hydrogen, C1-C8 alkyl, C2-C8 alkenyl, C2-C8 alkynyl, C3-C8 cycloalkyl, C3-C8 cycloalkenyl, -(C1-C8 alkylene)-OR, -(C1-C8 alkylene)-SR, -(C1-C8 alkylene)-(SO)R, -(C1-C8 alkylene)-(SO)₂R, -(C1-C8 alkylene)-(CO)OR, -(C1-C8 alkylene)-(CO)N(R)₂, -(C1-C8 alkylene)-(CO)NR(OR), -(C1-C8 alkylene)-O-N=C(R)₂, -(C1-C8 alkylene)-(CO)O-N=C(R)₂, aryl, heterocyclyl; or C1-C8 alkyl, C2-C8 alkenyl or C2-C8 alkynyl that are substituted by at least one group selected from halogen, C3-C8 cycloalkyl, C3-C8 cycloalkenyl, heterocyclyl, and aryl;
R₂ is hydrogen, C1-C8 alkyl, C2-C8 alkenyl, C2-C8 alkynyl, C3-C8 cycloalkyl, C3-C8 cycloalkyl C1-C8 alkyl, C3-C8 cycloalkenyl, C3-C8 cycloalkenyl C1-C8 alkyl, halo C1-C8 alkyl, halo C2-C8 alkenyl, halo C2-C8 alkynyl, aryl, heterocyclyl, aryl C1-C8 alkyl, heterocyclyl C1-C8 alkyl, -(C1-C8 alkylene)-OR, -(C1-C8 alkylene)-SR, cyano C1-C8 alkyl, -(CO)OR, -SO₂R, or -SO₂N(R)₂;
or R₁ and R₂ together form -(CH₂)ₙ- that is unsubstituted or substituted by at least one group selected from C1-C8 alkyl, halogen, and oxo;
n is 3, 4, or 5;
R₃ is O, NH, or N-(C1-C8 alkyl);
the aforementioned C3-C8 cycloalkyl, C3-C8 cycloalkenyl, aryl, or heterocyclyl are optionally substituted by at least one group selected from oxo, halogen, cyano, nitro, C1-C8 alkyl, C2-C8 alkenyl, C2-C8 alkynyl, C3-C8 cycloalkyl, C3-C8 cycloalkyl C1-C8 alkyl, halo C1-C8 alkyl, halo C2-C8 alkenyl, halo C2-C8 alkynyl, -OR, -SR, -(C1-C8 alkylene)-OR, -(C1-C8 alkylene)-SR, -O(CO)R, -(CO)R, -(CO)OR, -(CO)N(R)₂, -SO₂R, -OSO₂R, -N(R)₂, and -NR(OR);
R each independently represents hydrogen, C1-C8 alkyl, C2-C8 alkenyl, C2-C8 alkynyl; C1-C8 alkyl, C2-C8 alkenyl or C2-C8 alkynyl that are substituted by at least one group selected from halogen, hydroxy, C1-C8 alkoxy, cyano, tri C1-C8 alkylsilyl, phenyl, and C1-C8 alkoxycarbonyl; C3-C8 cycloalkyl, C3-C8 cycloalkyl C1-C8 alkyl, C3-C8 cycloalkenyl, C3-C8 cycloalkenyl C1-C8 alkyl, phenyl, heterocyclyl; phenyl or heterocyclyl that are substituted by at least one group selected from halogen, cyano, nitro, C1-C8 alkyl, halo C1-C8 alkyl, C1-C8 alkoxycarbonyl, C1-C8 alkylsulfanyl, C1-C8 alkylsulfonyl, C1-C8 alkoxy, halo C1-C8 alkoxy, phenyl, and halophenyl; or N(R)₂ together forms

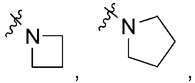

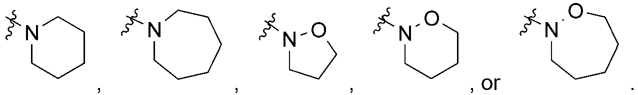

In another specific embodiment, Y is halogen, cyano, C1-C6 alkyl, C2-C6 alkenyl, C2-C6 alkynyl, C3-C6 cycloalkyl, C3-C6 cycloalkyl C1-C6 alkyl, C3-C6 cycloalkenyl, C3-C6 cycloalkenyl C1-C6 alkyl, halo C1-C6 alkyl, halo C2-C6 alkenyl, halo C2-C6 alkynyl, -OR, -SR, -(C1-C6 alkylene)-OR, -(C1-C6 alkylene)-O(CO)R, -(C1-C6 alkylene)-SR, -(CO)OR, or -(CO)R;
Z is hydrogen, halogen, C1-C6 alkyl, C2-C6 alkenyl, C2-C6 alkynyl, C3-C6 cycloalkyl, C3-C6 cycloalkyl C1-C6 alkyl, C3-C6 cycloalkenyl, C3-C6 cycloalkenyl C1-C6 alkyl, halo C1-C6 alkyl, halo C2-C6 alkenyl, halo C2-C6 alkynyl, cyano C1-C6 alkyl, -OR, -SR, -N(R)₂, -(C1-C6 alkylene)-OR, -(C1-C6 alkylene)-SR, -(C1-C6 alkylene)-N(R)₂, -(CO)OR, -(C1-C6 alkylene)-(CO)OR, -(C1-C6 alkylene)-(CO)N(R)₂, -(CO)N(R)₂, aryl, heterocyclyl, aryl C1-C6 alkyl, or heterocyclyl C1-C6 alkyl;
R₁ is hydrogen, C1-C6 alkyl, C2-C6 alkenyl, C2-C6 alkynyl, C3-C6 cycloalkyl, C3-C6 cycloalkenyl, -(C1-C6 alkylene)-OR, -(C1-C6 alkylene)-SR, -(C1-C6 alkylene)-(SO)R, -(C1-C6 alkylene)-(SO)₂R, -(C1-C6 alkylene)-(CO)OR, -(C1-C6 alkylene)-(CO)N(R)₂, -(C1-C6 alkylene)-(CO)NR(OR), -(C1-C6 alkylene)-O-N=C(R)₂, -(C1-C6 alkylene)-(CO)O-N=C(R)₂, aryl, heterocyclyl; or C1-C6 alkyl, C2-C6 alkenyl or C2-C6 alkynyl that are substituted by at least one group selected from halogen, C3-C6 cycloalkyl, C3-C6 cycloalkenyl, heterocyclyl, and aryl;
R₂ is hydrogen, C1-C6 alkyl, C2-C6 alkenyl, C2-C6 alkynyl, C3-C6 cycloalkyl, C3-C6 cycloalkyl C1-C6 alkyl, C3-C6 cycloalkenyl, C3-C6 cycloalkenyl C1-C6 alkyl, halo C1-C6 alkyl, halo C2-C6 alkenyl, halo C2-C6 alkynyl, aryl, heterocyclyl, aryl C1-C6 alkyl, heterocyclyl C1-C6 alkyl, -(C1-C6 alkylene)-OR, -(C1-C6 alkylene)-SR, cyano C1-C6 alkyl, -(CO)OR, -SO₂R, or -SO₂N(R)₂;
or R₁ and R₂ together form -(CH₂)ₙ- that is unsubstituted or substituted by at least one group selected from C1-C6 alkyl, halogen, and oxo;
n is 3, 4, or 5;
R₃ is O, NH, or N-(C1-C6 alkyl);
the aforementioned C3-C6 cycloalkyl, C3-C6 cycloalkenyl, aryl, or heterocyclyl are optionally substituted by at least one group selected from oxo, halogen, cyano, nitro, C1-C6 alkyl, C2-C6 alkenyl, C2-C6 alkynyl, C3-C6 cycloalkyl, C3-C6 cycloalkyl C1-C6 alkyl, halo C1-C6 alkyl, halo C2-C6 alkenyl, halo C2-C6 alkynyl, -OR, -SR, -(C1-C6 alkylene)-OR, -(C1-C6 alkylene)-SR, -O(CO)R, -(CO)R, -(CO)OR, -(CO)N(R)₂, -SO₂R, -OSO₂R, -N(R)₂, and -NR(OR);
R each independently represents hydrogen, C1-C6 alkyl, C2-C6 alkenyl, C2-C6 alkynyl; C1-C6 alkyl, C2-C6 alkenyl or C2-C6 alkynyl that are substituted by at least one group selected from halogen, hydroxy, C1-C6 alkoxy, cyano, tri C1-C6 alkylsilyl, phenyl, and C1-C6 alkoxycarbonyl; C3-C6 cycloalkyl, C3-C6 cycloalkyl C1-C6 alkyl, C3-C6 cycloalkenyl, C3-C6 cycloalkenyl C1-C6 alkyl, phenyl, heterocyclyl; phenyl or heterocyclyl that are substituted by at least one group selected from halogen, cyano, nitro, C1-C6 alkyl, halo C1-C6 alkyl, C1-C6 alkoxycarbonyl, C1-C6 alkylsulfanyl, C1-C6 alkylsulfonyl, C1-C6 alkoxy, halo C1-C6 alkoxy, phenyl, and halophenyl; or N(R)₂ together forms

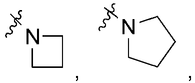

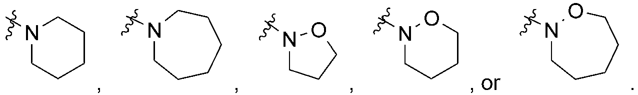

In another specific embodiment, R₃ is NH; Q₁ and Q₂ each independently represent O.

In another specific embodiment, R₁ is hydrogen, alkyl, alkenyl, alkynyl, cycloalkyl, cycloalkylalkyl, cycloalkenyl, cycloalkenylalkyl, haloalkyl, haloalkenyl, haloalkynyl, -alkylene-(CO)OR, -alkylene-(CO)N(R)₂, -alkylene-O-N=C(R)₂, aryl, heterocyclyl, arylalkyl, or heterocyclylalkyl.

In another specific embodiment, R₁ is hydrogen, C1-C8 alkyl, C2-C8 alkenyl, C2-C8 alkynyl, C3-C8 cycloalkyl, C3-C8 cycloalkyl C1-C8 alkyl, C3-C8 cycloalkenyl, C3-C8 cycloalkenyl C1-C8 alkyl, halo C1-C8 alkyl, halo C2-C8 alkenyl, halo C2-C8 alkynyl, -(C1-C8 alkylene)-(CO)OR, -(C1-C8 alkylene)-(CO)N(R)₂, -(C1-C8 alkylene)-O-N=C(R)₂, aryl, heterocyclyl, aryl C1-C8 alkyl, or heterocyclyl C1-C8 alkyl.

In another specific embodiment, R₁ is hydrogen, C1-C6 alkyl, C2-C6 alkenyl, C2-C6 alkynyl, C3-C6 cycloalkyl, C3-C6 cycloalkyl C1-C6 alkyl, C3-C6 cycloalkenyl, C3-C6 cycloalkenyl C1-C6 alkyl, halo C1-C6 alkyl, halo C2-C6 alkenyl, halo C2-C6 alkynyl, -(C1-C6 alkylene)-(CO)OR, -(C1-C6 alkylene)-(CO)N(R)₂, -(C1-C6 alkylene)-O-N=C(R)₂, aryl, heterocyclyl, aryl C1-C6 alkyl, or heterocyclyl C1-C6 alkyl.

In the definitions of the compounds represented by the above-mentioned general formula and all of the structural formulas hereinafter, the technical terms used, whether used alone or used in compound words, represent the following substituent groups: an alkyl group which has more than two carbon atoms and may be linear or branched. For example, the alkylene in the compound word "-alkylene-OR" may be -CH₂-, -CH₂CH₂-, -CH(CH₃)-, -C(CH₃)₂-, etc. An alkyl group is, for example, C1 alkyl: methyl; C2 alkyl: ethyl; C3 alkyl: propyl such as n-propyl or isopropyl; C4 alkyl: butyl such as n-butyl, isobutyl, tert-butyl, or 2-butyl; C5 alkyl: pentyl such as n-pentyl; C6 alkyl: hexyl such as n-hexyl, isohexyl, or 1,3-dimethylbutyl. Similarly, alkenyl is, for example, vinyl, allyl, 1-methylprop-2-en-1-yl, 2-methylprop-2-en-1-yl, but-2-en-1-yl, but-3-en-1-yl, 1-methylbut-3-en-1-yl, or 1-methylbut-2-en-1-yl. Alkynyl is, for example, ethynyl, propargyl, but-2-yn-1-yl, but-3-yn-1-yl, or 1-methylbut-3-yn-1-yl. A multiple bond may be at any position of each unsaturated group. Cycloalkyl is a saturated carbocyclic ring system having for example three to six carbon atoms such as cyclopropyl, cyclobutyl, cyclopentyl, or cyclohexyl. Similarly, cycloalkenyl is monocyclic alkenyl having for example three to six carbon ring members such as cyclopropenyl, cyclobutenyl, cyclopentenyl, or cyclohexenyl, wherein a double bond can be at any position. A halogen is fluorine, chlorine, bromine, or iodine.

Unless otherwise specified, the "aryl" of the present invention includes, but is not limited to, phenyl, naphthyl, 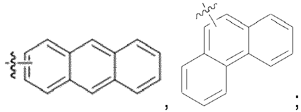 the "heterocyclyl" not only includes, but is not limited to, saturated or unsaturated non-aromatic cyclic groups 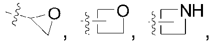 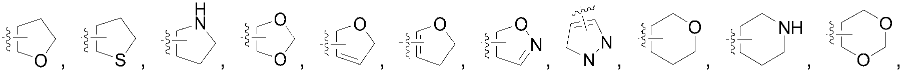 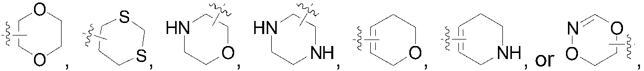 etc., but also includes, but is not limited to, heteroaryl, that is an aromatic cyclic group having for example 3 to 6 ring atoms and also optionally fused with a benzo ring. One to four (for example, 1, 2, 3, or 4) heteroatoms of the ring atoms are selected from oxygen, nitrogen, and sulfur, for example

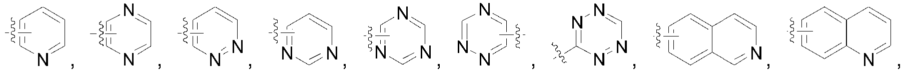

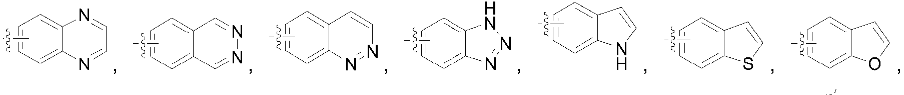

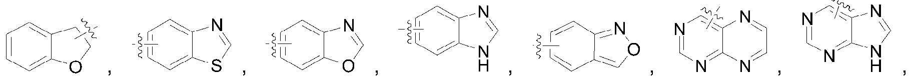

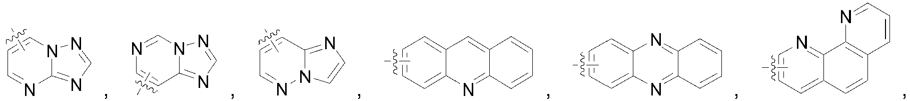

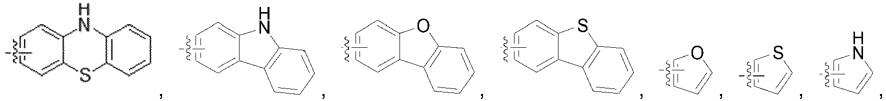

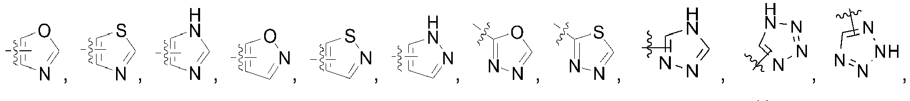

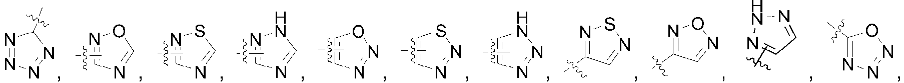

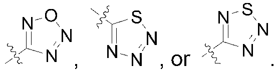

If one group is substituted by a group, this should be understood to mean that the group is substituted by one or more groups, which may be the same or different, selected from the mentioned groups. In addition, the same or different substitution characters contained in the same or different substituent groups are independently selected, which may be the same or different. This is also applicable to a ring system formed by different atoms and units. Meanwhile, the scope of the claims will exclude those compounds chemically unstable under standard conditions which are known to a person skilled in the art.

In addition, unless specifically defined, the term "substituted by at least one group" herein refers to being substituted by, for example, 1, 2, 3, 4, or 5 groups; a group (including heterocyclyl, aryl, etc.) without being specified a linking site may be linked at any position, including a site connected to C or N; if it is substituted, the substituent group may also substitute at any position as long as the valence bond theory is complied with. For example, the heteroaryl 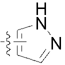 substituted by a methyl may represent 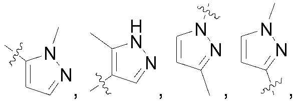 etc.

The present invention provides an isoxazoline compound with chiral center(s) as shown in Formula I' or a salt thereof: 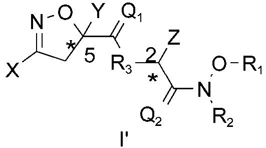
wherein, the carbon atom(s) (C*) at position 2 and/or 5 is a chiral center, and the definitions of substituents R₁, R₂, R₃, Q₁, Q₂, X, Y, and Z are as described as above and Z is not hydrogen;
based on the contents of stereoisomers that have R- and S-configuration at position 2, it has a stereochemical purity of 60%-100% (*S*), preferably 70%-100% (*S*), more preferably 80%-100% (*S*), further preferably 90%-100% (*S*), even further preferably 95%-100% (*S*); or it has a stereochemical purity of 60%-100% (*R*), preferably 70%-100% (*R*), more preferably 80%-100% (*R*), further preferably 90%-100% (*R*), even further preferably 95%-100% (*R*);
and/or on an occasion of the carbon atom at position 5 being a chiral center, based on the contents of stereoisomers that have *R*- and *S*-configuration at this position, it has a stereochemical purity of 60%-100% (*S*), preferably 70%-100% (*S*), more preferably 80%-100% (*S*), further preferably 90%-100% (*S*), even further preferably 95%-100% (*S*); or it has a stereochemical purity of 60%-100% (*R*), preferably 70%-100% (*R*), more preferably 80%-100% (*R*), further preferably 90%-100% (*R*), even further preferably 95%-100% (*R*).

Among them, "stereochemical purity" refers to the percentage of the amount of the stereoisomers to the total amount of stereoisomers with chiral center(s).

In the present invention, the stereochemical configuration at the position marked with * in Formula I is determined as the main (*R*) or (*S*) according to the Cahn-Ingold-Prelog system. However, the subject matter of the present invention also relates to all stereoisomers at other positions encompassed by Formula I, and mixtures thereof. Such compounds of Formula I contain, for example, one or more additional asymmetric carbon atoms or other double bonds that are not specified in Formula I. It is to be understood that the present invention comprises pure isomers and mixtures thereof of pure isomers enriched to varying degrees, wherein, the asymmetric carbon atom at the position marked with * is in *R*-configuration or *S*-configuration; or in the mixture, a compound or a compound of the same chemical structure has *R*-configuration or *S*-configuration at the position marked with *, or is present in such a proportion where the compound with *R*-configuration or *S*-configuration is predominantly present (at least 60% having *R*-configuration or *S*-configuration); meanwhile, other asymmetric carbon atoms may exist in racemic forms or may be resolved to varying degrees. As long as the stereochemical configuration at a position marked with * is eligible, possible stereoisomers defined by particular spatial forms such as enantiomers, diastereoisomers, *Z*- and *E*-isomers, are all encompassed by Formula I, and may be obtained from mixtures of stereoisomers using conventional methods, or may be prepared by stereoselective reactions in combination with the use of stereochemically pure starting materials.

The present invention also includes any keto-enol tautomeric form, a mixture and salt thereof, if various functional groups are present.

Stereoisomers are obtainable from the mixtures prepared by optical resolution. Stereoisomers may also be selectively prepared by employing stereoselective reactions and using optically active starting materials and/or auxiliaries. For optical resolution, conventional methods can often be employed (see *Textbooks of Stereochemistry*), such as those described below for resolving mixtures into diastereoisomers, for example, physical methods, e.g., crystallization, chromatography, particularly column chromatography and high-pressure liquid chromatography, distillation methods carried out under reduced pressure as needed, extraction and other methods. Chromatographic separation on a chiral solid phase is usually adopted to separate residual mixtures of enantiomers. Suitable for a preparative amount or for use on an industrial scale are methods such as crystallization of diastereomeric salts, which can be obtained from compounds using optically active acids, and if acidic groups are present, optically active bases can be employed as needed.

A method for preparing the isoxazoline compound comprises the following steps:
(1) subjecting the compound shown in the general formula II and the compound shown in the general formula III or its salt (e.g., a hydrochloride) to reaction to produce the compound as shown in the general formula I, and the reaction equation is as follows: 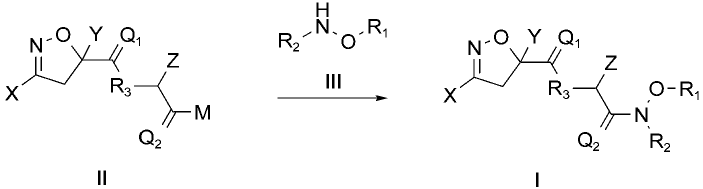
or (2) subjecting the compound shown in the general formula IV and the compound shown in the general formula V or its salt (e.g., a hydrochloride) to reaction to produce the compound as shown in the general formula I, and the reaction equation is as follows: 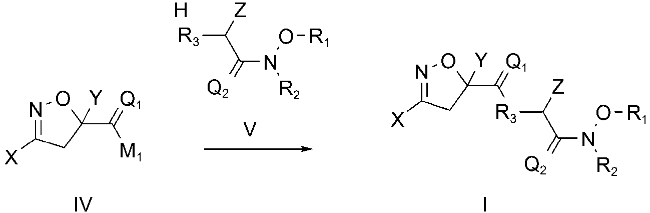
or (3) subjecting the compound shown in the general formula VI and the compound shown in the general formula VII to reaction to produce the compound as shown in the general formula I, and the reaction equation is as follows: 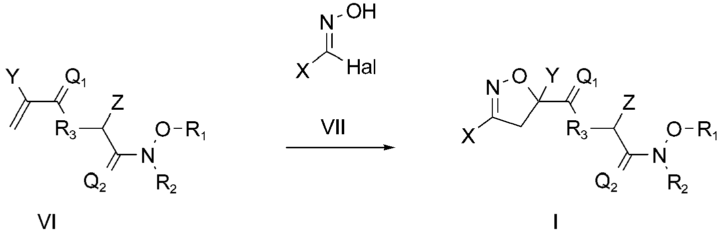
wherein, M and M₁ each independently represent OH or halogen; Hal represents halogen; the definitions of substituents R₁, R₂, R₃, Q₁, Q₂, X, Y, and Z are as described as above.

In a specific embodiment, the reactions (1) and (2) are carried out in the presence of a solvent, respectively; in another specific embodiment, a base and/or a condensing agent is added during the reactions.

In another specific embodiment, the base is selected from at least one of inorganic bases (e.g., K₂CO₃, Na₂CO₃, C_{S2}CO₃, NaHCO₃, KHCO₃, KF, CsF, KI, Nal, K₃PO₄, K₂HPO₄, NaOH, KOH, NaH, KH, etc.) and organic bases (e.g., DMAP, pyrazole, triethylamine, DIEA/DIPEA, potassium trimethylsilanolate, AcOK, AcONa, MeONa, EtONa, t-BuONa, etc.).

In another specific embodiment, the condensing agent is selected from at least one of Py-BOP, Py-AOP, EDCI, HOBT, DCC, HBTU, and HATU.

In another specific embodiment, the solvent is selected from at least one of DMF, DMA, acetonitrile, dichloroethane, DMSO, dioxane, dichloromethane, tetrahydrofuran, and ethyl acetate.

In a specific embodiment, the reaction (3) is carried out in the presence of a base and a solvent.

In another specific embodiment, the base is selected from at least one of inorganic bases (e.g., K₂CO₃, Na₂CO₃, C_{S2}CO₃, NaHCO₃, KHCO₃, KF, CsF, KI, Nal, K₃PO₄, K₂HPO₄, NaOH, KOH, NaH, KH, etc.) and organic bases (e.g., DMAP, pyrazole, triethylamine, DIEA/DIPEA, potassium trimethylsilanolate, AcOK, AcONa, MeONa, EtONa, t-BuONa, etc.).

In another specific embodiment, the solvent is selected from at least one of methanol, ethanol, isopropanol, DMF, DMA, acetonitrile, dichloroethane, DMSO, dioxane, dichloromethane, tetrahydrofuran, and ethyl acetate.

In another specific embodiment, when M is OH, the compound shown in Formula II is prepared by hydrolysis of the compound shown in Formula IV, and the chemical reaction equation is as follows:

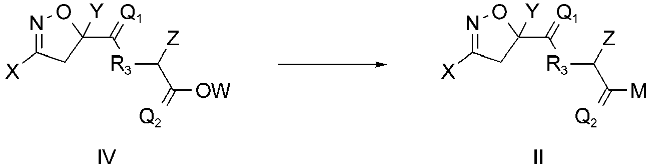

The compound shown in Formula IV is prepared by reaction between the compound shown in Formula V and the compound shown in Formula VI, and the chemical reaction equation is as follows: 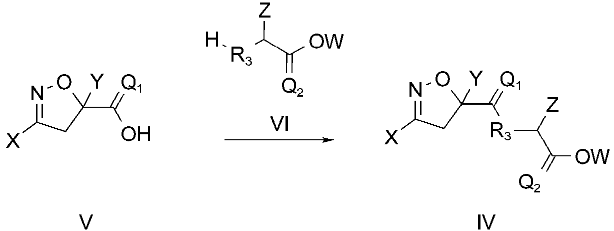 wherein, W represents C1-C6 alkyl or phenyl.

In addition, the compound of the present invention can be prepared with reference to the methods set forth in CN201280017007.3, etc.

An intermediate is as shown in Formula II, III, V, or VI.

A herbicidal composition comprises a herbicidally effective amount of at least one of the isoxazoline compounds; preferably, also comprising a formulation auxiliary; more preferably, also comprising an additional active ingredient.

A method for controlling weeds comprises applying a herbicidally effective amount of at least one of the isoxazoline compounds or the herbicidal composition to a plant or a weedy area.

At least one of the isoxazoline compounds or the herbicidal composition has use in weed control; preferably, the isoxazoline compound is used to control weeds among useful crops, and the useful crops are transgenic crops or crops treated by genome editing techniques.

The compounds of Formula I of the present invention have outstanding herbicidal activity against a broad spectrum of monocotyledonous and dicotyledonous harmful plants of economic importance. The active substances of the present invention also act effectively on perennial weeds which grow from root stocks, rhizomes, or other perennial organs and are difficult to control. In this context, it is generally immaterial whether the substances are applied before sowing, before emergence, or after emergence. Representative examples of monocotyledonous and dicotyledonous weed florae which can be controlled by the compound of the present invention may be specifically mentioned, without limiting to certain species. Examples of weed species on which the active substances act efficiently include the monocotyledons such as annual *Avena, Lolium, Alopecurus, Phalaris, Echinochloa, Digitaria, Setaria* and *Cyperus,* as well as perennial *Agropyron, Cynodon, Imperata* and *Sorghum,* and perennial *Cyperus.*

In the case of dicotyledonous weed species, the spectrum of action extends to species such as annual *Galium, Viola, Veronica, Lamium, Stellaria, Amaranthus, Sinapis, Ipomoea, Sida, Matricaria* and *Abutilon,* as well as perennial weeds *Convolvulus, Cirsium, Rumex,* and *Artemisia.* The active substances of the present invention perform outstanding control of harmful plants such as *Echinochloa, Sagittaria, Alisma, Eleocharis, Scirpus* and *Cyperus* under the specific condition of paddy rice growing. If the compounds of the present invention are applied to soil surface prior to germination, the weed seedlings are either prevented completely from emerging, or the weeds stop growing when reaching the cotyledon stage and eventually die completely after three to four weeks. In particular, the compounds of the present invention exhibit excellent activity against *Apera spica venti, Matsumurella chinense, Fallopia convolvulus, Stellaria media, Veronica hederifolia, Veronica persica, Viola tricolor, Amaranthus, Galium,* and *Kochia.*

Although the compounds of the present invention have excellent herbicidal activity against monocotyledonous and dicotyledonous weeds, there is no damage at all to crop plants of economic importance such as wheat, barley, rye, rice, maize, sugarbeet, cotton and soybean, or the damage is negligible. In particular, the compounds have excellent compatibility with cereals such as wheat, barley, and maize, in particular wheat. Therefore, the compounds of the present invention are highly suitable for selectively controlling undesired plants in plantings for agricultural or ornamental use.

Owing to their herbicidal properties, these active substances may be employed for controlling harmful plants in plantings of genetically engineered plants that are known or to be introduced. Transgenic plants usually have advantageous traits, for example, resistance to certain pesticides, in particular to certain herbicides; resistance to plant diseases or pathogenic microorganisms of plant diseases such as certain insects or microorganisms including fungi, bacteria, or viruses. Other particular traits relate to the following conditions of the product, for example, quantity, quality, storage stability, composition, and special ingredients. Thus, it is known that the obtained transgenic plant product has an increased starch content, or modified starch quality, or a different fatty acid composition.

The compounds of Formula I of the present invention or salts thereof are preferably used in plantings of transgenic crops and ornamental plants of economic importance, for example, cereals such as wheat, barley, rye, oats, millet, rice, manioc, and maize; or in plantings of vegetable plants such as sugarbeet, cotton, soybean, rapeseed, potato, tomato, pea and the like. The compounds of Formula I are preferably used as herbicides in plantings of useful plants which are resistant, or have been made resistant by genetic engineering, against the toxic effects of the herbicides.

Conventional ways for breeding plants which have modified traits compared to known plants include, for example, conventional breeding methods and breeding of mutant strains. In other words, novel plants having improved traits may be generated with the aid of genetic engineering methods (see, e.g., EP-0221044 A, EP-0131624 A). For example, several methods have been described:
- changing crop plants using genetic engineering to modify the starch synthesis in plants (e.g., WO 92/11376, WO 92/14827, WO 91/19806);
- transgenic crop plants resistant to certain herbicides, including glufosinate herbicides (e.g., EP-0242236 A, EP-0242246 A), or glyphosate herbicides (WO 92/00377), or sulfonylurea herbicides (EP-0257993 A, US-5013659 A);
- transgenic crop plants, for example cotton, able to produce *Bacillus thuringiensis* toxins (Bt toxins) which impart resistance to certain pests that invade the plants (EP-0142924 A, EP-0193259 A);
- transgenic crop plants having a modified fatty acid composition (WO 91/13972).

Numerous molecular biology techniques which allow preparation of a transgenic plant having a modified trait are known (see, e.g., Sambrook et al., 1989, Molecular Cloning, A Laboratory Manual, 2nd edition, Cold Spring Harbor Laboratory Press, Cold Spring Harbor, N.Y.; or Winnacker, "Gene und Klone" [Genes and Clones], VCH Weinheim, 2nd edition, 1996; or Christou, "Trends in Plant Science", 1 (1996) 423-431). In order to carry out genetic engineering manipulations, it is possible to introduce a nucleic acid molecule into a plasmid, allowing mutagenesis or a sequence change to occur by recombination of the DNA sequence. Using the above-mentioned standard processes, it is possible to, for example, substitute bases, remove partial sequences, or add natural or synthetic sequences. To link DNA fragments with each other, it is possible to attach adaptors or linkers to the fragments.

A plant cell having a gene product of reduced activity may be prepared by the following methods, for example, by expressing at least one appropriate antisense-RNA and sense-RNA to achieve a cosuppression effect, or by expressing at least one appropriately constructed ribozyme which specifically cleaves the transcripts of the above-mentioned gene product.

To this end, it is possible to employ a DNA molecule which comprises all the coding sequences of the gene product including any flanking sequence that may be present and a DNA molecule which comprises only parts of the coding sequences that must be long enough to cause an antisense effect in the cells. It is also possible to use sequences which have a high degree of homology but are not entirely identical to the coding sequences of the gene product.

When nucleic acid molecules are expressed in a plant, the synthesized protein may be localized in any desired compartment of the plant cell. However, to achieve localization in a certain compartment, it is possible, for example, to link the coding region with the DNA sequences to ensure localization in the certain location. Such sequences are known to a person skilled in the art (see, e.g., Braun et al., EMBO J., 11 (1992), 3219-3227; Wolter et al., Proc. Natl. Acad. Sci. USA, 65 (1988), 846-850; Sonnewald et al., Plant J., 1 (1991), 95-106).

The transgenic plant cells can be recombined onto a whole plant using known techniques. A transgenic plant may be of any desired plant species, i.e., a monocotyledonous and dicotyledonous plant. In this manner, it is possible to obtain a transgenic plant having a modified trait by overexpression, suppression, or inhibition of a homologous (= natural) gene or gene sequence, or by expression of a heterologous (= foreign) gene or gene sequence.

When using the active substances of the present invention in a transgenic crop, in addition to the inhibitory effect against harmful plants which can be observed in other crops, there are frequently special effects on the corresponding transgenic crop, for example, improving or broadening the spectrum of weeds which can be controlled, modifying the application amount during application, excellent combination of the drug resistance of the preferred transgenic crop and herbicide performance, as well as effects on the growth and the yield of the transgenic crop plant. The present invention therefore also provides use of the compounds as herbicides for controlling harmful plants among transgenic crop plants.

In addition, the compounds of the present invention are able to significantly regulate the growth of a crop plant. These compounds are employed for the targeted control of plant constituents and for facilitating harvesting by engaging in plant metabolism in a regulating manner, for example by provoking desiccation and stunted growth. Furthermore, they are also suitable for regulating and inhibiting undesirable plant growth without destroying the growth of crop plants. Inhibition of plant growth plays a key role in many monocotyledonous and dicotyledonous crops as it can reduce or completely prevent lodging.

The compounds of the present invention may be applied in customary formulations in the form of wettable powders, emulsifiable concentrates, sprayable solutions, dusts, or granules. The present invention therefore also provides herbicidal compositions comprising the compounds of Formula I. The compounds of Formula I may be formulated in numerous ways depending on the prevailing physical parameters in biology and/or chemistry. Examples of suitable formulation options are: wettable powders (WP), water-soluble powders (SP), water-soluble concentrates, emulsifiable concentrates (EC), emulsions such as oil-in-water and water-in-oil emulsions (EW), sprayable solutions, suspension concentrates (SC), oil dispersions (OD), oil- or water-based dispersions, oil-miscible solutions, dust powders (DP), capsule suspensions (CS), seed-dressing compositions, granules for broadcasting and soil application, spray granules, coating granules and adsorption granules, water-dispersible granules (WG), water-soluble granules (SG), ULV (Ultra-low volume) formulations, microcapsules, and waxes. These individual formulation types are known and described in the following literature, for example, Winnacker-Küchler, "Chemische Technologie" [Chemical Technology], Volume 7, C. Hauser Verlag Munich, 4th edition, 1986; Wade van Valkenburg, "Pesticide Formulations", Marcel Dekker, N.Y., 1973; K. Martens, "Spray Drying" Handbook, 3rd edition, 1979, G. Goodwin Ltd. London.

Necessary formulation auxiliaries such as inert materials, surfactants, solvents and other additives are likewise known and described in the following documents, for example, Watkins, "Handbook of Insecticide Dust Diluents and Carriers", 2nd edition, Dorland Books, Caldwell N.J.; H. v. Olphen, "An Introduction to Clay Colloid Chemistry", 2nd edition, J. Wiley & Sons, N.Y.; C. Marsden, "Solvents Guide", 2nd edition, Interscience, N.Y. 1963; "McCutcheon's Detergents and Emulsifiers Annual", MC Publ. Corp., Ridgewood N.J.; Sisley and Wood, "Encyclopedia of Surface Active Agents", Chem. Publ. Co. Inc., N.Y., 1964; Schönfeldt, "Grenzflüchenaktive Äthylenoxidaddkte" [Surface-active ethylene oxide adducts], Wiss. Verlagagesell., Stuttgart, 1976; Winnacker-Küchler, "Chemische Technologie" [Chemical Technology], Volume 7, C. Hauser Verlag Munich, 4th edition, 1986.

Wettable powders are uniformly dispersible in water which contain, in addition to the active substance, a diluent or inert substance, ionic and nonionic surfactant (wetting agent, dispersant), for example polyethoxylated alkyl phenol, polyethoxylated fatty alcohol, polyethoxylated fatty amine, fatty alcohol polyglycol ether sulfate, alkane sulfonate, alkylphenyl sulfonate, sodium lignosulfonate, sodium 2,2'-dinaphthylmethane-6,6'-disulfonats, sodium dibutylnaphthalenesulfonate, or sodium methyl oleoyl taurate. To prepare the wettable powders, the herbicidally active substance is finely ground, for example, in a customary apparatus such as hammer mill, fan mill, and air-jet mill, and mixed with formulation auxiliaries simultaneously or sequentially.

Emulsifiable concentrates are prepared by dissolving the active substance in an organic solvent, for example butanol, cyclohexanone, dimethylformamide, xylene, or aromatic compounds with relatively high boiling point, or hydrocarbons or mixtures of the solvents, with the addition of one or more ionic and/or nonionic surfactants (emulsifiers). Examples of emulsifiers which can be used are calcium alkylarylsulfonates such as calcium dodecylbenzene sulfonate, or nonionic emulsifiers such as fatty acid polyglycol esters, alkylaryl polyglycol ethers, fatty alcohol polyglycol ethers, propylene oxide-ethylene oxide condensation products, alkyl polyethers, sorbitan esters such as sorbitan fatty acid esters, or polyoxyethylene sorbitan esters such as polyoxyethylene sorbitan fatty acid esters.

Dusts are obtained by grinding the active substance with finely-divided solid substances, for example talc, natural clays such as kaolin, bentonite and pyrophyllite, or diatomaceous earth. Water- or oil-based suspensions may be prepared by the following methods, for example, by wet milling using a commercially customary bead mill, with or without the addition of the surfactants mentioned above in the case of another formulation type.

Emulsions such as oil-in-water emulsions (EW) may be prepared by means of stirrers, colloid mills and/or static mixers using aqueous organic solvents, and, if desired, the surfactants as mentioned above in the case of another formulation type may be added.

Granules may be prepared by the following methods that either spraying the active substance onto the adsorptive and granulating with inert materials, or concentrating the active substance onto the surface of carriers such as sand and kaolinite and granulating inert materials by means of adhesive binders such polyvinyl alcohol, sodium polyacrylate, or mineral oils. Suitable active substances may also be granulated in the manner which is customary for the preparation of fertilizer granules. If desired, fertilizers may be mixed in. Water-dispersible granules are prepared by customary methods such as spray-drying, fluidized-bed granulation, disk granulation, or mixing using high-speed mixers and extrusion without solid inert materials.

For the preparation methods of granules using disk, fluidized-bed, extruder and spray, see the following processes in, for example, "Spray-Drying Handbook" 3rd edition, 1979, G. Goodwin Ltd., London; J. E. Browning, "Agglomeration", Chemical and Engineering, 1967, pages 147 ff.; "Perry's Chemical Engineer's Handbook", 5th edition, McGraw-Hill, New York 1973, pp. 8-57. For further details on the formulation of crop protection products, see, for example G. C. Klingman, "Weed Control as a Science", John Wiley and Sons Inc., New York, 1961, pages 81-96 and J. D. Freyer, S. A. Evans, "Weed Control Handbook", 5th edition, Blackwell Scientific Publications, Oxford, 1968, pages 101-103.

An agrochemical formulation generally contains from 0.1 to 99% by weight, in particular from 0.1 to 95%, of the active substance of Formula I. In a wettable powder, the concentration of the active substance is, for example, from about 10 to 99% by weight, and the remainder to 100% by weight consists of customary formulation constituents. In an emulsifiable concentrate, the concentration of the active substance may be from about 1 to 90%, preferably from 5 to 80%, by weight. A dust formulation contains from 1 to 30% by weight of the active substance, preferably from 5 to 20% by weight of the active substance in usual cases, while a sprayable solution contains from about 0.05 to 80% by weight, preferably from 2 to 50%, of the active substance. In the case of water-dispersible granules, the content of the active substance mainly depends on whether the active substance is liquid or solid and on auxiliaries, fillers, etc. that are used during granulation. In water-dispersible granules, the content of the active substance, for example, is between 1 and 95% by weight, preferably between 10 and 80% by weight.

In addition, formulations of the active substances may include tackifiers, wetting agents, dispersants, emulsifiers, penetrants, preservatives, antifreeze agents, solvents, fillers, carriers, colorants, defoamers, evaporation suppressors as well as pH and viscosity modifiers which are usually customary in all cases.

Based on these formulations, it is also possible to produce mixtures with other pesticidally active substances, for example insecticides, acaricides, herbicides and fungicides, and also with safeners, fertilizers and/or plant growth regulators, in a manner of pre-mix or tank mix.

In mixed formulations or tank-mix formulations, suitable active substances which can be mixed with the active substances of the present invention are the known substances described, for example in World Herbicide New Product Technology Handbook, China Agricultural Science and Farming Techniques Press, 2010. 9 and in the literature cited herein. For example, the herbicidally active substances mentioned below may be mixed with the compounds of Formula I (noted that the compounds are either named by the "common name" in accordance with the International Organization for Standardization (ISO) or by the chemical names, accompanied with a customary code number if appropriate): acetochlor, butachlor, alachlor, propisochlor, metolachlor, S-metolachlor, pretilachlor, propachlor, ethachlor, napropamide, napropamide-M, propanil, mefenacet, diphenamid, diflufenican, ethaprochlor, beflubutamid, bromobutide, dimethenamid, dimethenamid-P, etobenzanid, flufenacet, thenylchlor, metazachlor, isoxaben, flamprop-M-methyl, flamprop-M-propyl, allidochlor, pethoxamid, chloranocryl, cypromid, mefluidide, monalide, delachlor, prynachlor, terbuchlor, xylachlor, dimethachlor, cisanilide, trimexachlor, clomeprop, propyzamide, pentanochlor, carbetamide, benzoylprop-ethyl, cyprazole, butenachlor, tebutam, benzipram, quinonamid, dichlofluanid, naproanilide, diethatyl-ethyl, naptalam, flufenacet, EL-177, benzadox, chlorthiamid, chlorophthalimide, isocarbamide, picolinafen, atrazine, simazine, prometryn, cyanatryn, simetryn, ametryn, propazine, dipropetryn, SSH-108, terbutryn, terbuthylazine, triaziflam, cyprazine, proglinazine, trietazine, prometon, simeton, aziprotryne, desmetryn, dimethametryn, procyazine, mesoprazine, sebuthylazine, secbumeton, terbumeton, methoprotryne, cyanatryn, ipazine, chlorazine, atraton, pendimethalin, eglinazine-ethyl, cyanuric acid, indaziflam, chlorsulfuron, metsulfuron-methyl, bensulfuron-methyl, chlorimuron-ethyl, tribenuron-methyl, thifensulfuron-methyl, pyrazosulfuron-ethyl, mesosulfuron, iodosulfuron-methyl sodium, foramsulfuron, cinosulfuron, triasulfuron, sulfometuron methyl, nicosulfuron, ethametsulfuron-methyl, amidosulfuron, ethoxysulfuron, cyclosulfamuron, rimsulfuron, azimsulfuron, flazasulfuron, monosulfuron, monosulfuron-ester, flucarbazone-sodium, flupyrsulfuron-methyl, halosulfuron-methyl, oxasulfuron, imazosulfuron, primisulfuron, propoxycarbazone, prosulfuron, sulfosulfuron, trifloxysulfuron, triflusulfuron-methyl, tritosulfuron, sodium metsulfuron methyl, flucetosulfuron, HNPC-C9908, orthosulfamuron, propyrisulfuron, metazosulfuron, acifluorfen, fomesafen, lactofen, fluoroglycofen, oxyfluorfen, chlornitrofen, aclonifen, ethoxyfen-ethyl, bifenox, nitrofluorfen, chlomethoxyfen, fluorodifen, fluoronitrofen, furyloxyfen, nitrofen, TOPE, DMNP, PPG1013, AKH-7088, halosafen, chlortoluron, isoproturon, linuron, diuron, dymron, fluometuron, benzthiazuron, methabenzthiazuron, cumyluron, ethidimuron, isouron, tebuthiuron, buturon, chlorbromuron, methyldymron, phenobenzuron, SK-85, metobromuron, metoxuron, monolinuron, monuron, siduron, fenuron, fluothiuron, neburon, chloroxuron, noruron, isonoruron, cycluron, thiazfluron, tebuthiuron, difenoxuron, parafluron, methylamine tribunil, karbutilate, trimeturon, dimefuron, monisouron, anisuron, methiuron, chloreturon, tetrafluron, phenmedipham, phenmedipham-ethyl, desmedipham, asulam, terbucarb, barban, propham, chlorpropham, rowmate, swep, chlorbufam, carboxazole, chlorprocarb, fenasulam, BCPC, CPPC, carbasulam, butylate, benthiocarb, vernolate, molinate, triallate, dimepiperate, esprocarb, pyributicarb, cycloate, di-allate, EPTC, ethiolate, orbencarb, pebulate, prosulfocarb, tiocarbazil, CDEC, dimexano, isopolinate, methiobencarb, 2,4-D-butyl, MCPA-sodium, 2,4-D-isooctyl, MCPA-isooctyl, 2,4-D-sodium, 2,4-D-dimethylammonium, MCPA-thioethyl, MCPA, dichlorprop, salts of dichlorprop-P, 2,4-DB, mecoprop, salts of mecoprop, MCPB, 2,4,5-T, fenoprop, 2,4,5-TB, MCPA- ammonium, dicamba, erbon, chlorfenac, disul, 2,3,6-TBA, chloramben, methoxy-2,3,6-TBA, diclofop-methyl, fluazifop-butyl, fluazifop-P-butyl, haloxyfop-methyl, haloxyfop-P, quizalofop-ethyl, quizalofop-P-ethyl, fenoxaprop-ethyl, fenoxaprop-P-ethyl, propaquizafop, cyhalofop-butyl, metamifop, clodinafop-propargyl, fenthiaprop-ethyl, chlorazifop-propargyl, poppenate-methyl, trifopsime, isoxapyrifop, paraquat, diquat, oryzalin, ethalfluralin, isopropalin, nitralin, profluralin, prodinamine, benfluralin, fluchloraline, dinitramina, dipropalin, chlornidine, methalpropalin, dinoprop, glyphosate, anilofos, glufosinate ammonium, amiprophos-methyl, sulphosate, piperophos, bialaphos, bensulide, butamifos, fosamine-ammonium, 2,4-DEP, H-9201, DMPA, imazapyr, imazethapyr, imazaquin, imazamox, imazamox-ammonium, imazapic, imazamethabenz-methyl, fluroxypyr, fluroxypyr-isooctyl ester, clopyralid, picloram, trichlopyr, dithiopyr, haloxydine, pyriclor, thiazopyr, fluridone, aminopyralid, diflufenzopyr, triclopyr-butotyl, cliodinate, sethoxydim, clethodim, cycloxydim, alloxydim, profoxydim, butroxydim, tralkoxydim, tepraloxydim, buthidazole, metribuzin, hexazinone, metamitron, ethiozin, ametridione, amibuzin, bromoxynil, bromoxynil octanoate, ioxynil octanoate, ioxynil, dichlobenil, diphenylacetonitrile, pyraclonil, chloroxynil, iodobonil, flumetsulam, florasulam, penoxsulam, metosulam, cloransulam-methyl, diclosulam, pyroxsulam, benzofluor, bispyribac, pyribenzoxim, pyriftalid, pyriminobac-methyl, pyrithiobac-sodium, benzobicylon, mesotrione, sulcotrione, tembotrione, tefuryltrione, bicyclopyrone, ketospiradox, isoxaflutole, isoxachlortole, fenoxasulfone, methiozolin, fluazolate, pyraflufen-ethyl, pyrazolynate, difenzoquat, pyrazoxyfen, benzofenap, nipyraclofen, pyrasulfotole, topramezone, pyroxasulfone, cafenstrole, flupoxam, amitrole, amicarbazone, azafenidin, carfentrazone, sulfentrazone, bencarbazone, benzfendizone, butafenacil, bromacil, isocil, lenacil, terbacil, flupropacil, cinidon-ethyl, flumiclorac, flumioxazin, flumipropyn, MK-129, flumezin, (sodium)pentachlorophenol, dinoseb, dinoterb, dinoterb acetate, dinosam, DNOC, chloronitrophene, medinoterb acetate, dinofenate, oxadiargyl, oxadiazon, pentoxazone, profluazol, fluthiacet-methyl, fentrazamide, flufenpyr-ethyl, pyrazon, brompyrazon, metflurazon, kusakira, dimidazon, oxapyrazon, norflurazon, pyridafol, quinclorac, quinmerac, bentazone, pyridate, oxaziclomefone, benazolin-ethyl, clomazone, cinmethylin, pyribambenz-isopropyl, pyribambenz-propyl, indanofan, sodium chlorate, dalapon, trichloroacetic acid, monochloroacetic acid, hexachloroacetone, flupropanate, cyperquat, bromofenoxim, epronaz, methazole, flurtamone, benfuresate, ethofumesate, tioclorim, chlorthal, fluorochloridone, tavron, acrolein, bentranil, tridiphane, chlorfenprop-methyl, thidiazimin, phenisopham, busoxinone, methoxyphenone, saflufenacil, clacyfos, chloropon, alorac, diethamquat, etnipromid, iprymidam, ipfencarbazone, thiencarbazone-methyl, pyrimisulfan, chlorflurazole, tripropindan, sulglycapin, prosulfalin, cambendichlor, aminocyclopyrachlor, rodethanil, benoxacor, fenclorim, flurazole, fenchlorazole, cloquintocet-mexyl, oxabetrinil, MG191, cyometrinil, DKA-24, mefenpyr-diethyl, furilazole, fluxofenim, isoxadifen-ethyl, dichlormid, halauxifen-methyl, DOW848, UBH-509, D489, LS 82-556, KPP-300, NC-324, NC-330, KH-218, DPX-N8189, SC-0744, DOWCO535, DK-8910, V-53482, PP-600, MBH-001, KIH-9201, ET751, KIH-6127, and KIH-2023.

For use, the commercially available formulations are, if needed, diluted in customary manners, for example diluted with water in the cases of wettable powders, emulsifiable concentrates, dispersions, and water-dispersible granules. For products in the form of dusts, granules for soil application, or solutions for broadcasting and spray, usually no further dilution with inert materials is required prior to use. The required application amount of the compounds of Formula I varies with the external conditions such as temperature, humidity, the nature of the used herbicide and the like. It may have a large variation range, for example, between 0.001 and 1.0 kg a.i./ha, or more active substances, but preferably between 0.005 and 750 g a.i./ha, in particular between 0.005 and 250 g a.i./ha.

### Detailed Embodiments of the Invention

The following examples are used to illustrate the present invention and should not be construed as limiting the present invention in any way. The scope of rights claimed by the present invention is described in the Claims.

In view of the economy and diversity of the compounds, some compounds were preferably synthesized. Among the many synthesized compounds, selected ones are listed in Table 1 below. The specific compound structure and corresponding chemical data are as set forth in Table 1. The compounds in Table 1 are only to better illustrate the present invention but do not limit the present invention. For a person skilled in the art, it should not be understood that the scope of the above-mentioned subject matters of the present invention is limited to the following compounds.

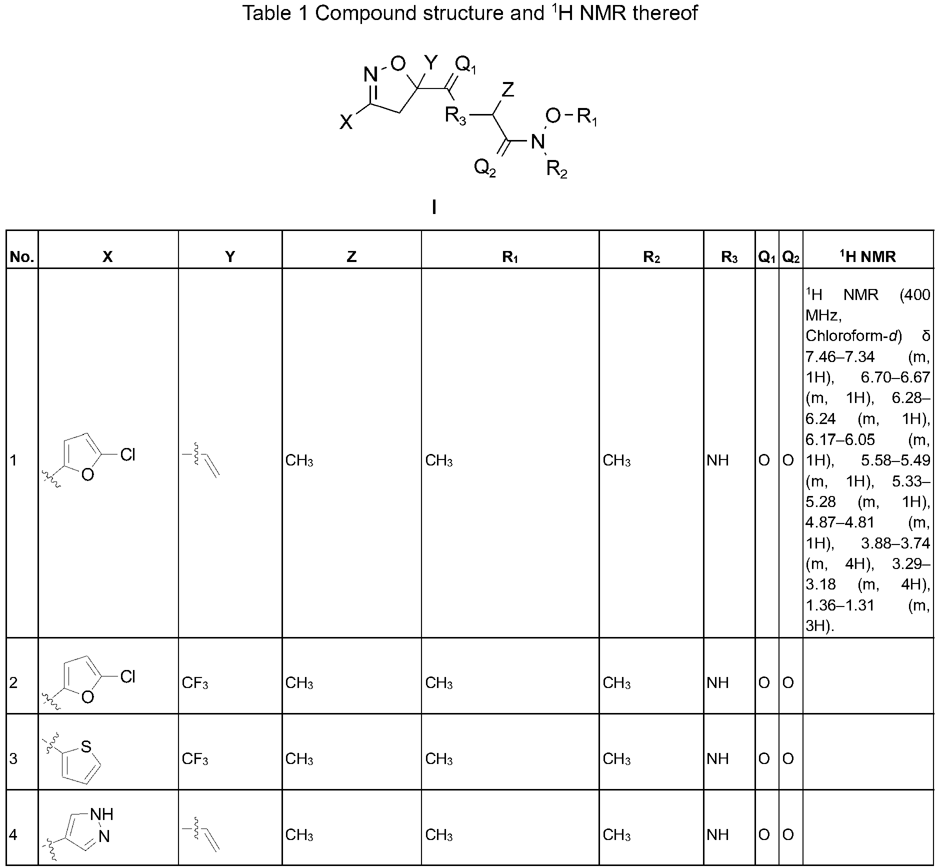

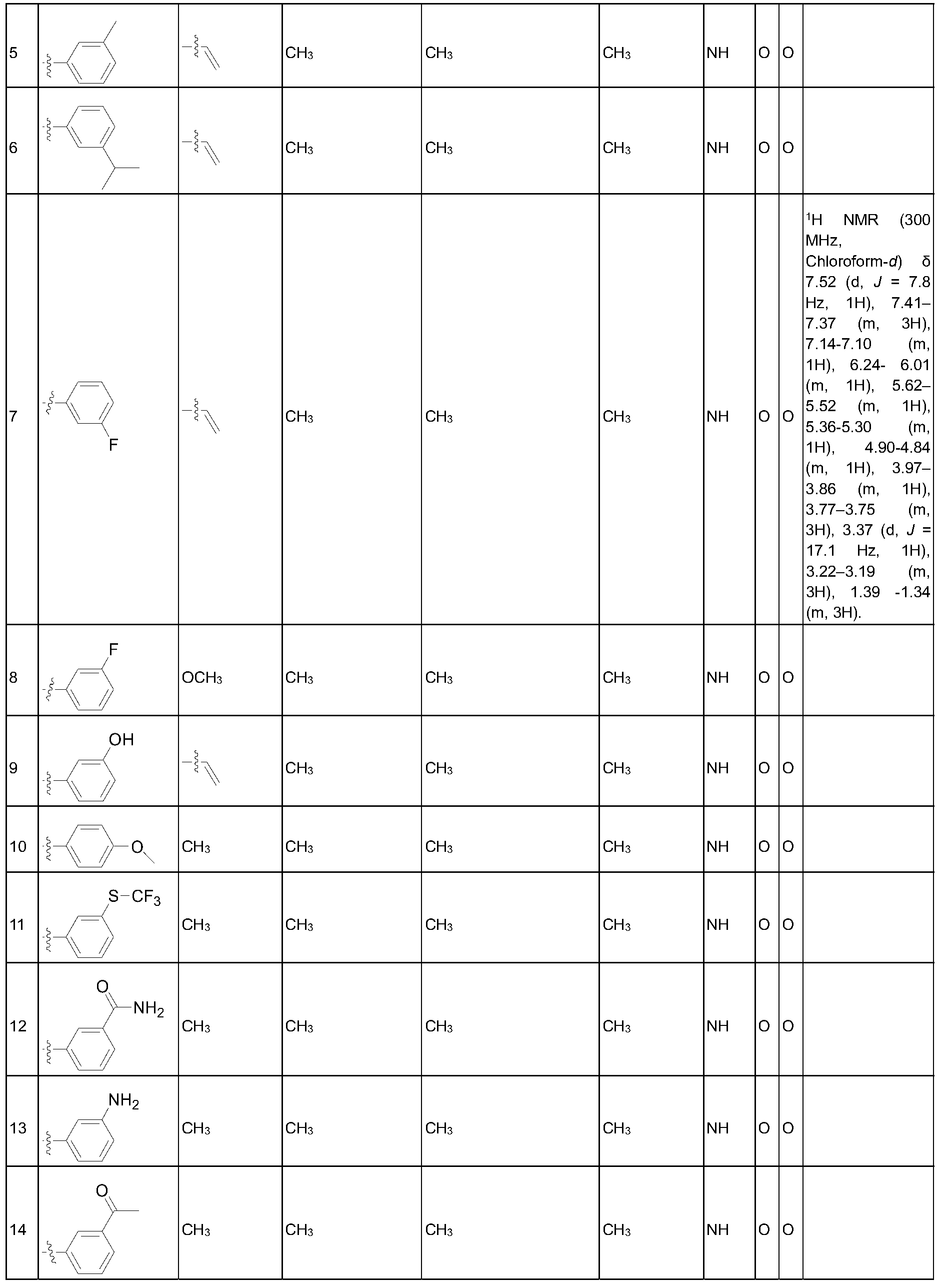

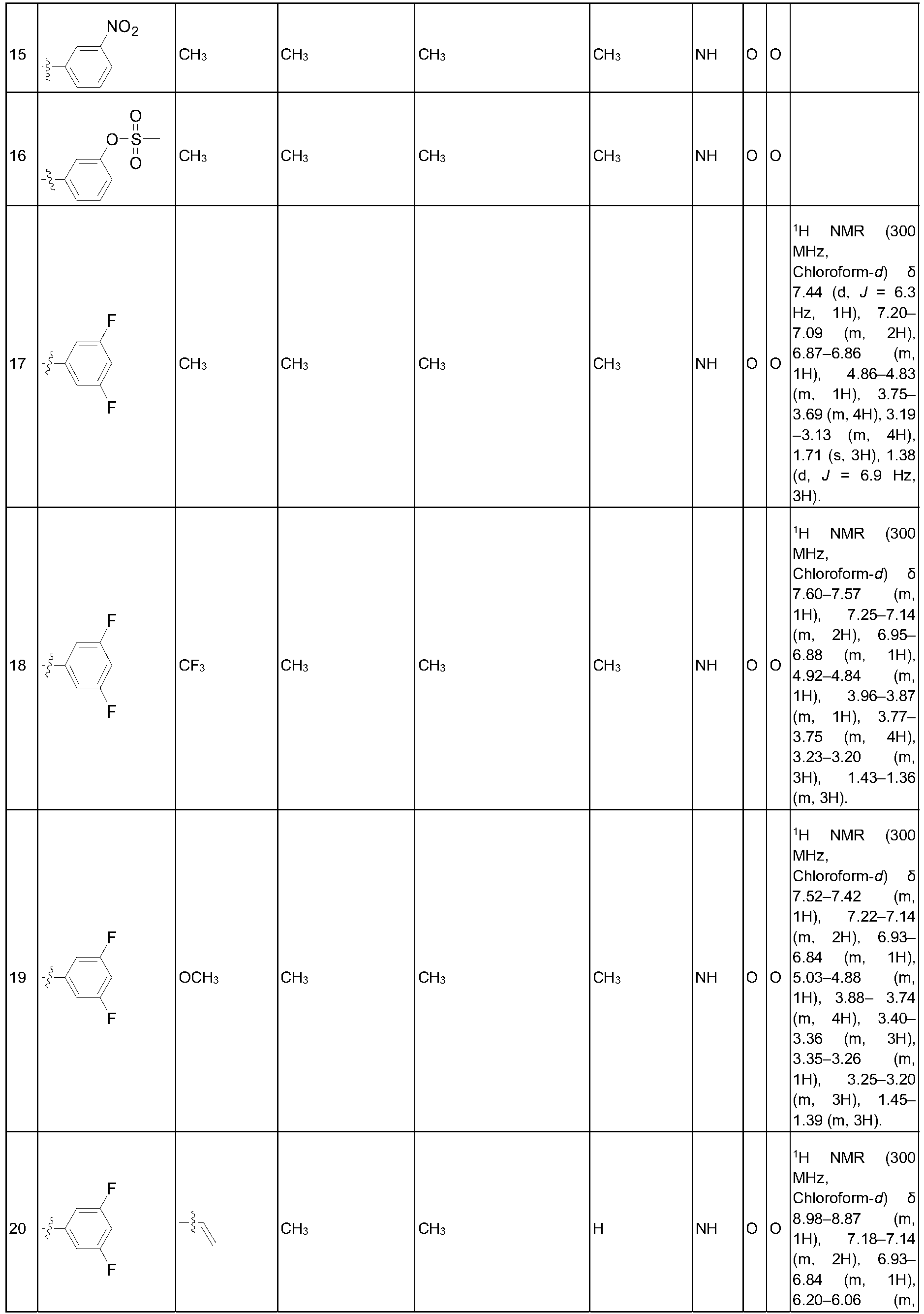

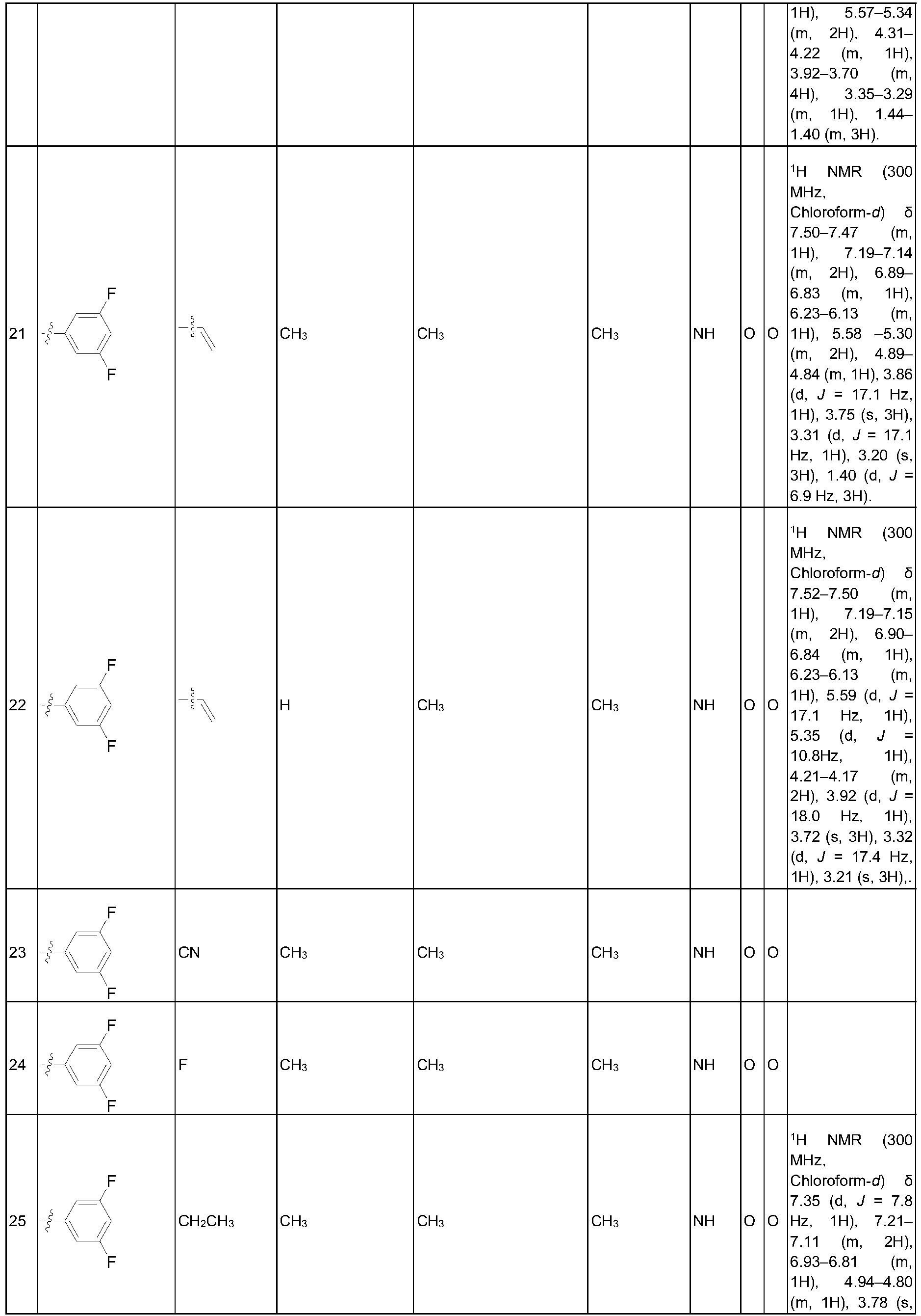

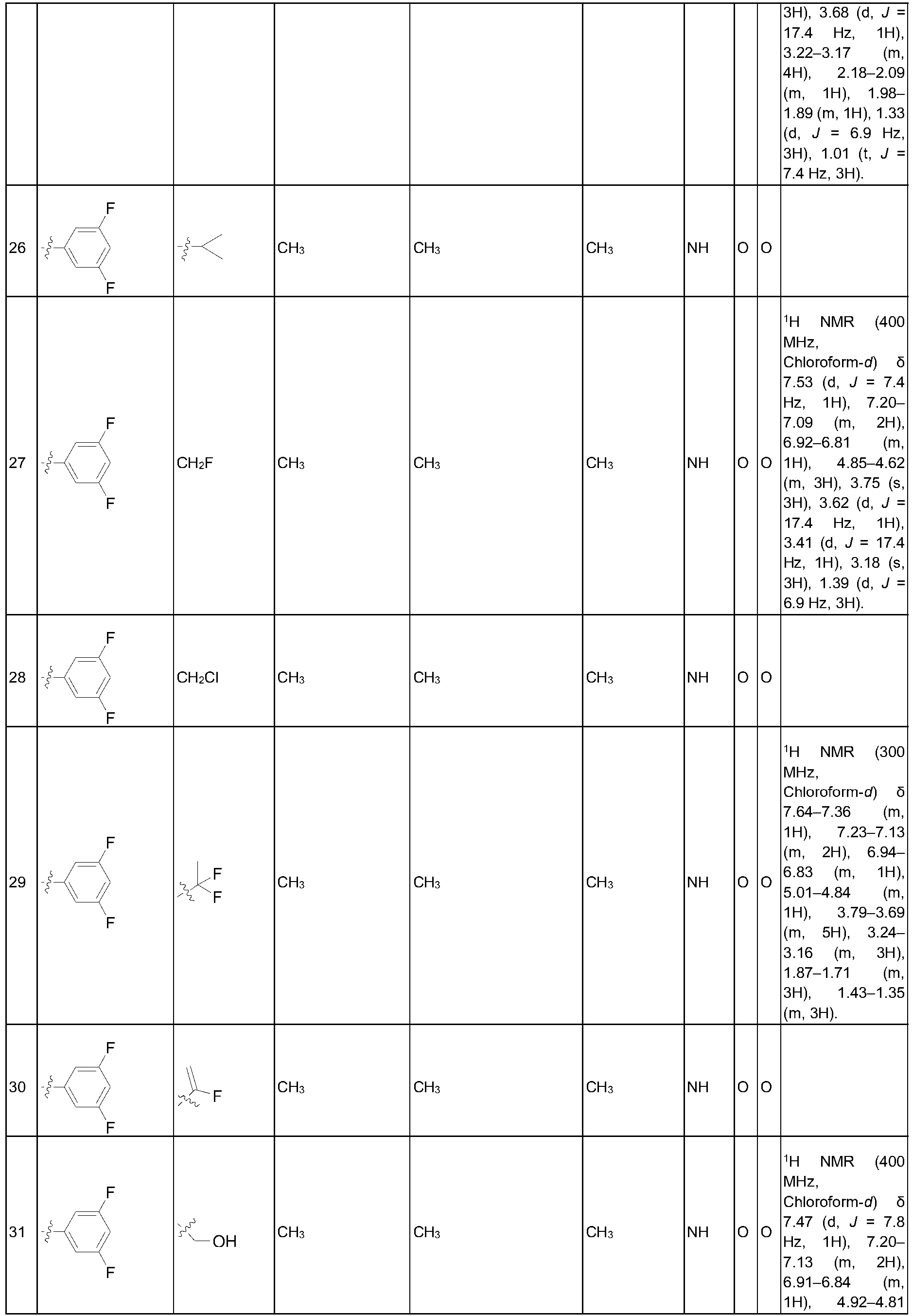

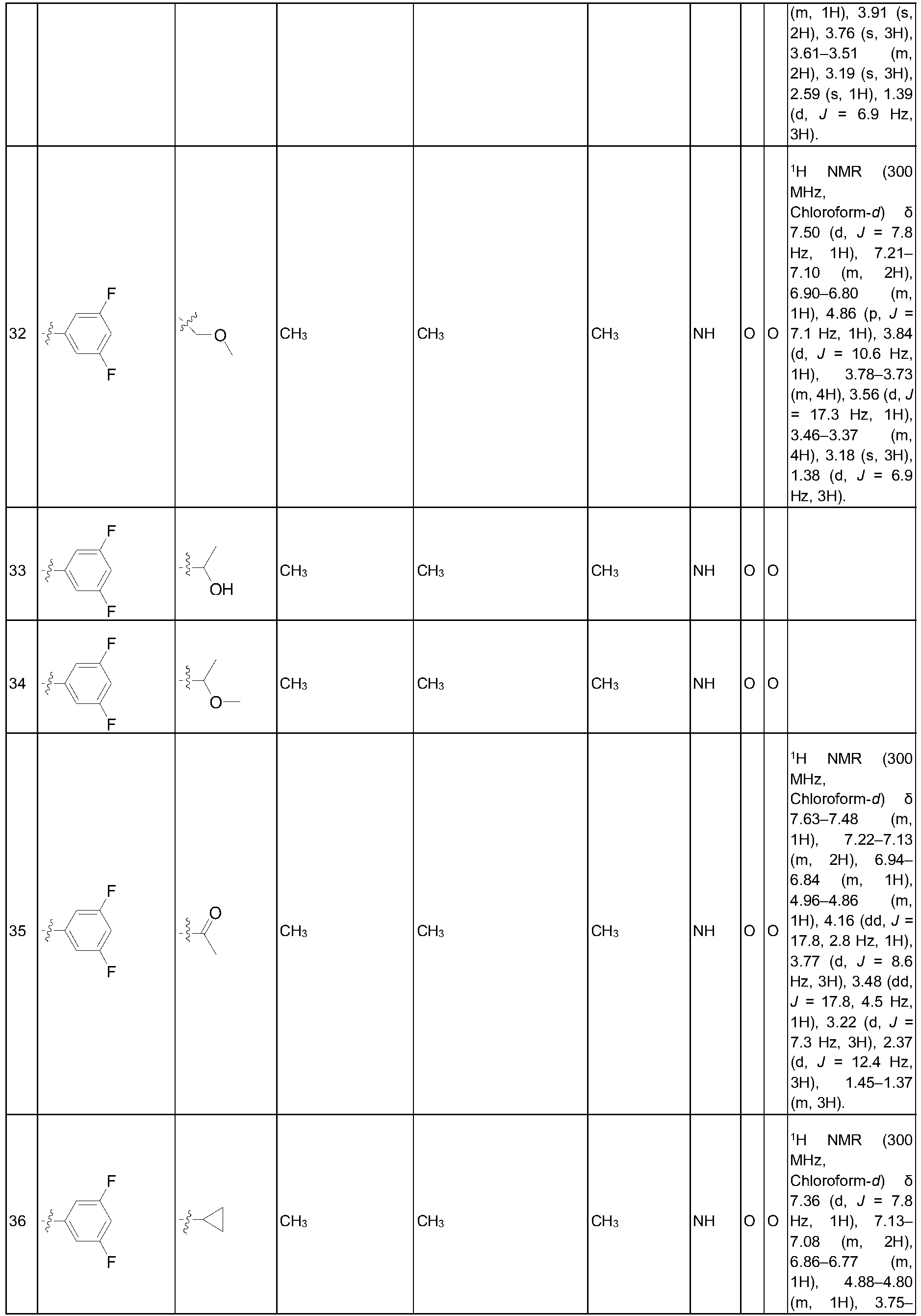

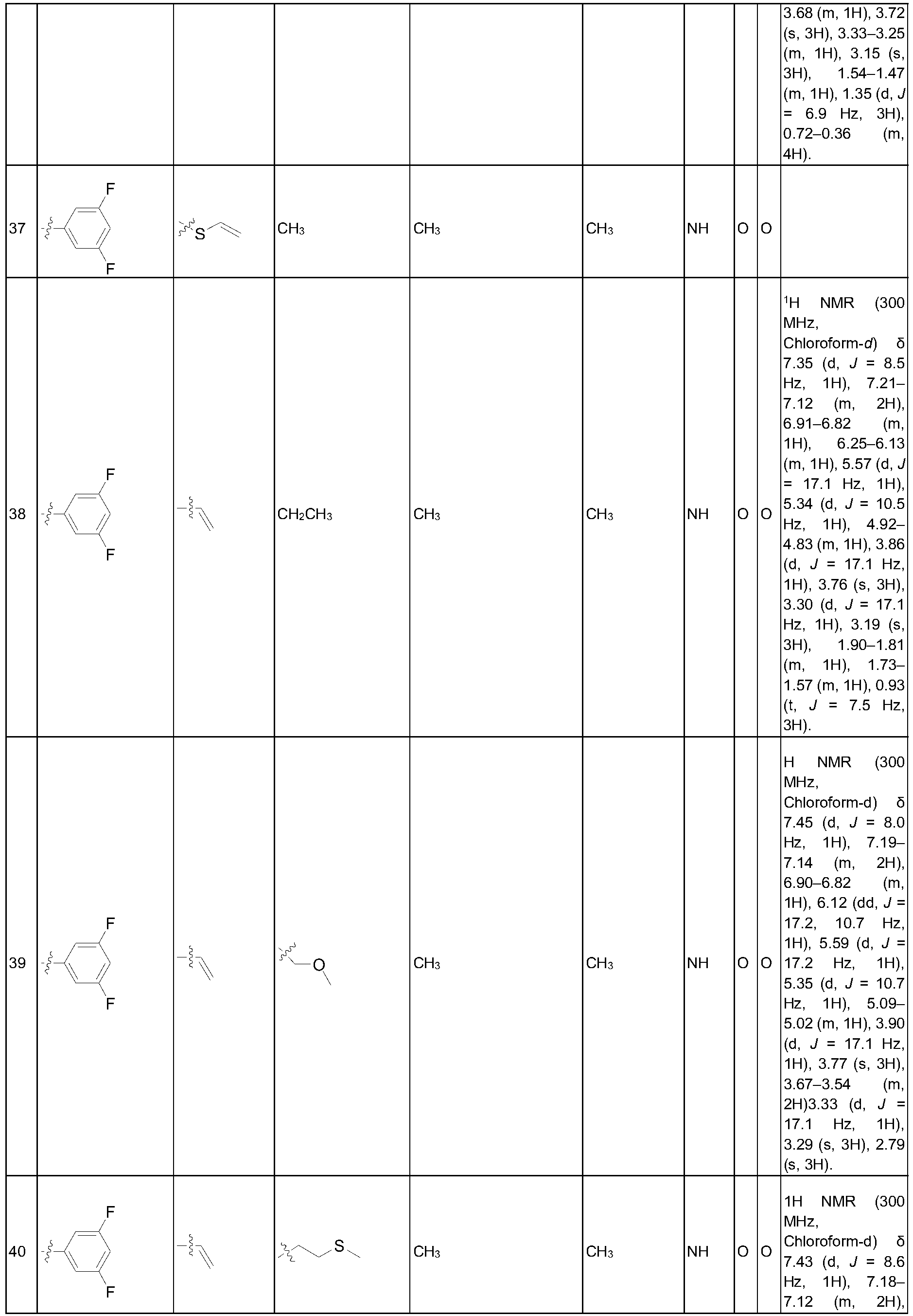

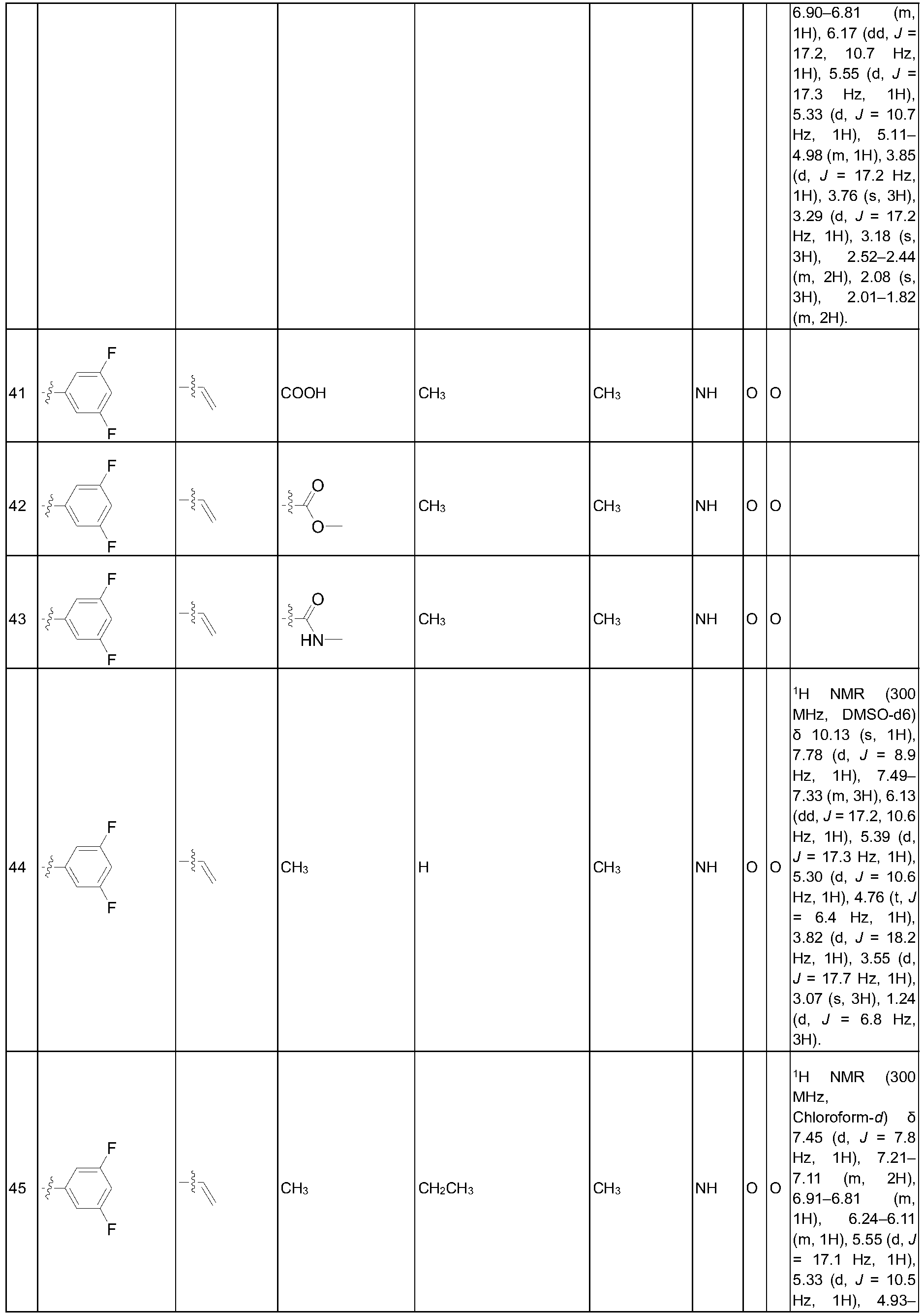

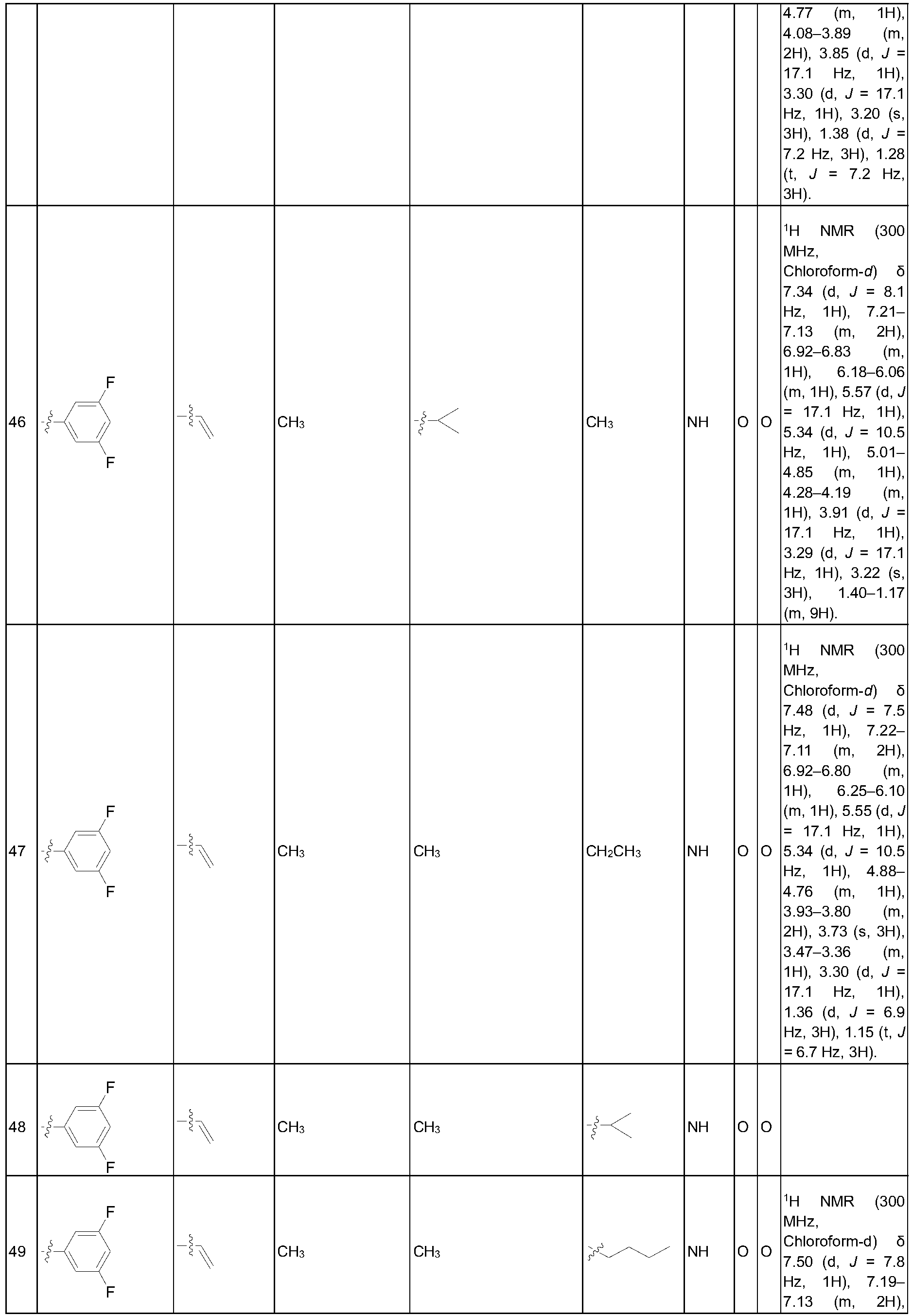

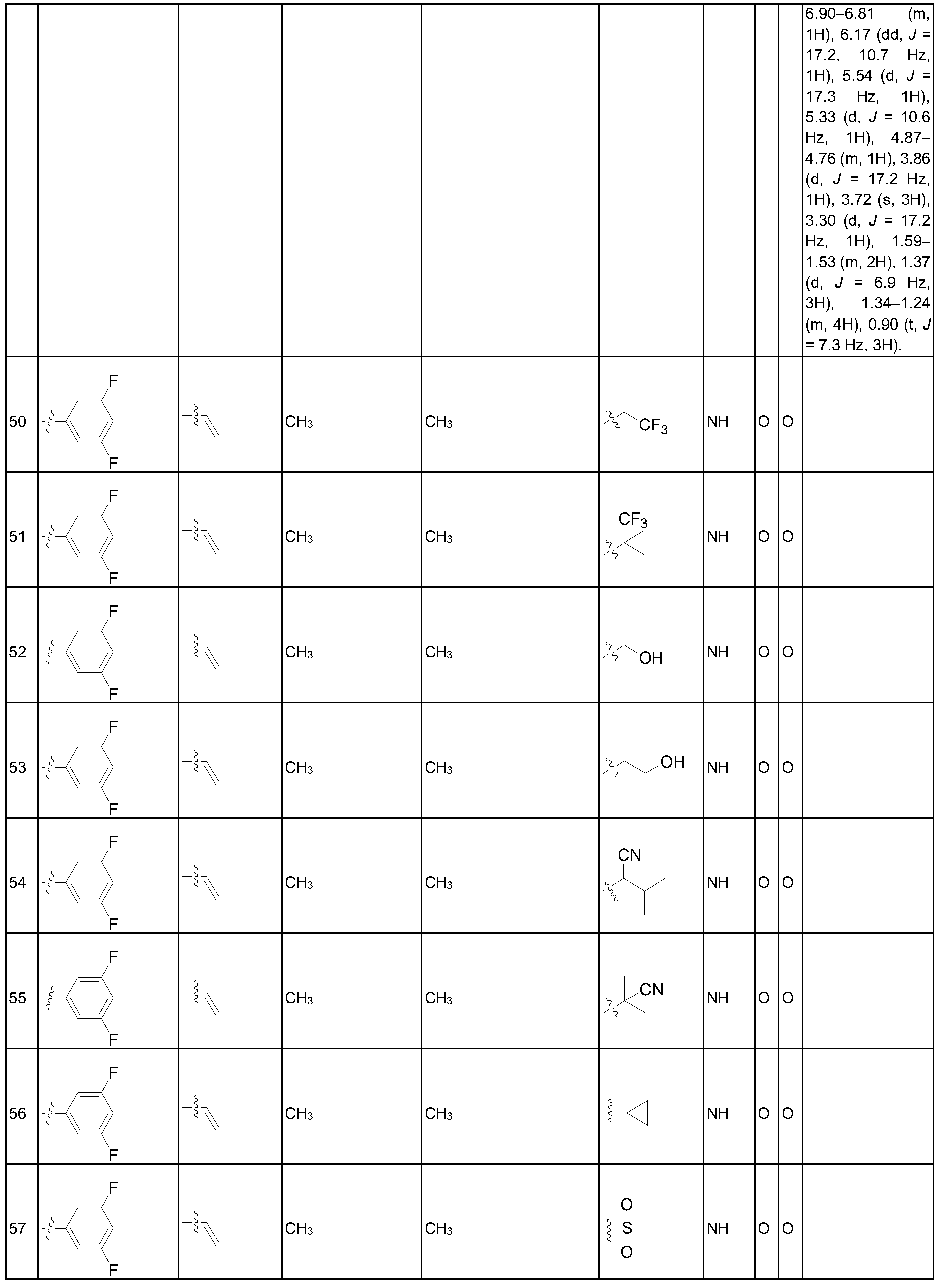

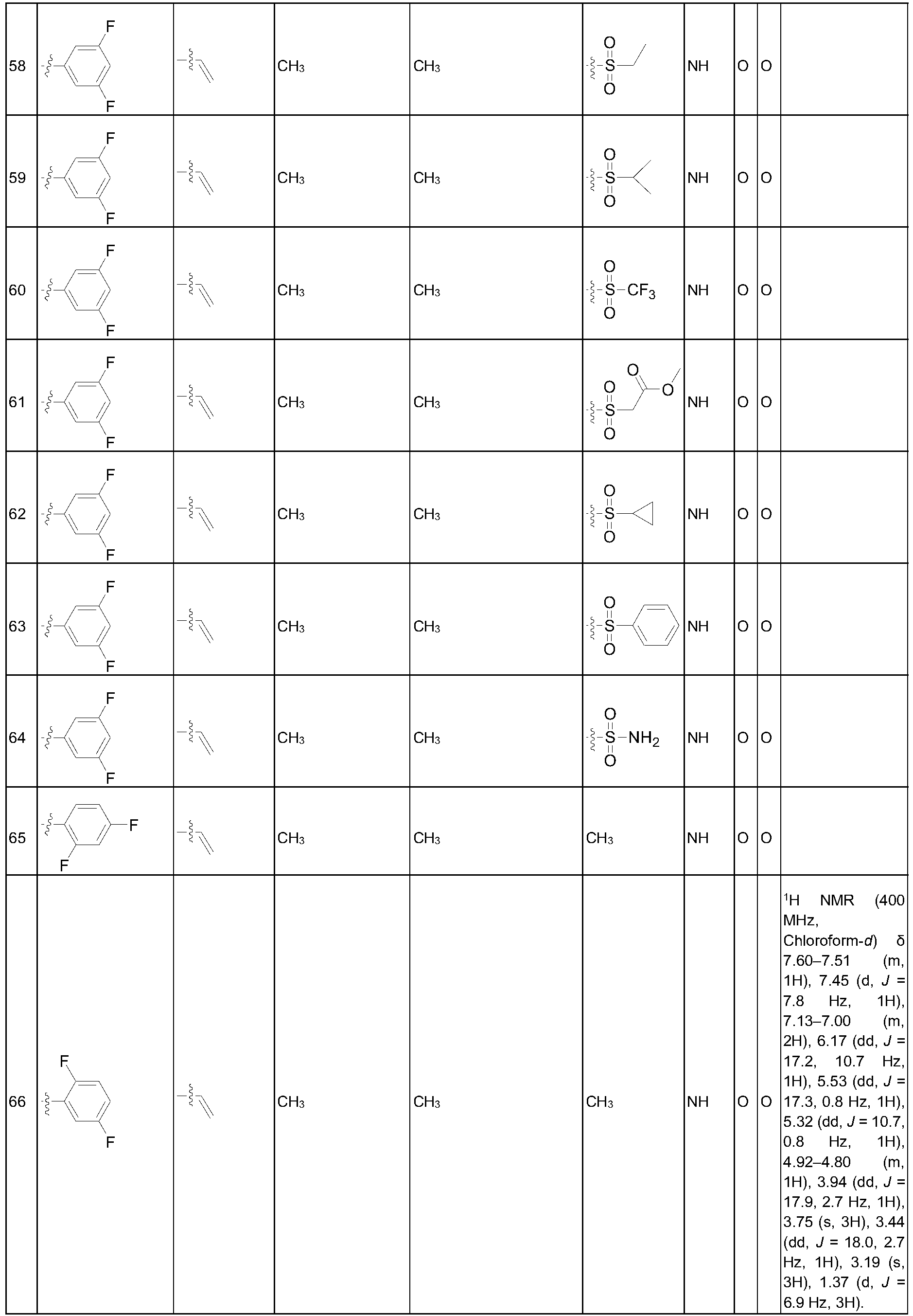

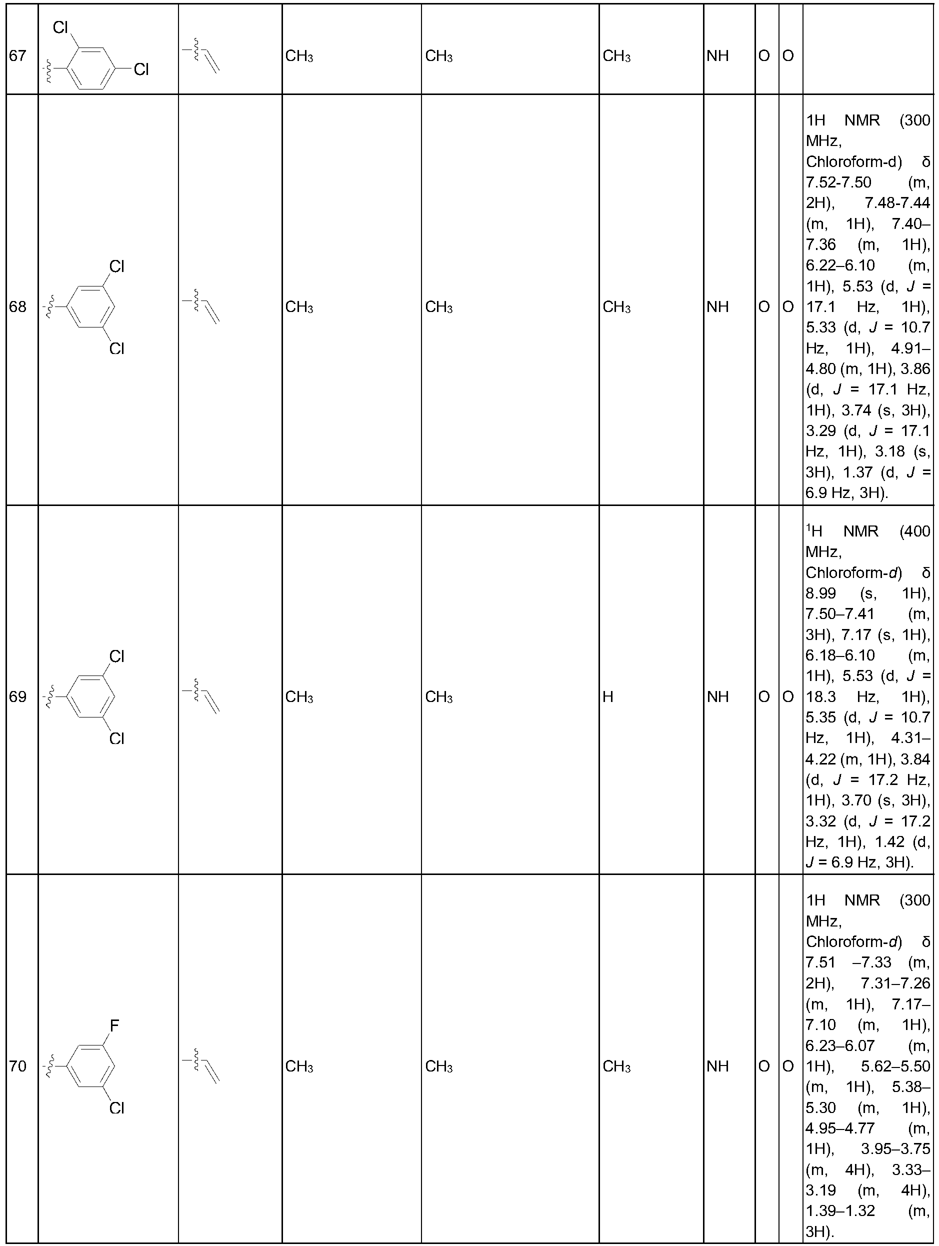

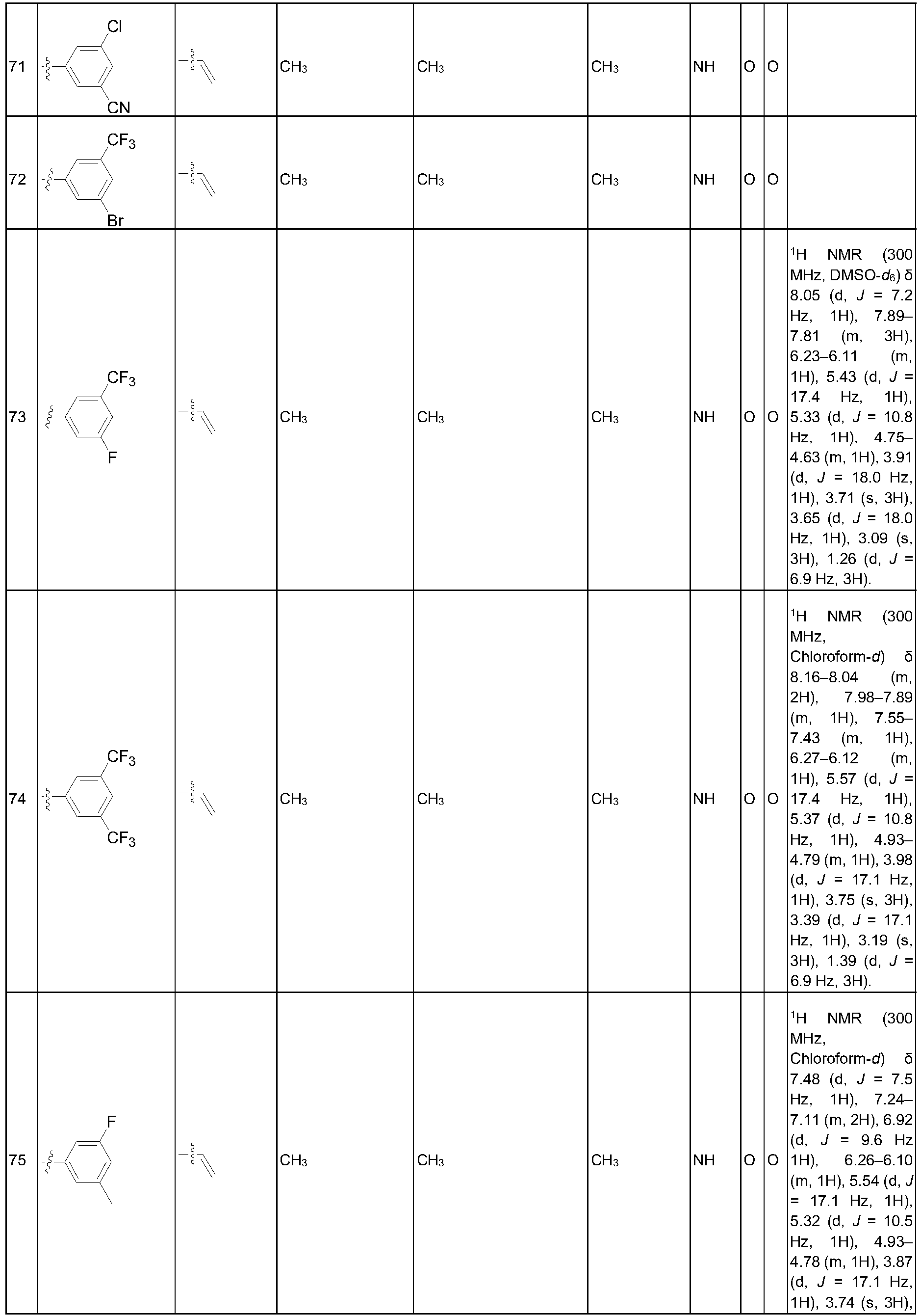

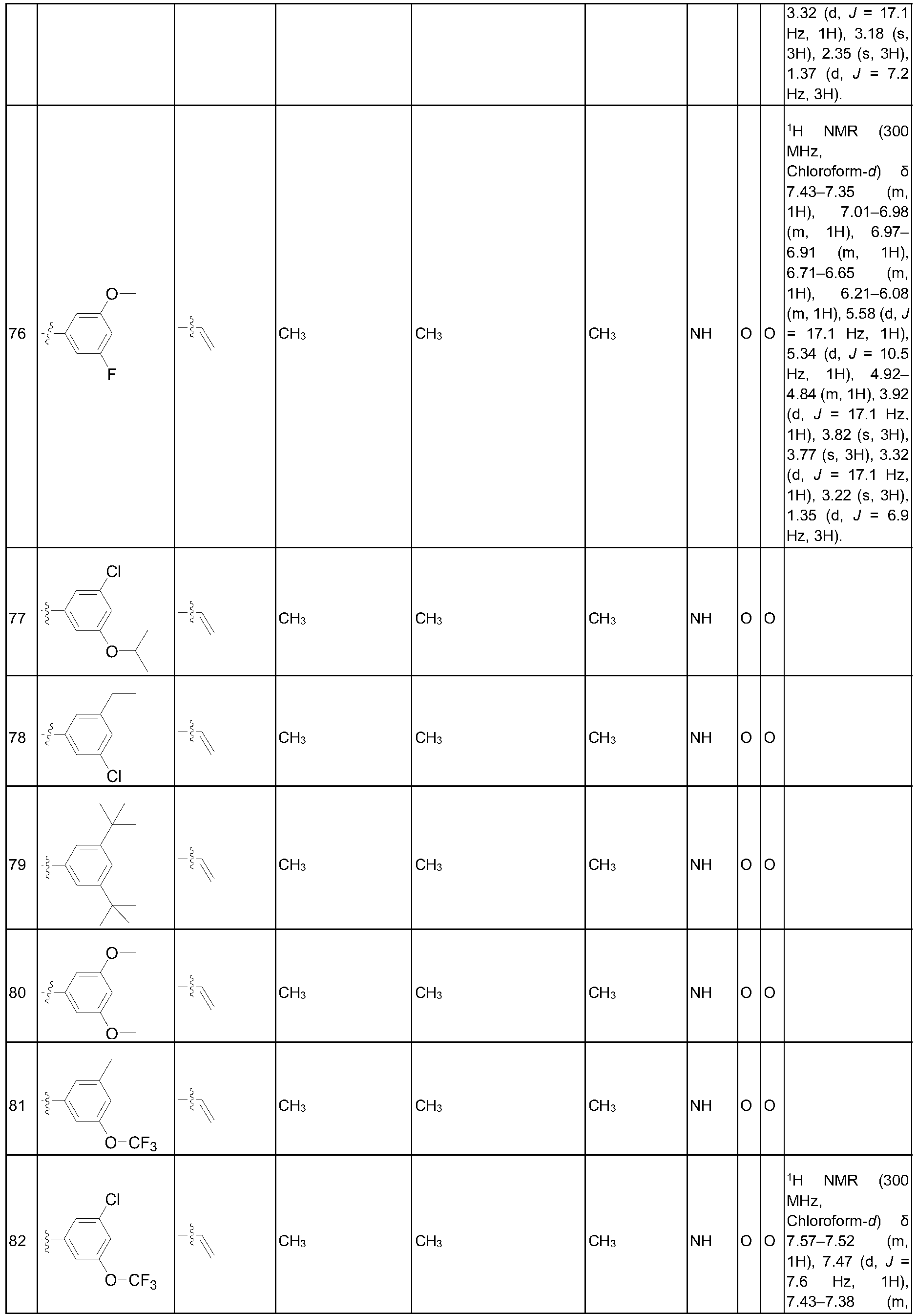

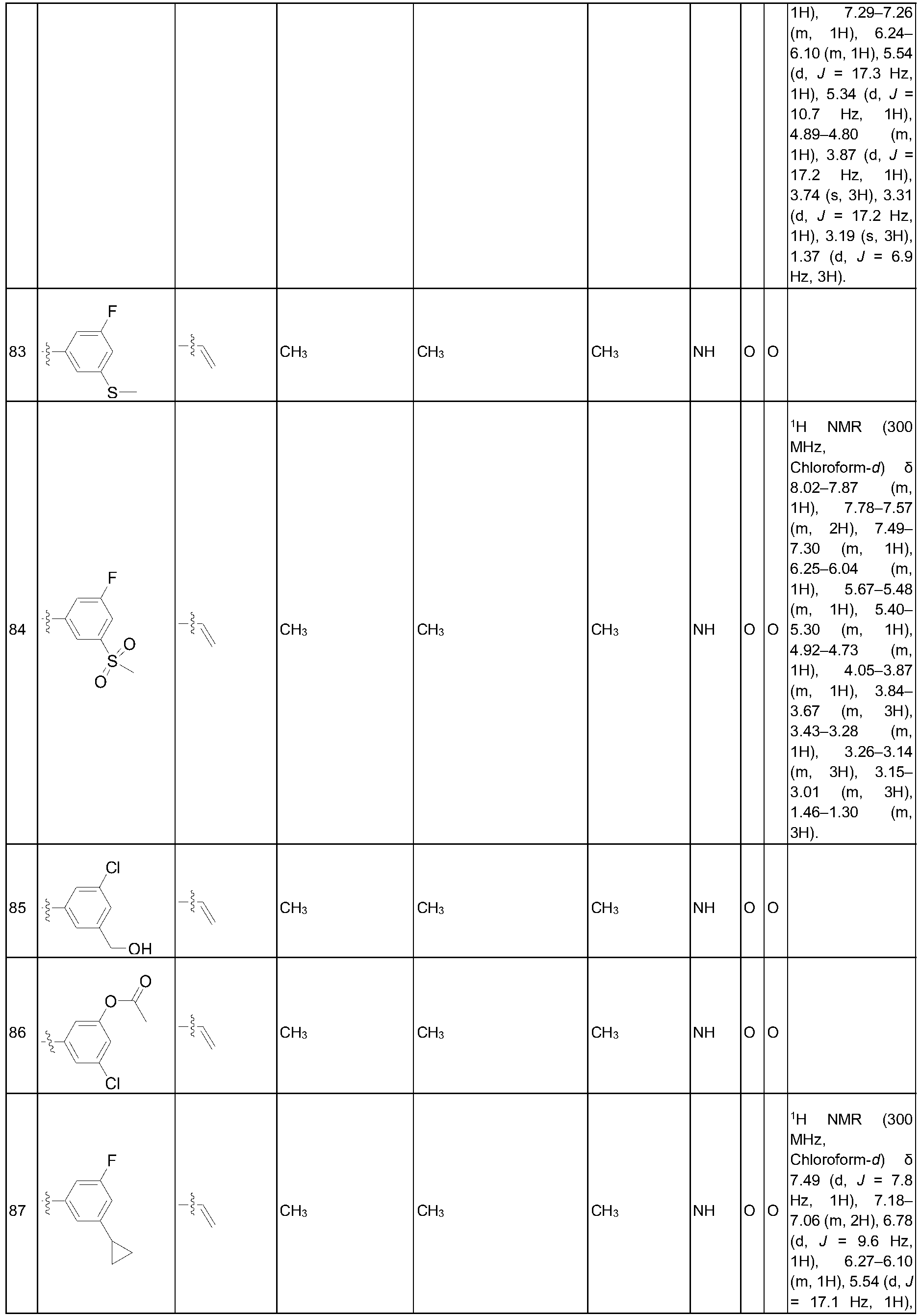

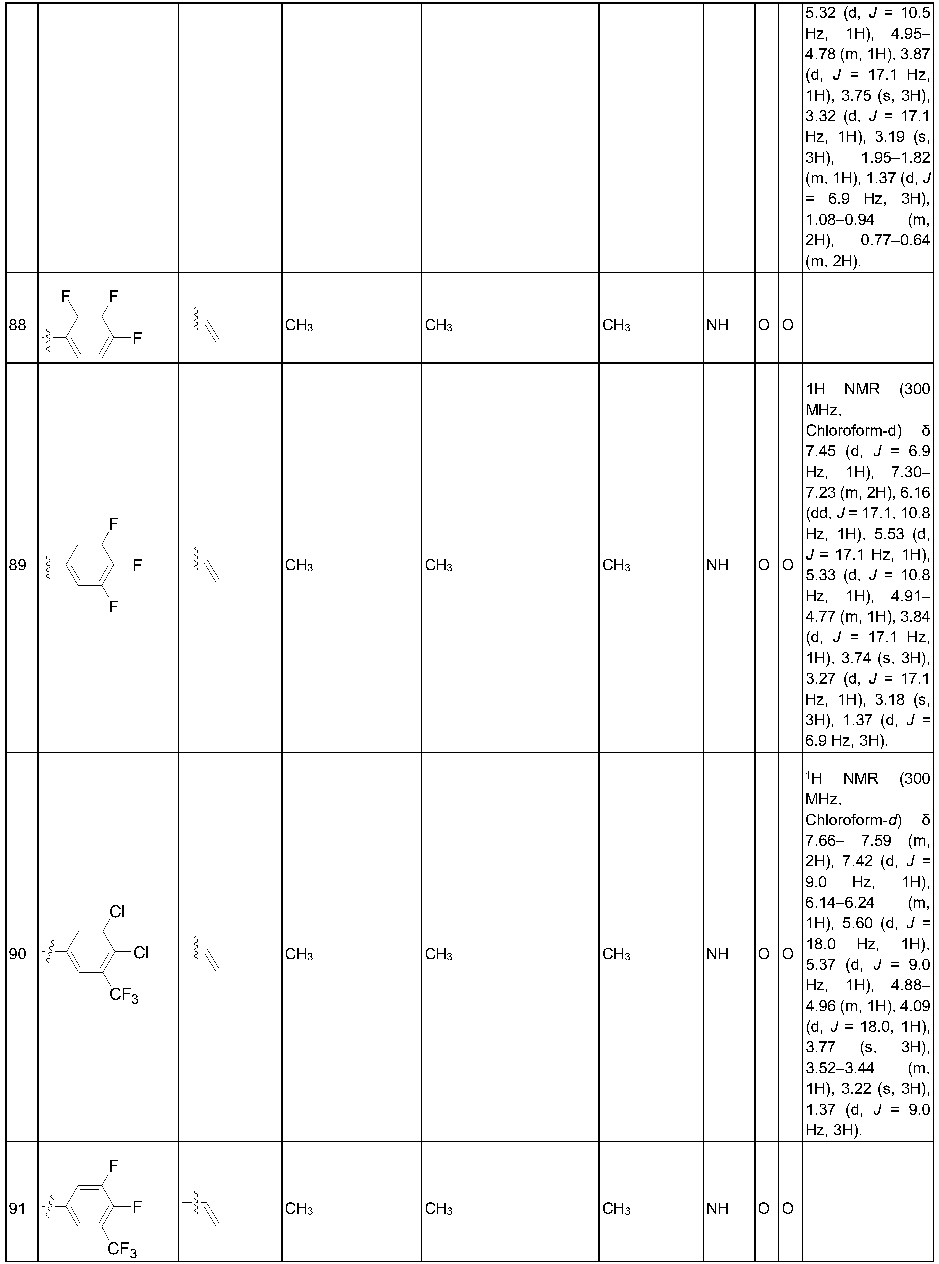

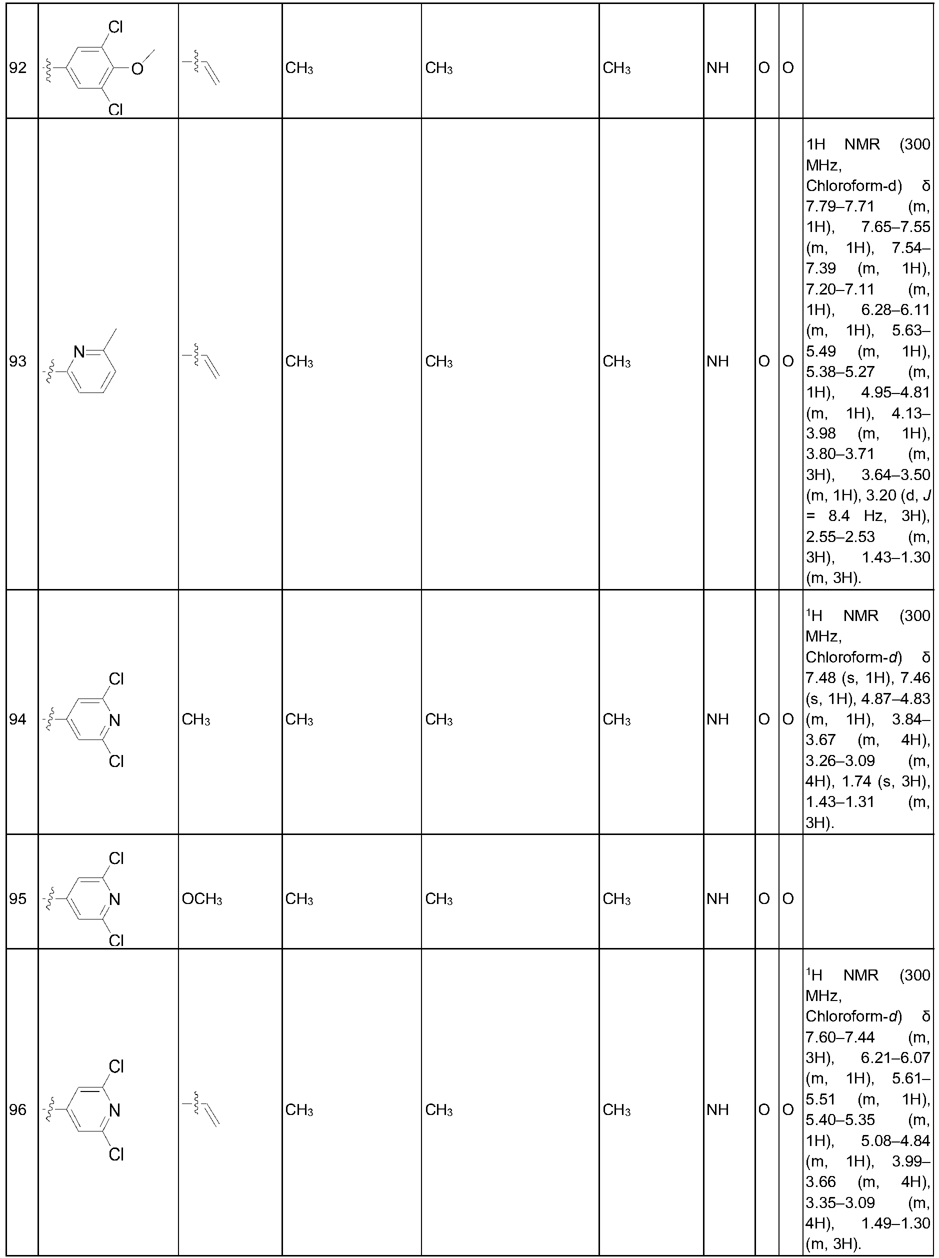

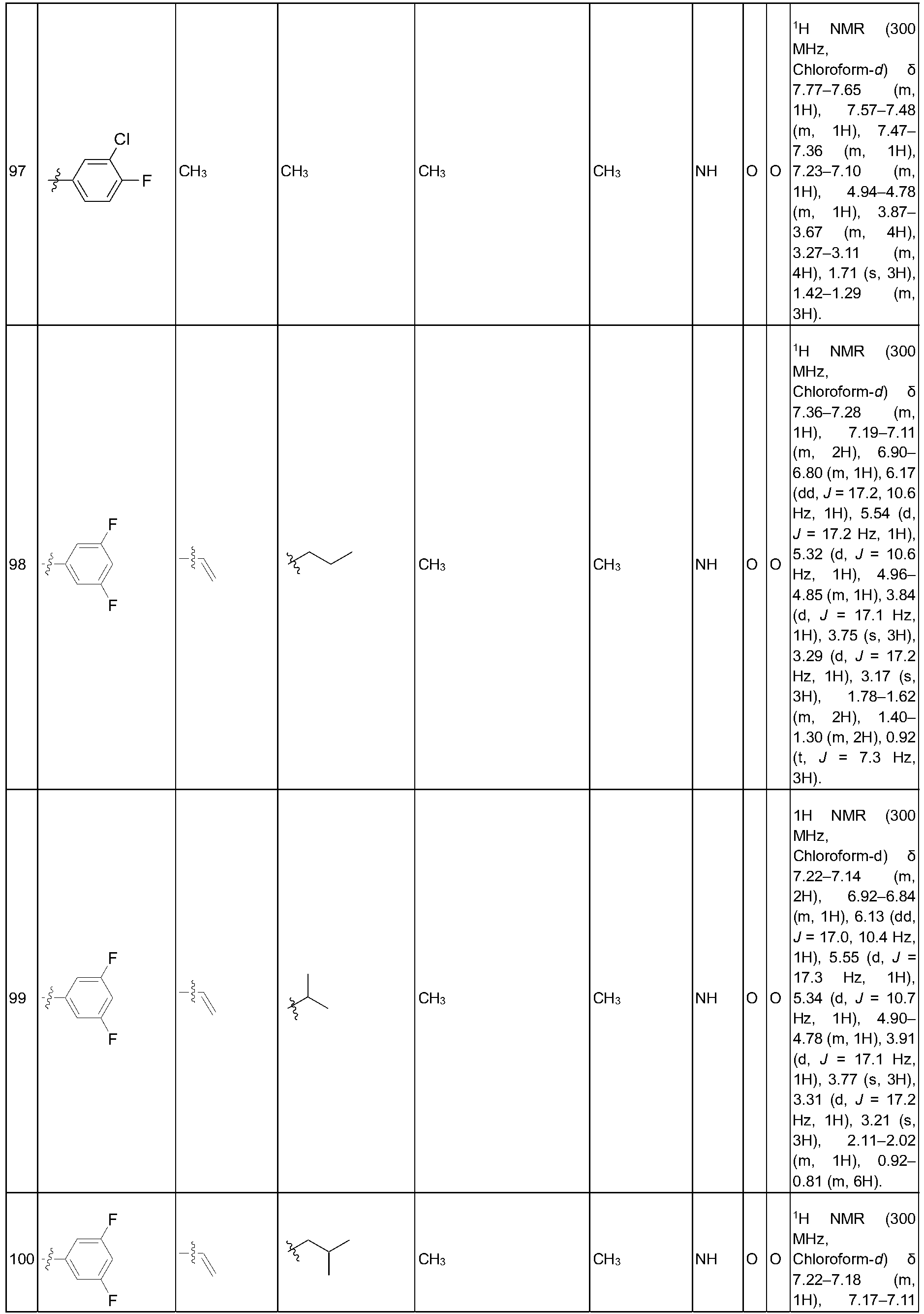

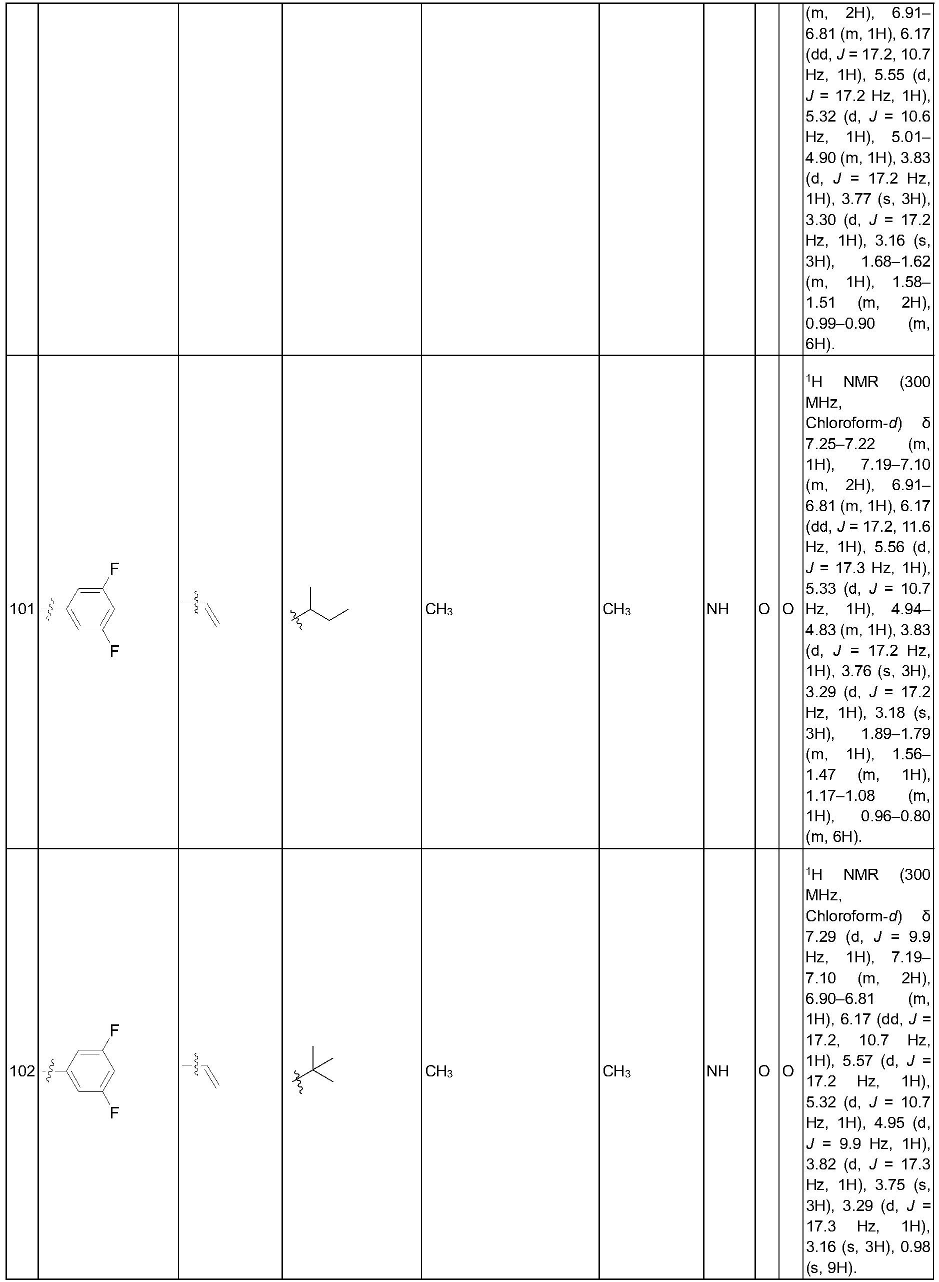

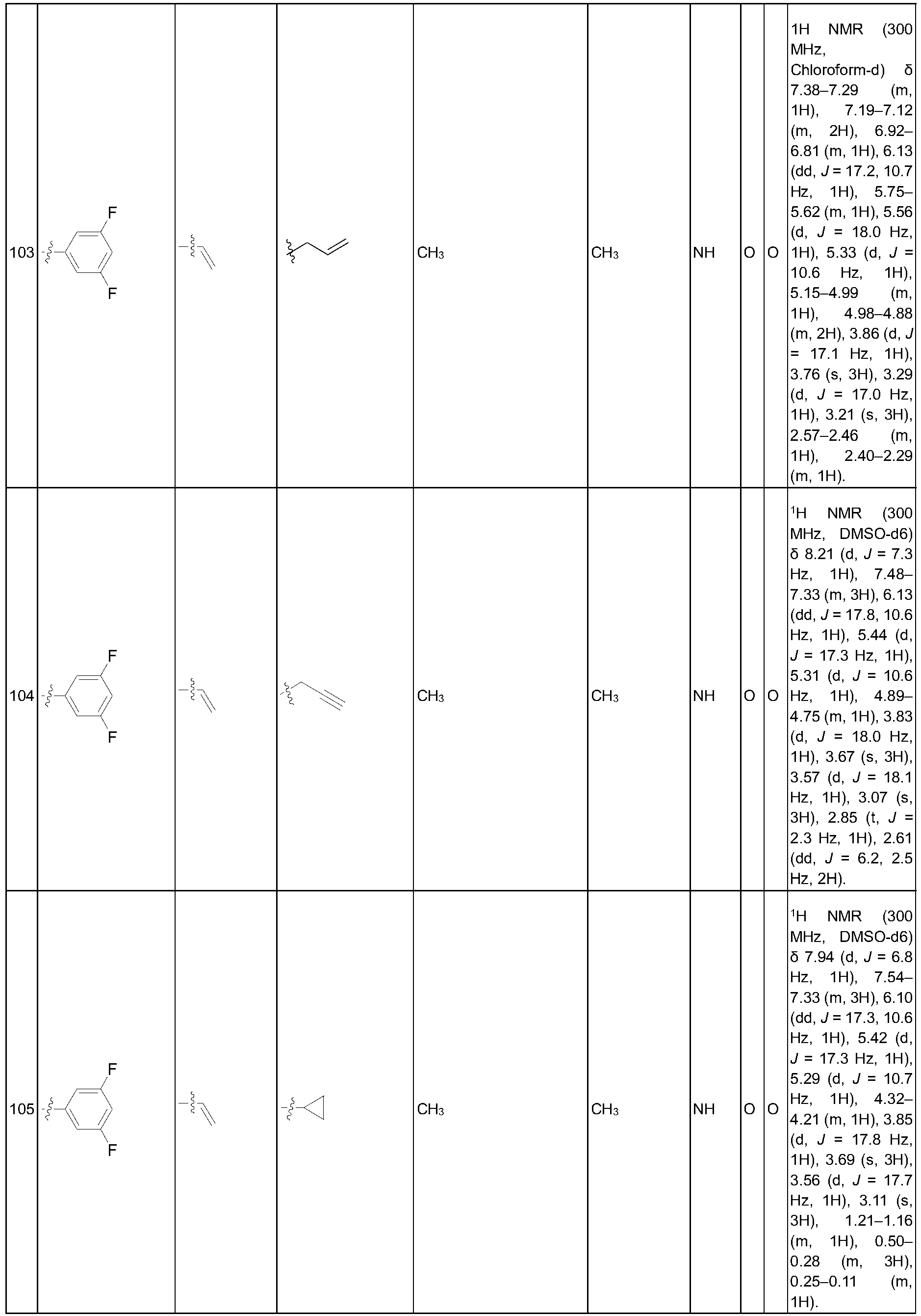

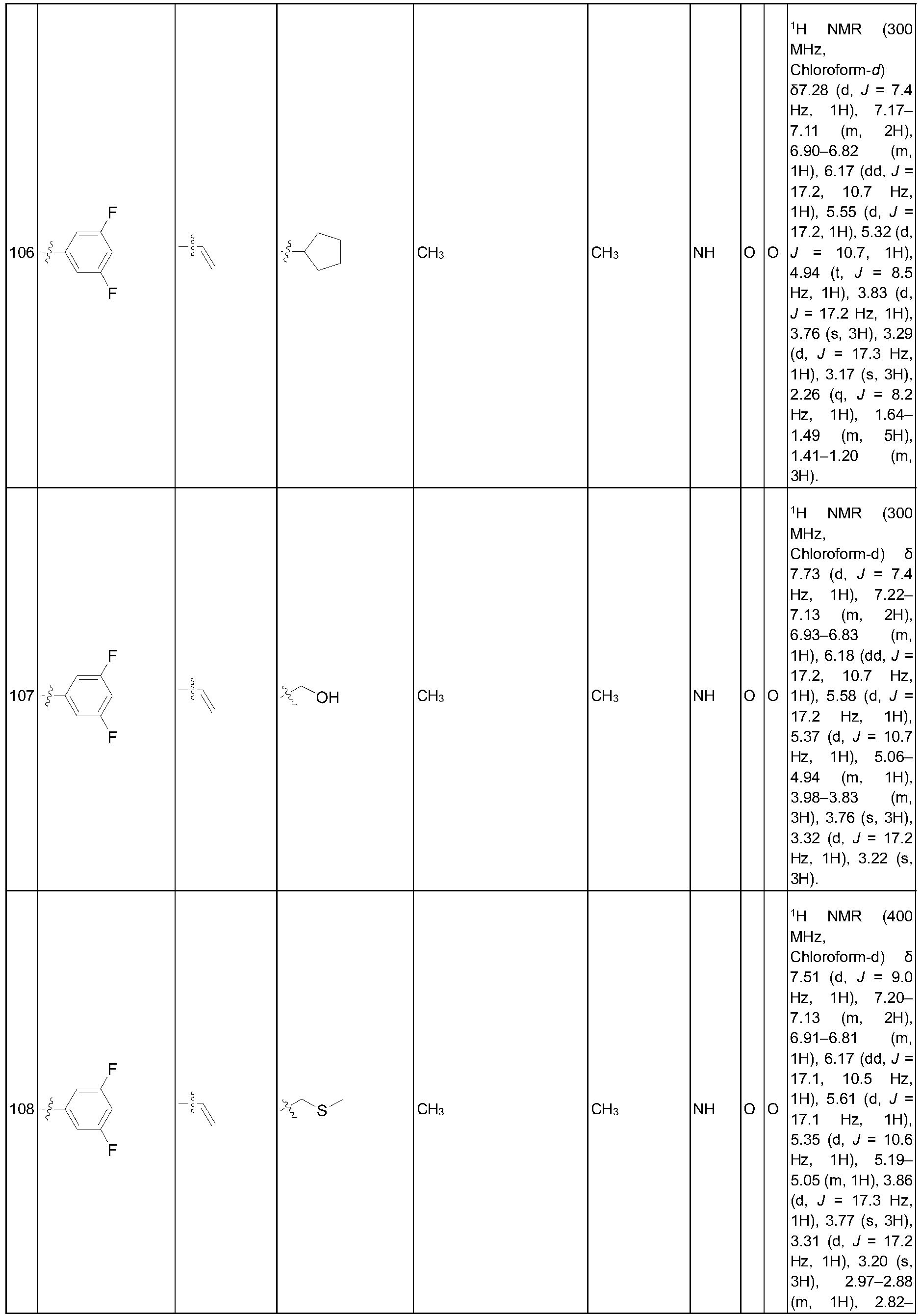

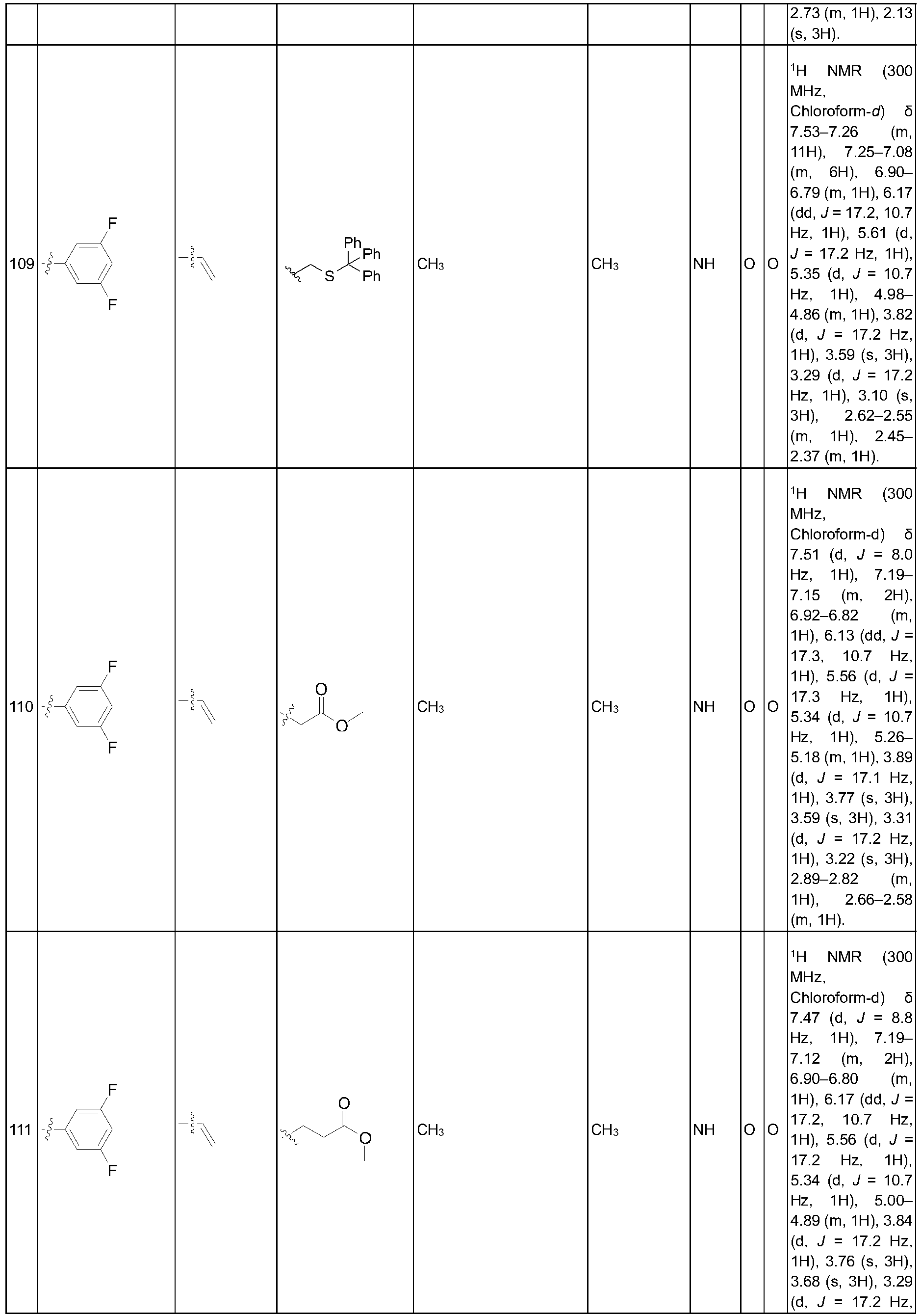

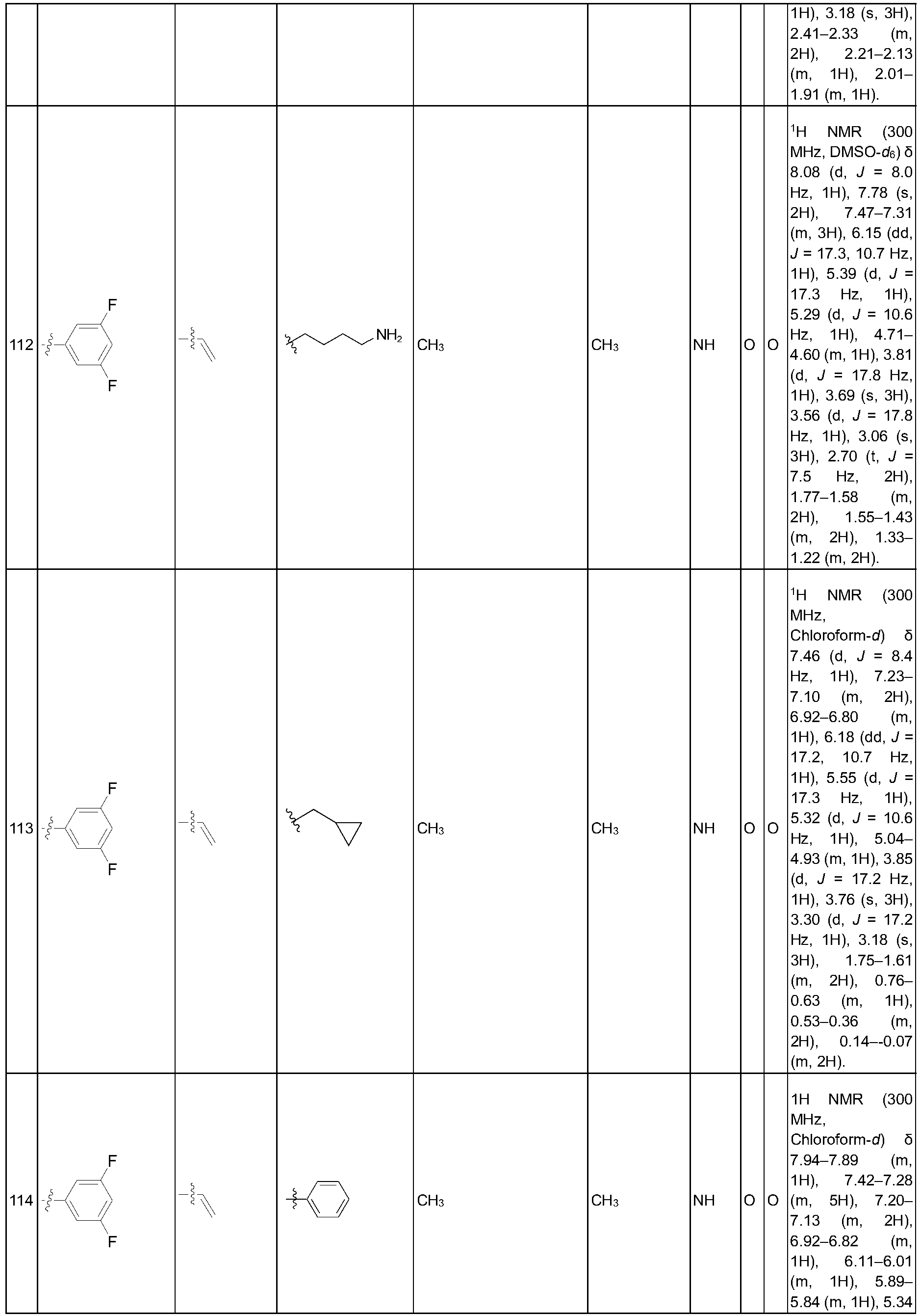

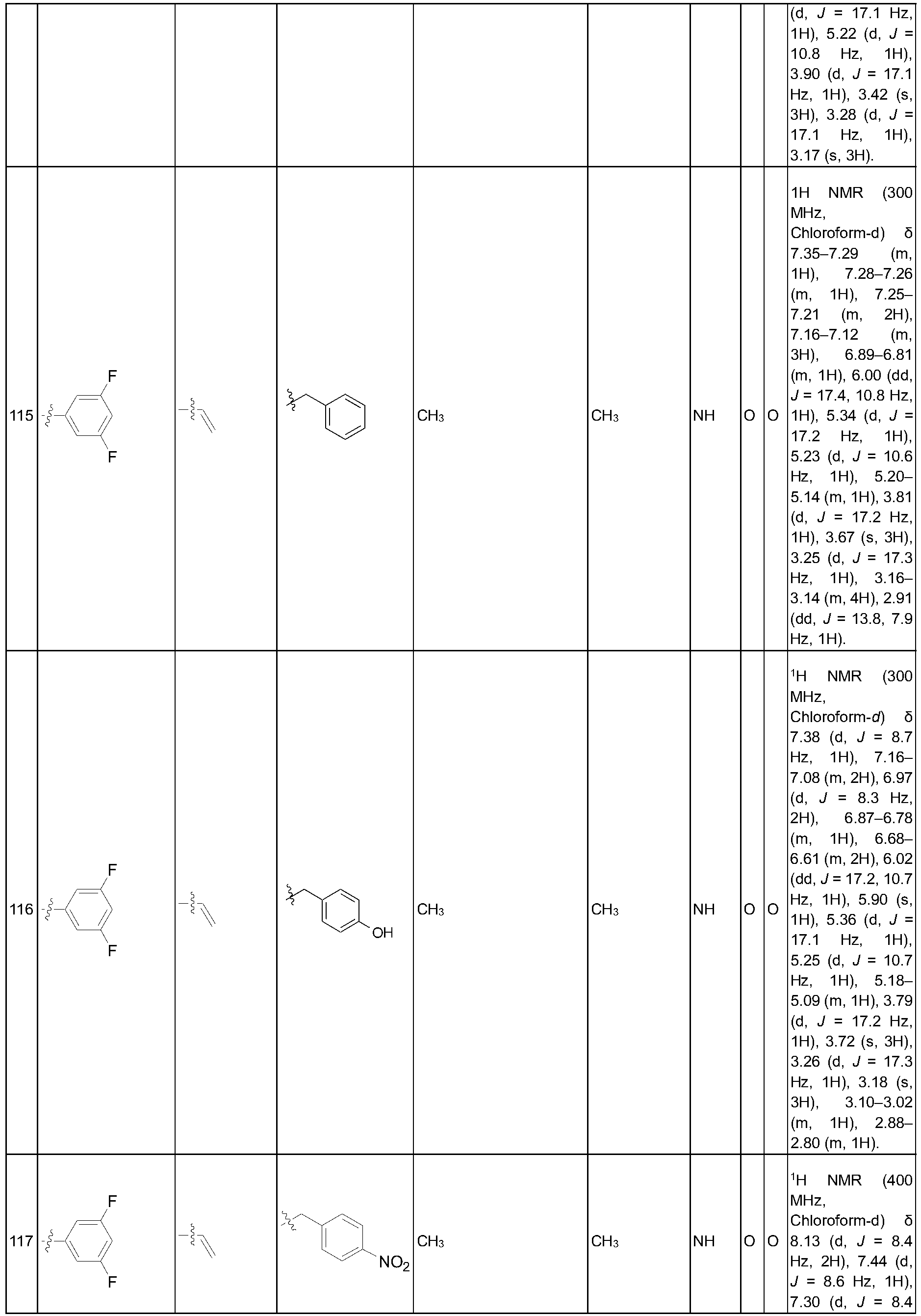

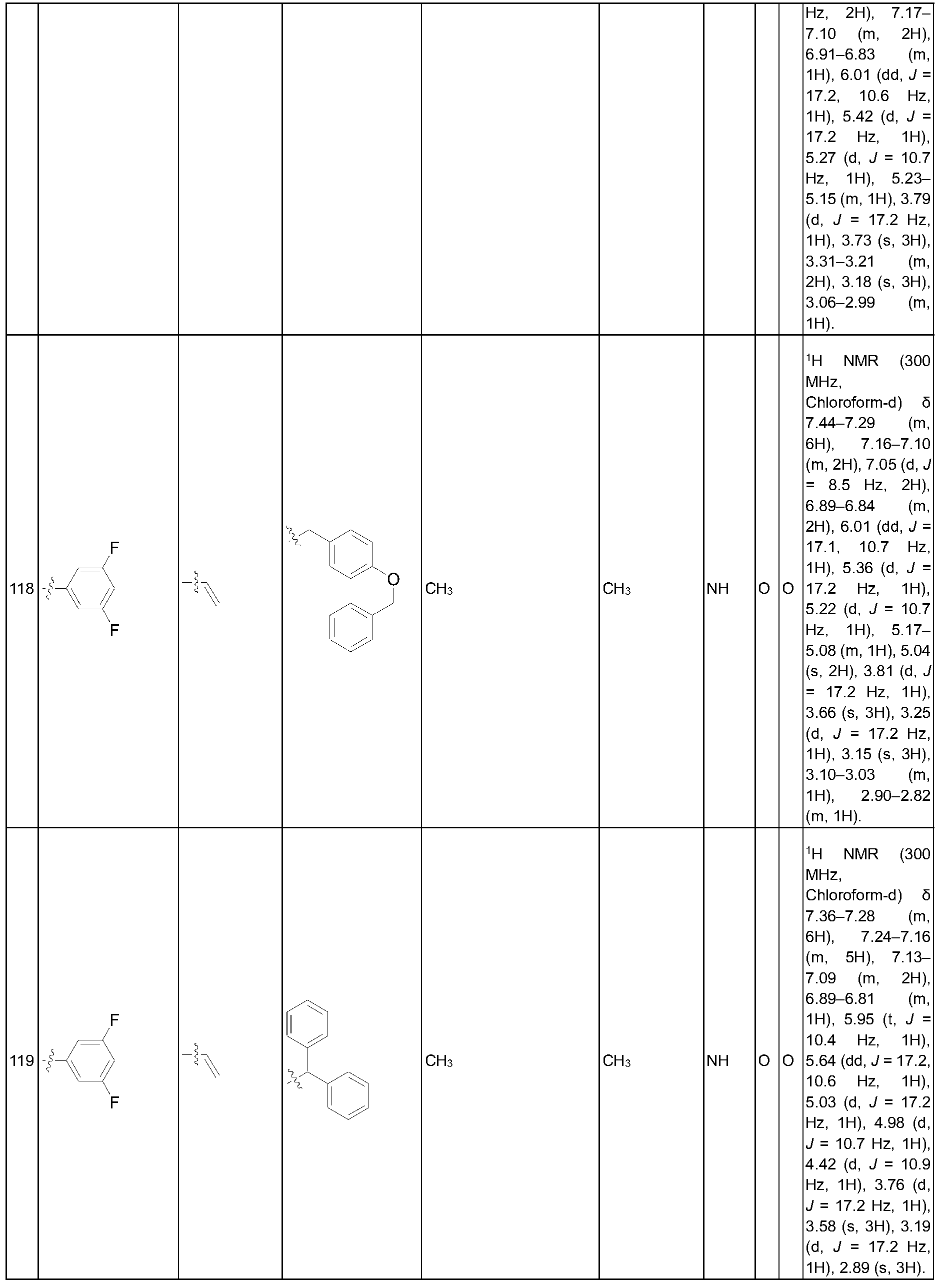

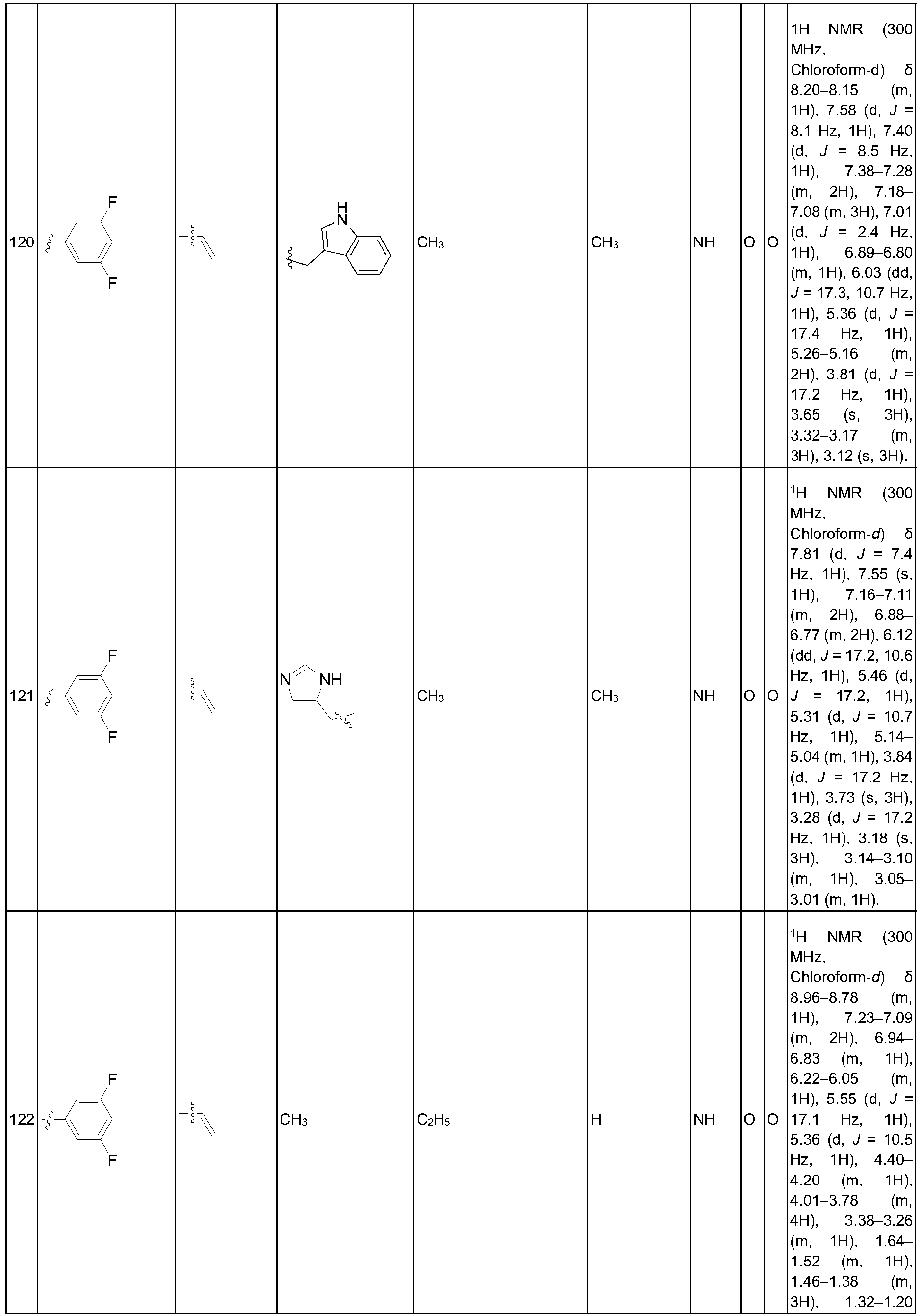

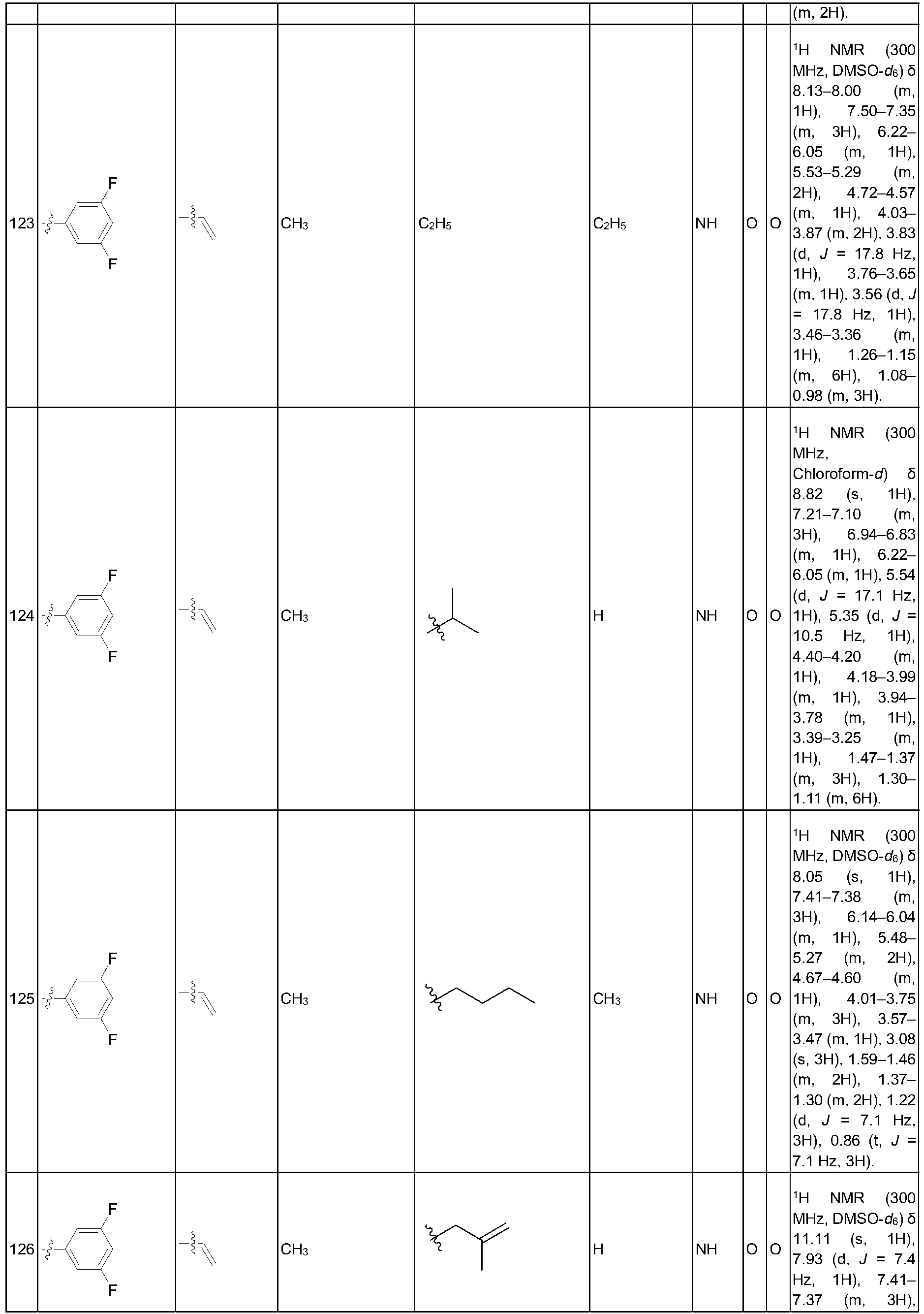

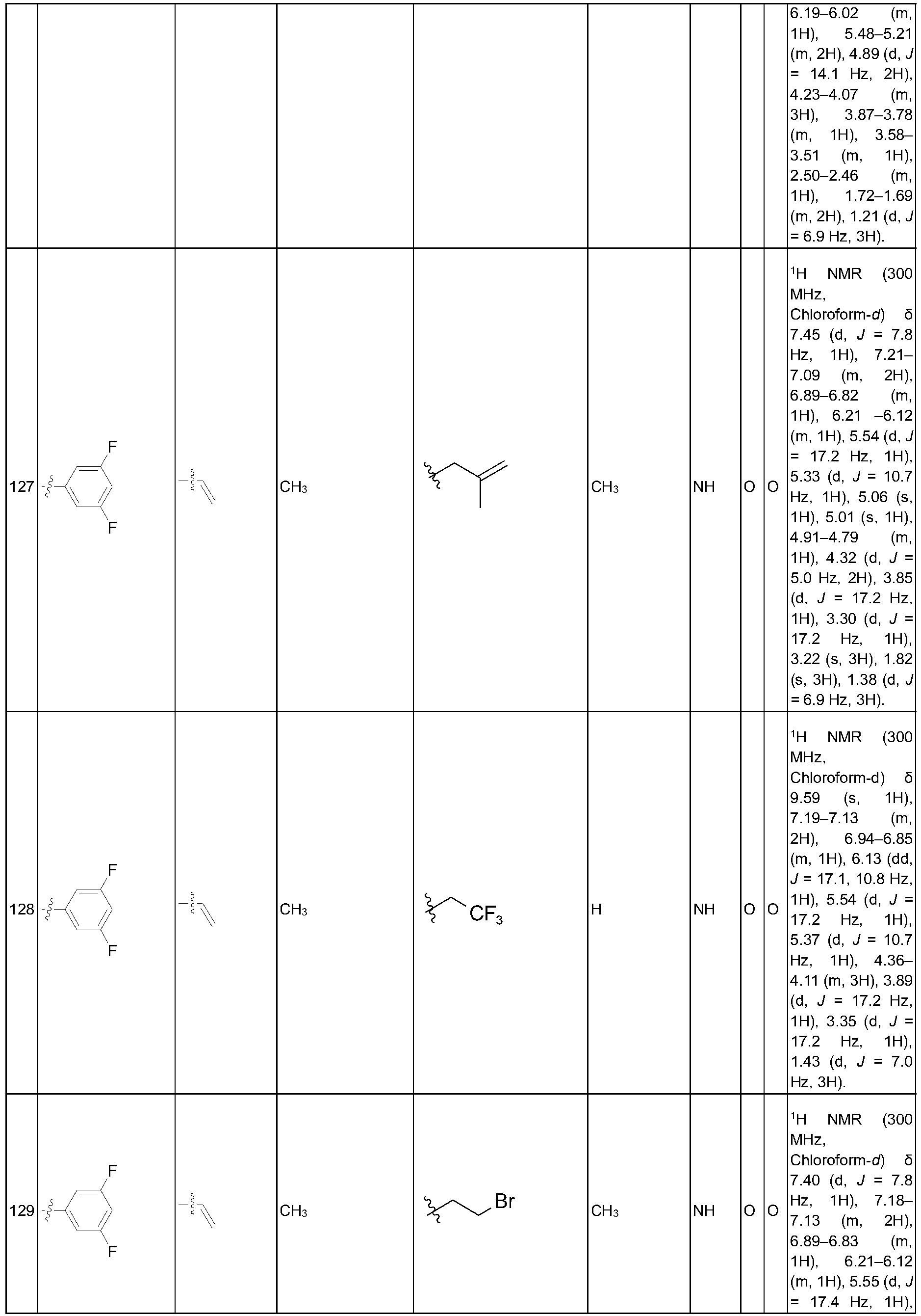

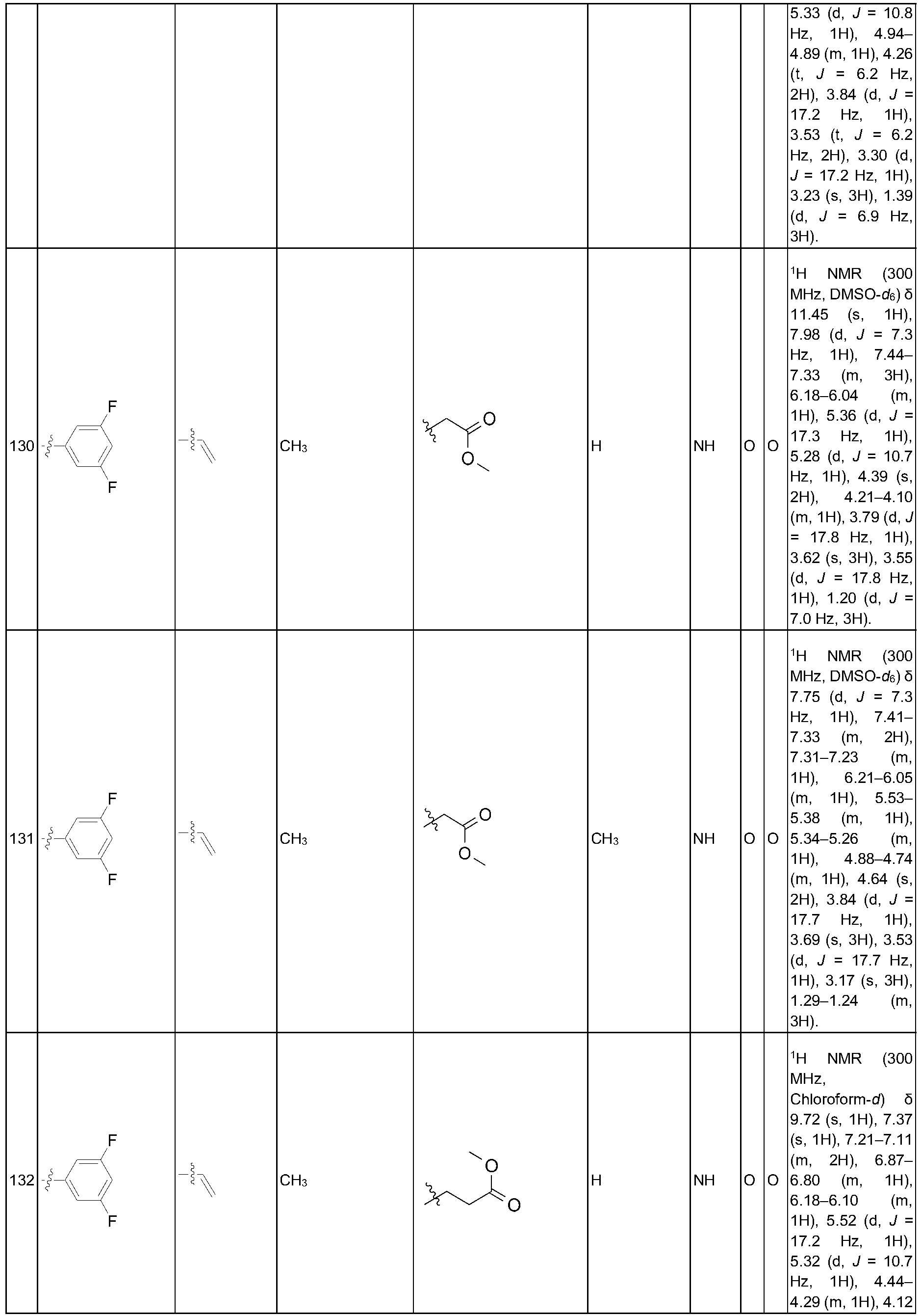

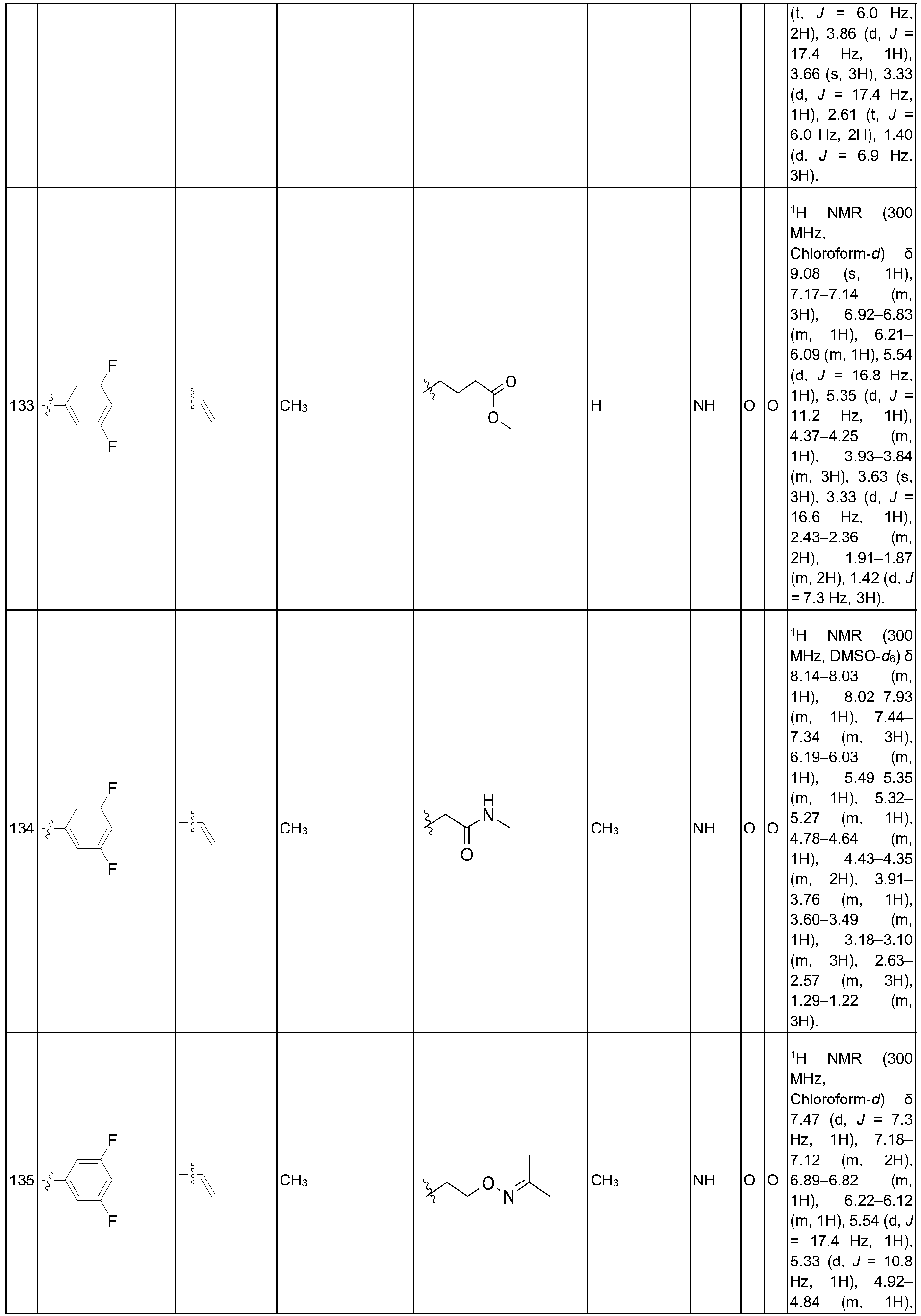

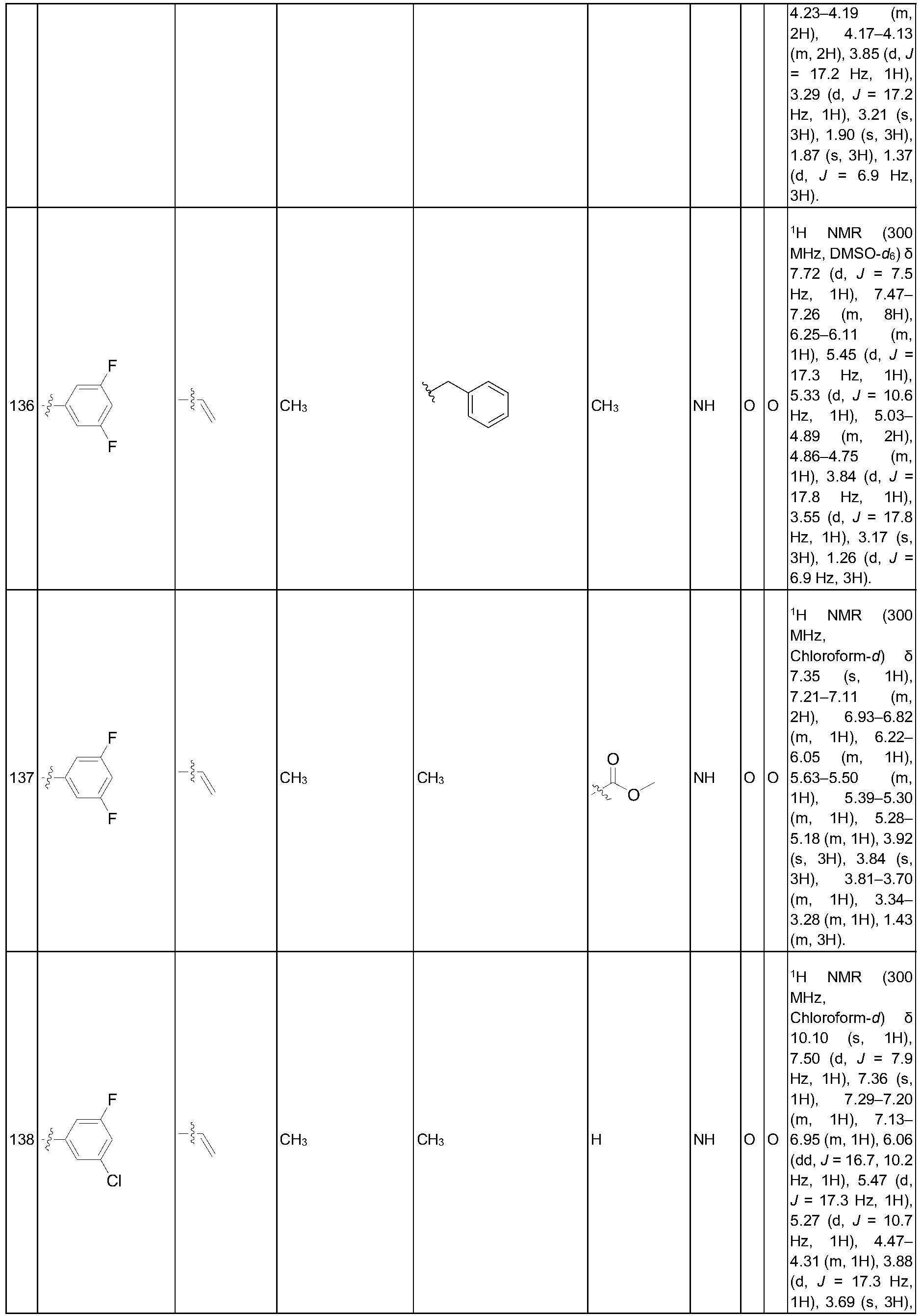

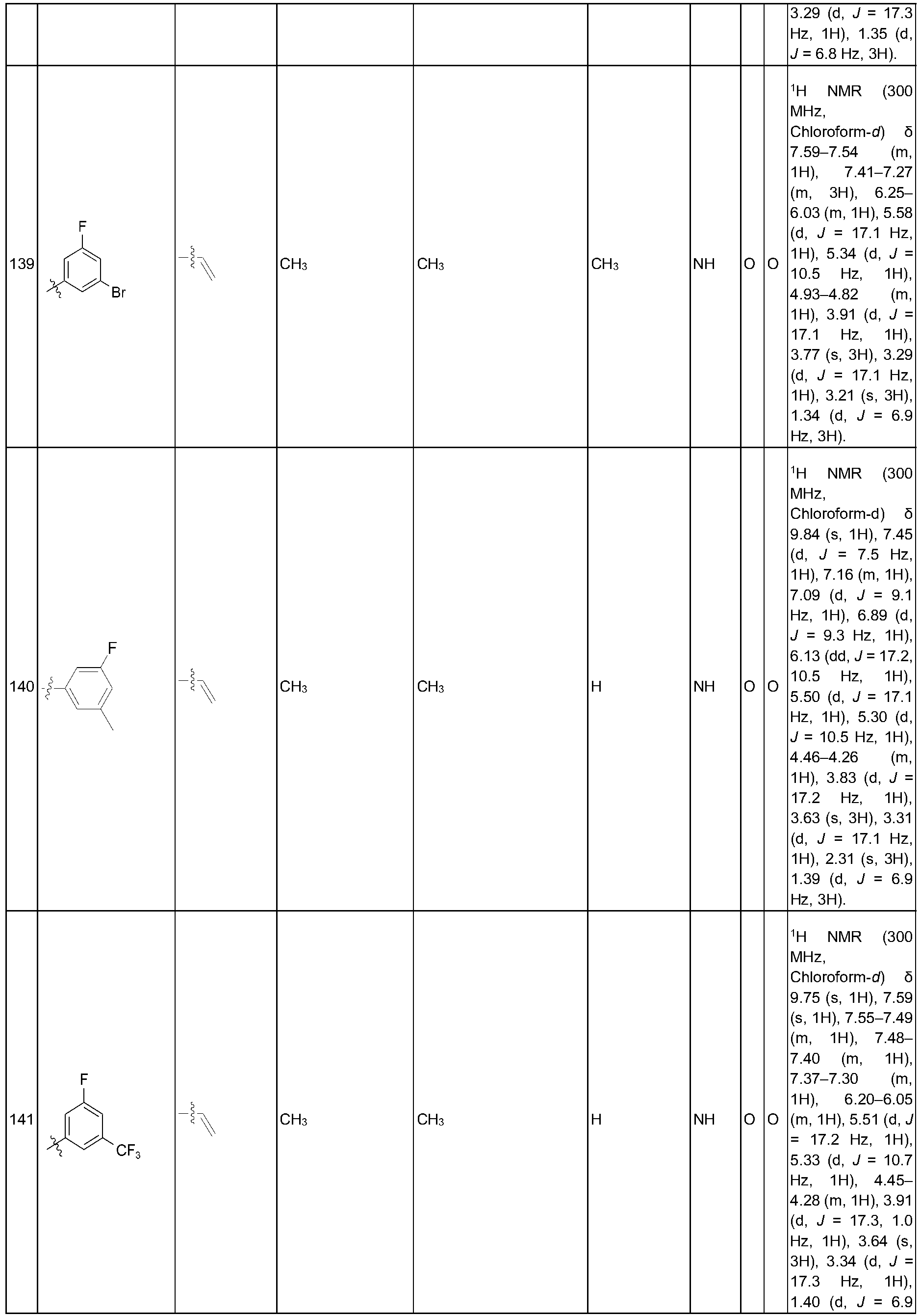

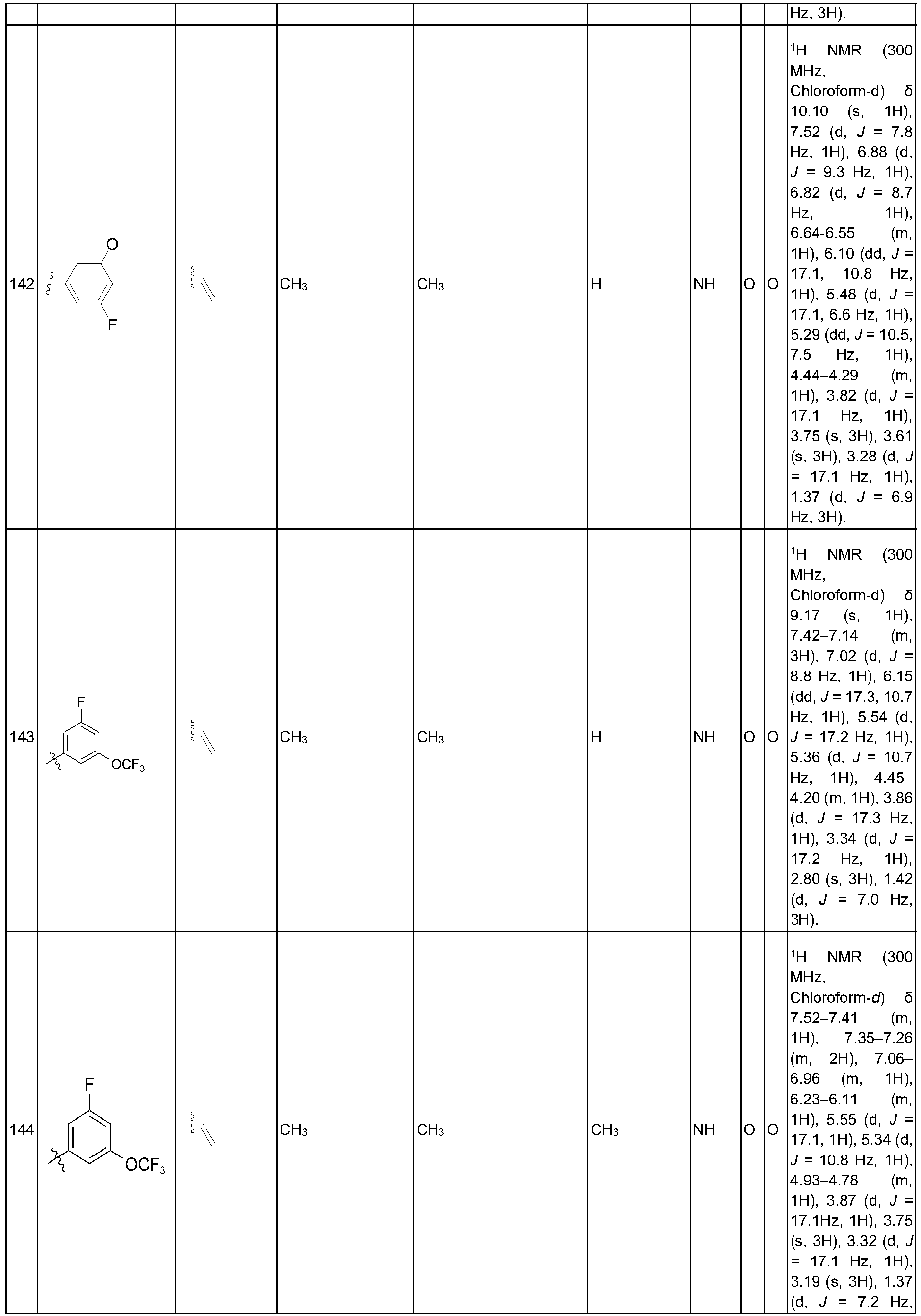

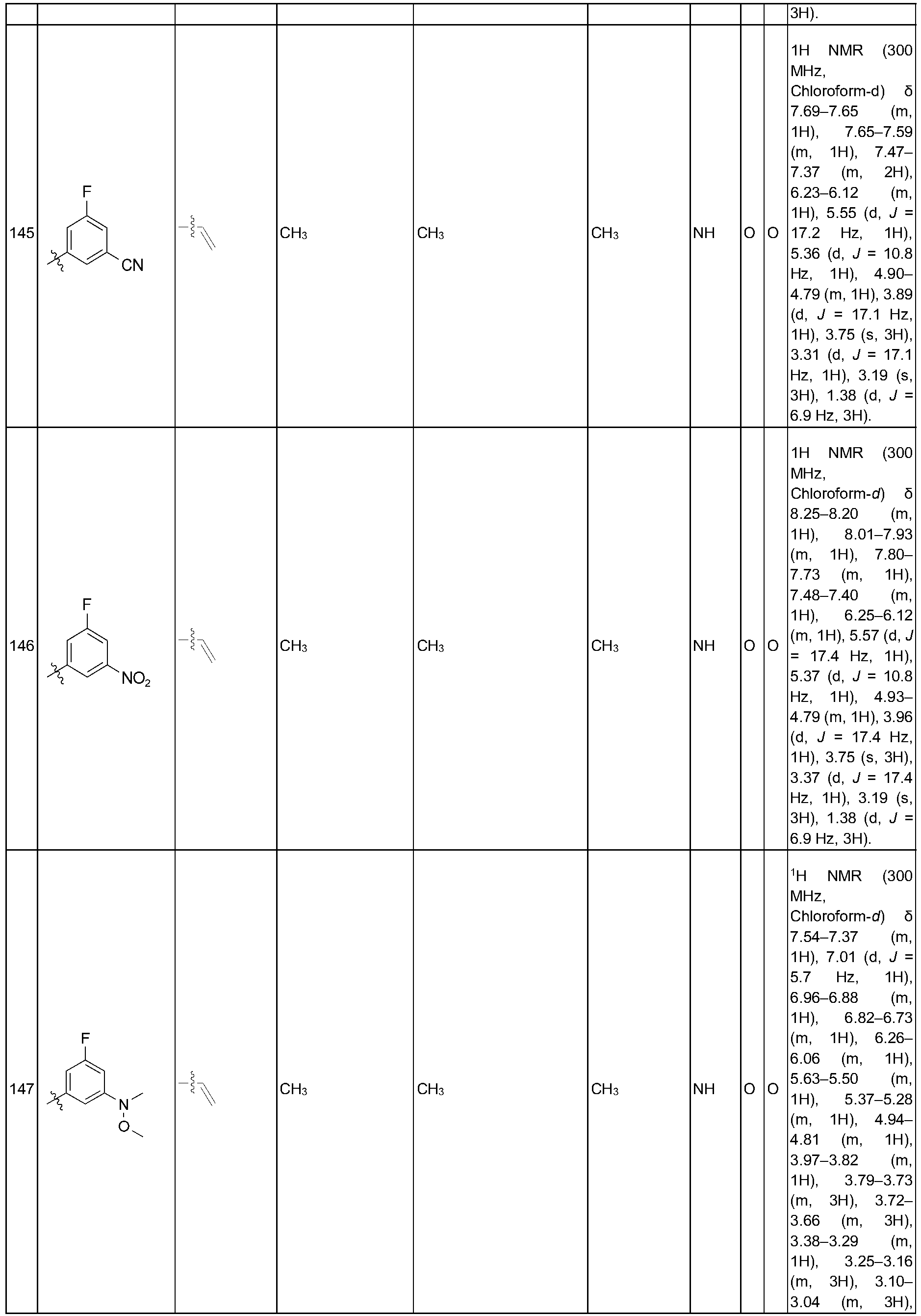

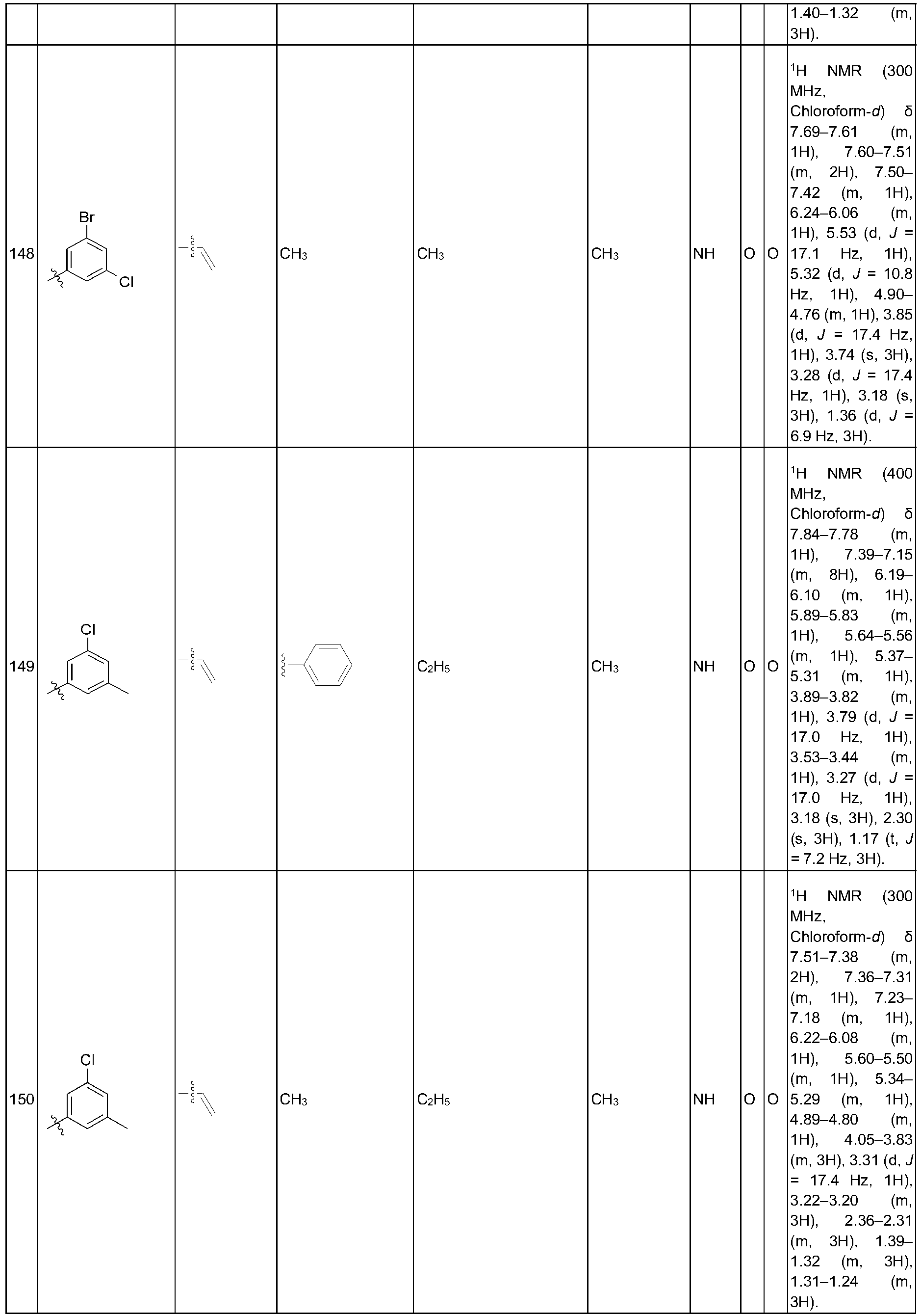

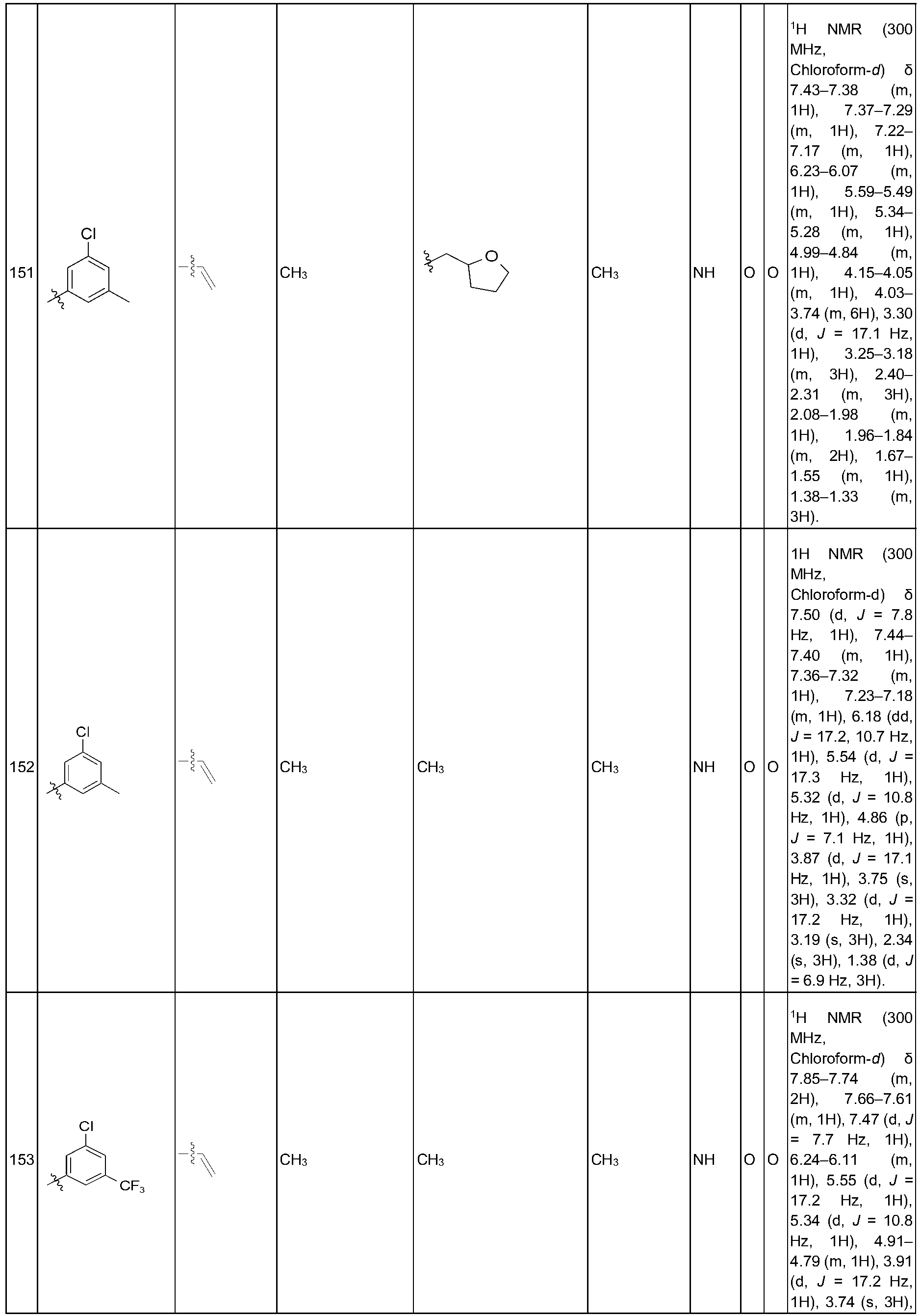

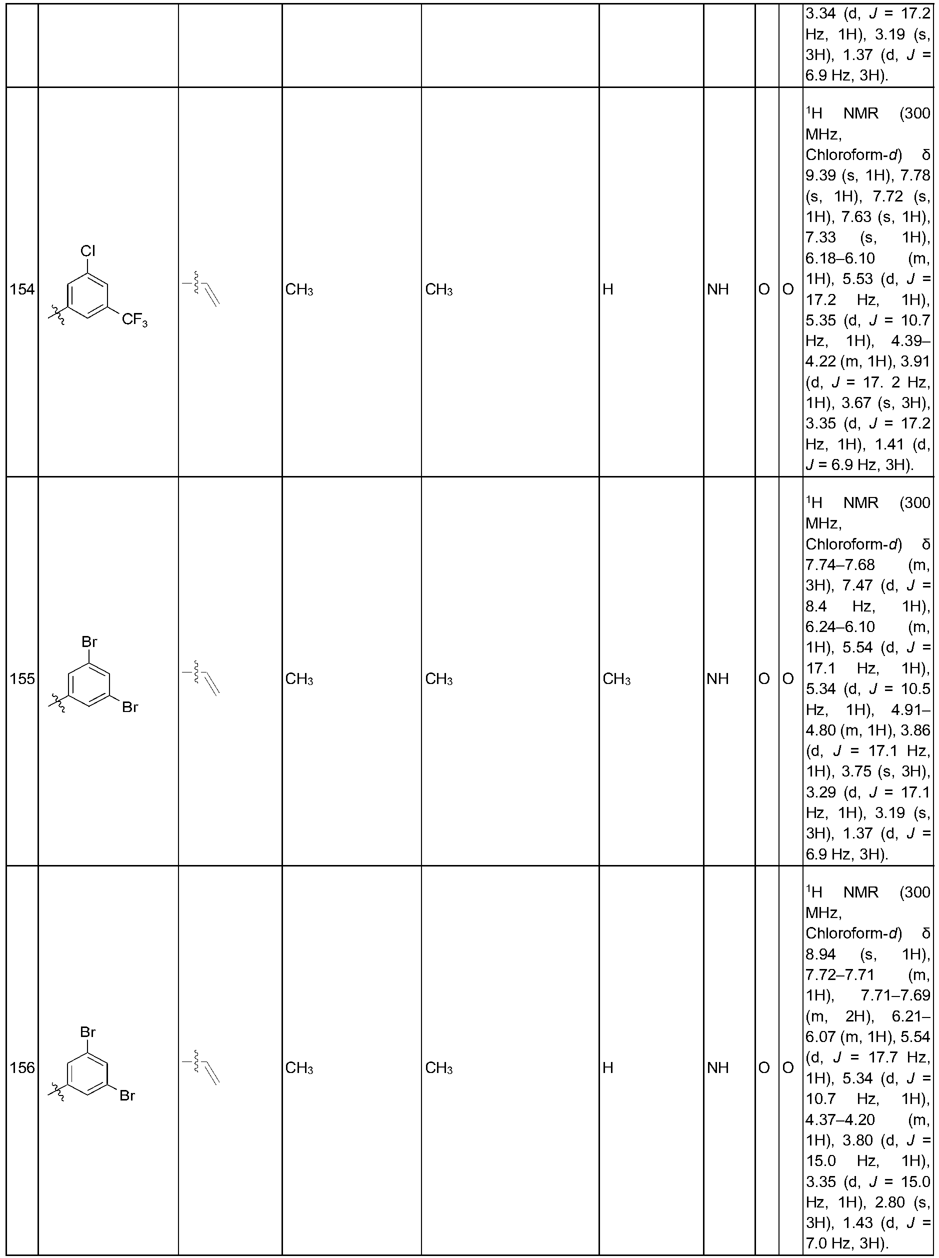

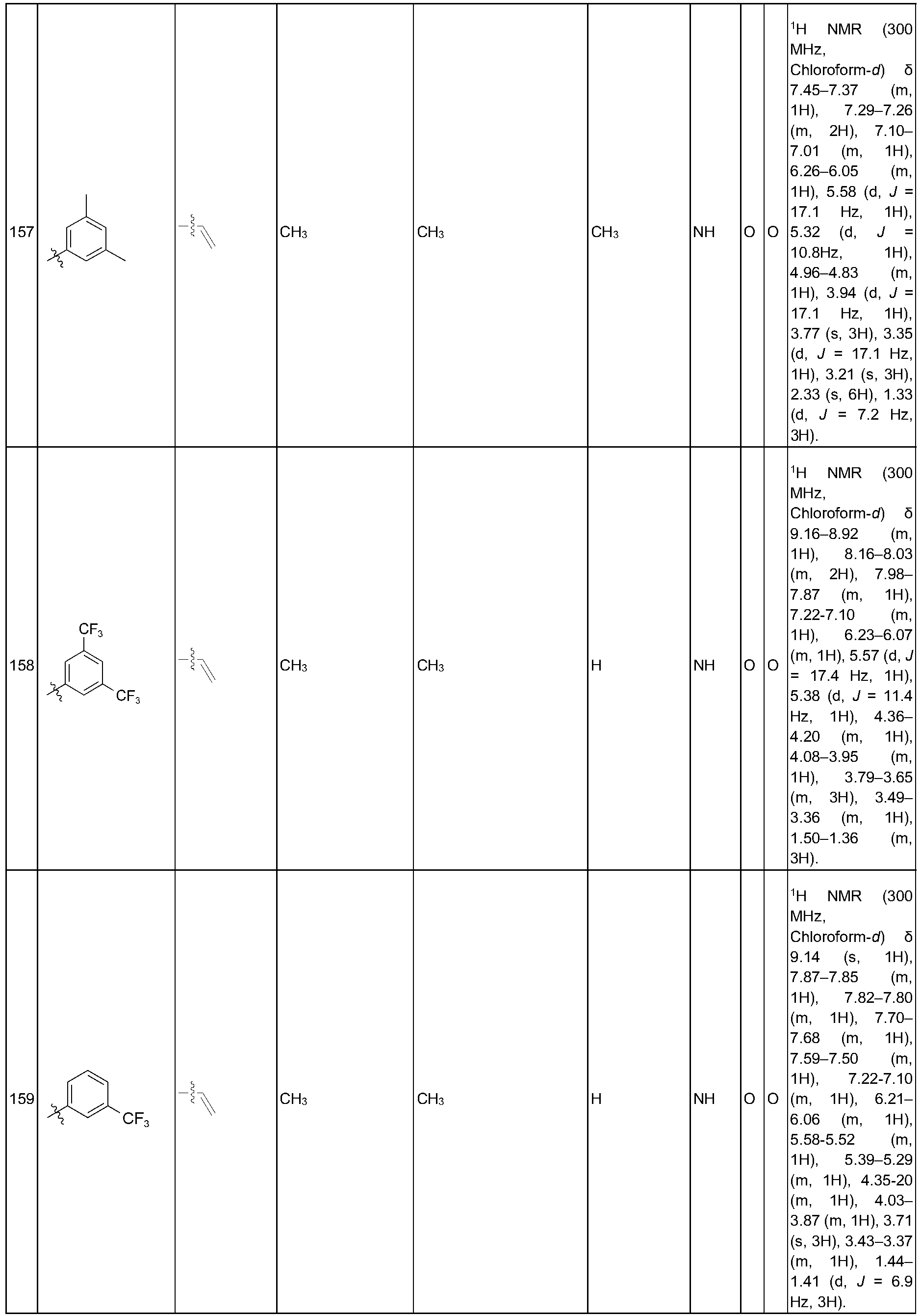

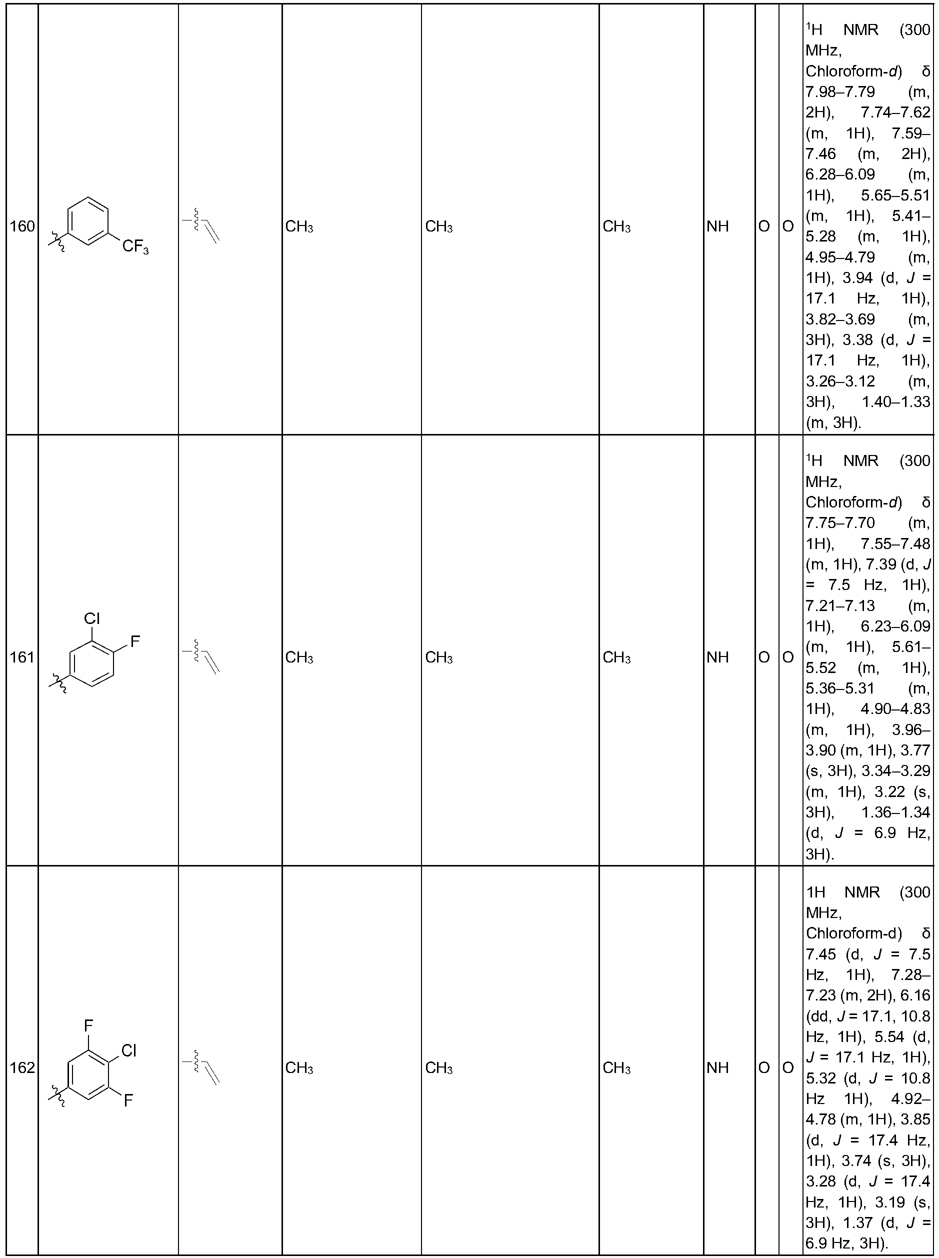

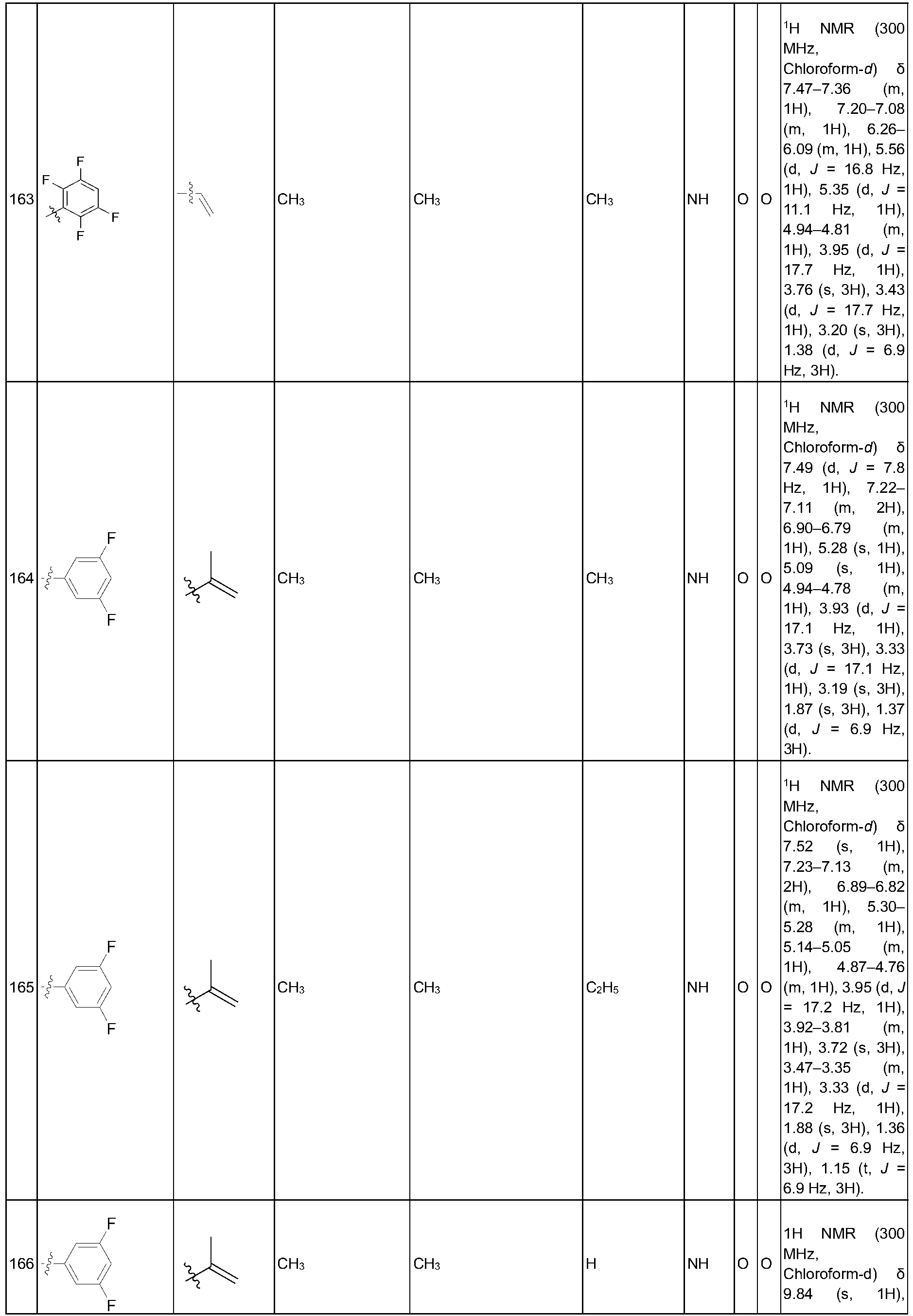

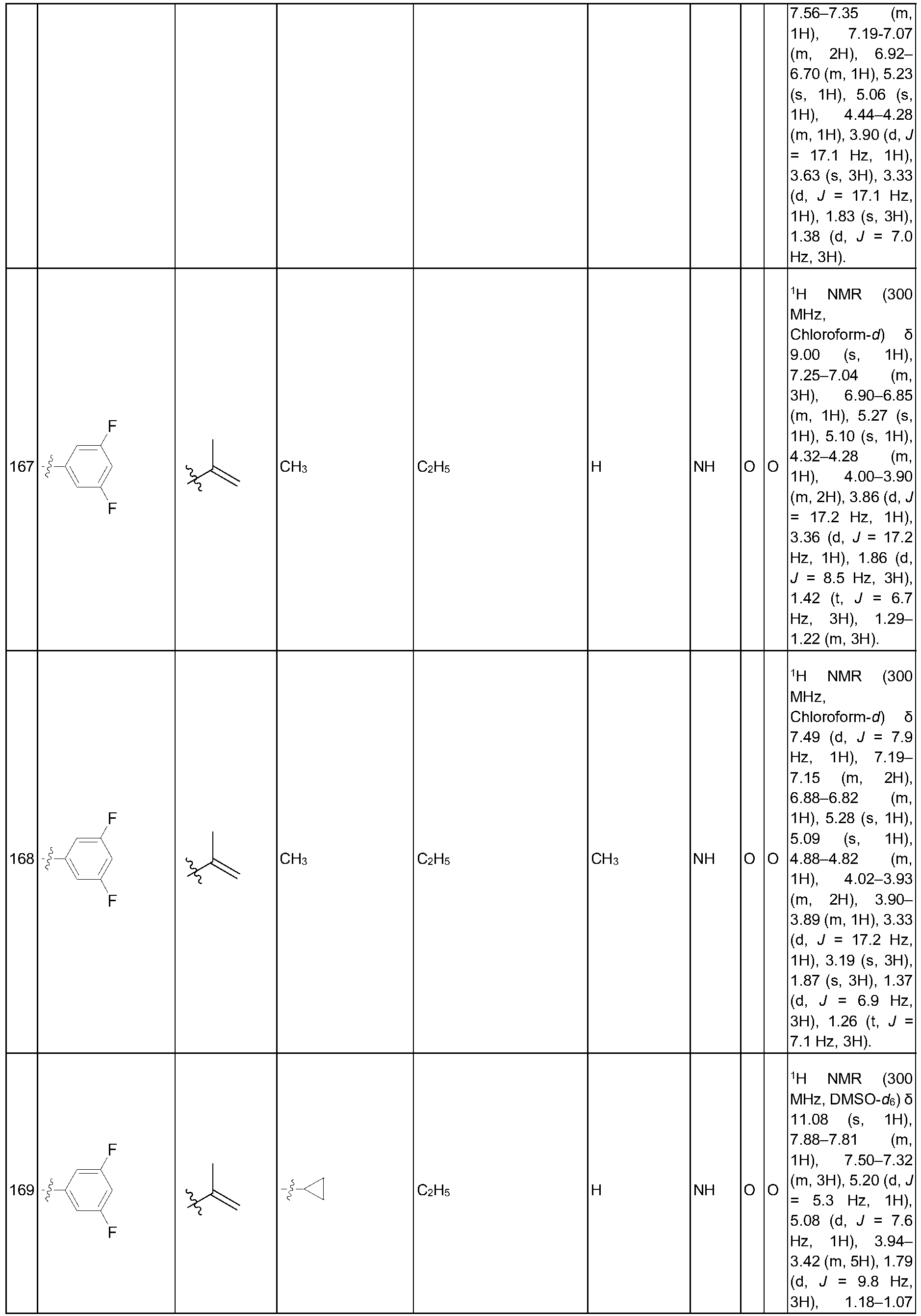

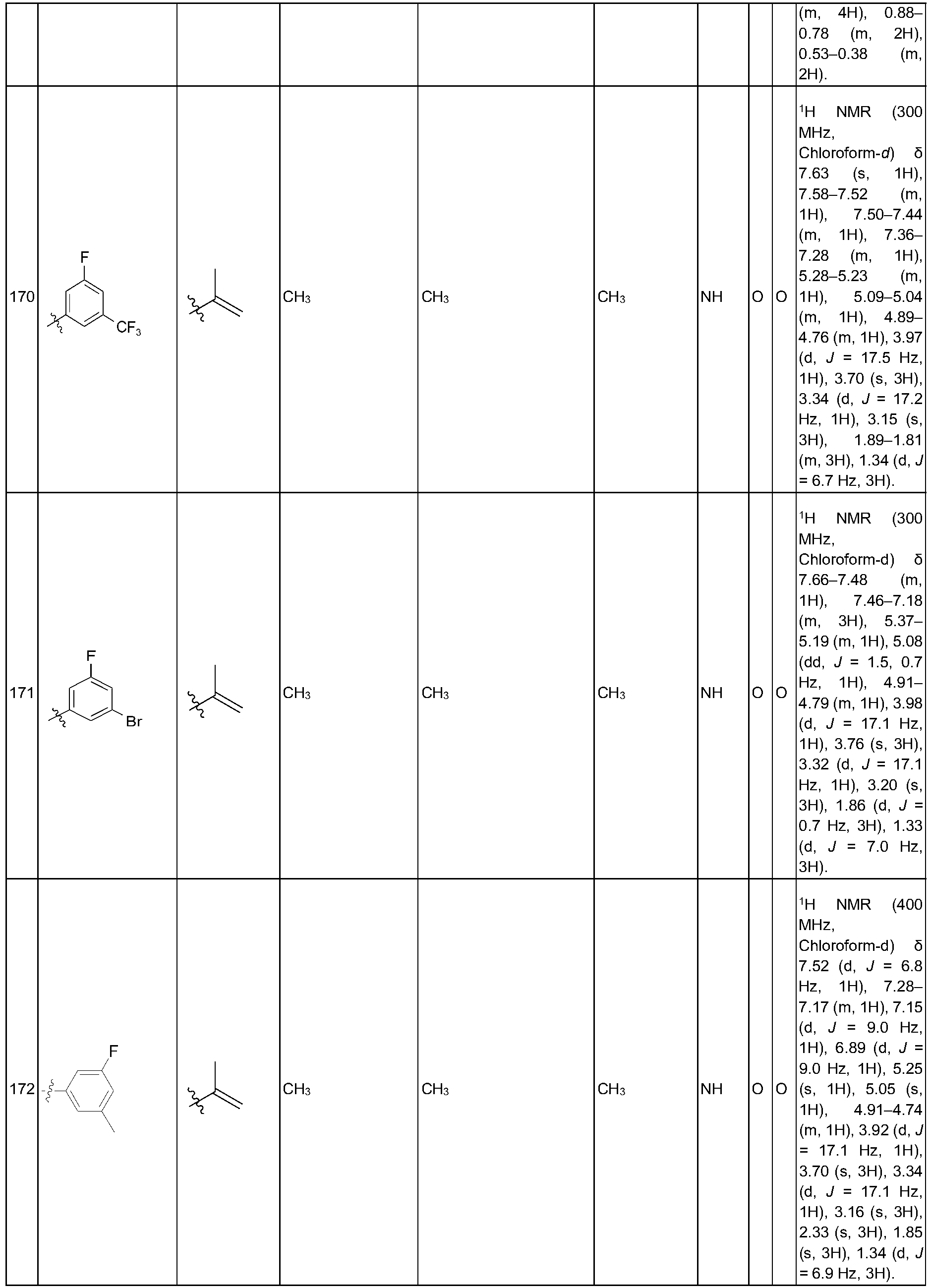

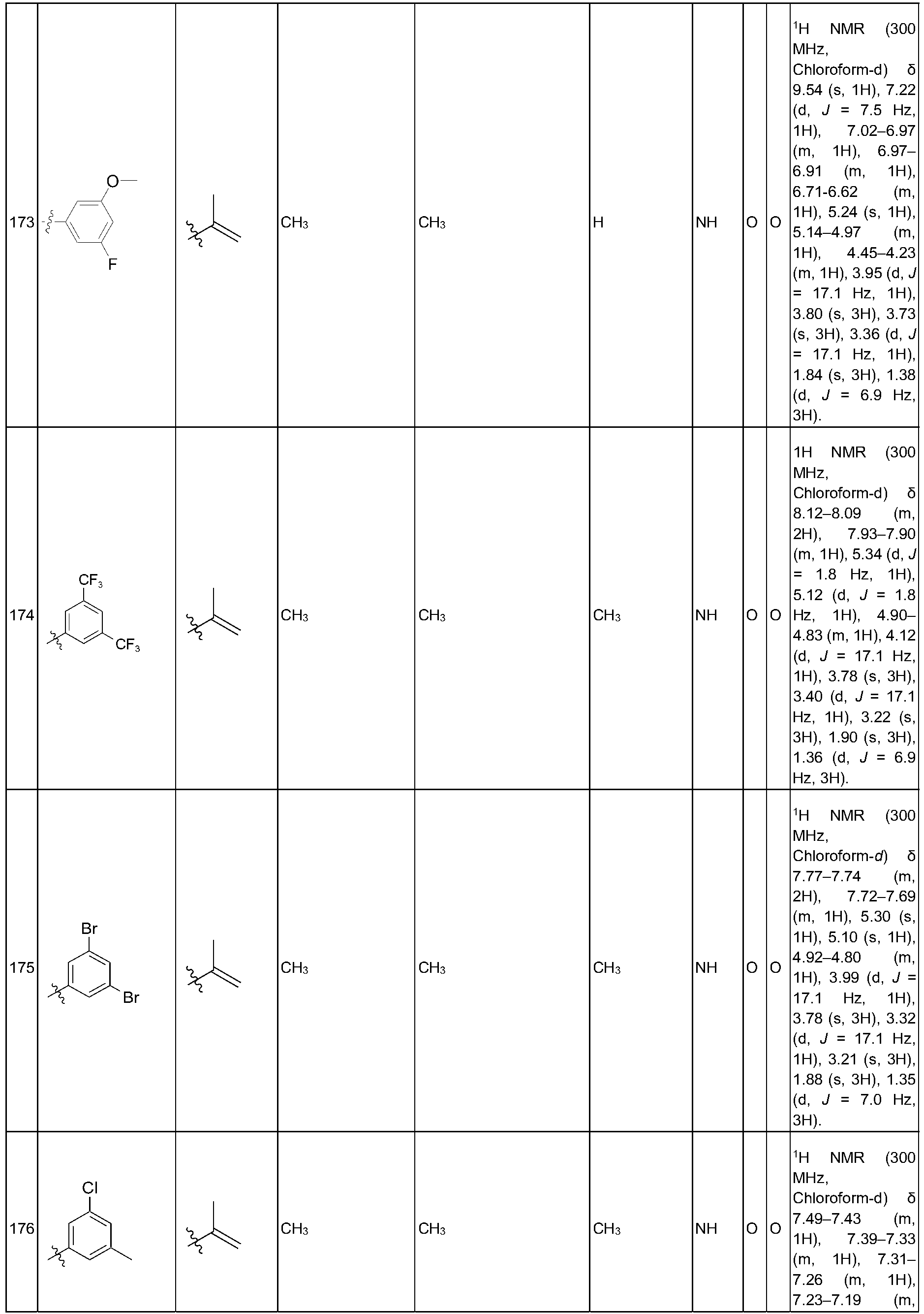

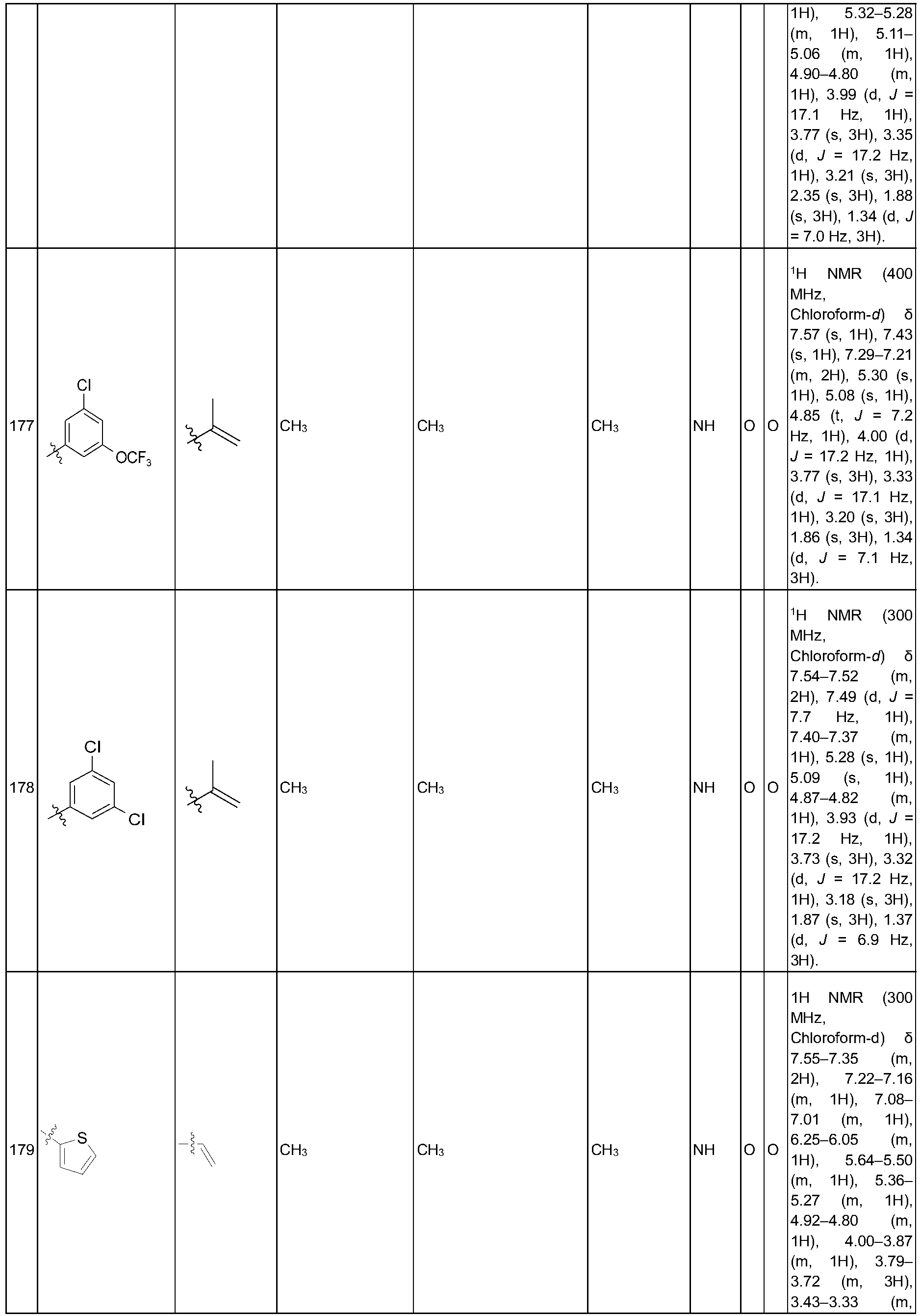

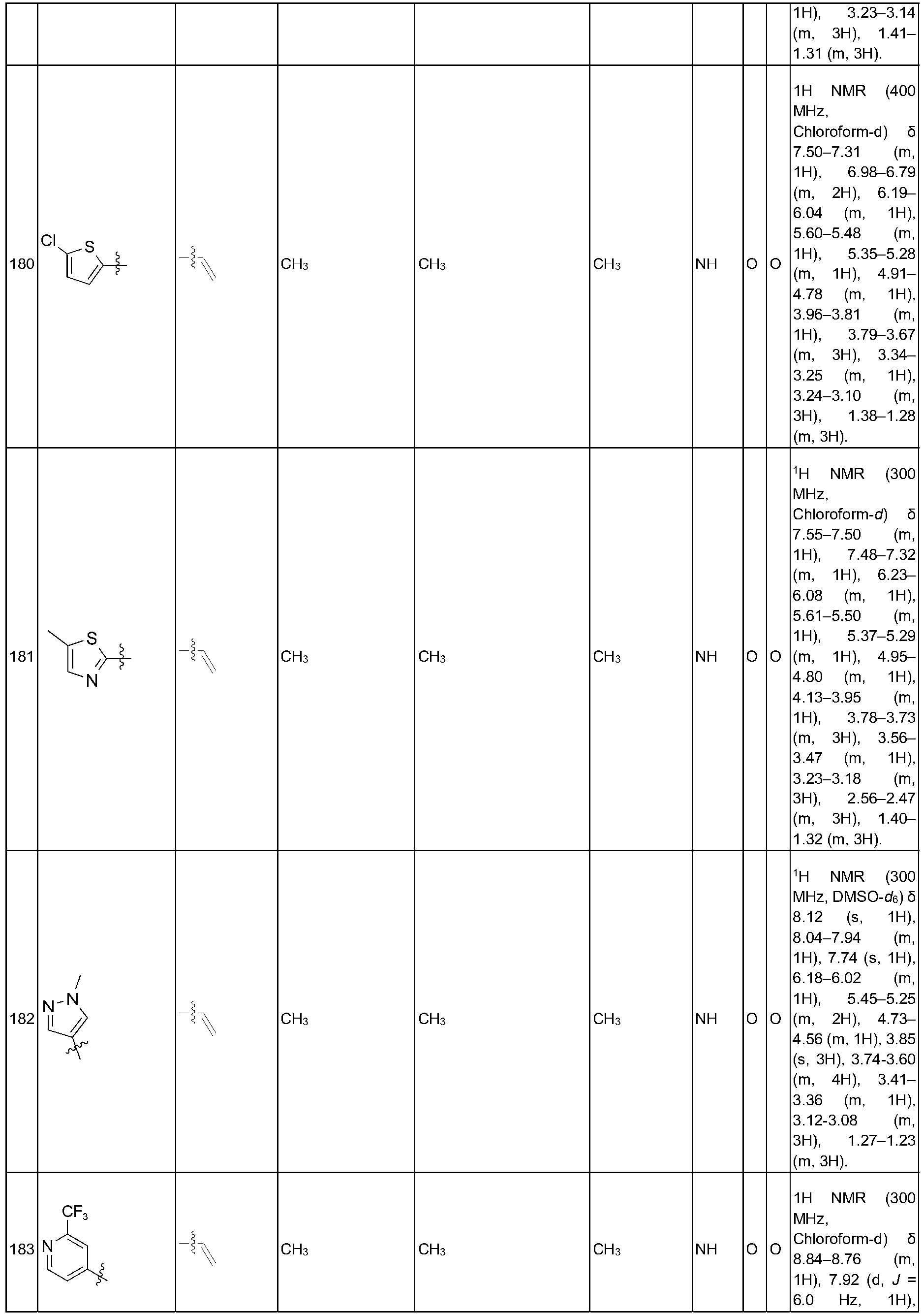

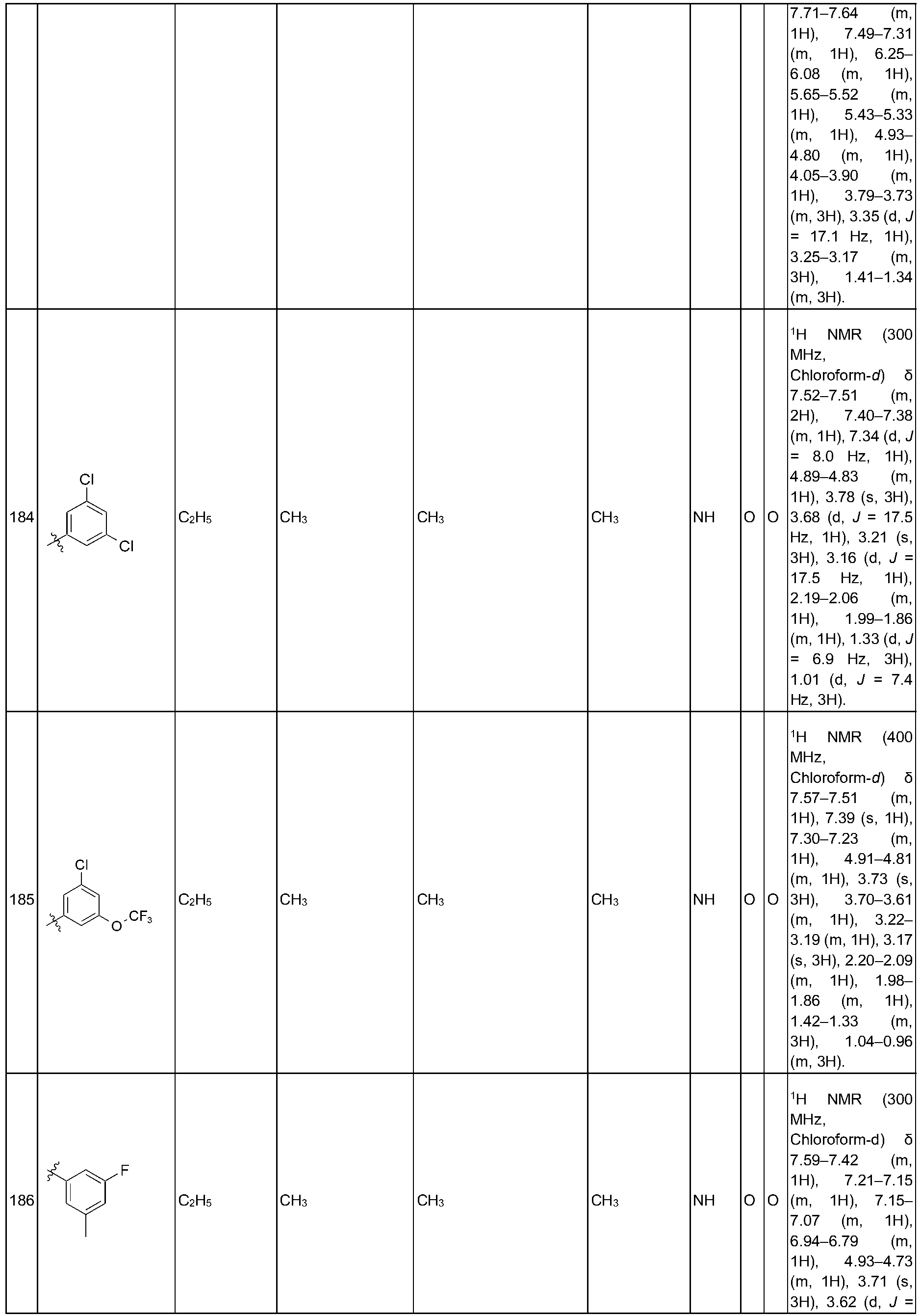

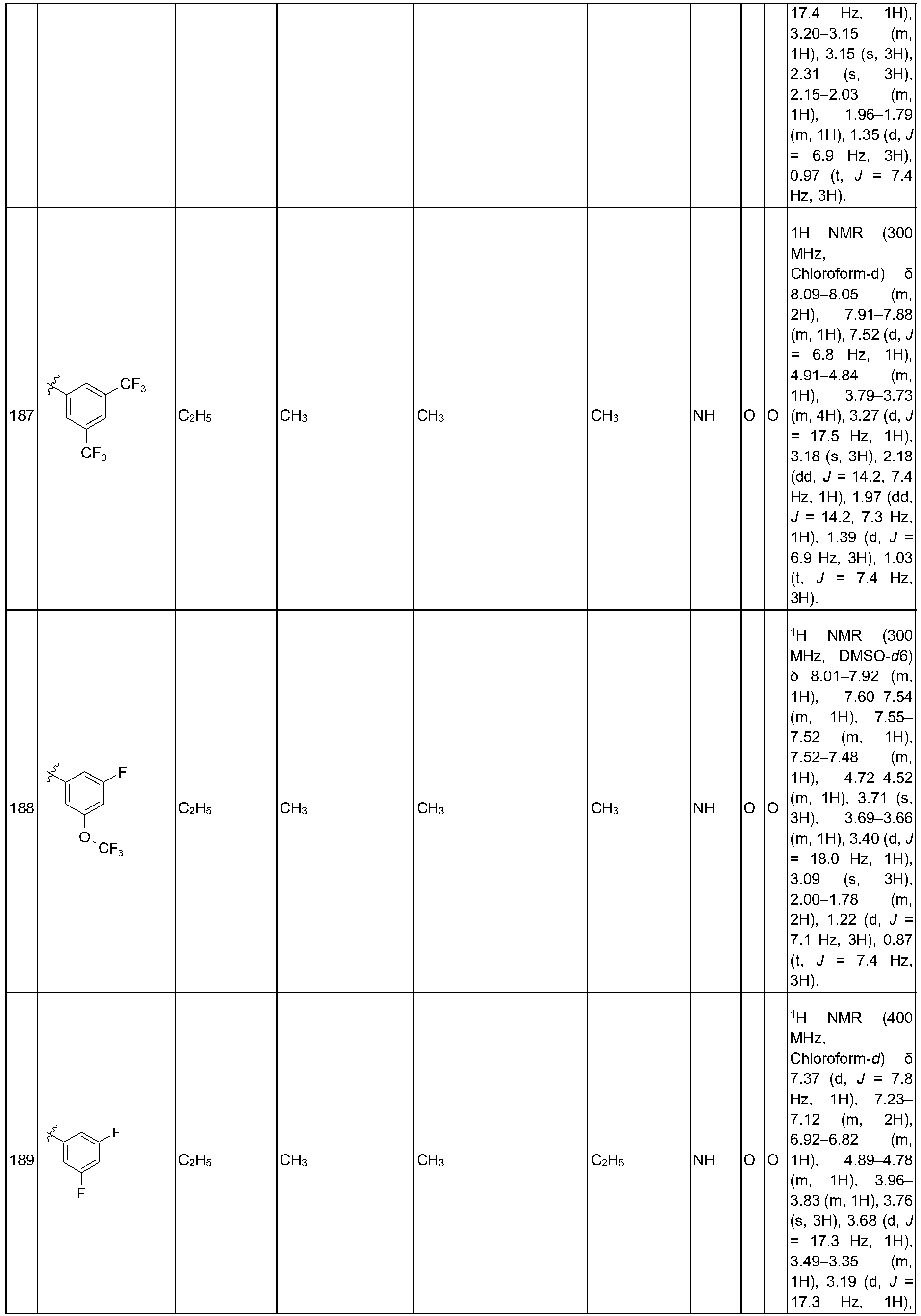

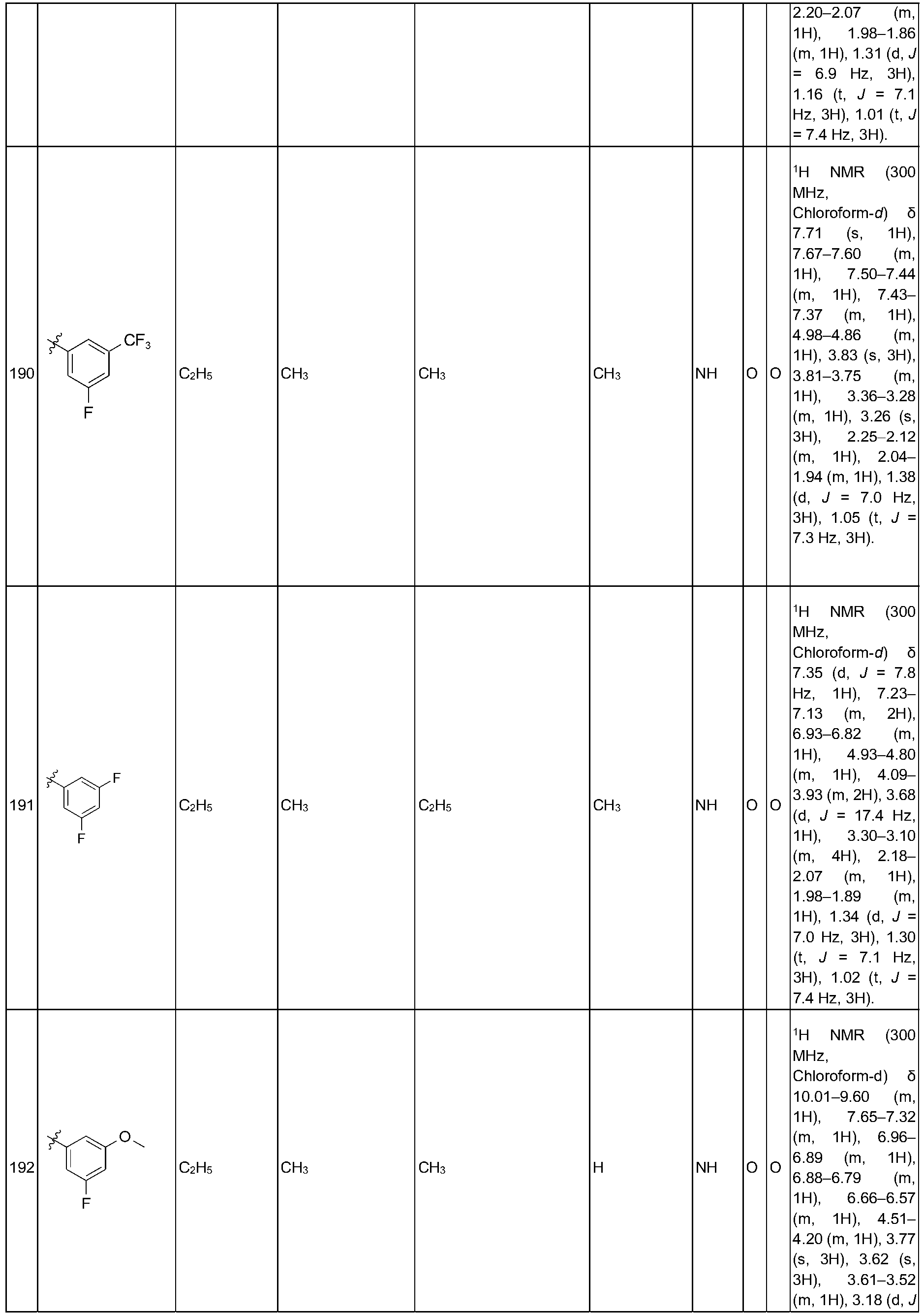

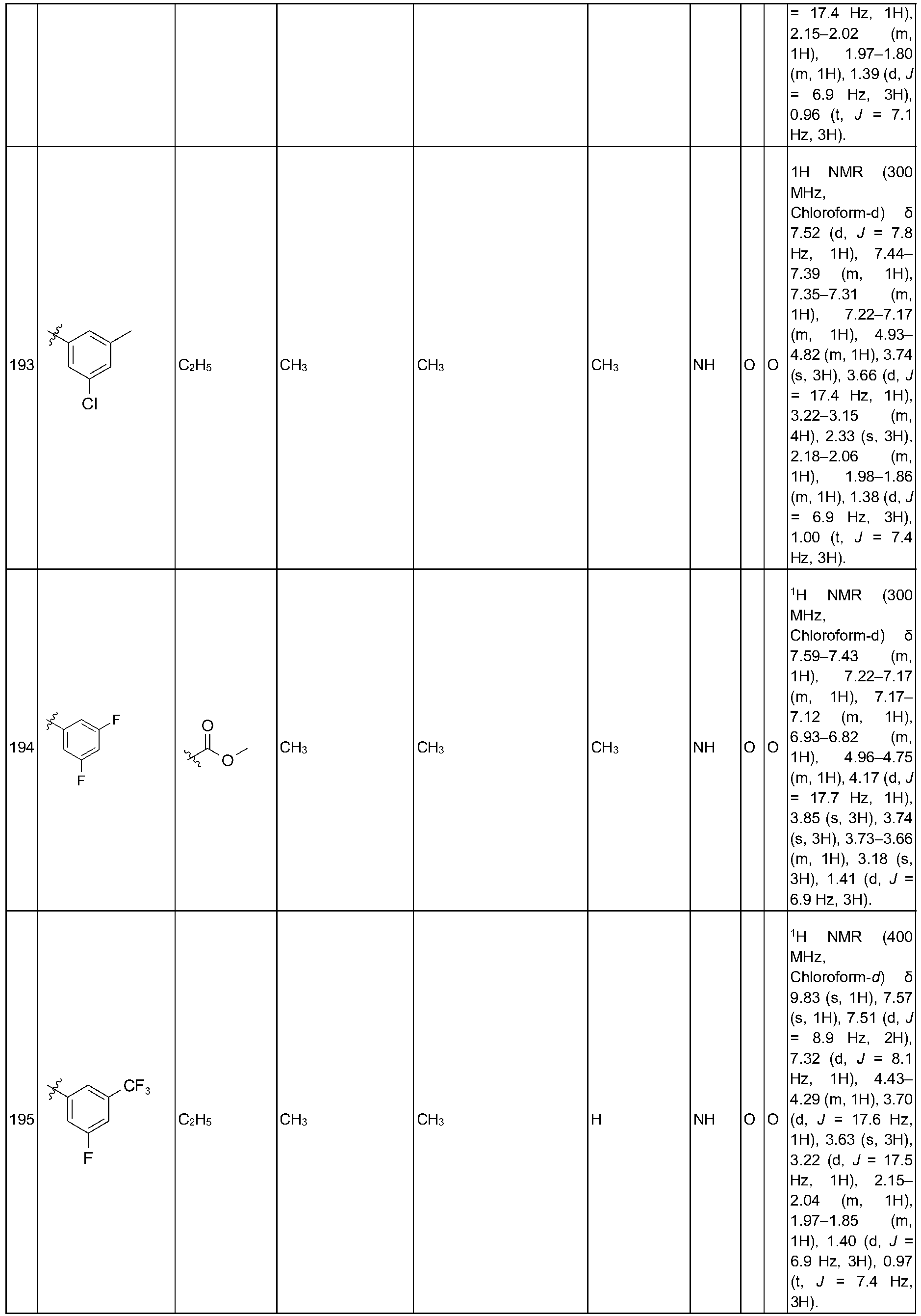

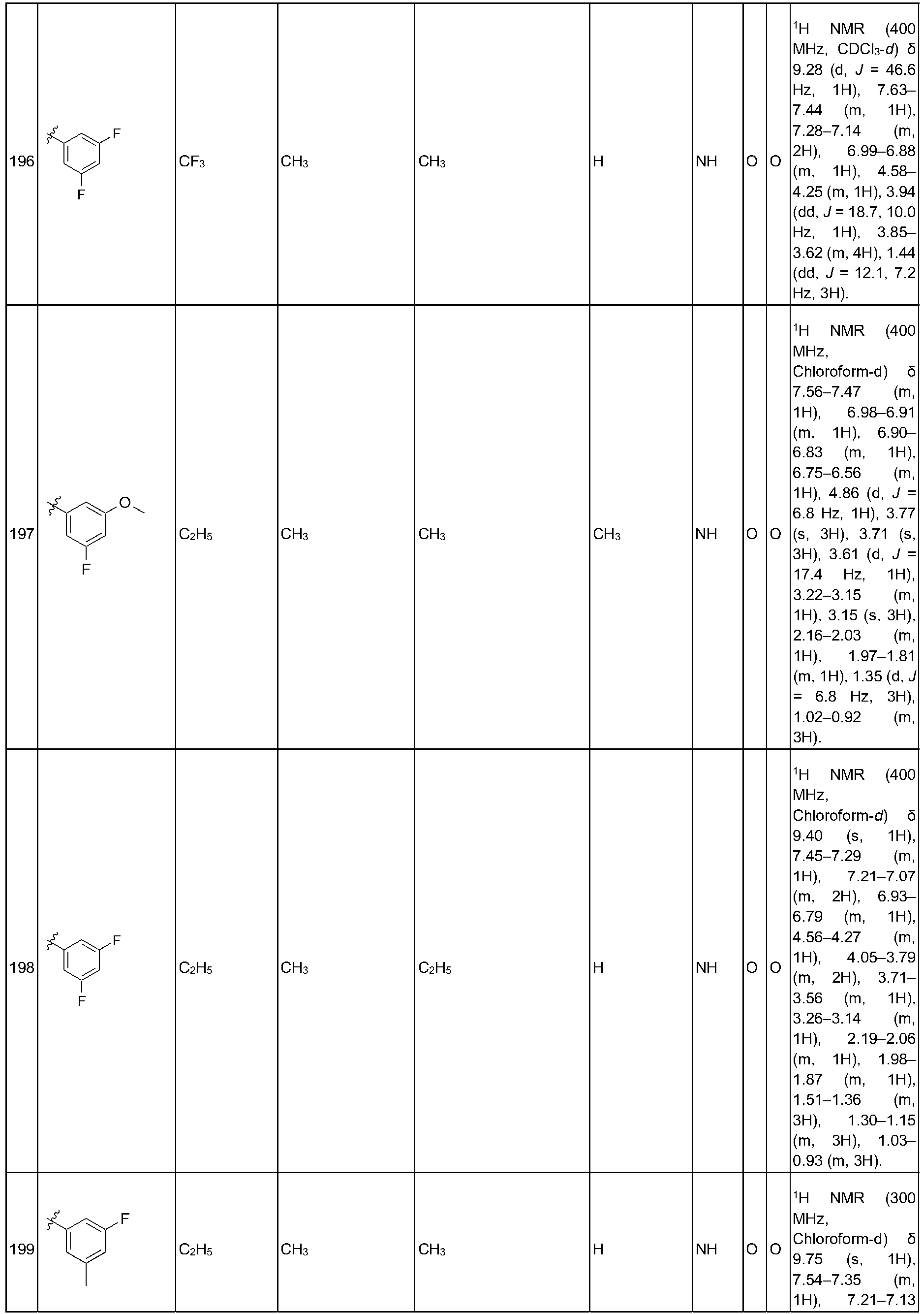

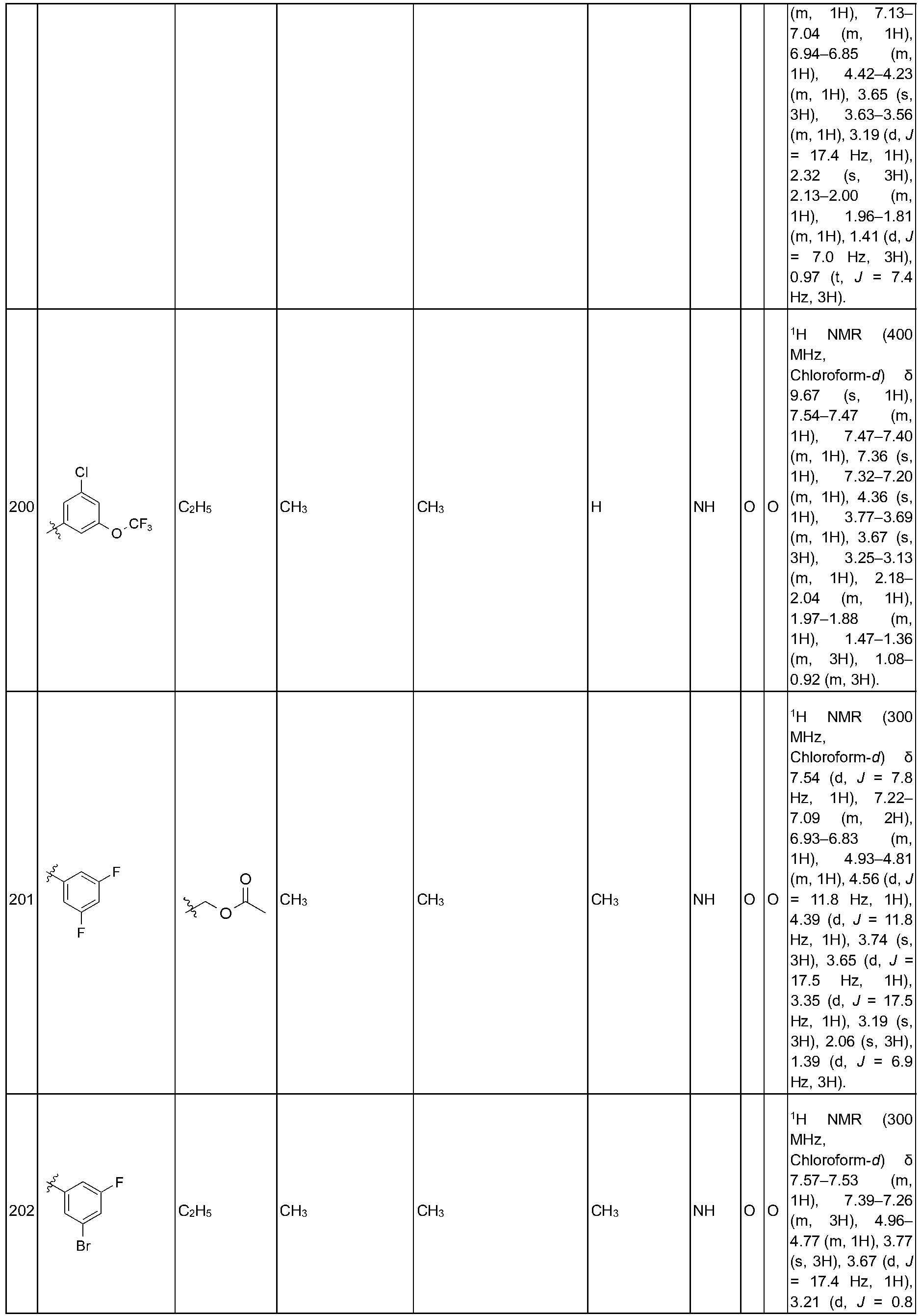

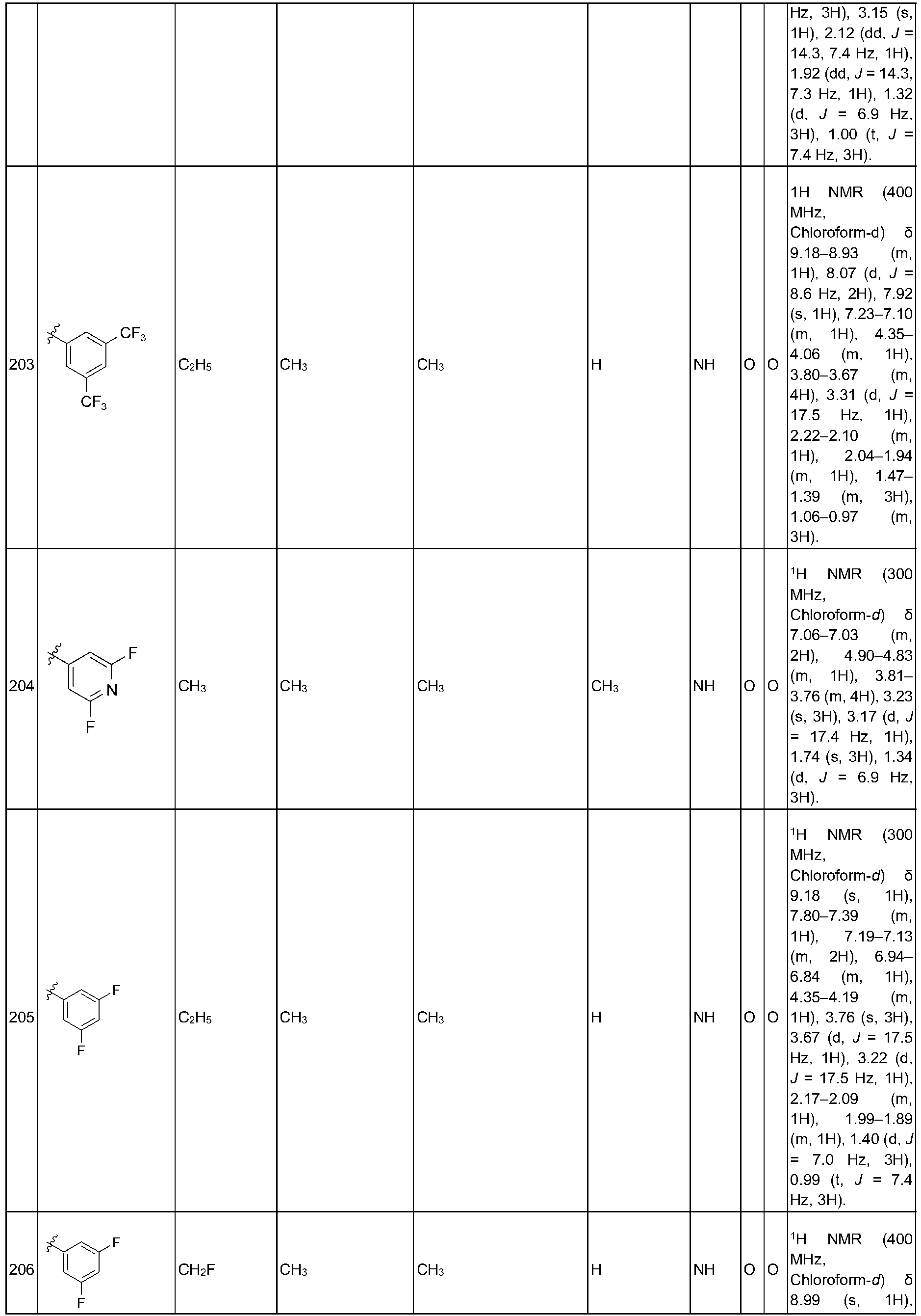

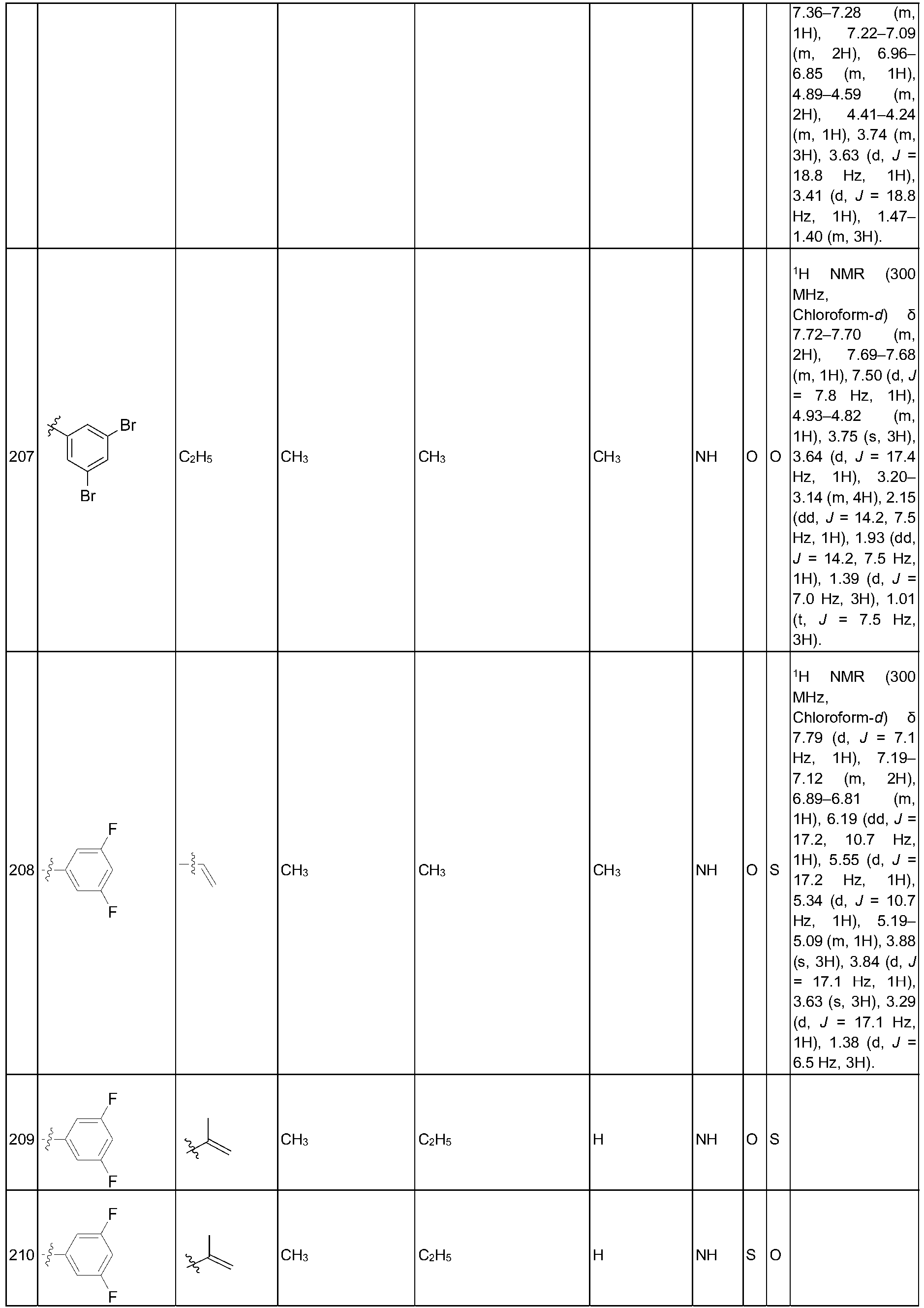

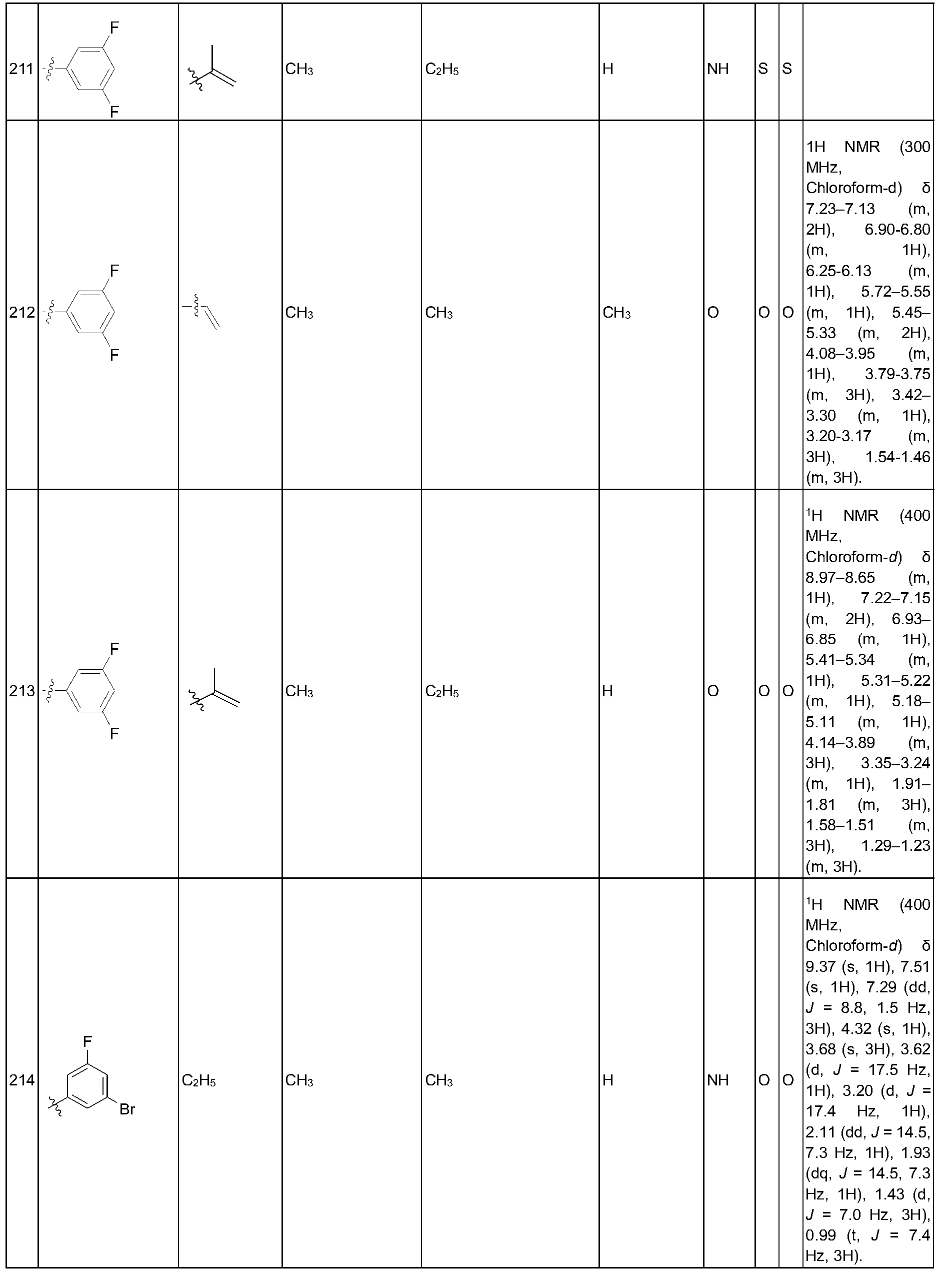

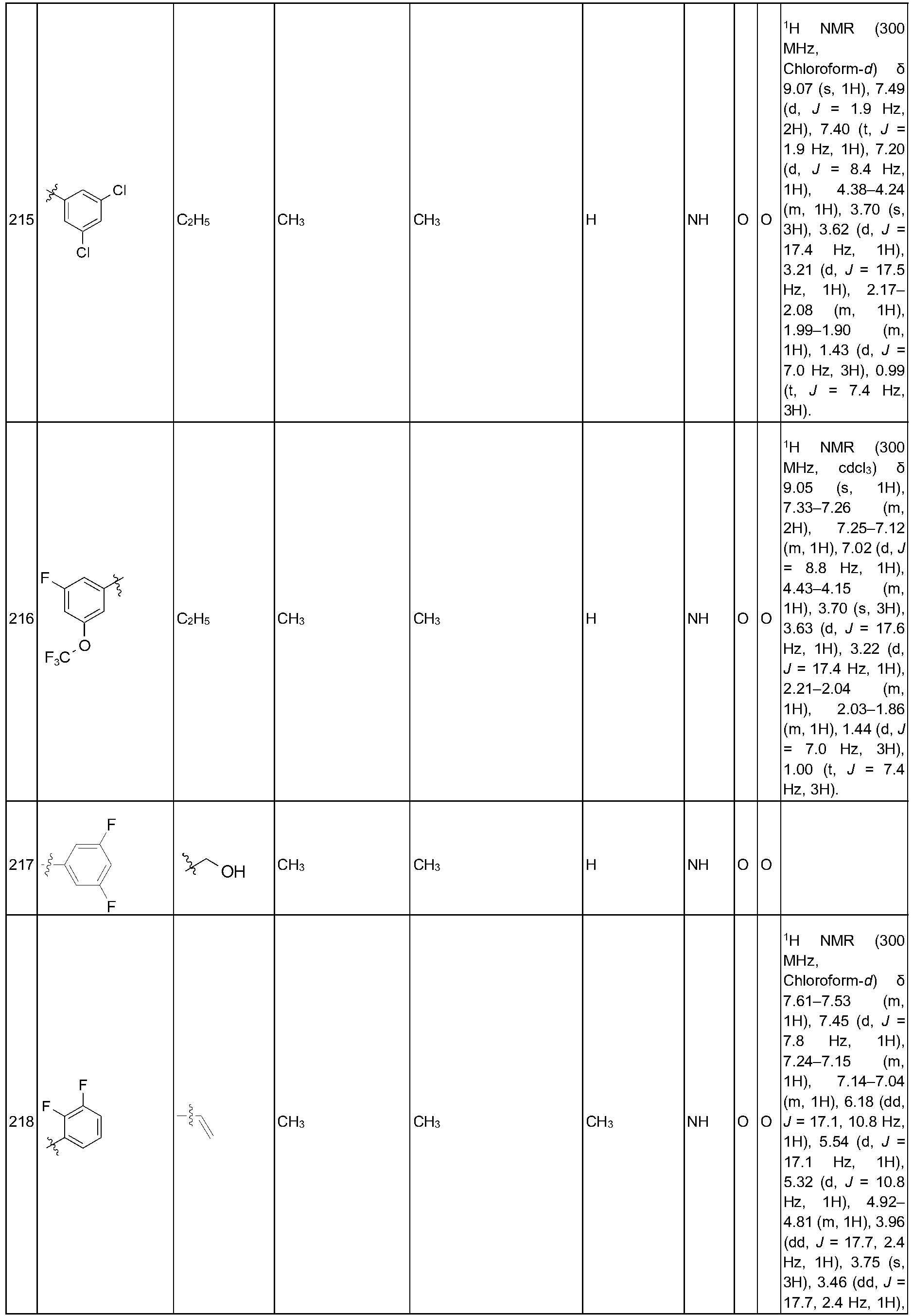

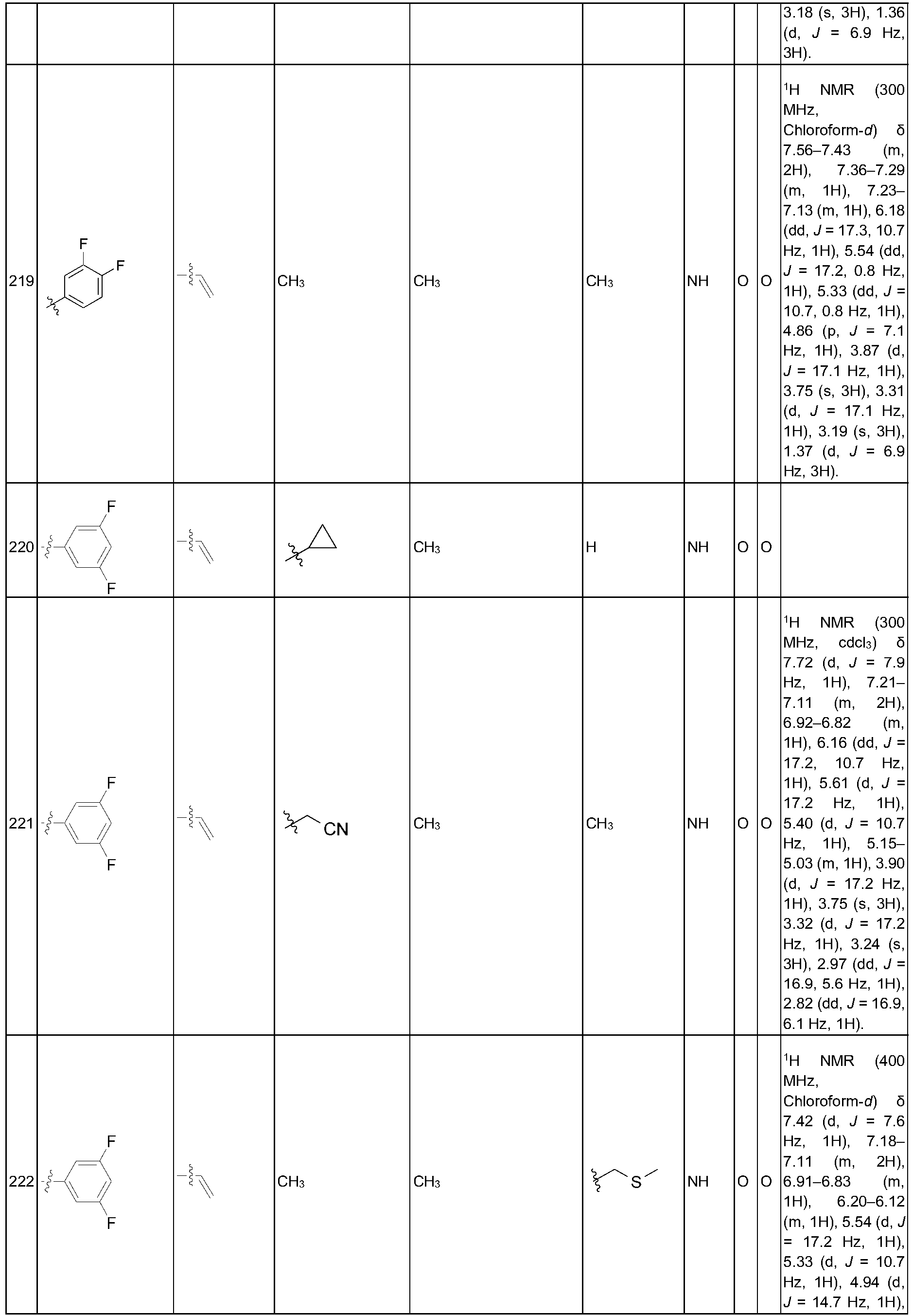

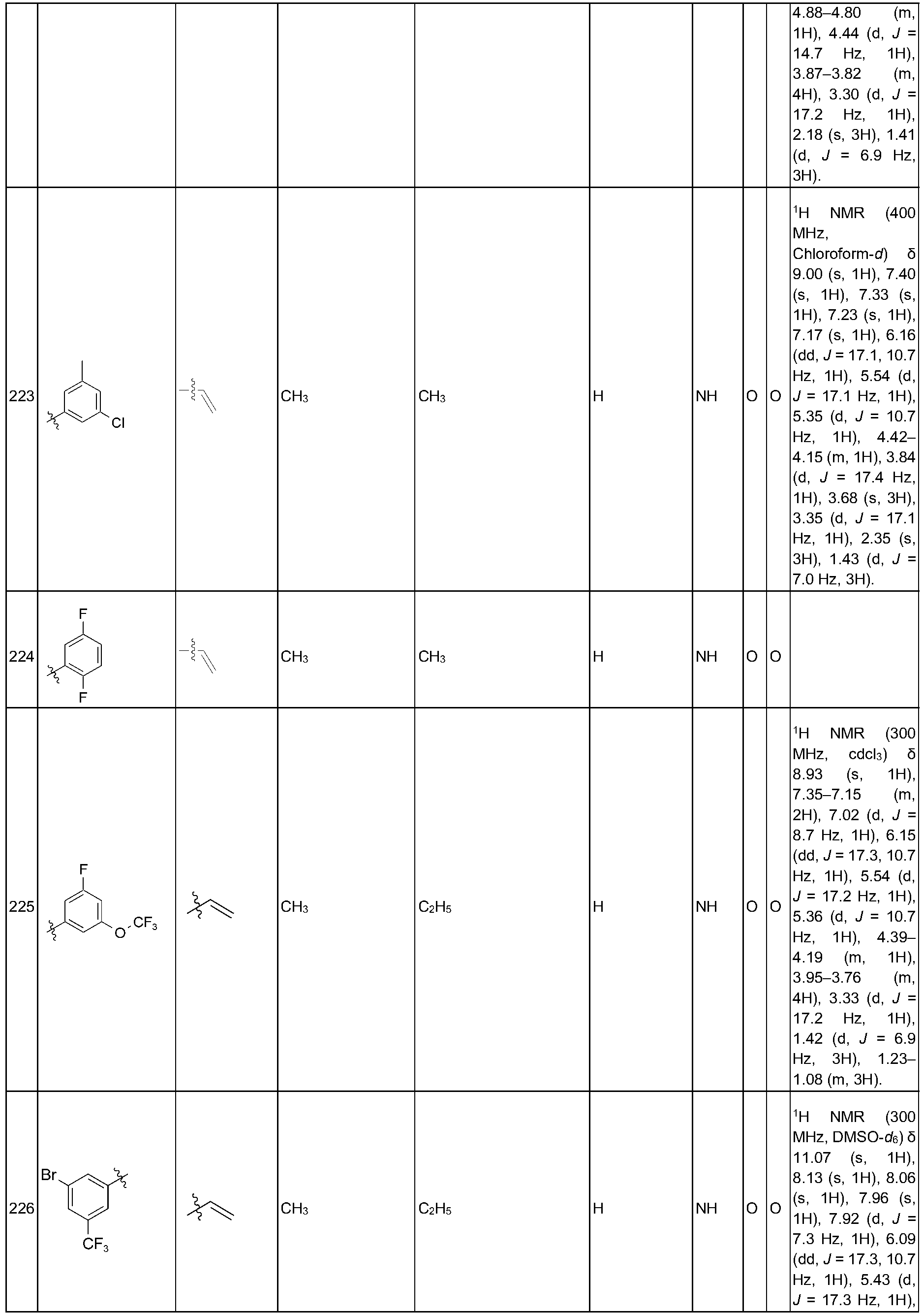

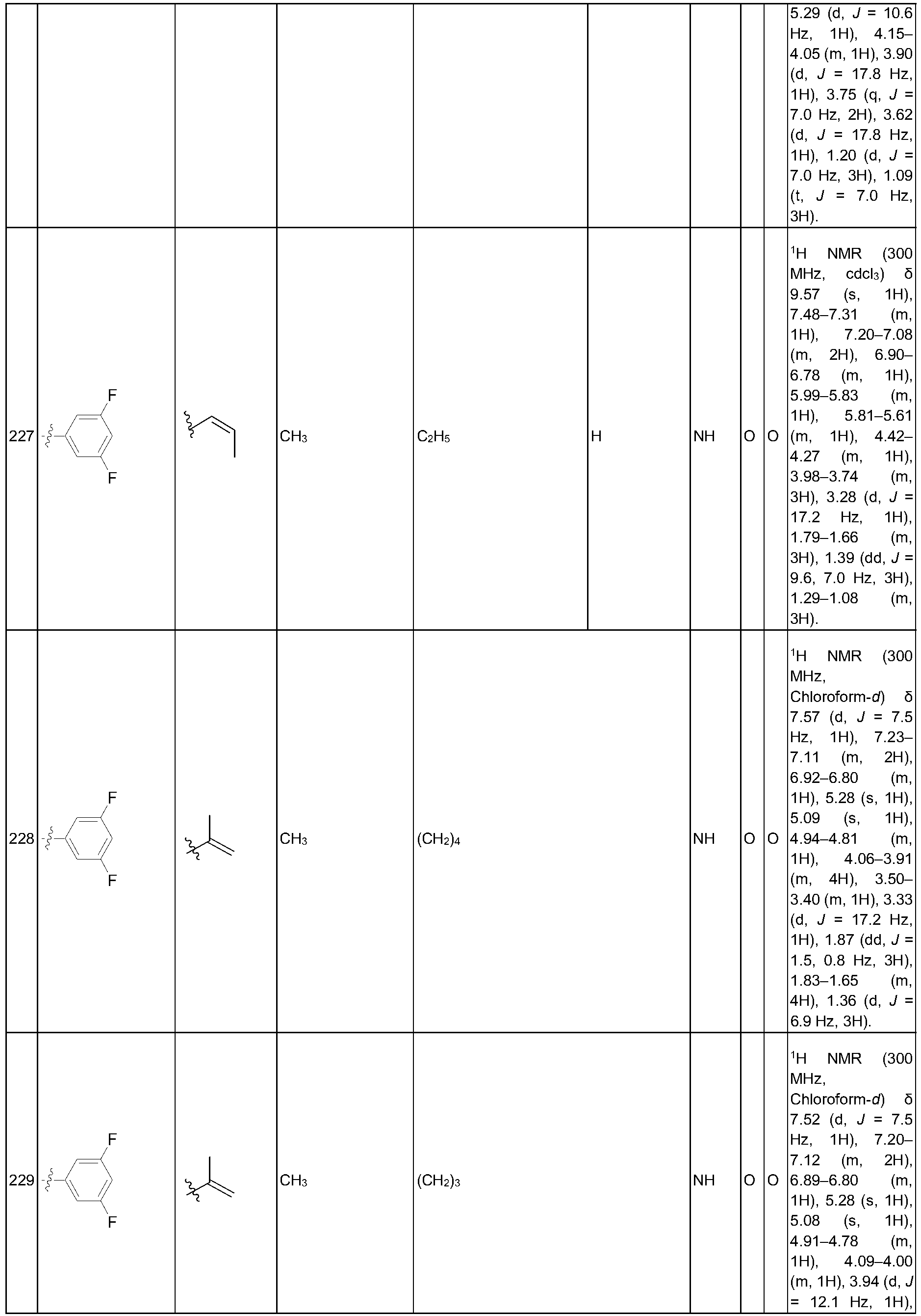

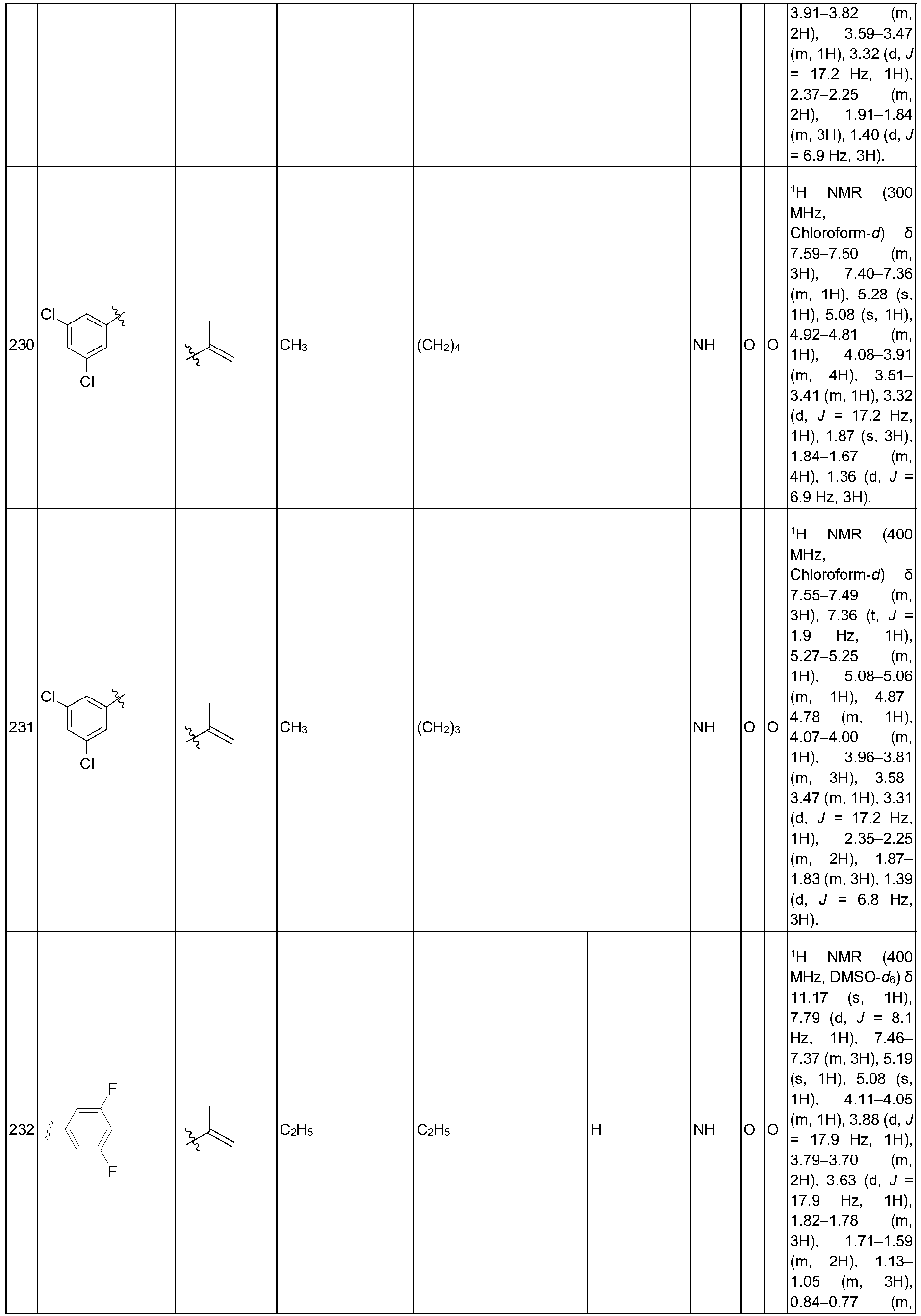

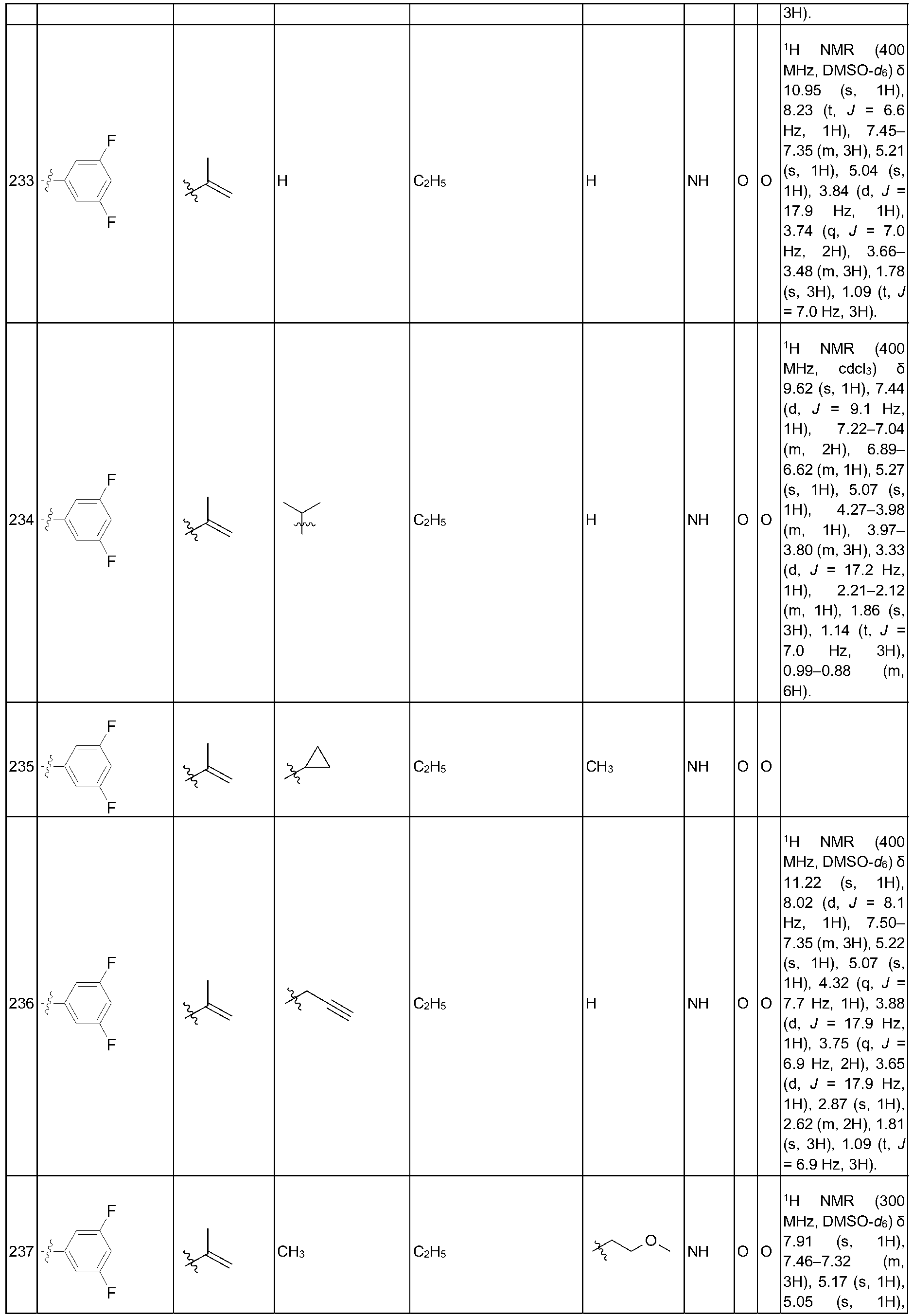

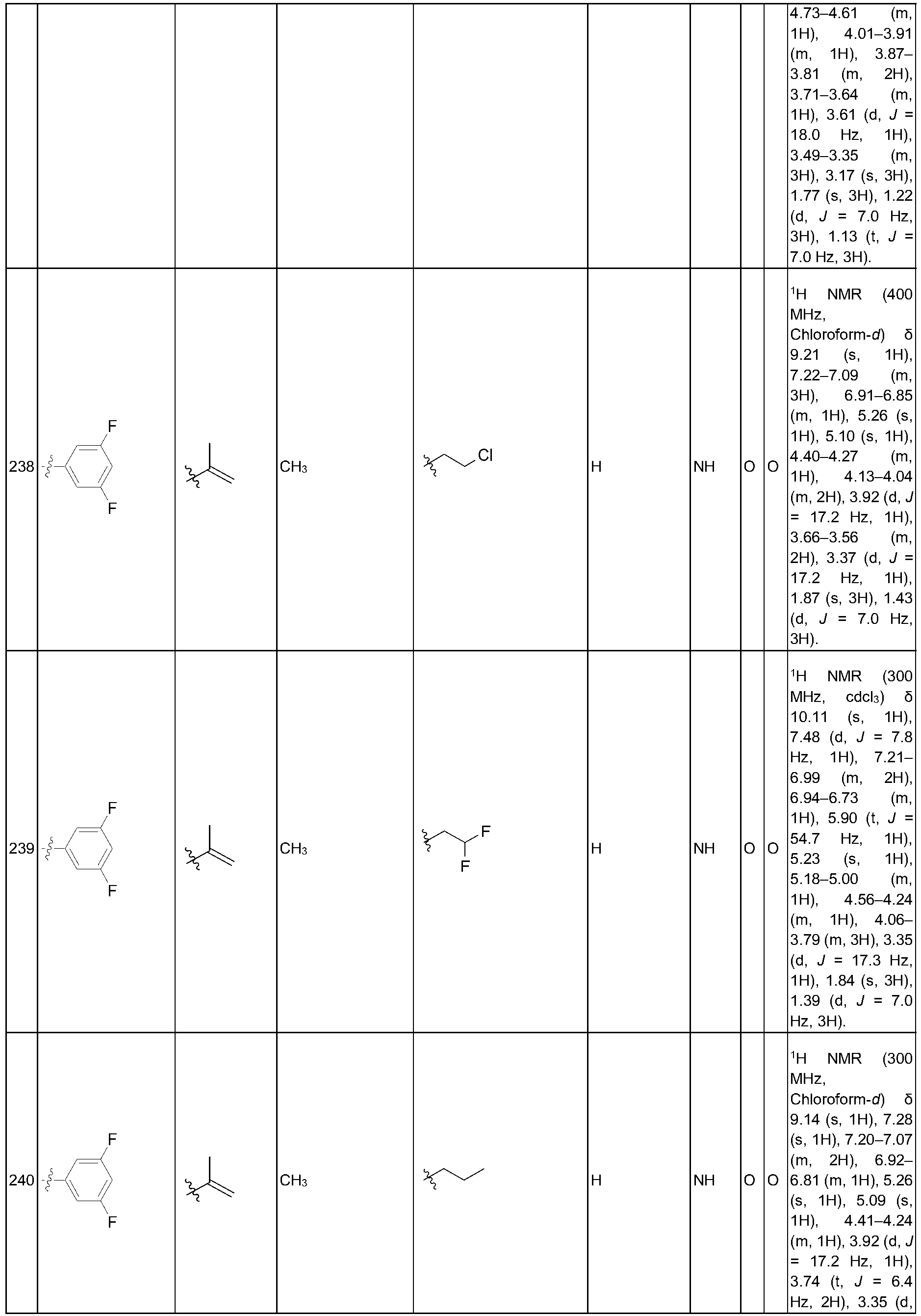

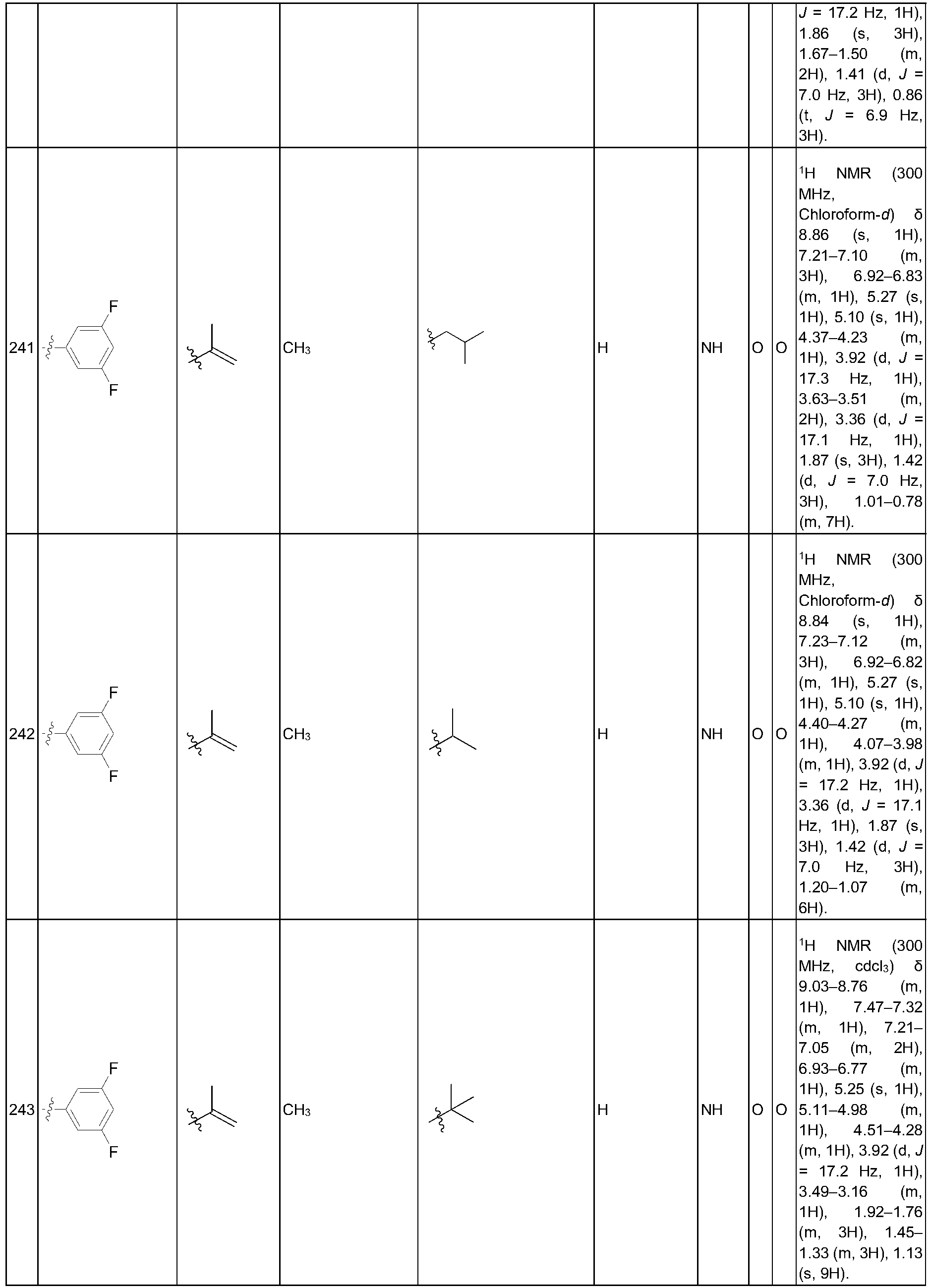

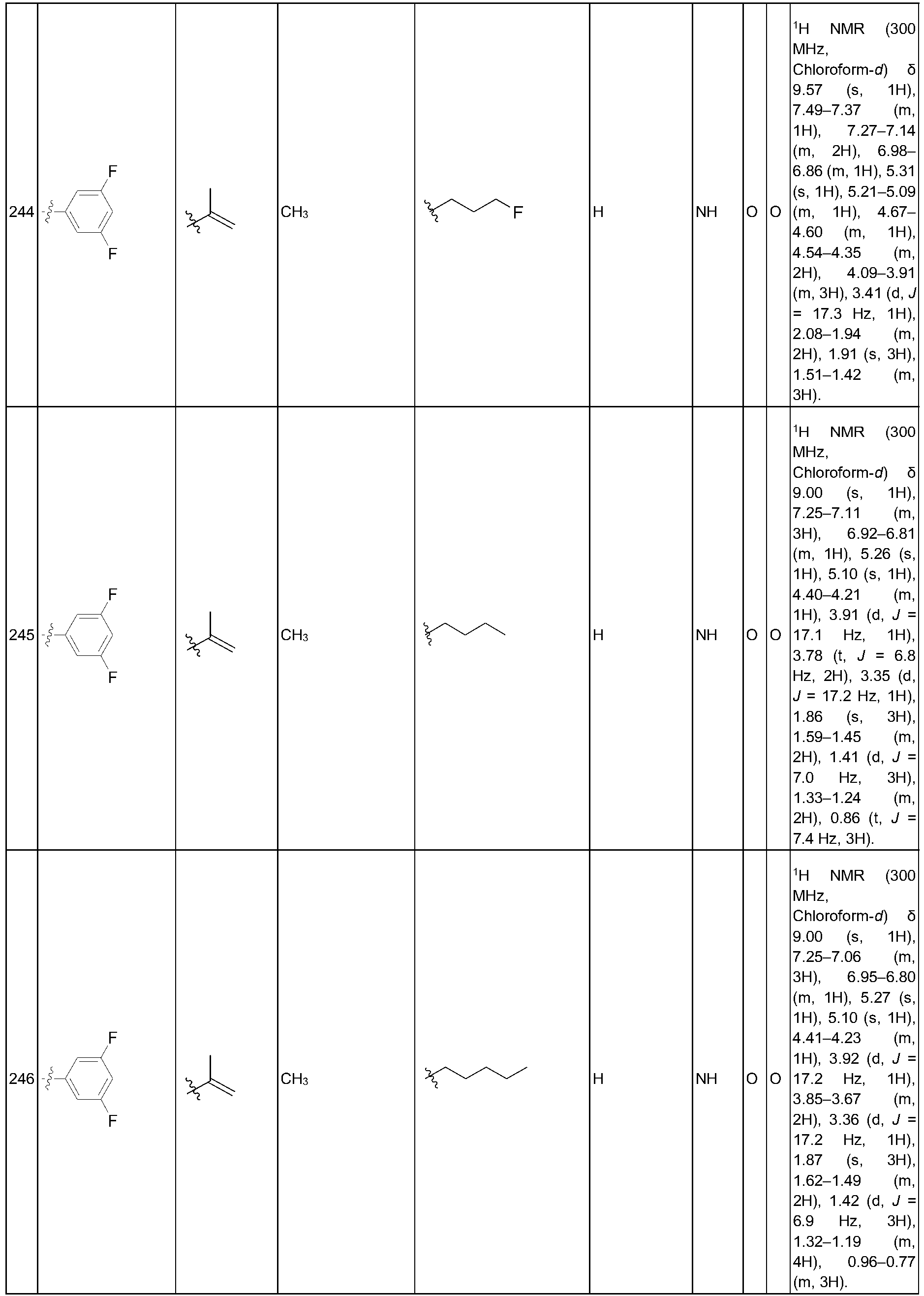

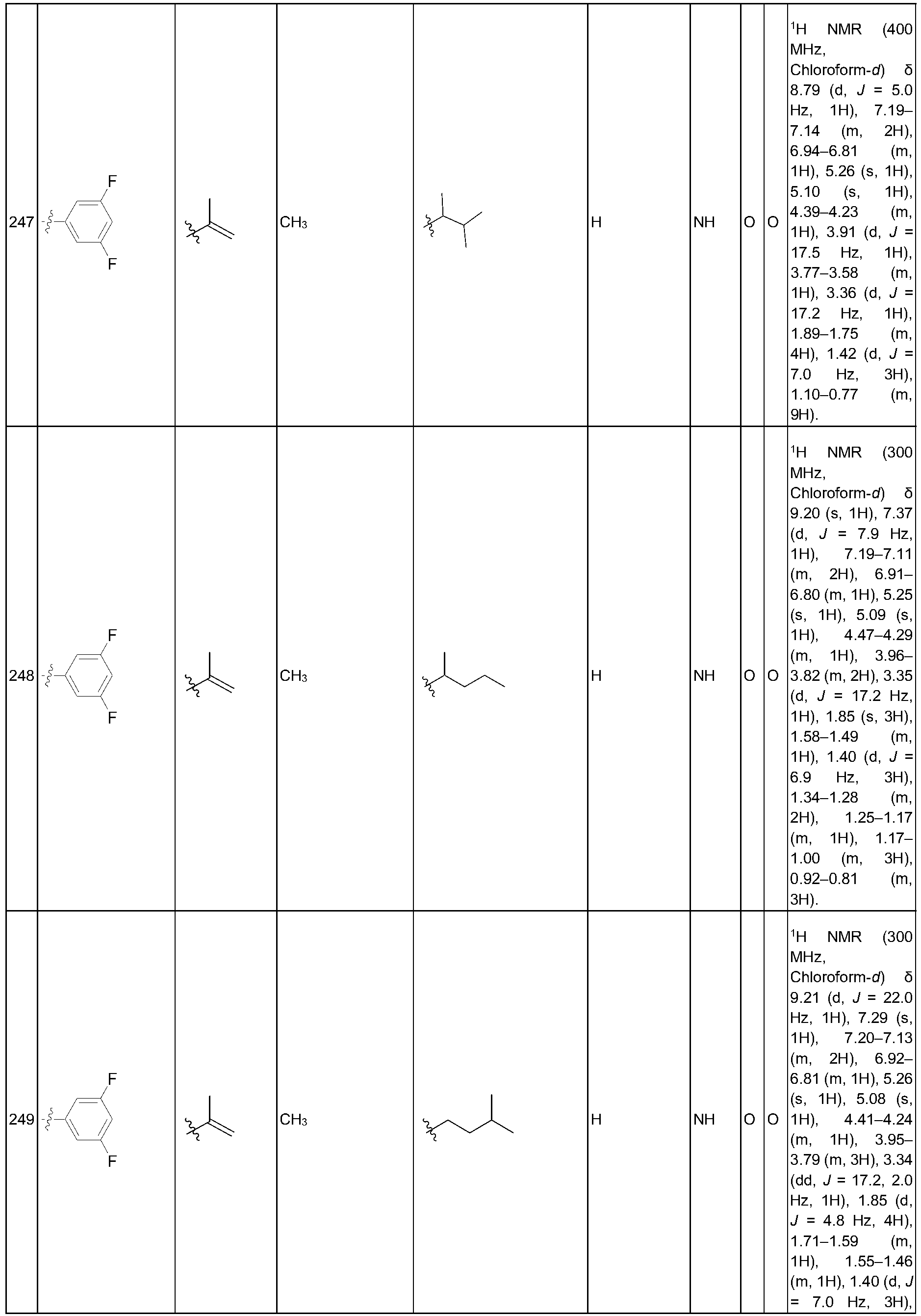

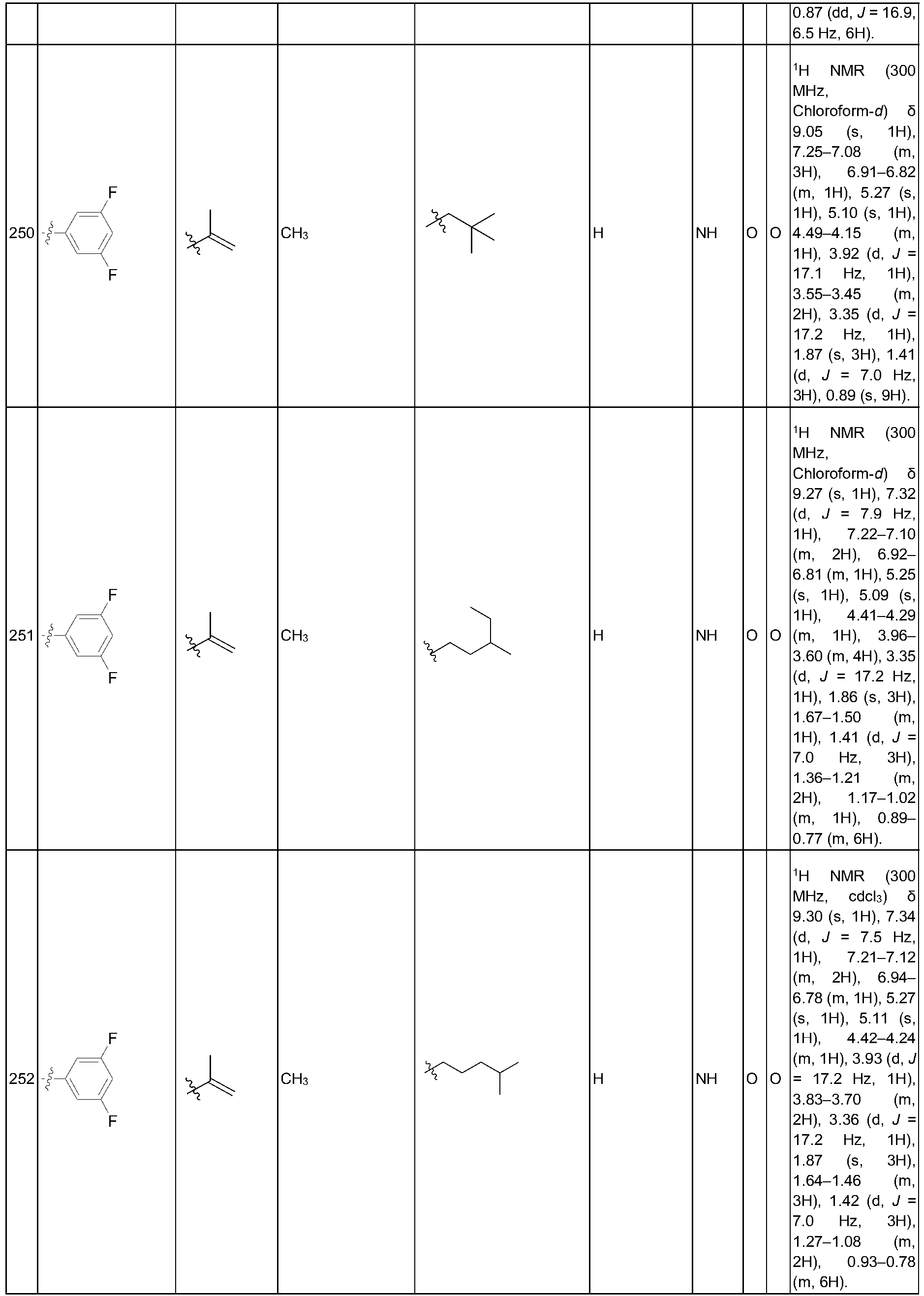

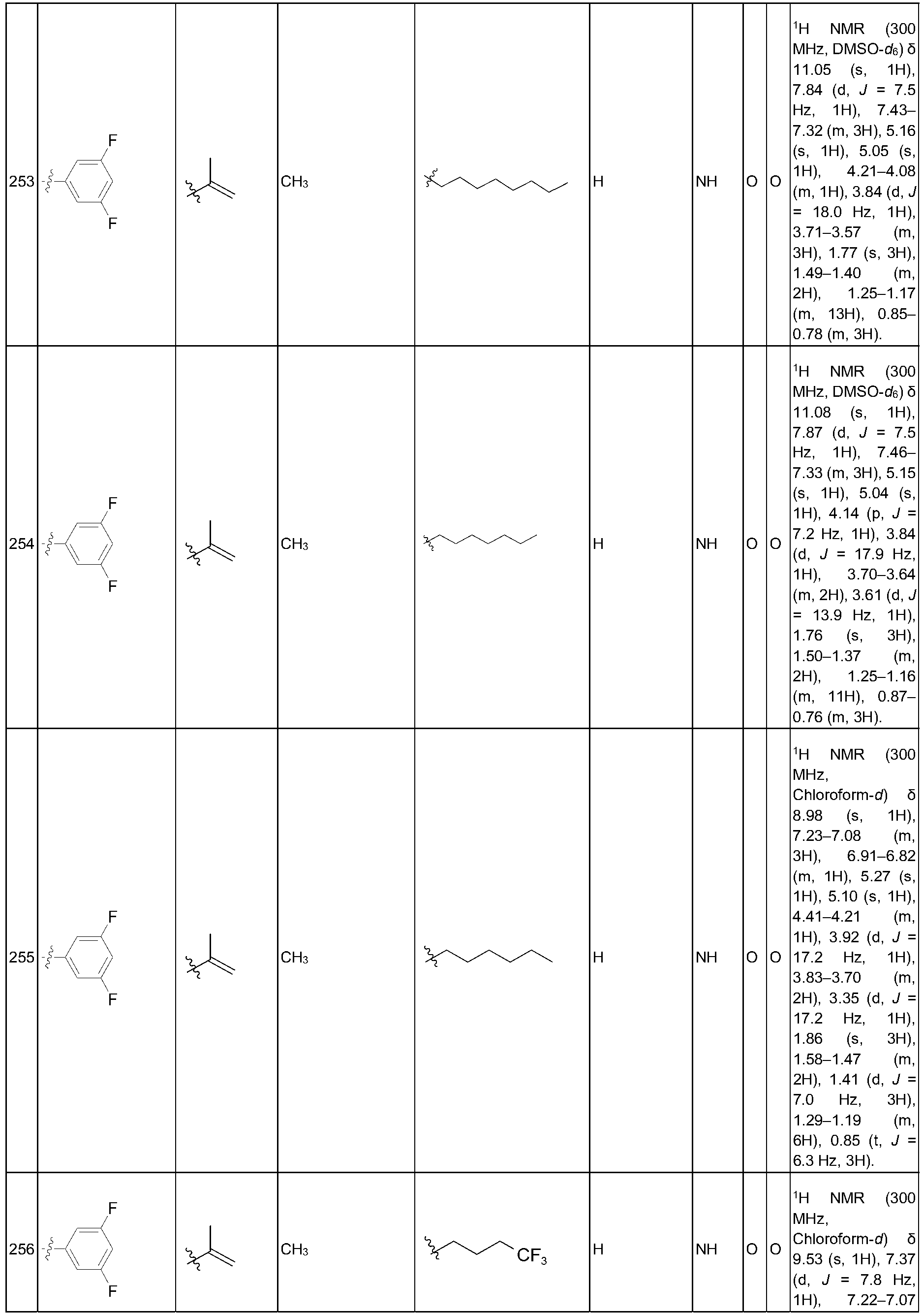

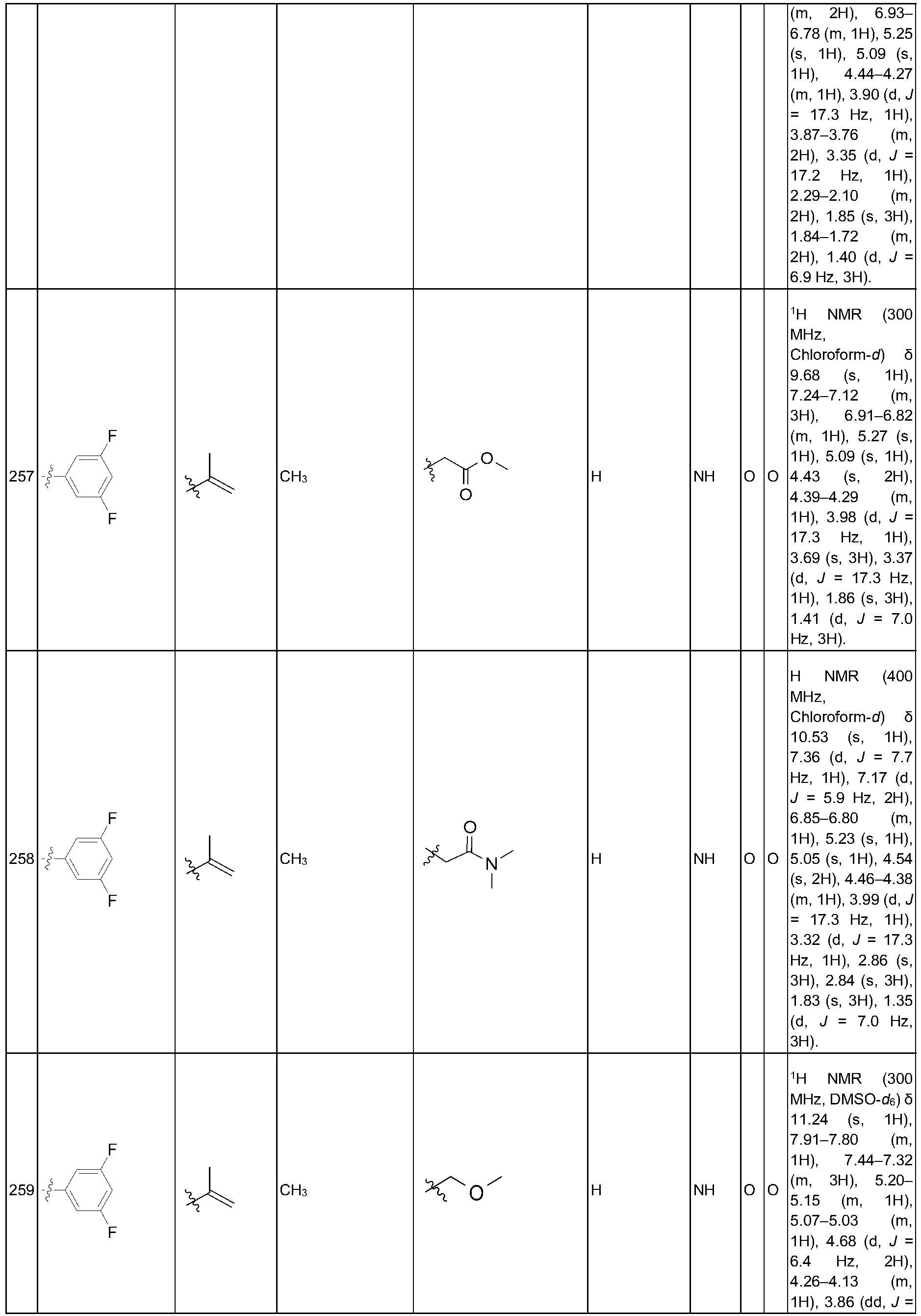

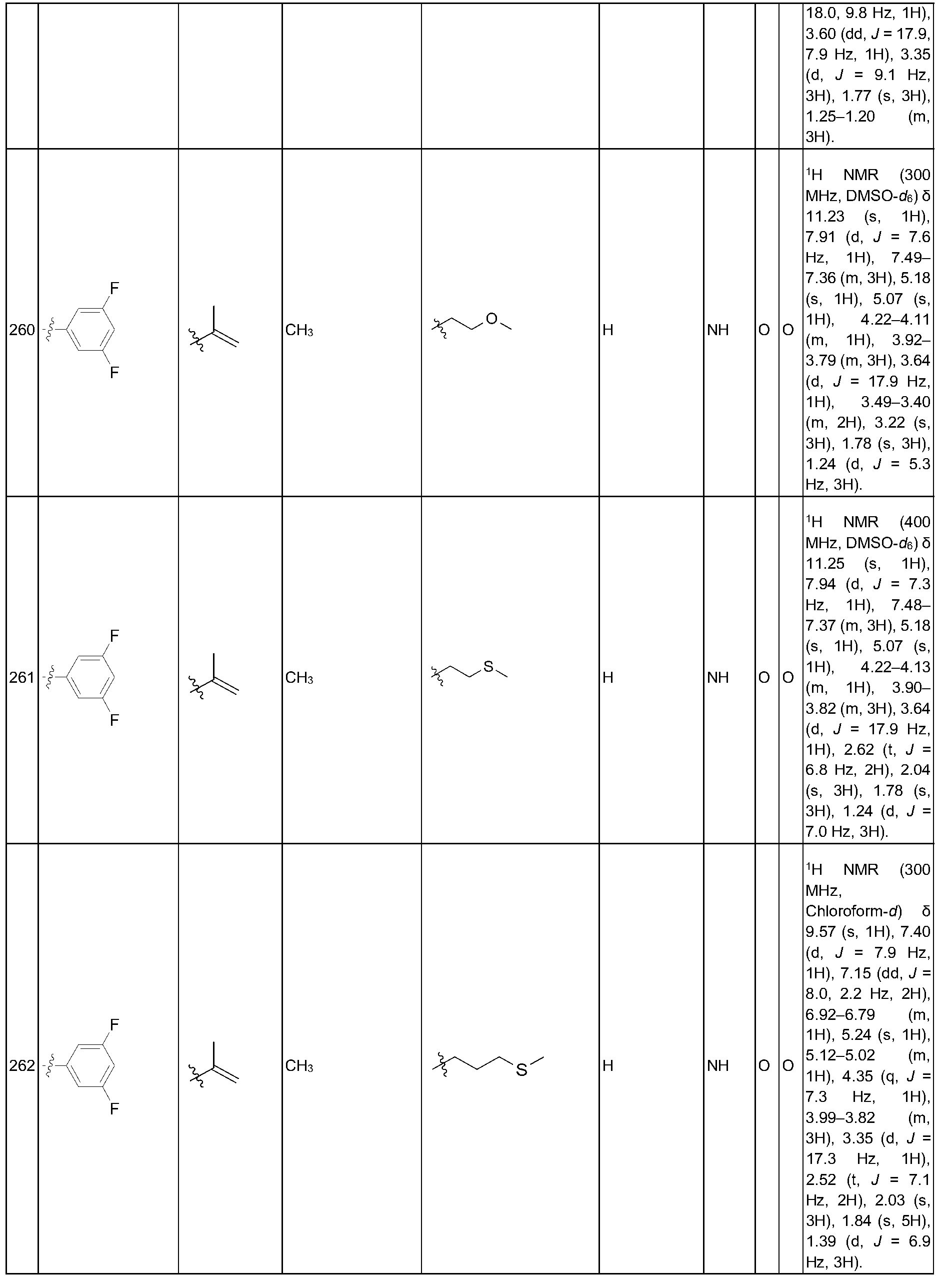

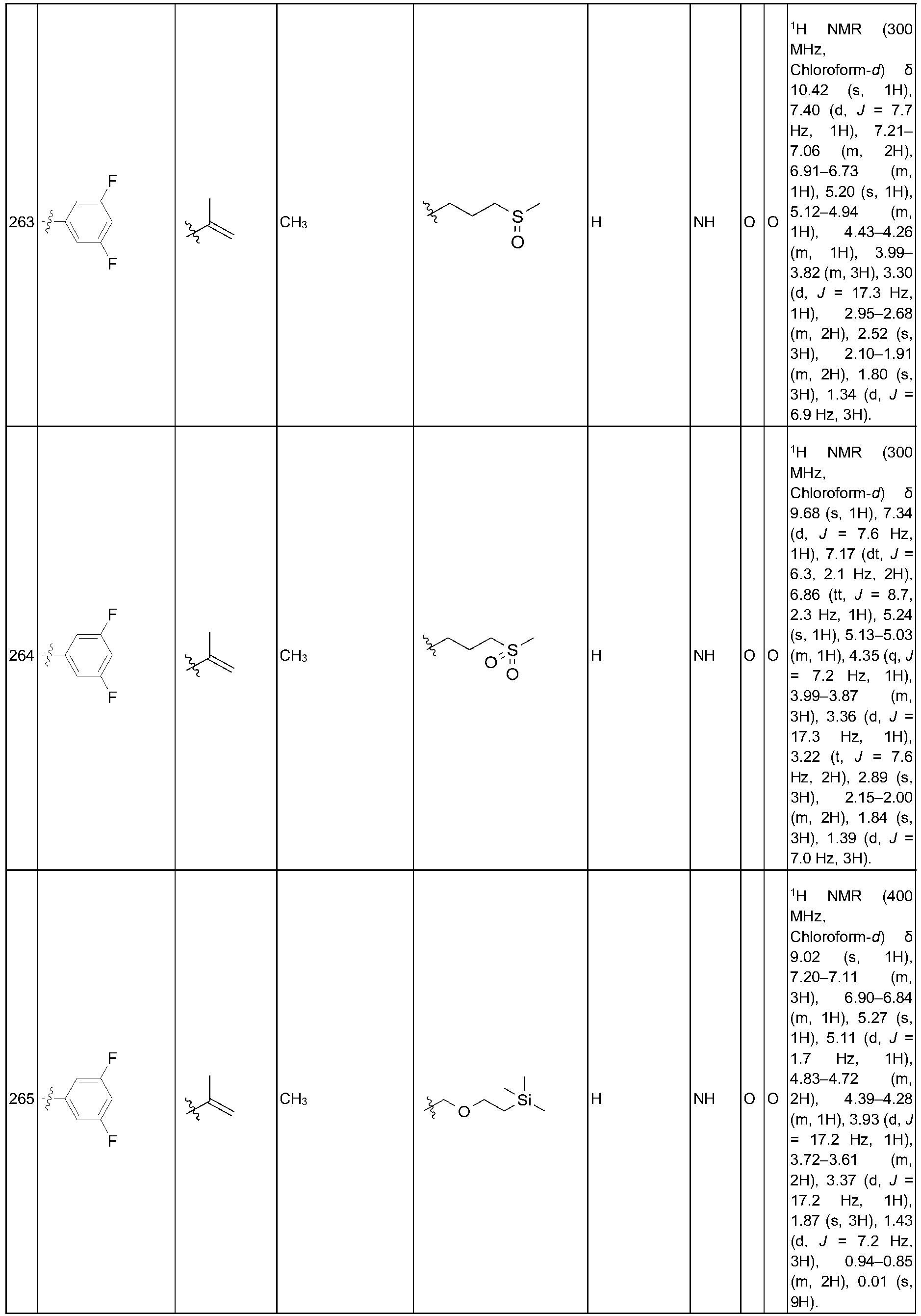

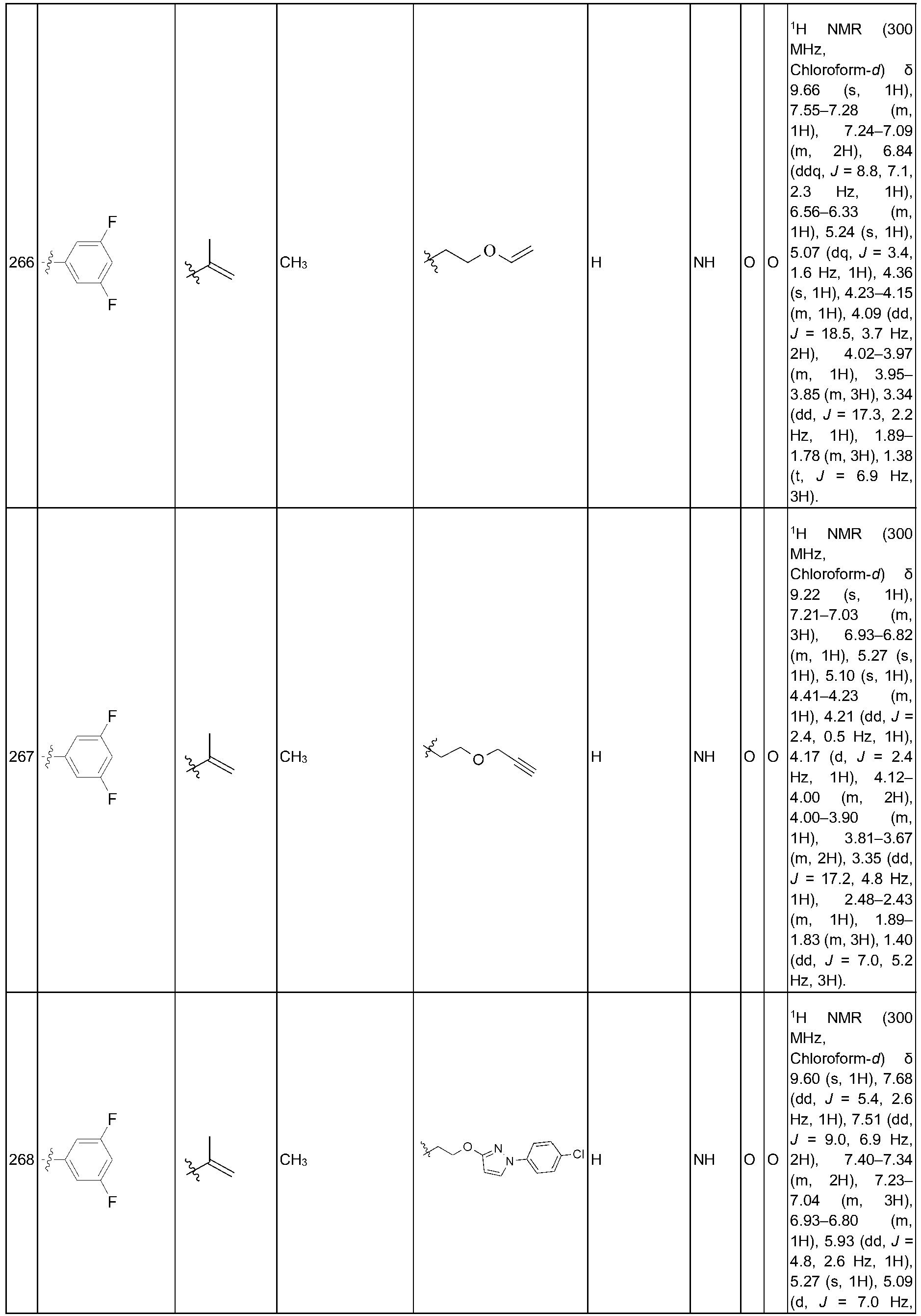

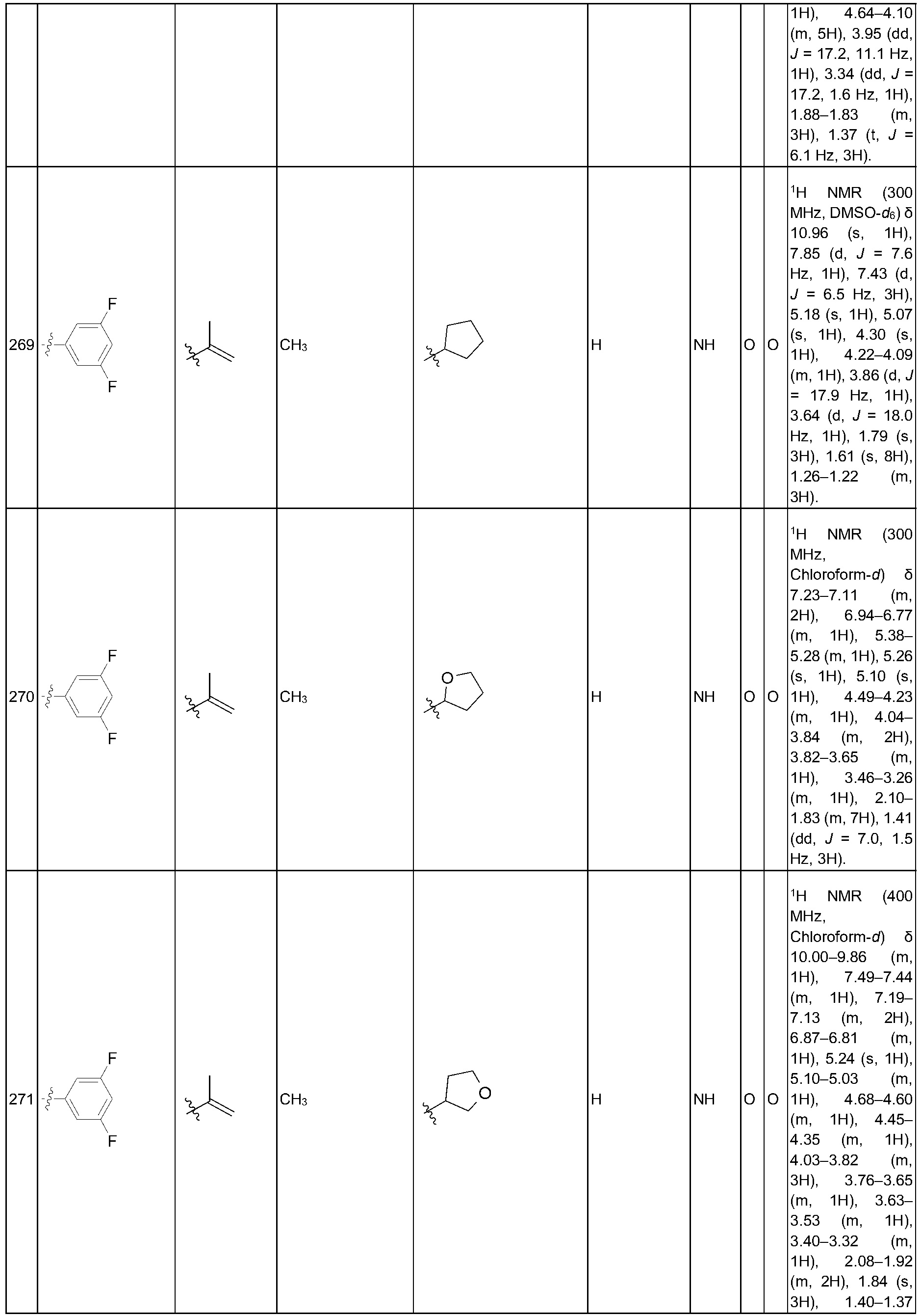

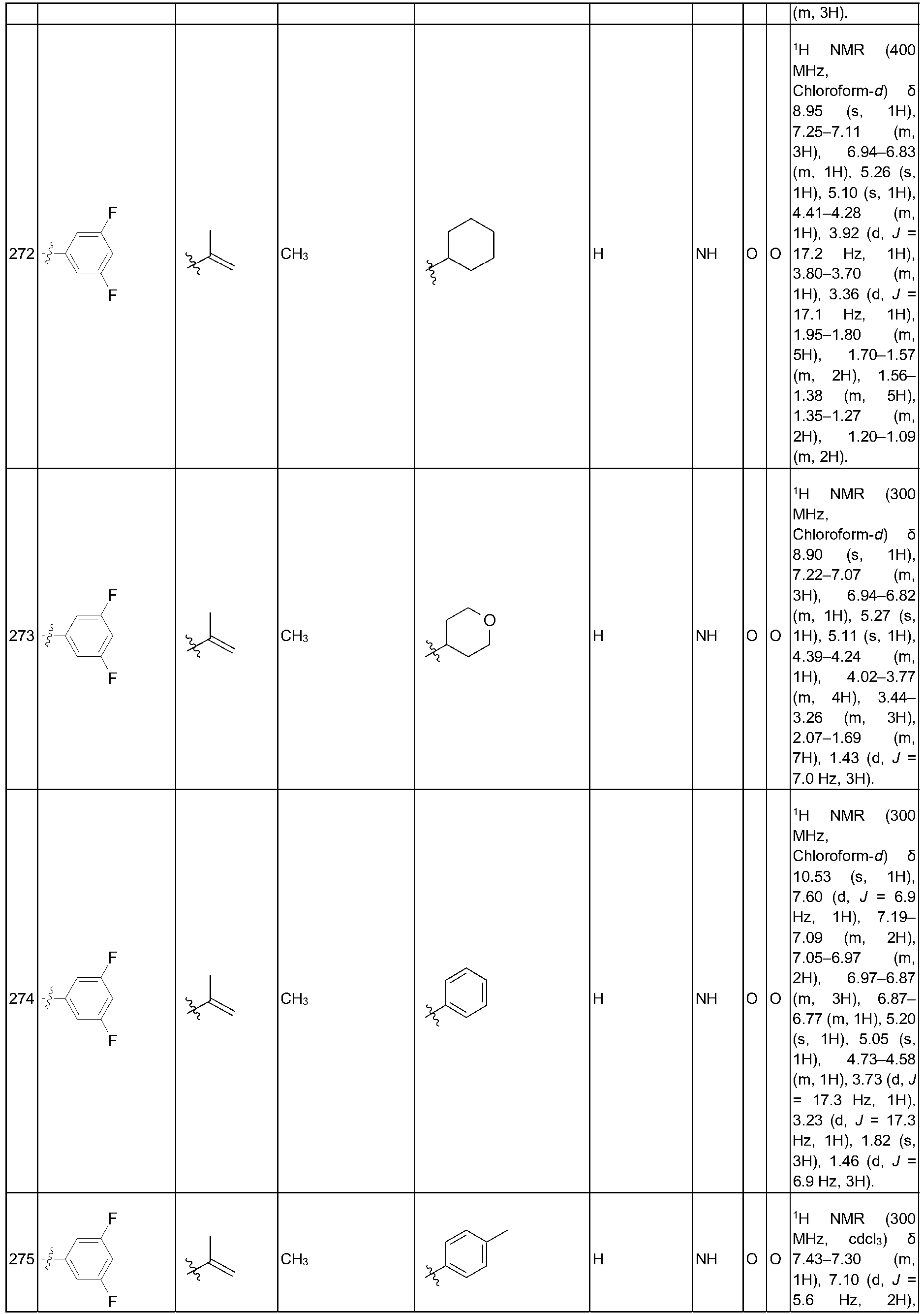

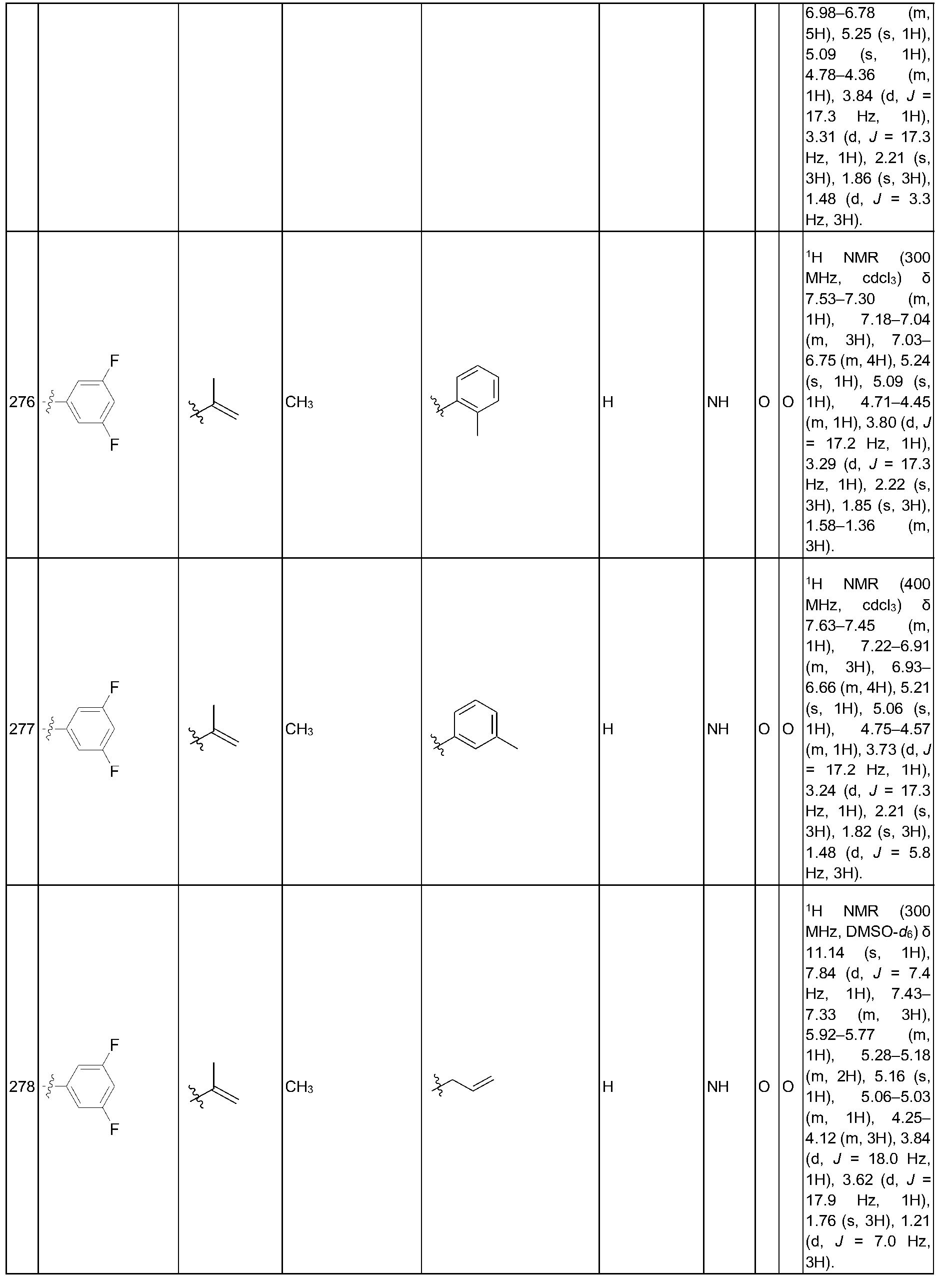

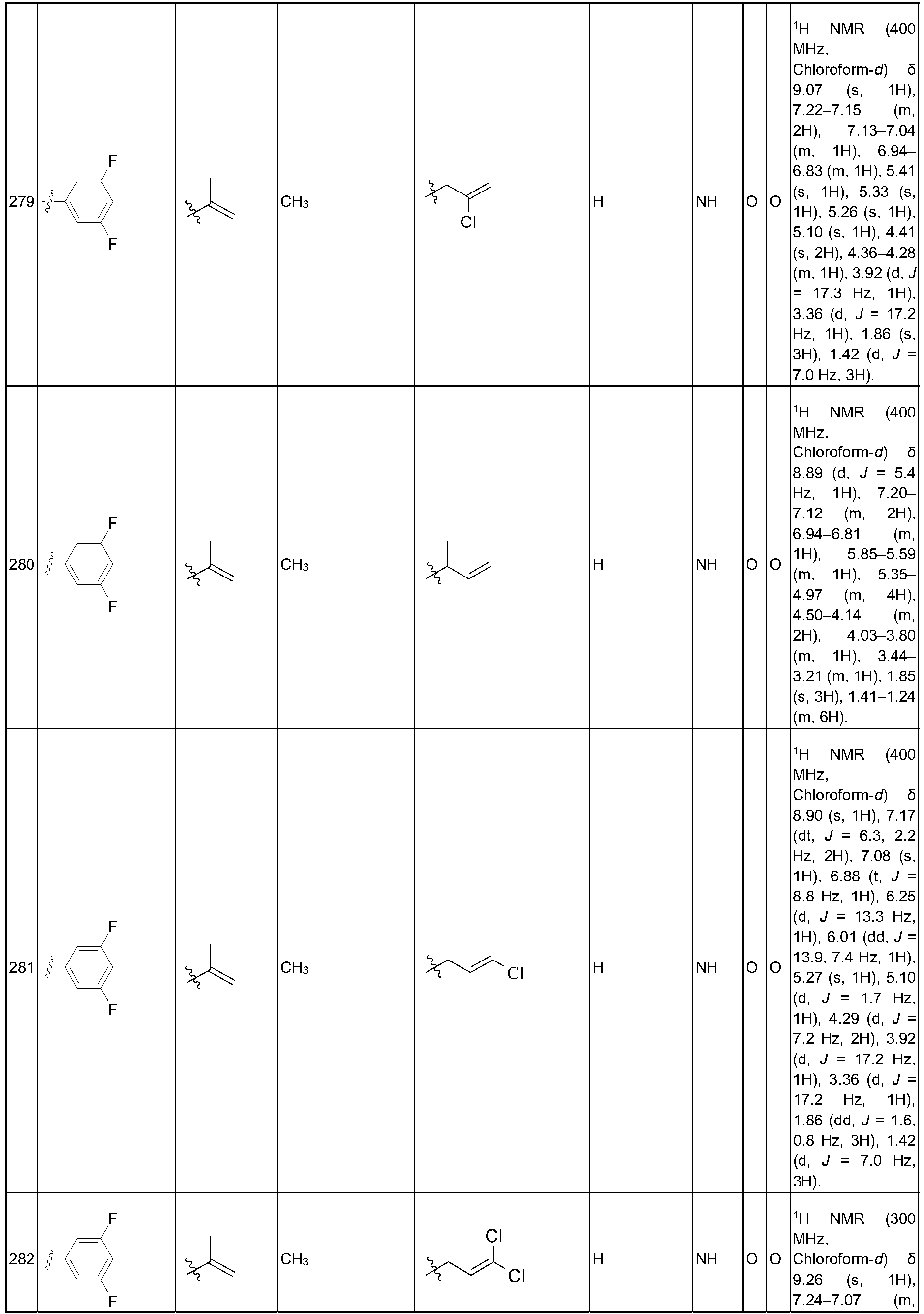

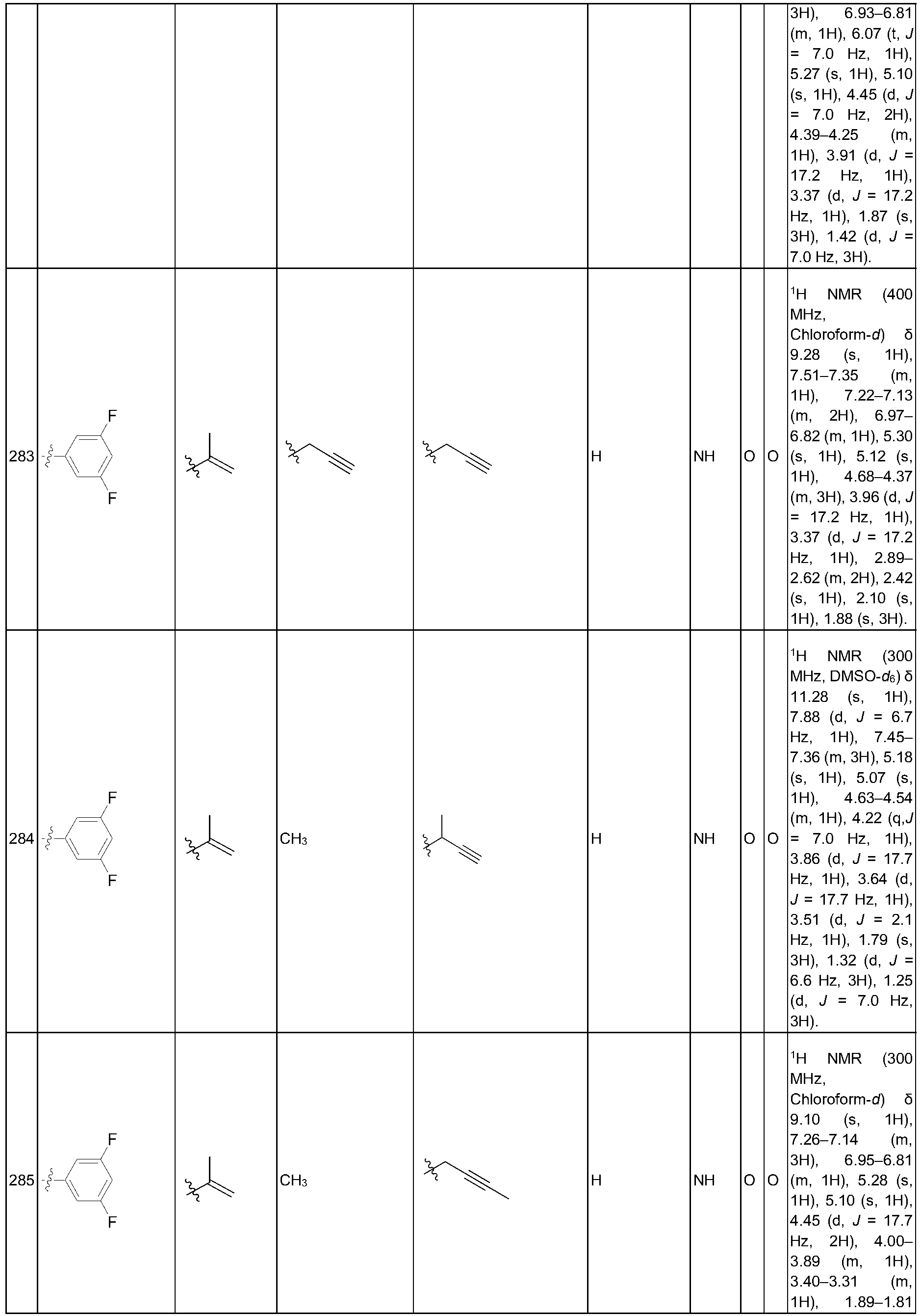

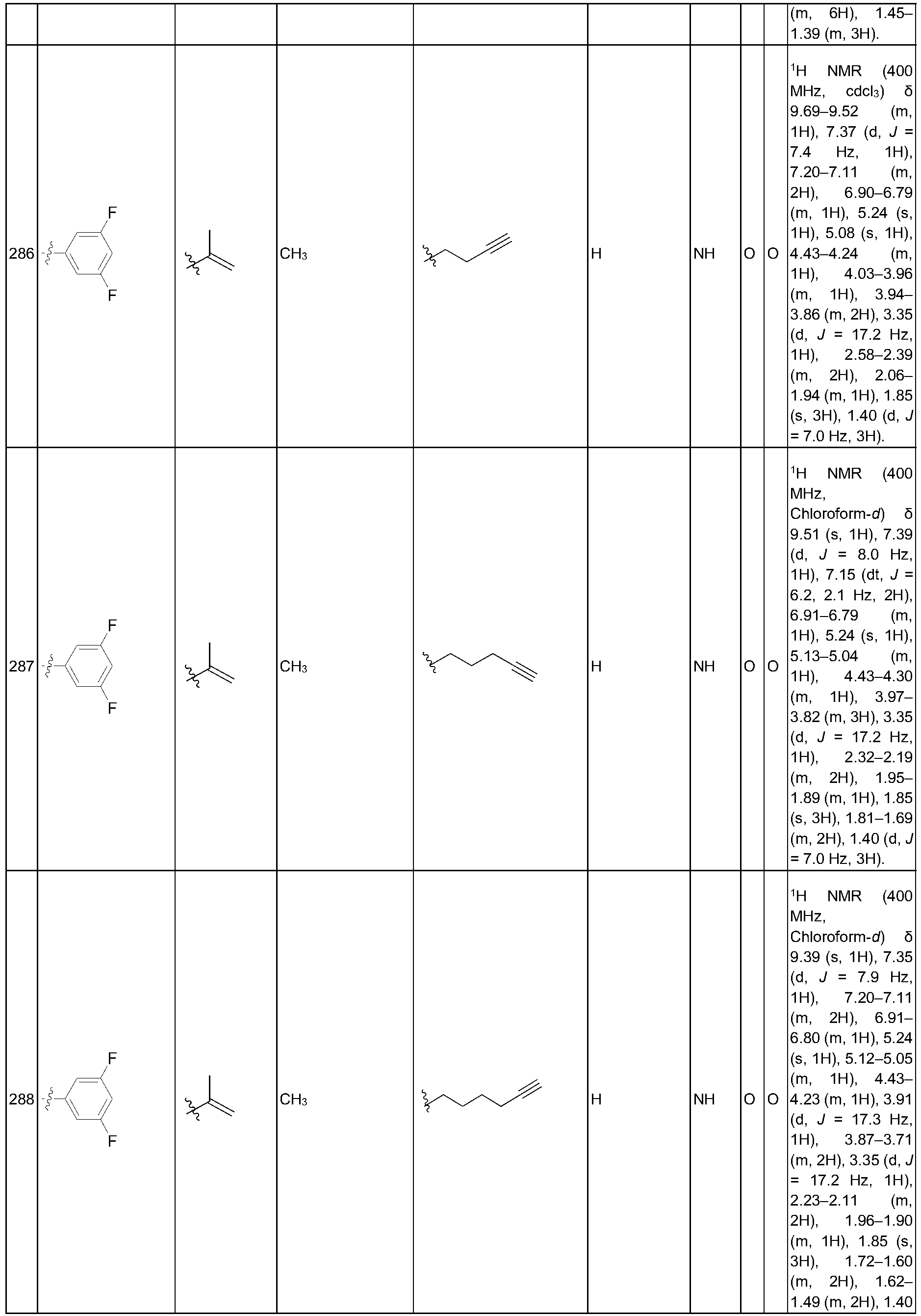

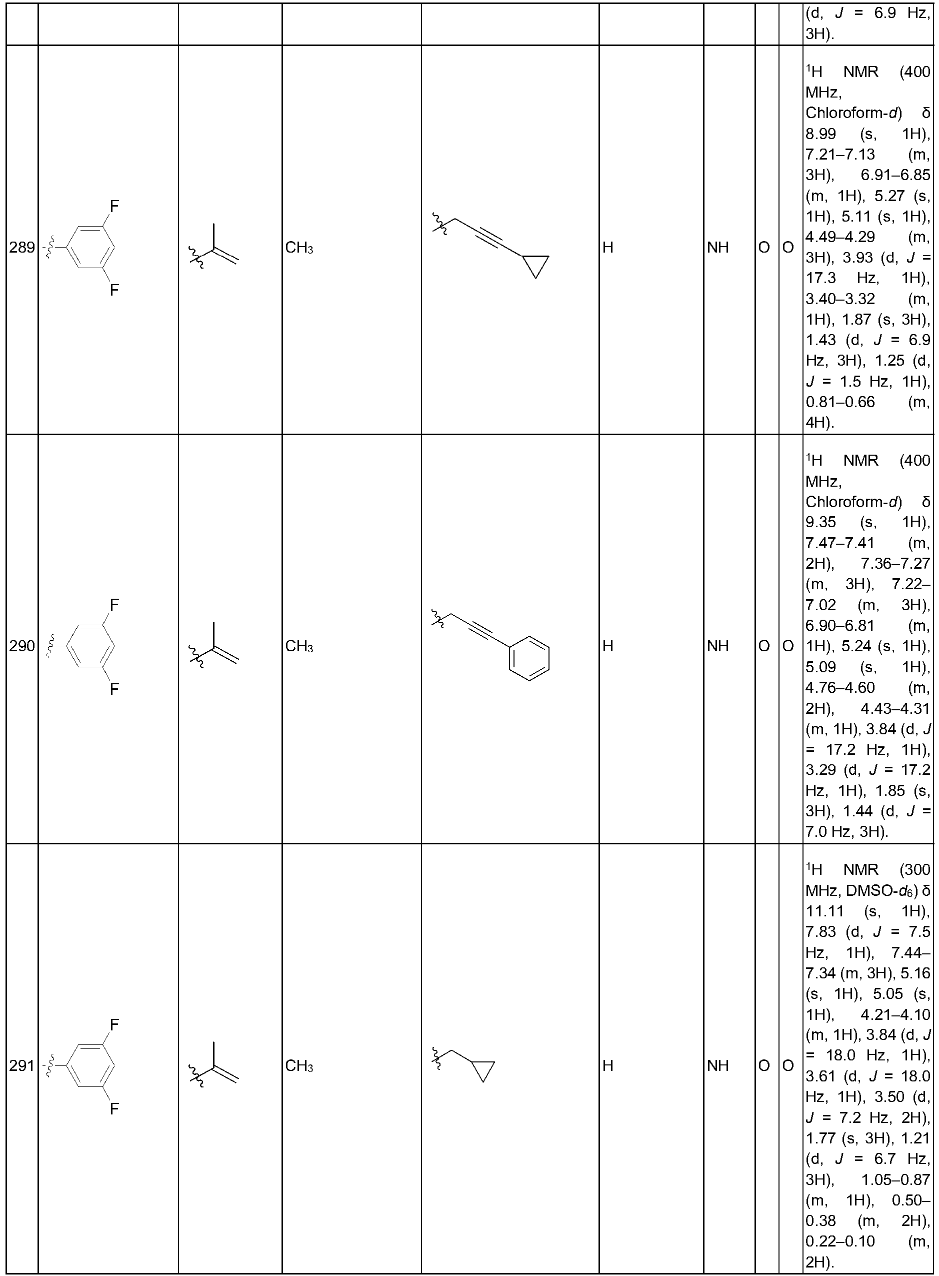

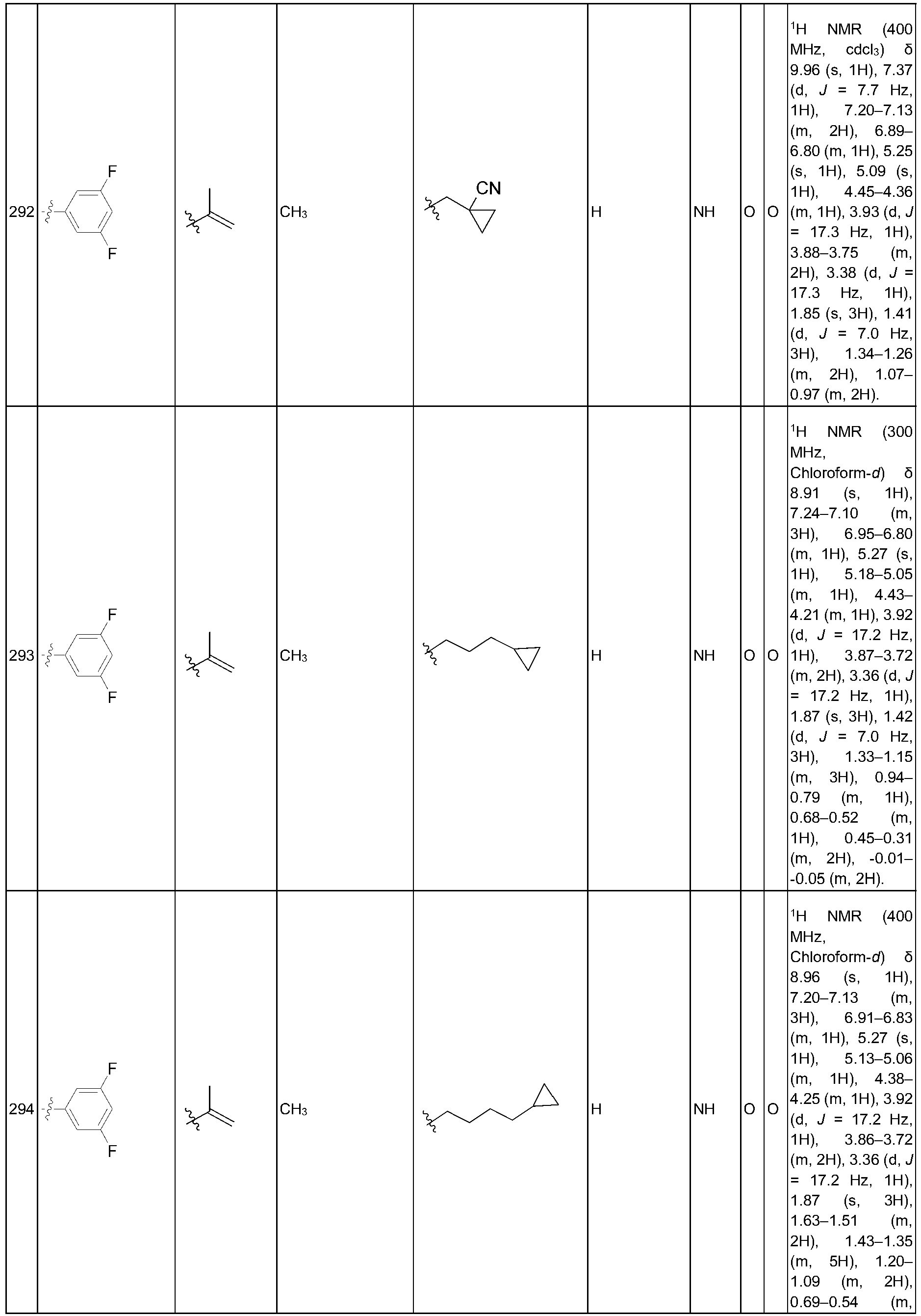

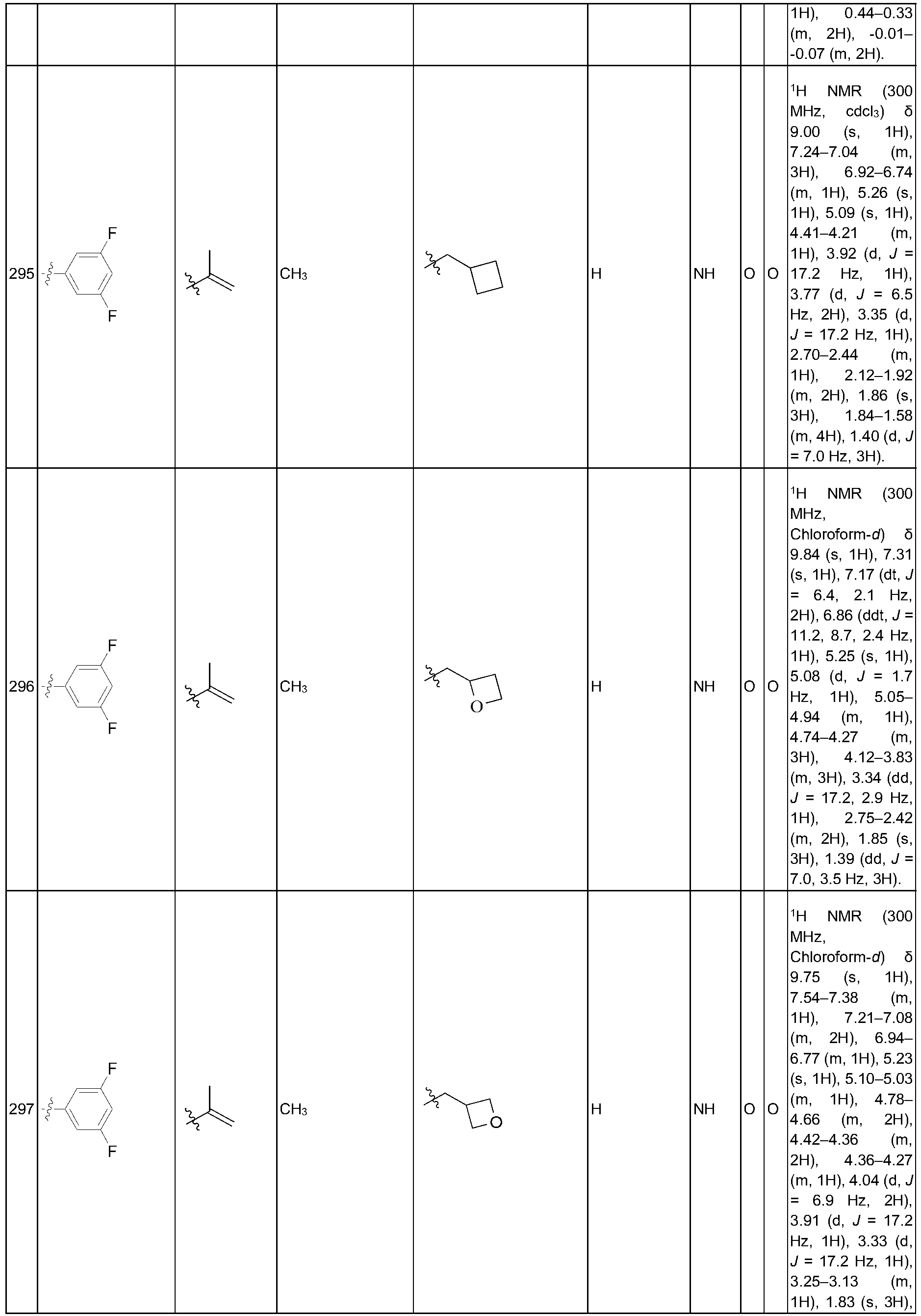

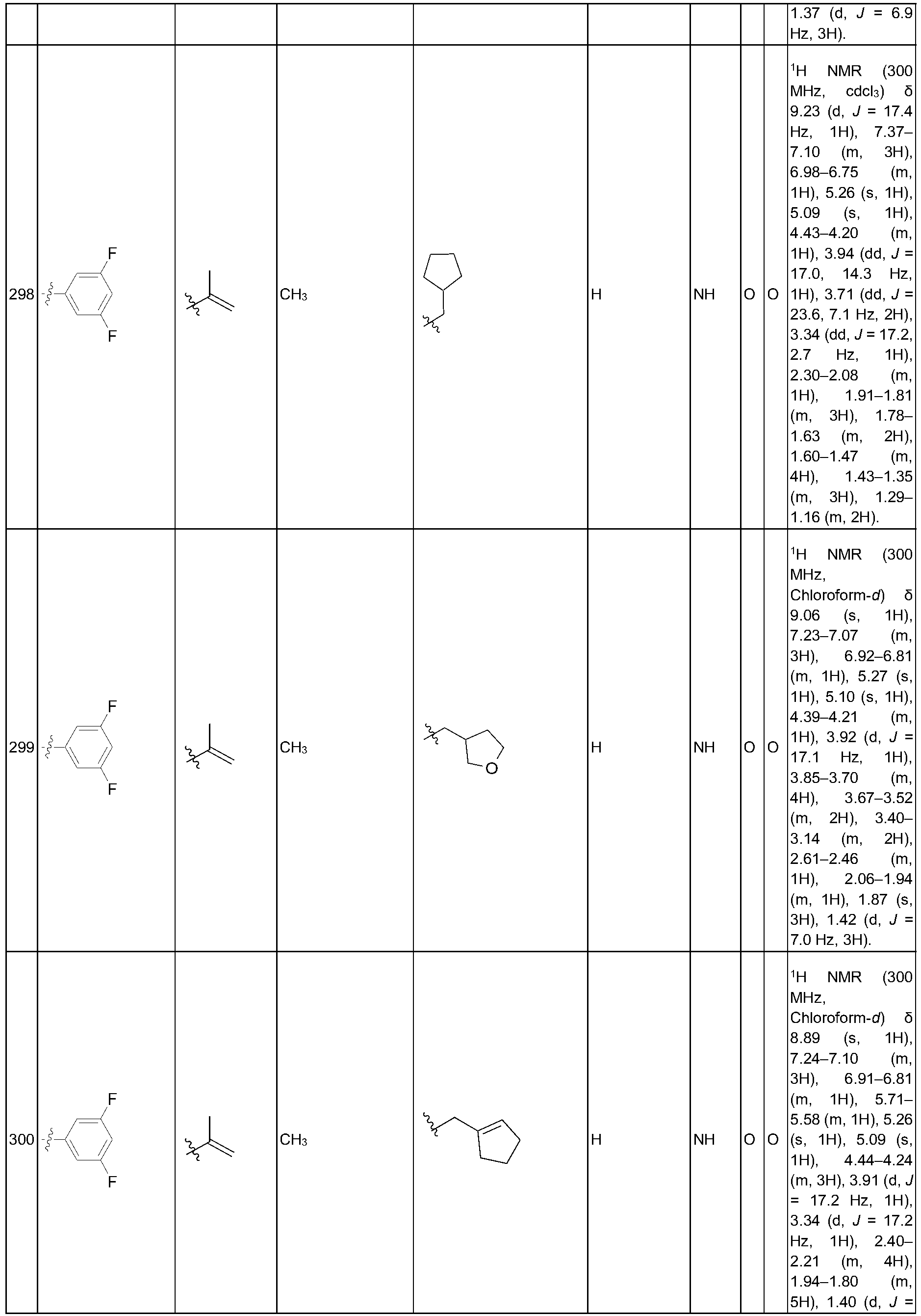

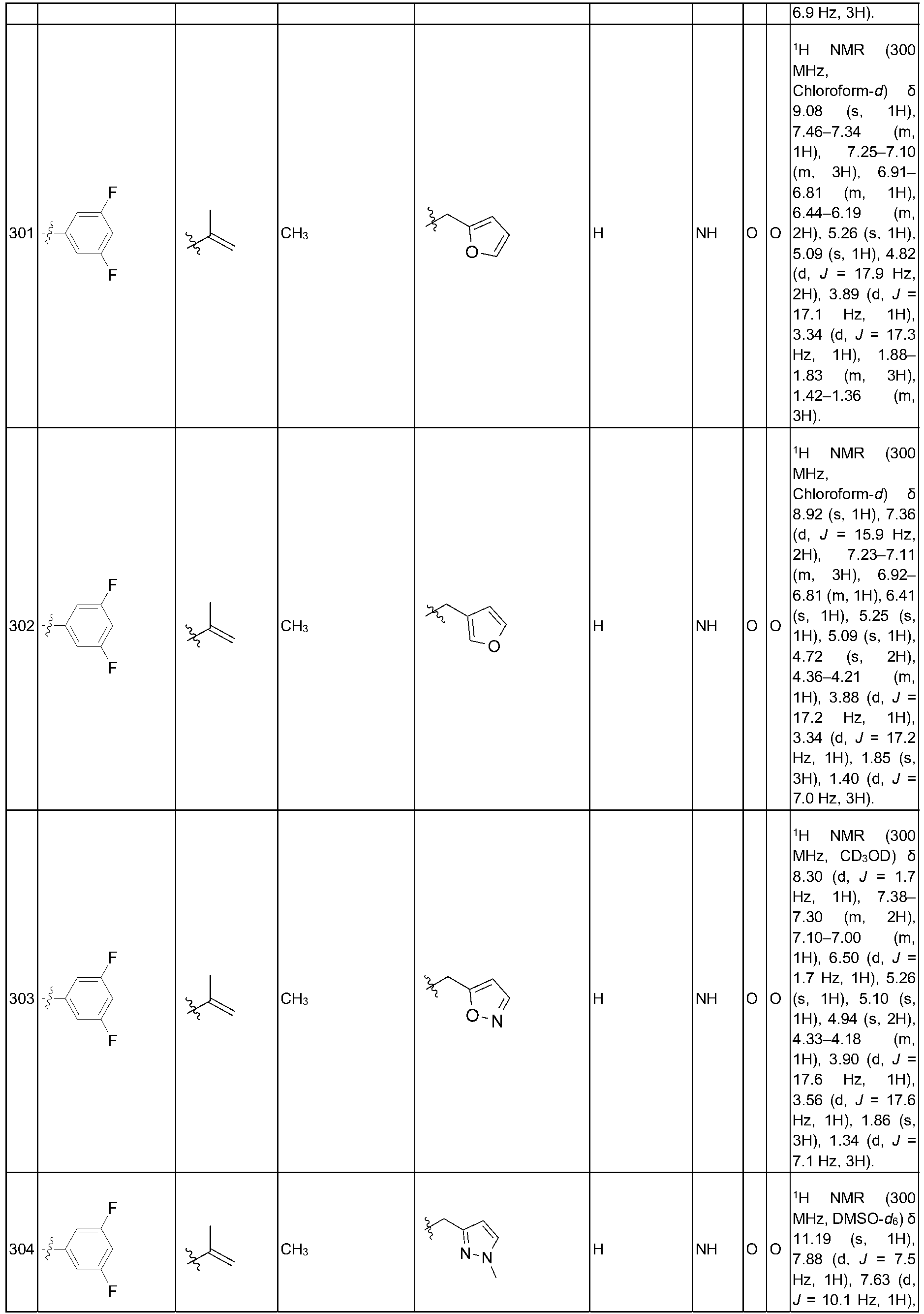

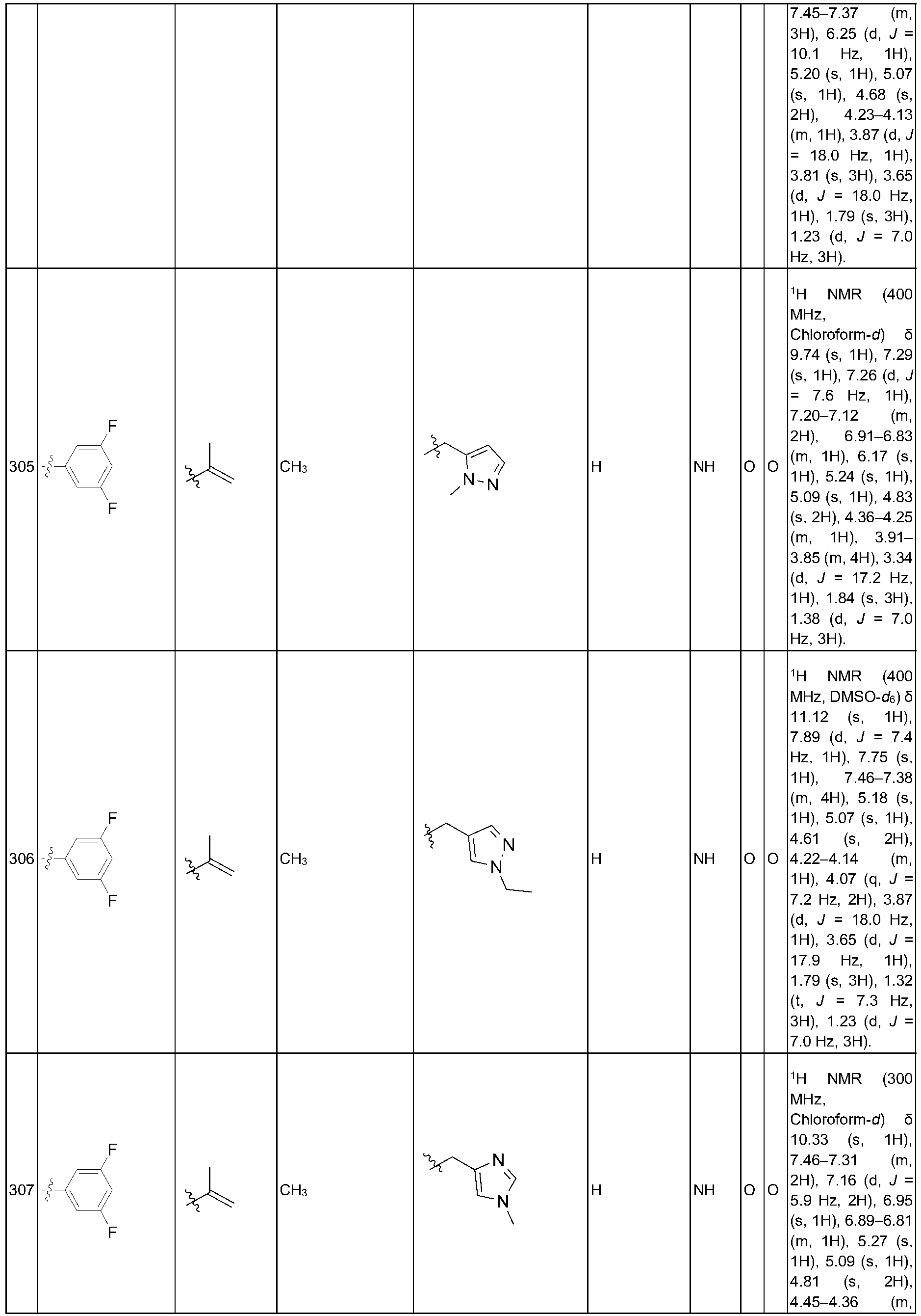

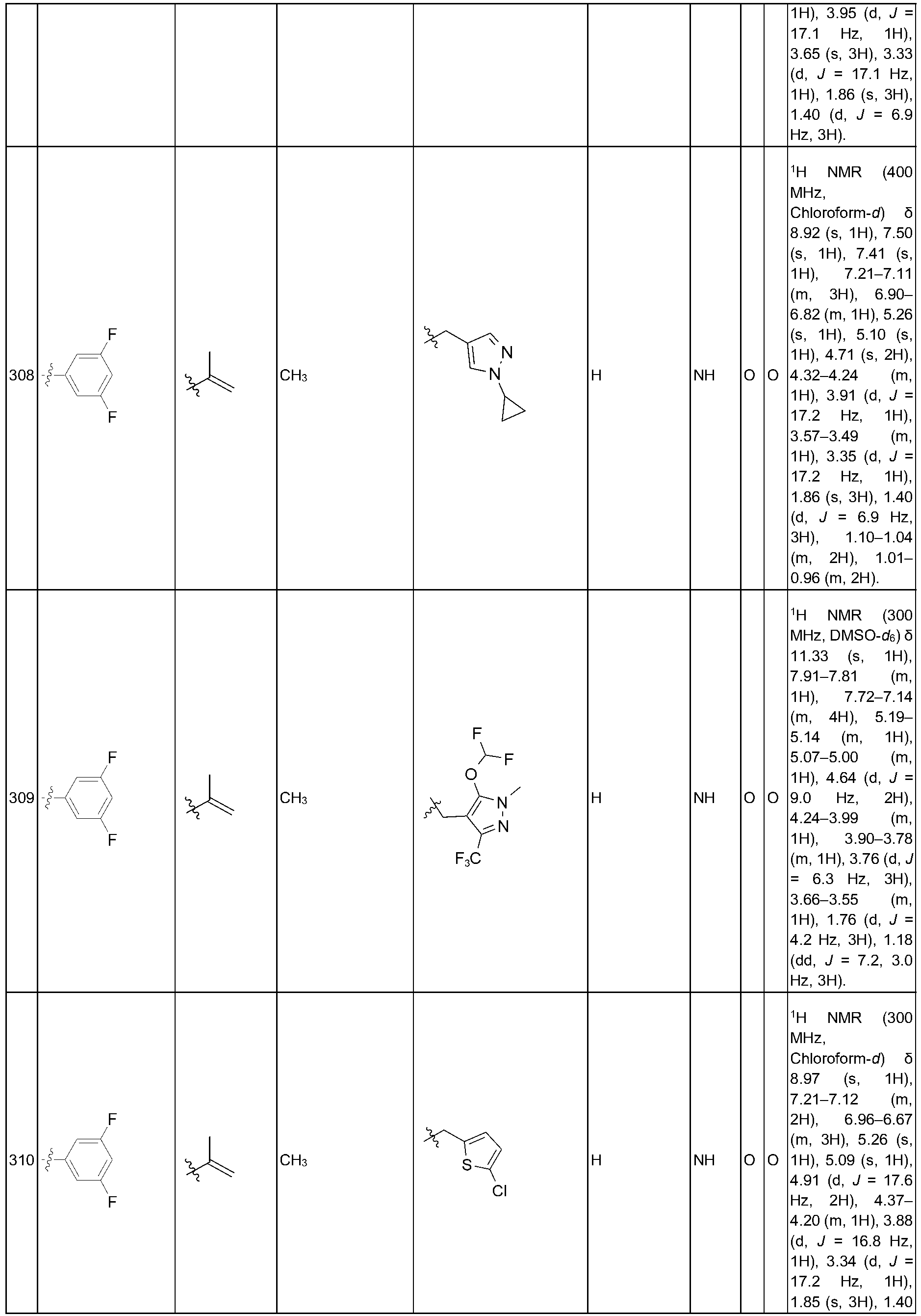

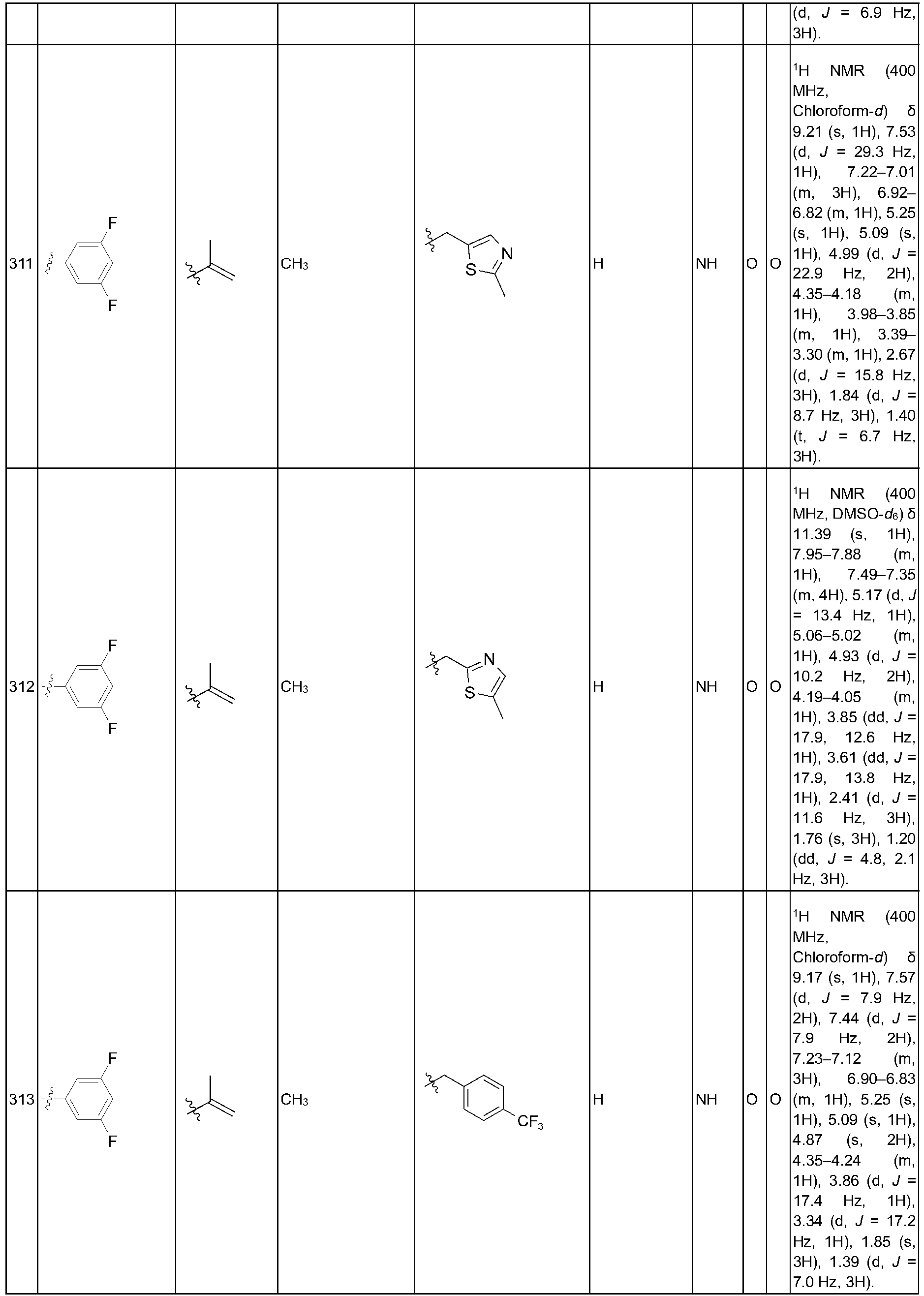

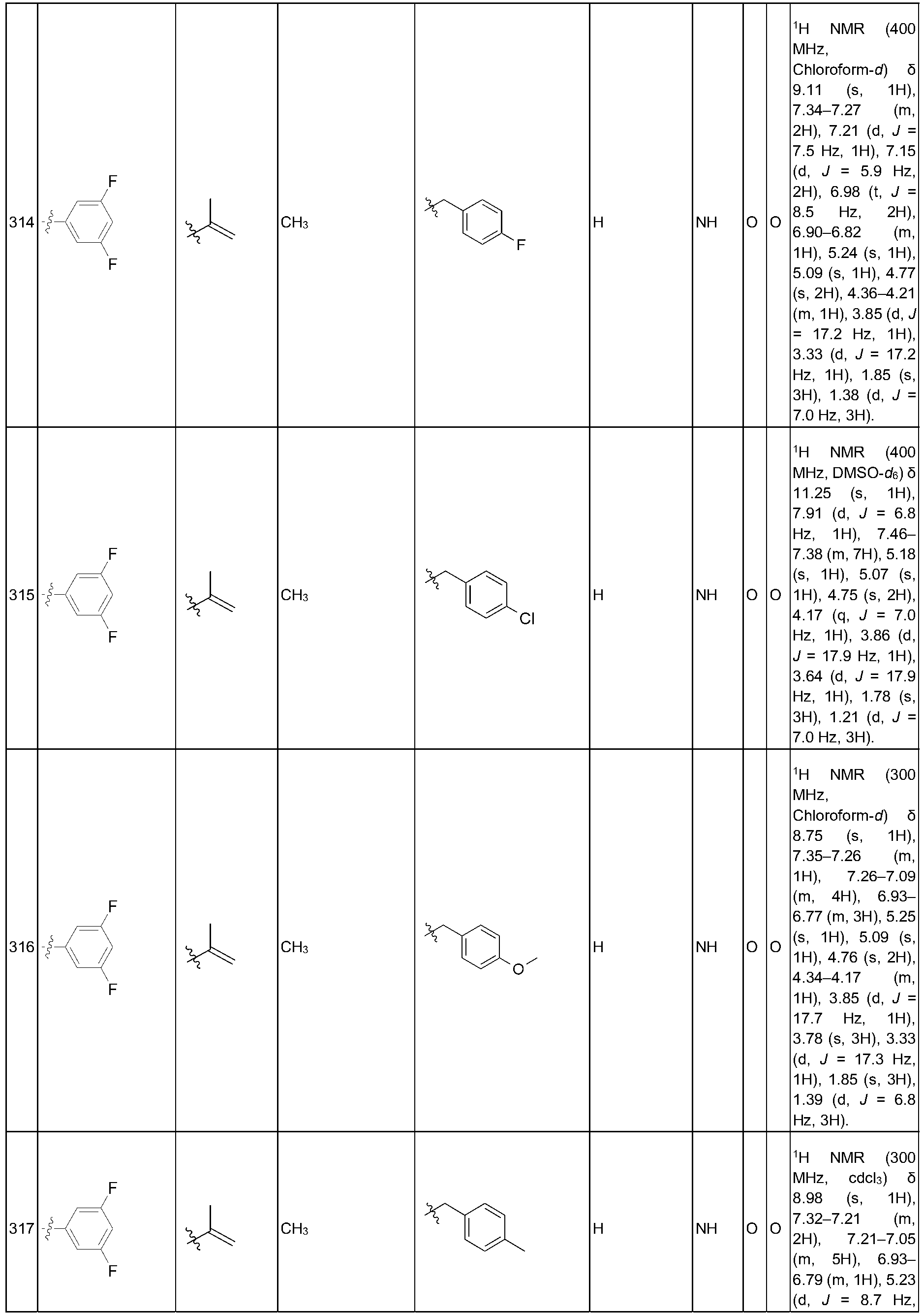

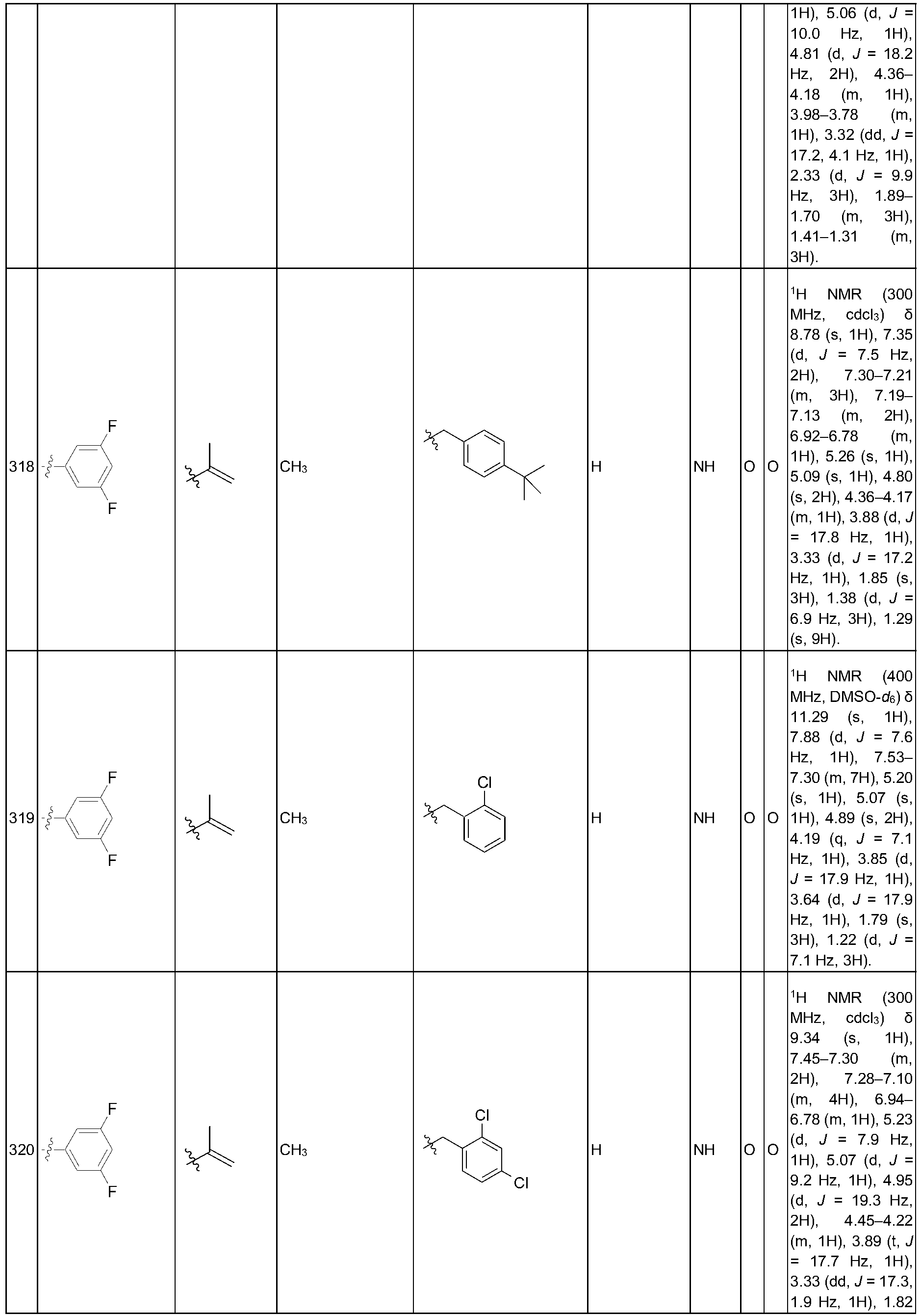

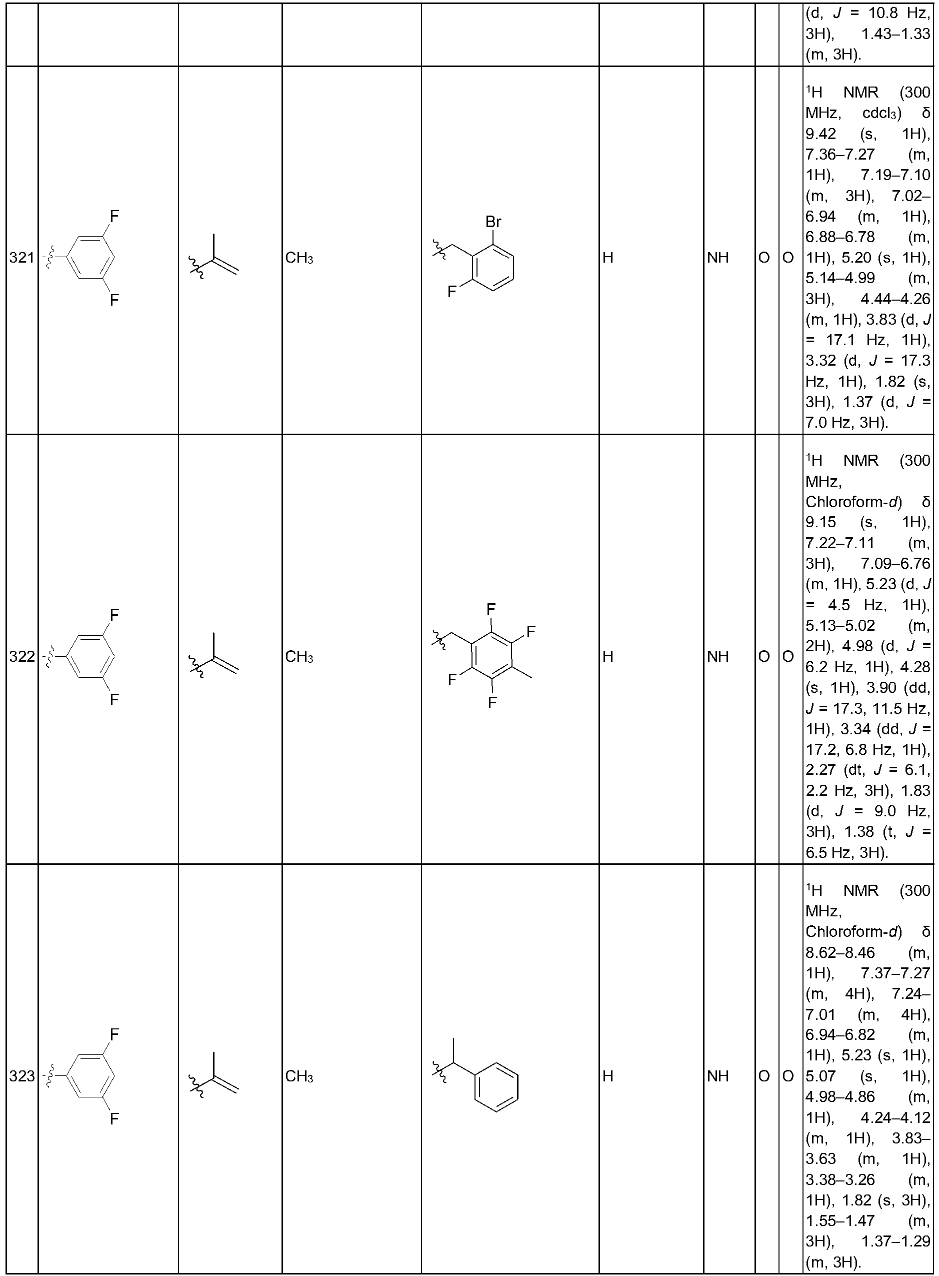

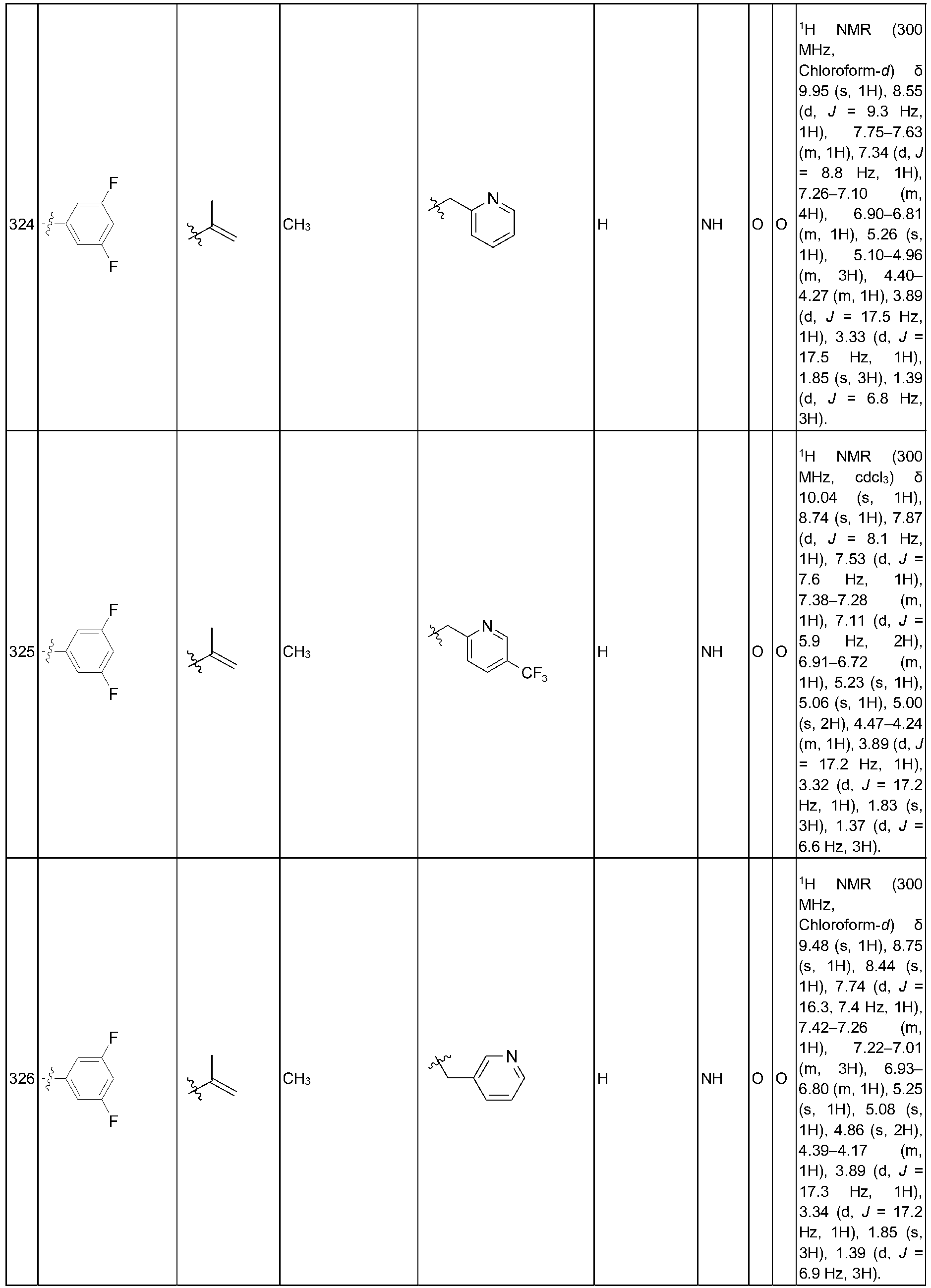

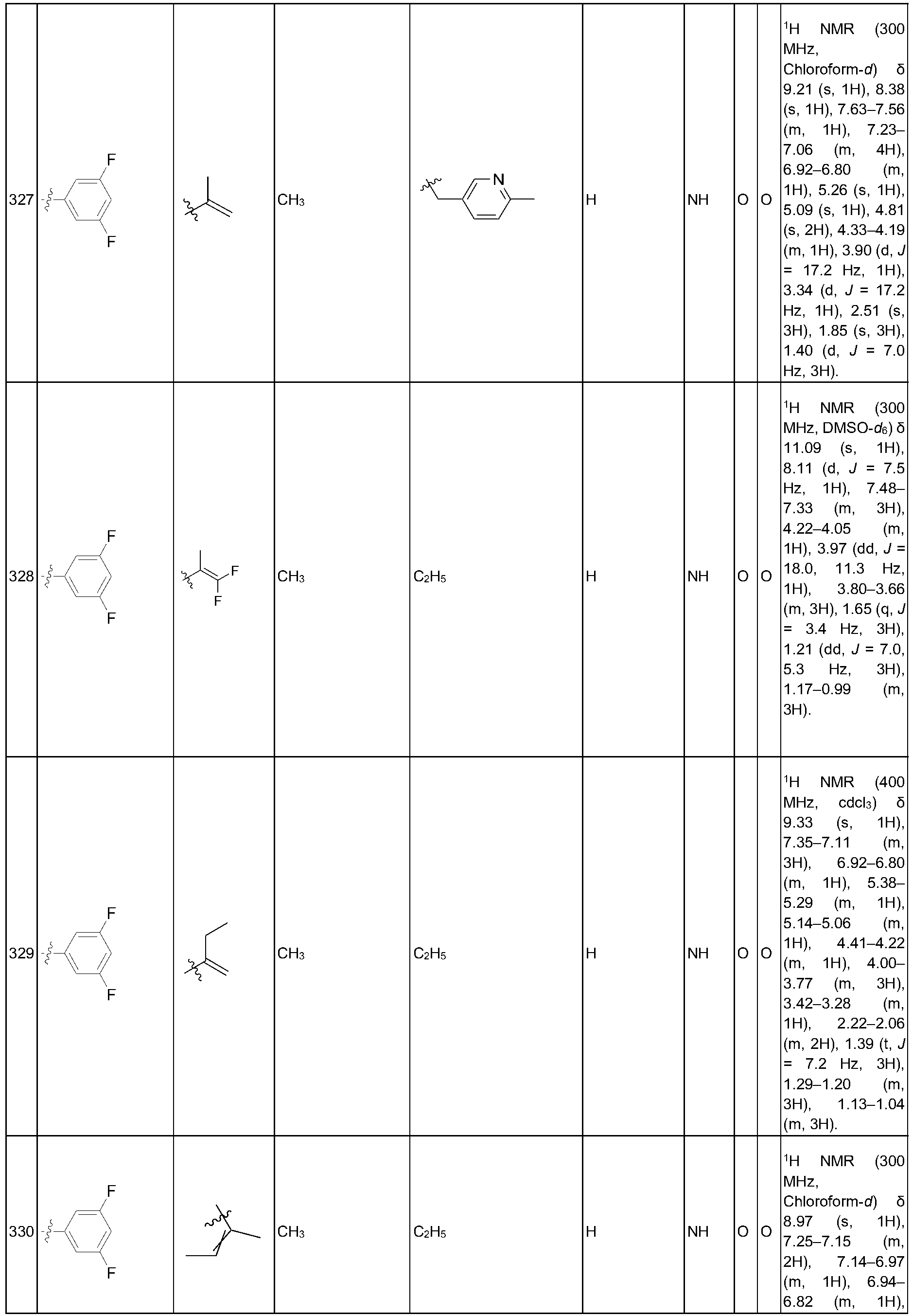

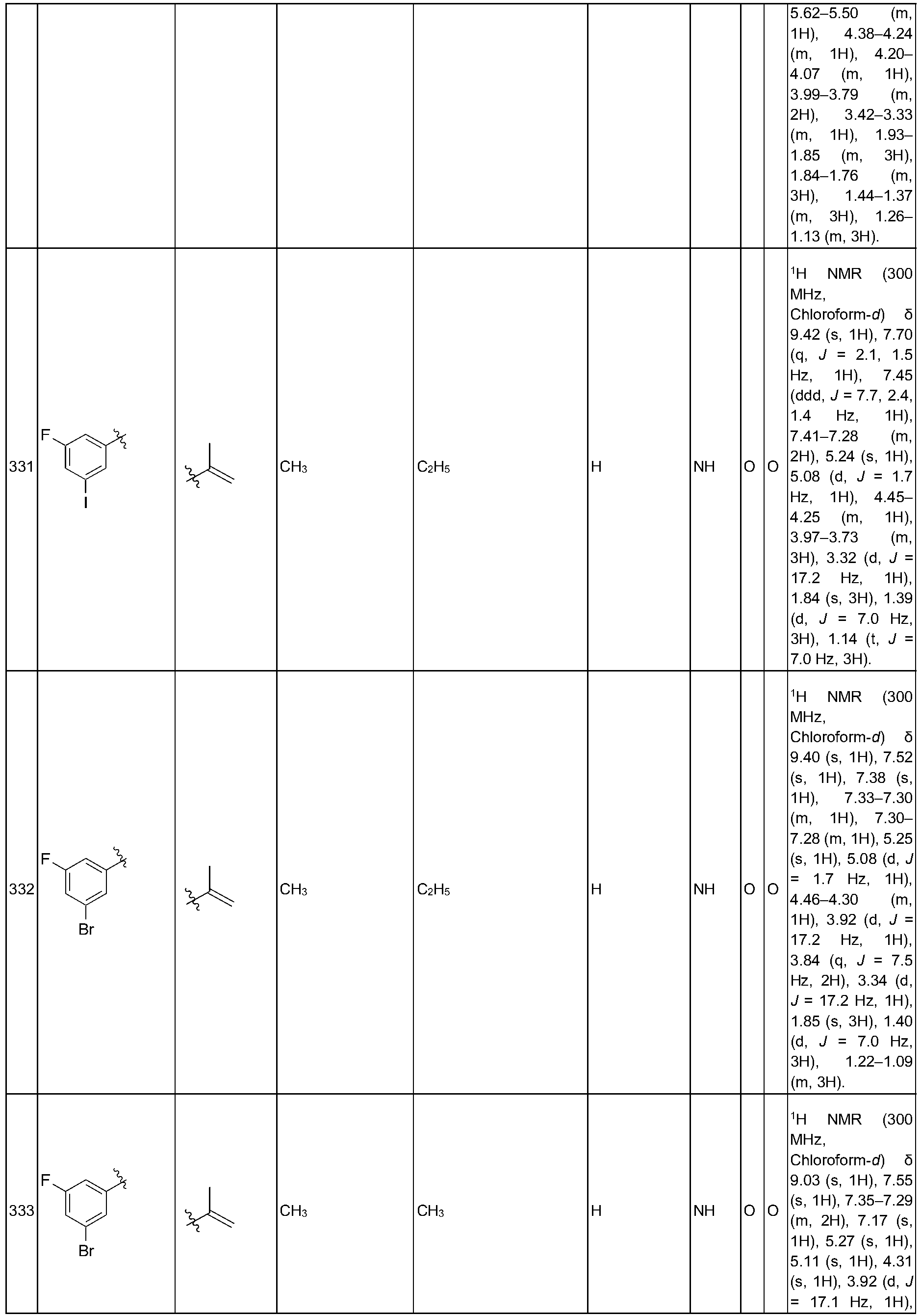

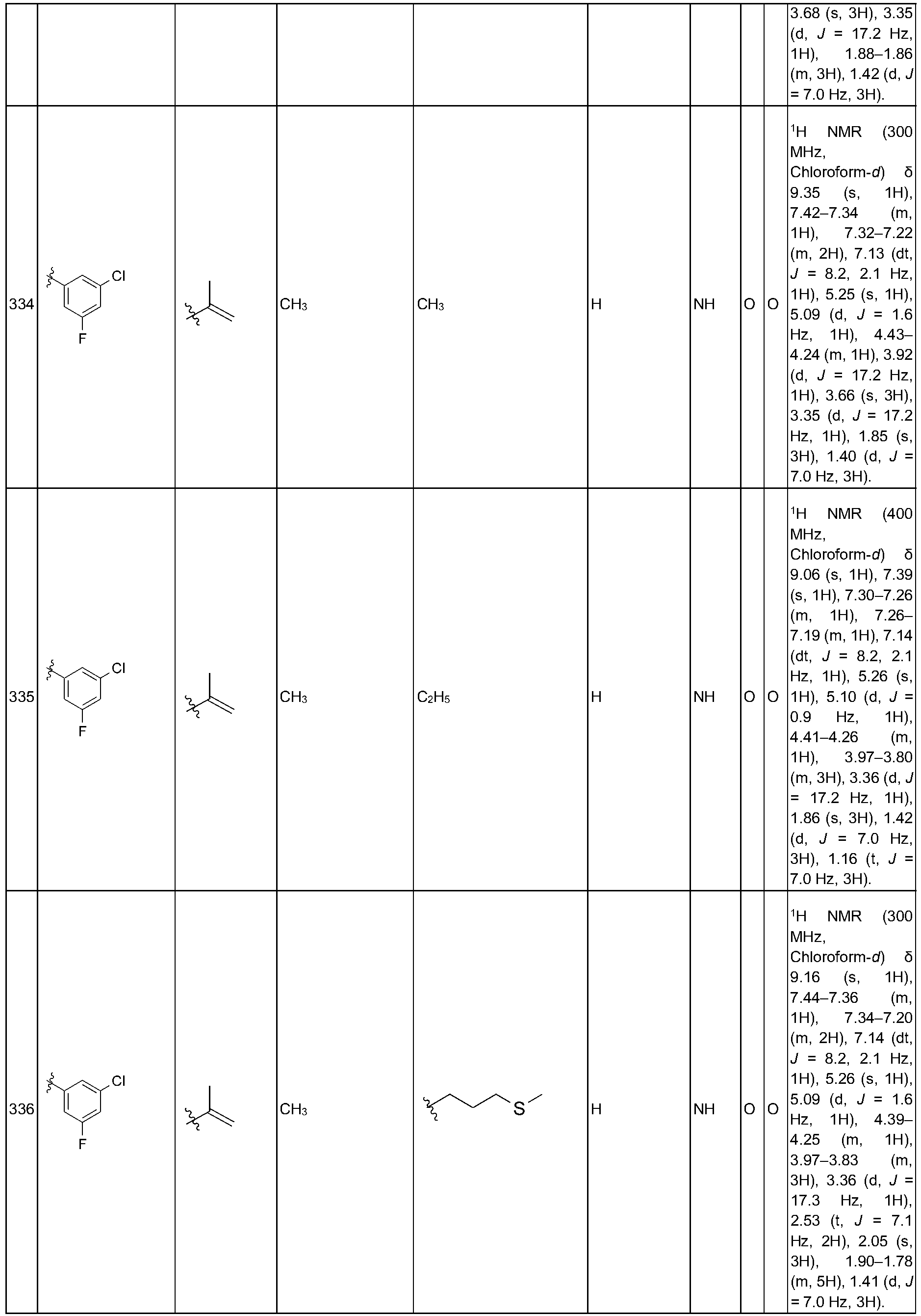

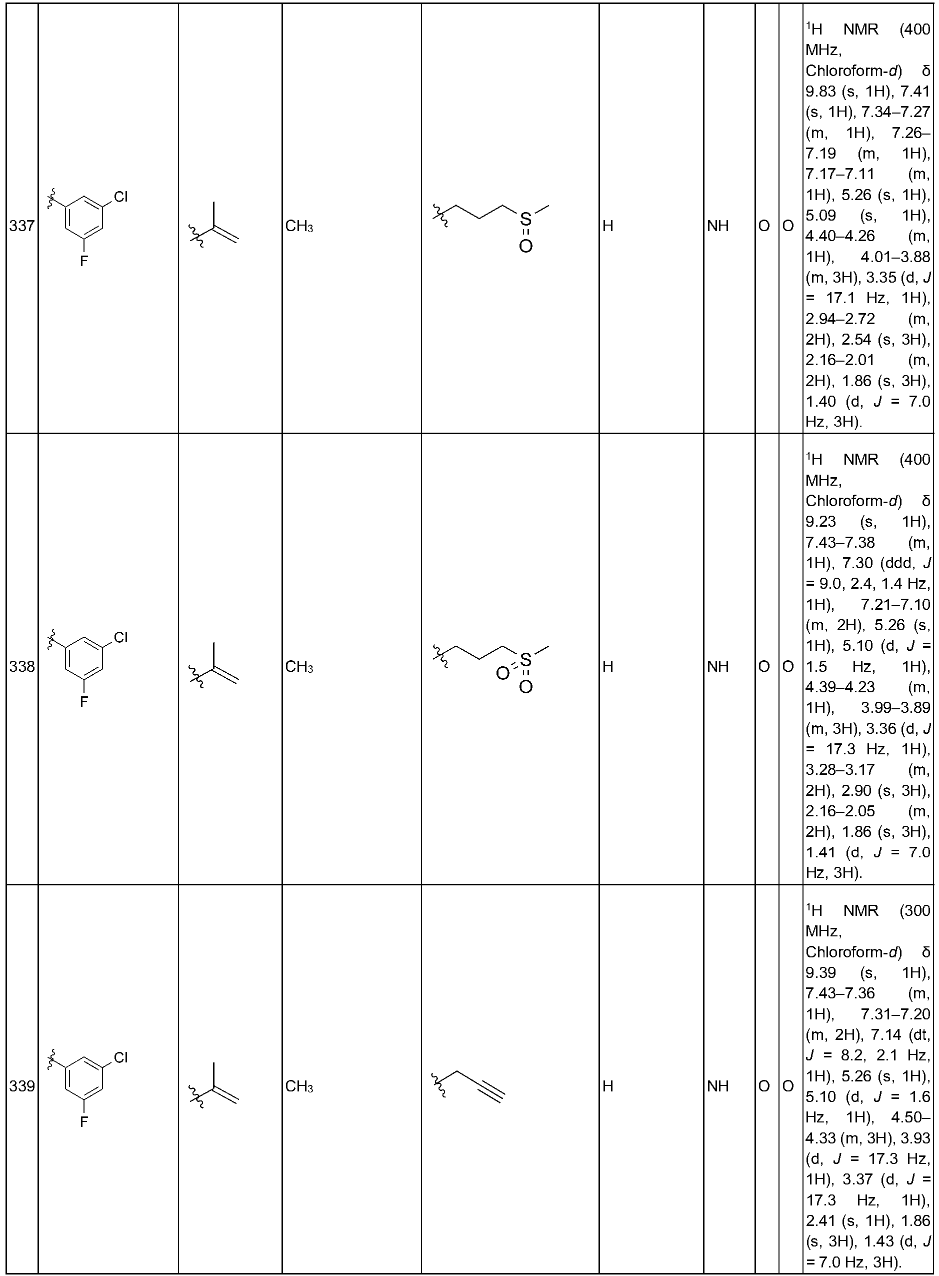

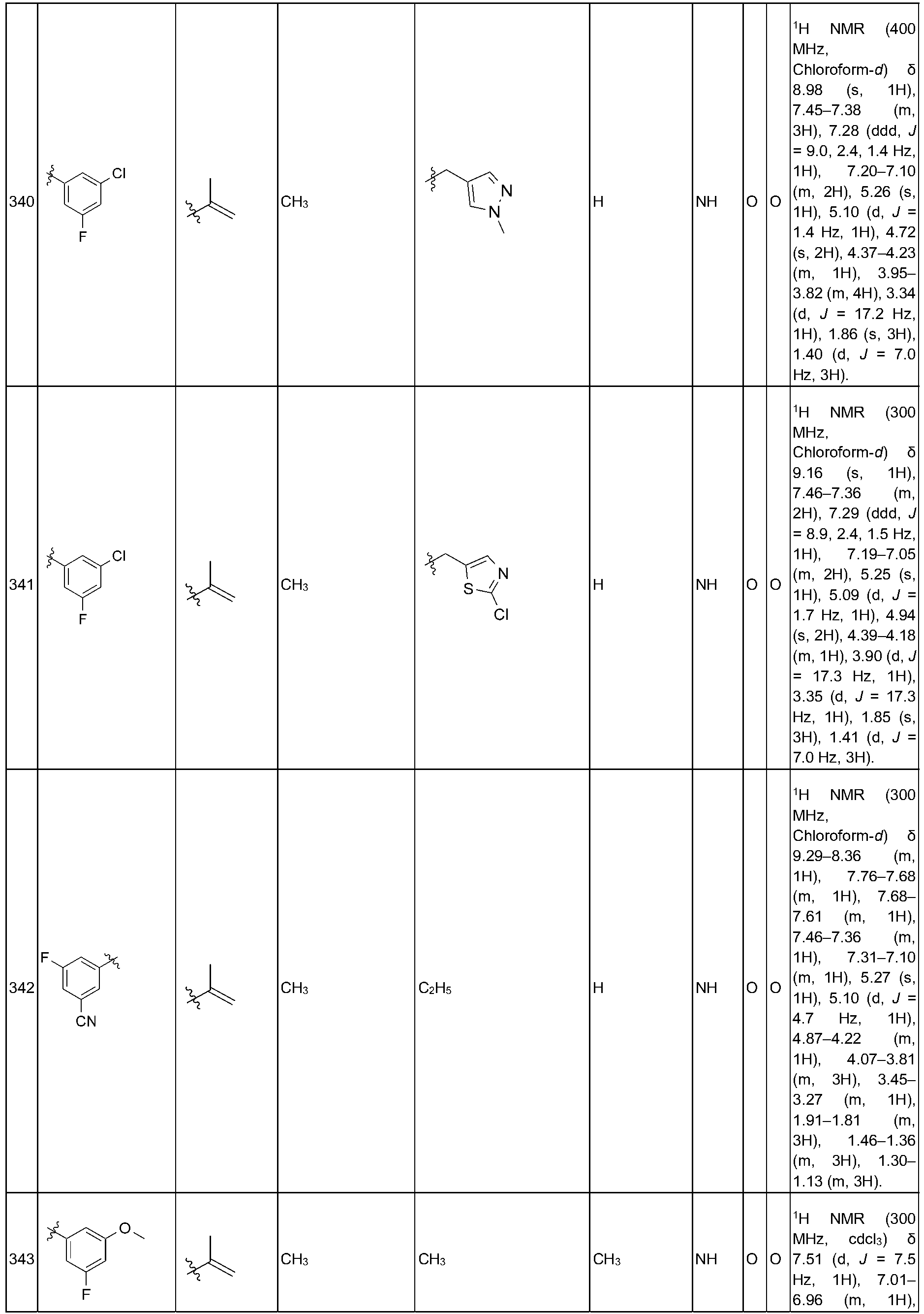

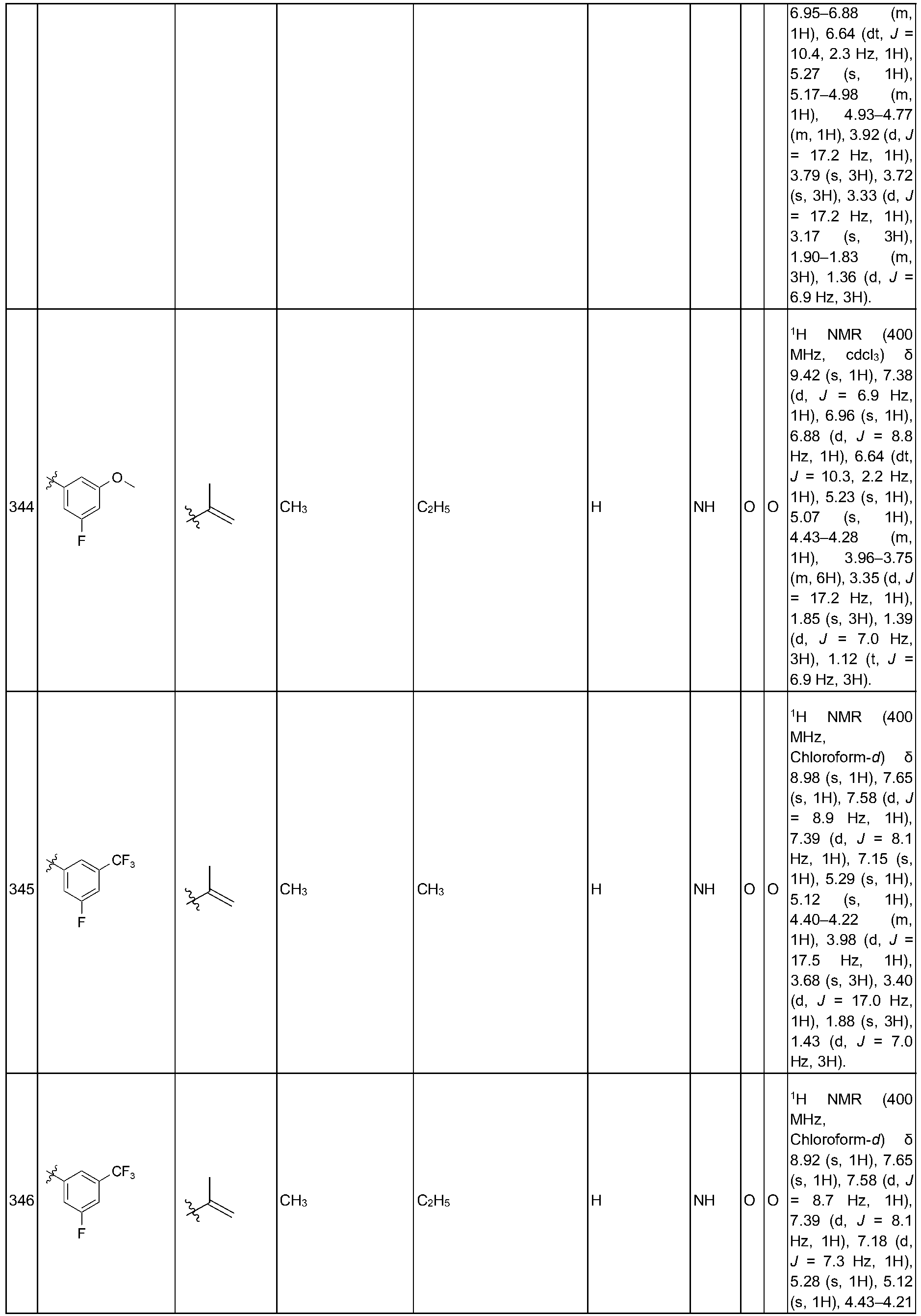

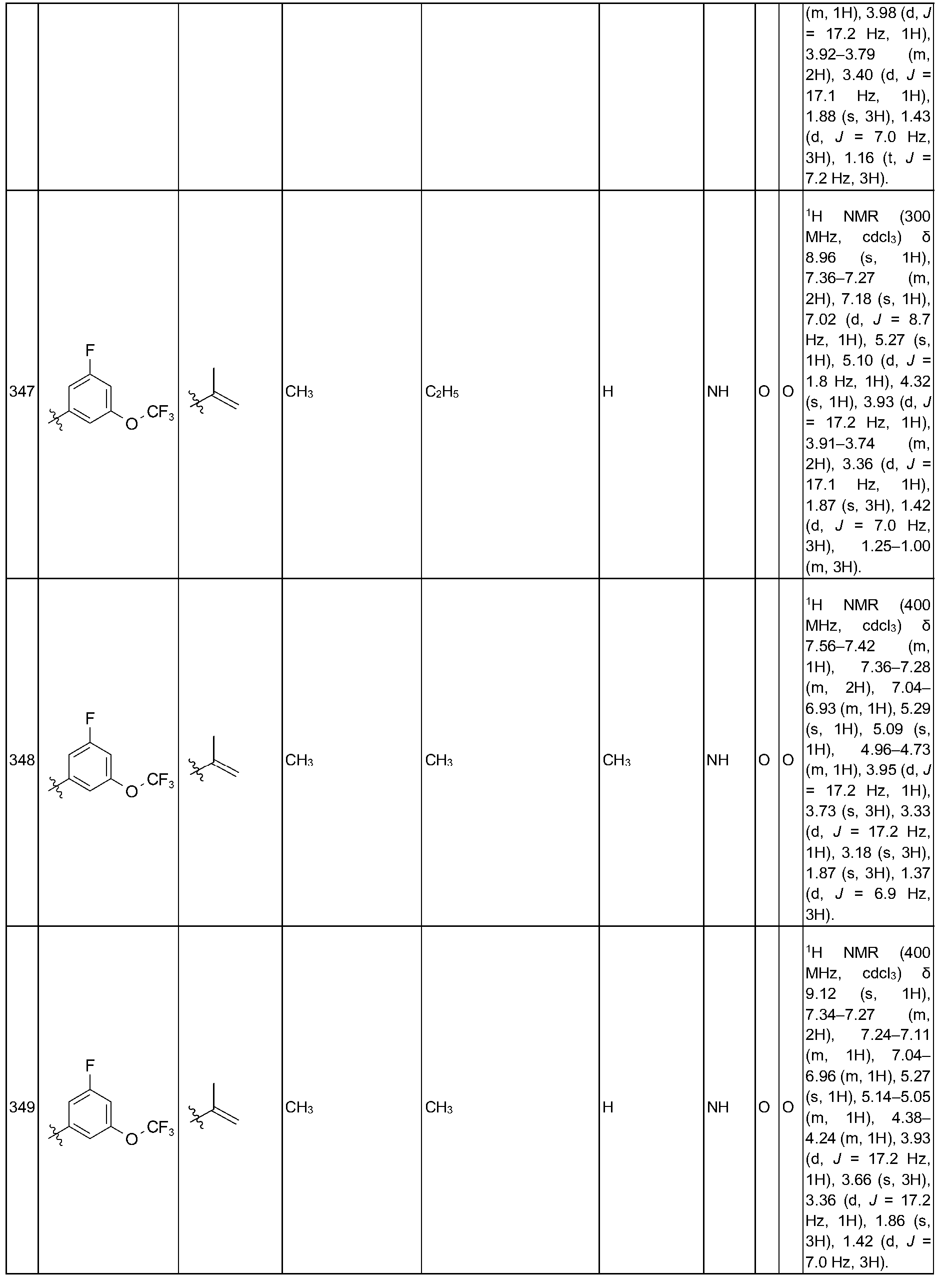

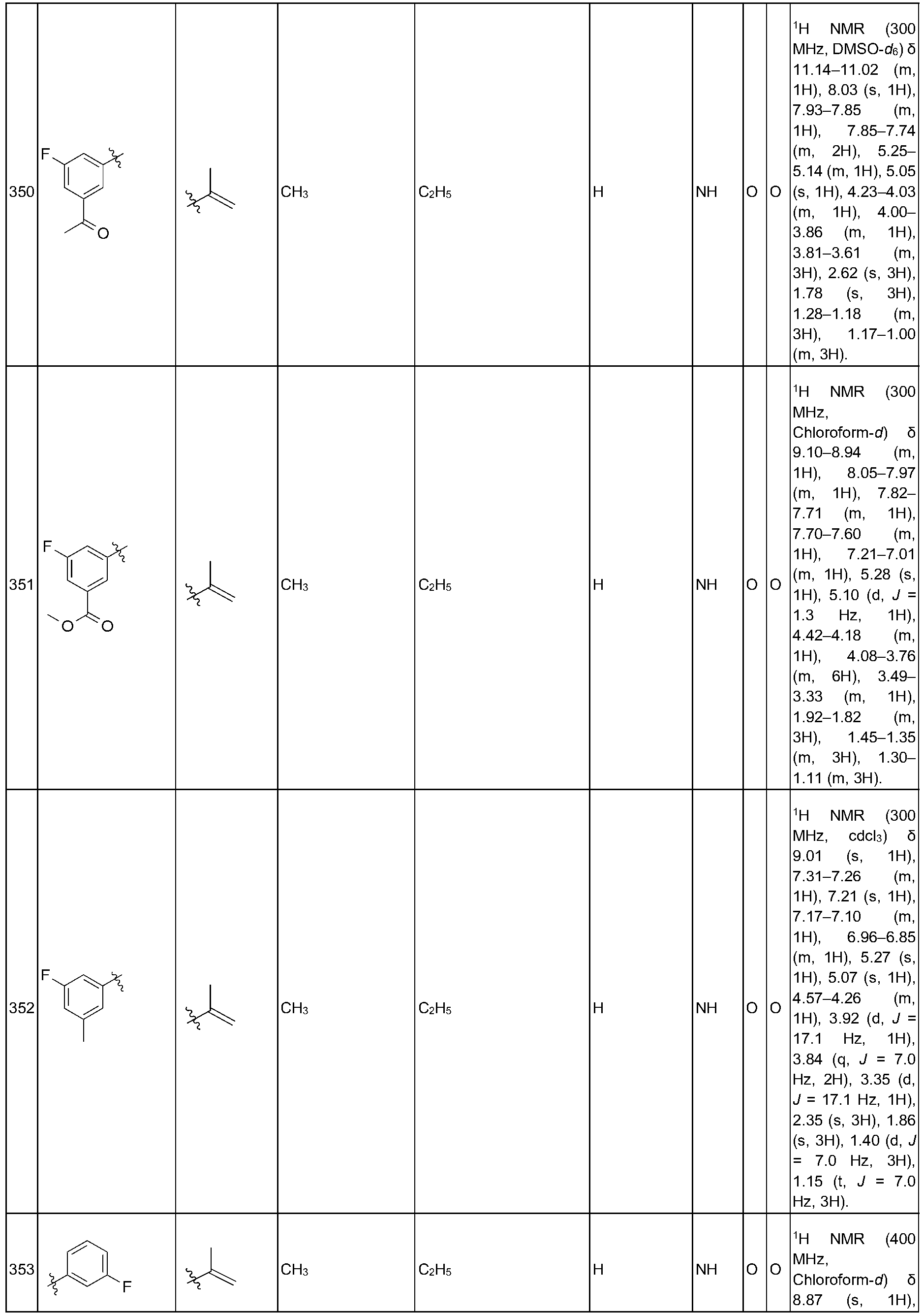

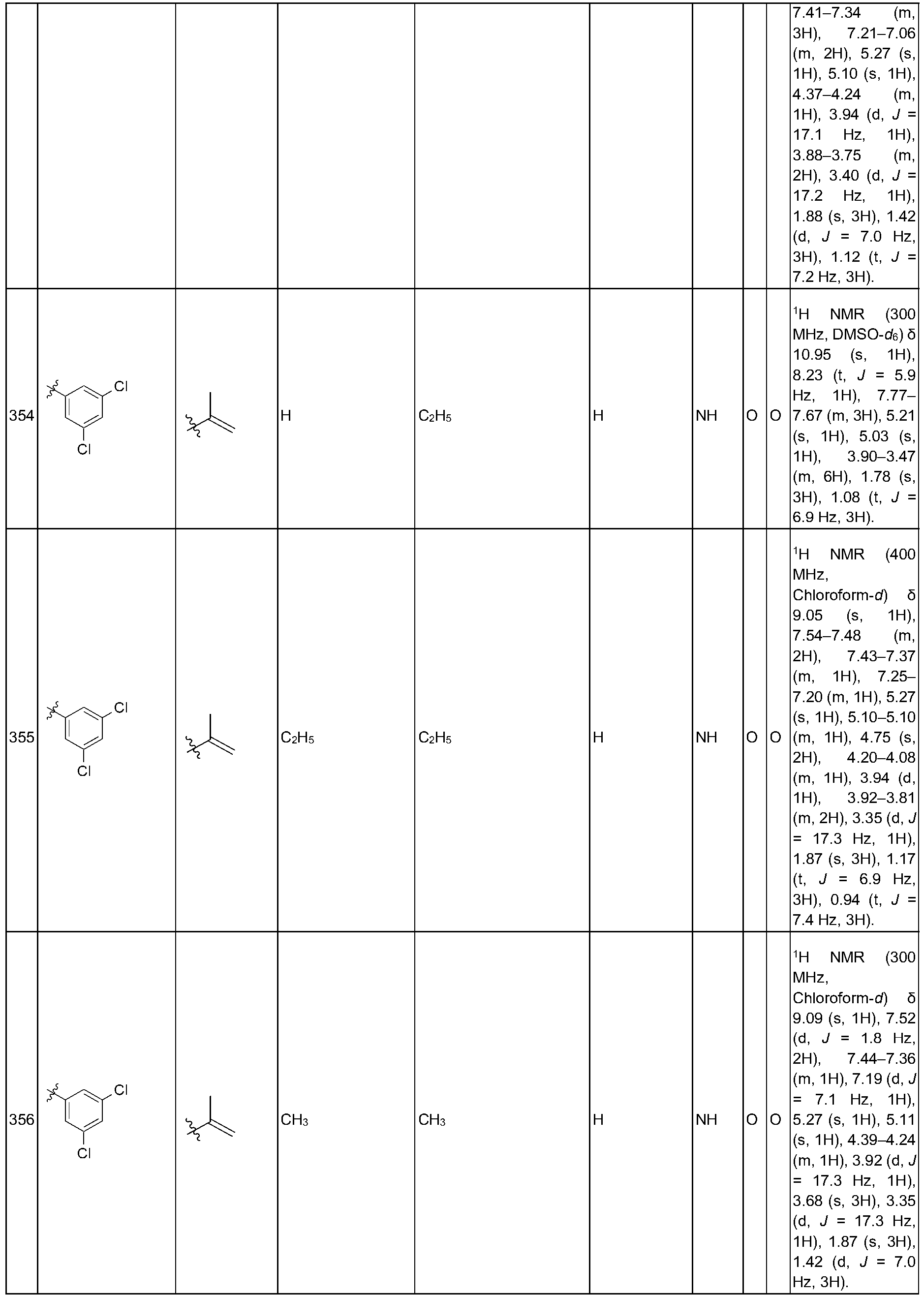

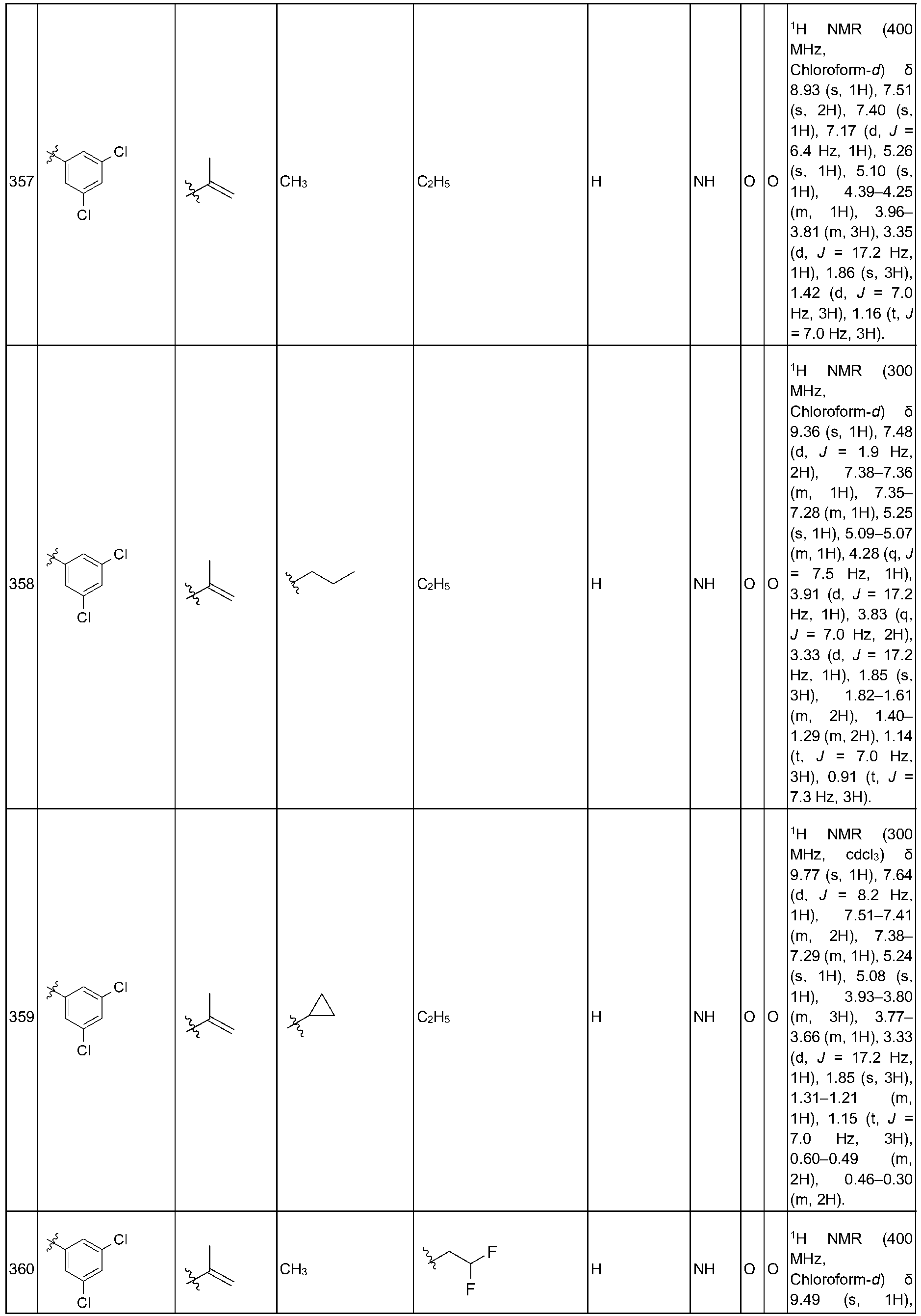

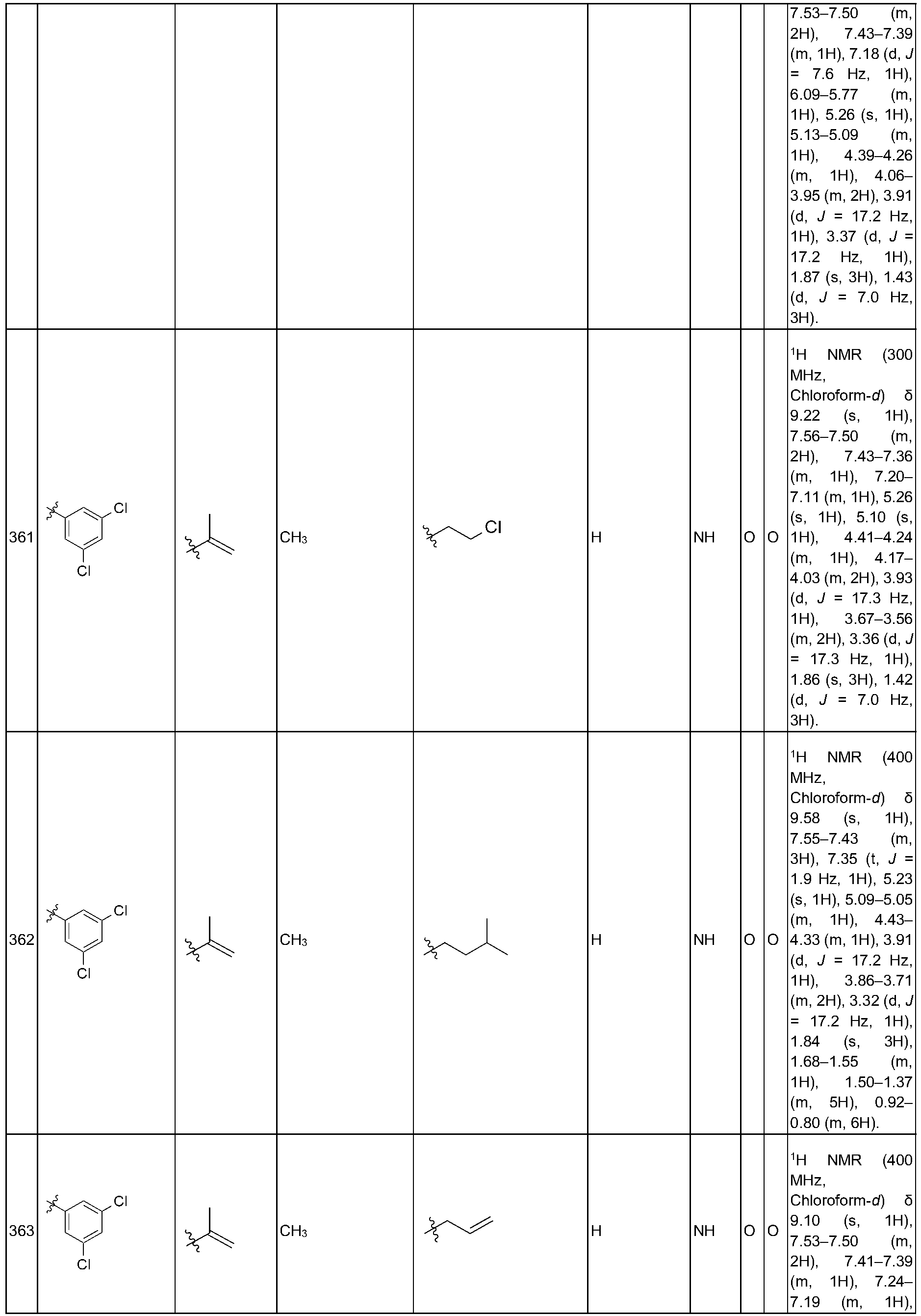

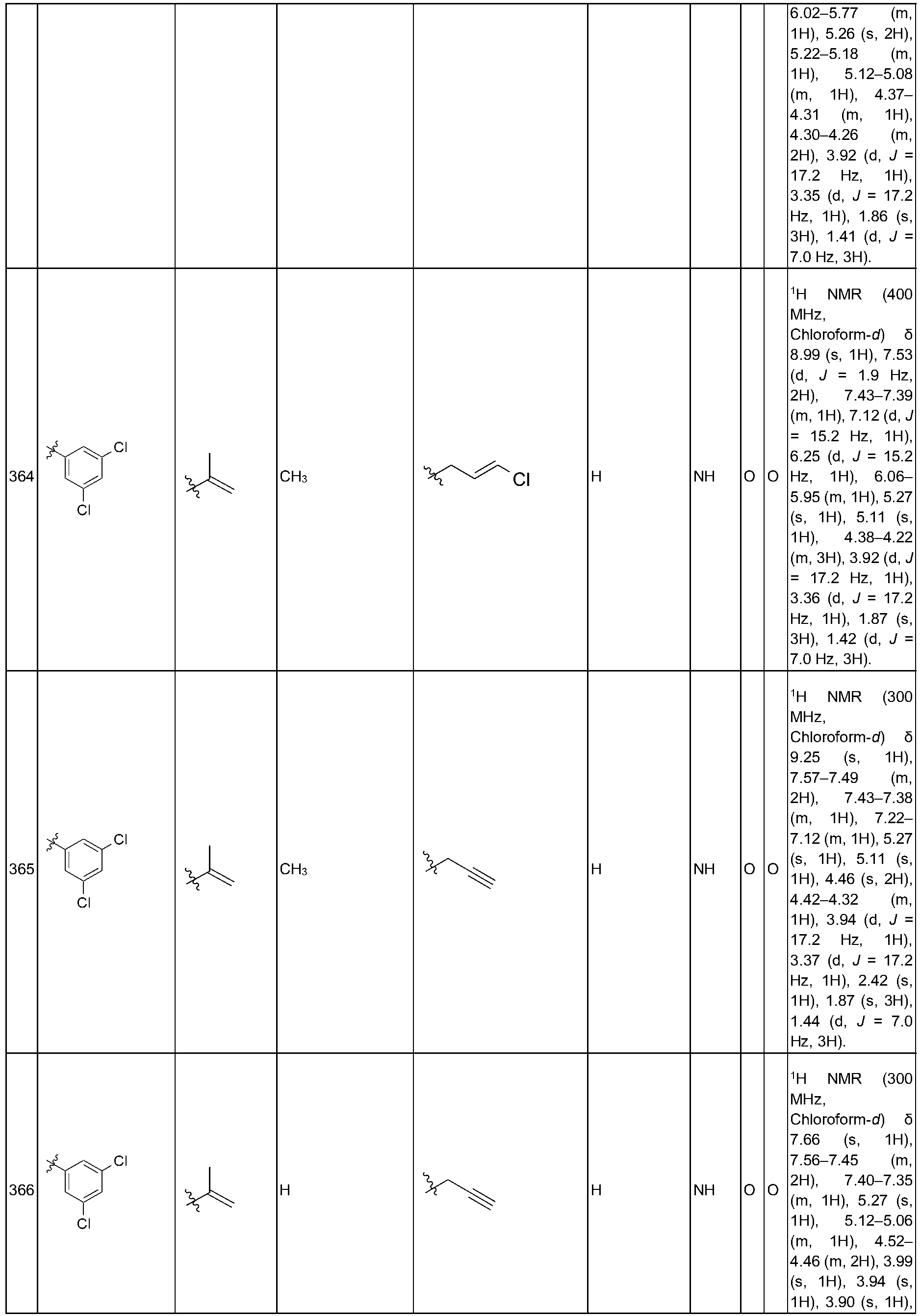

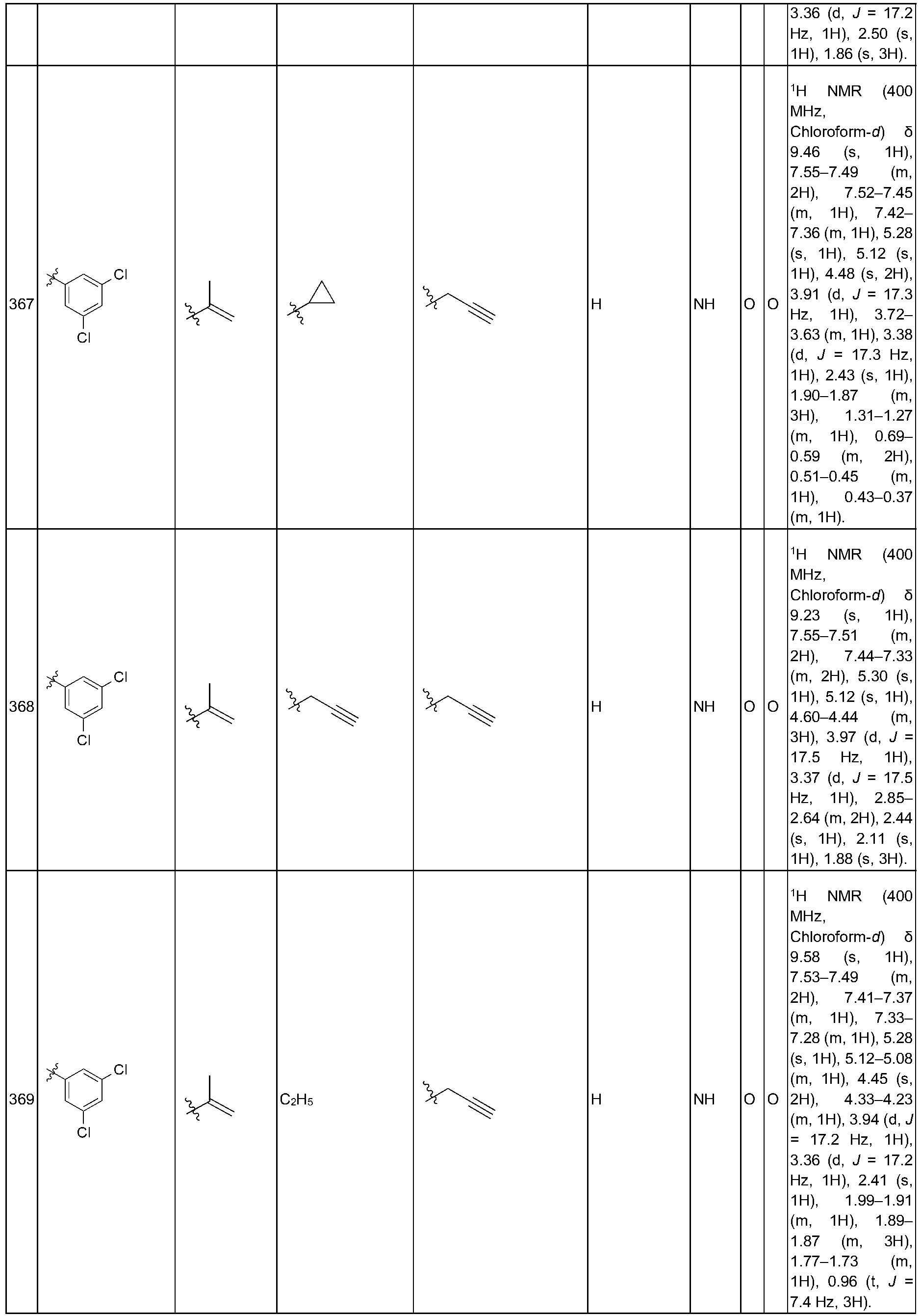

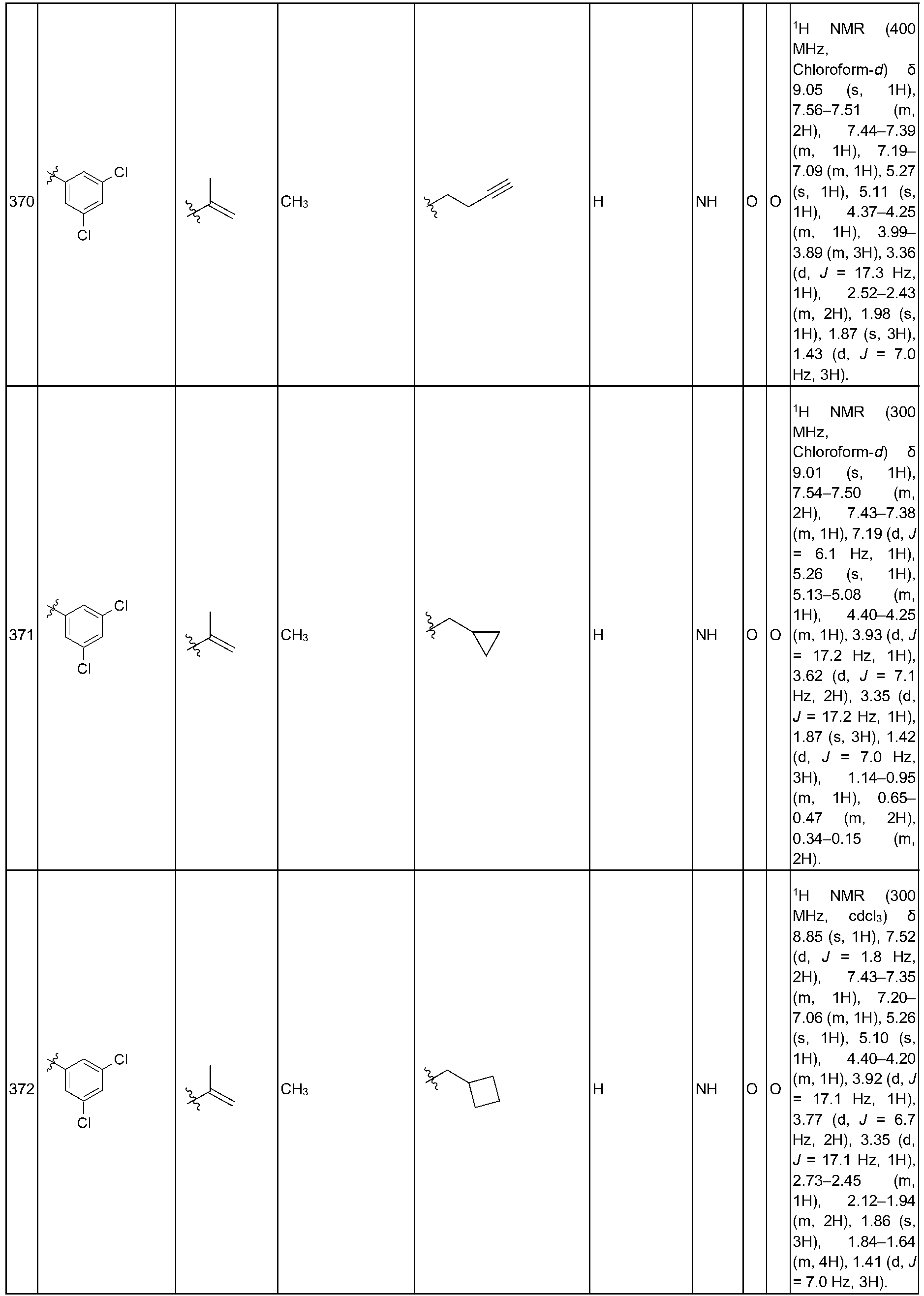

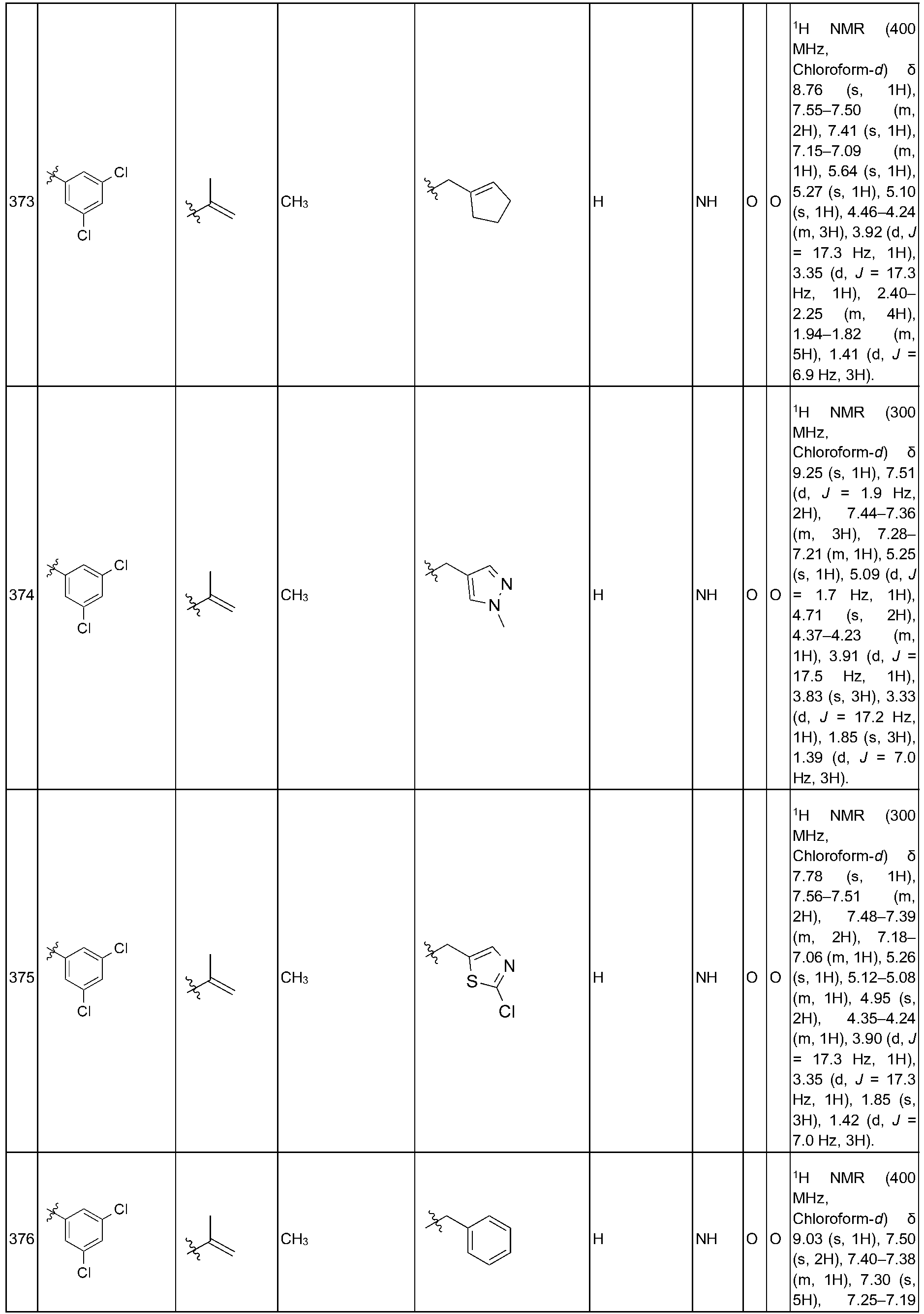

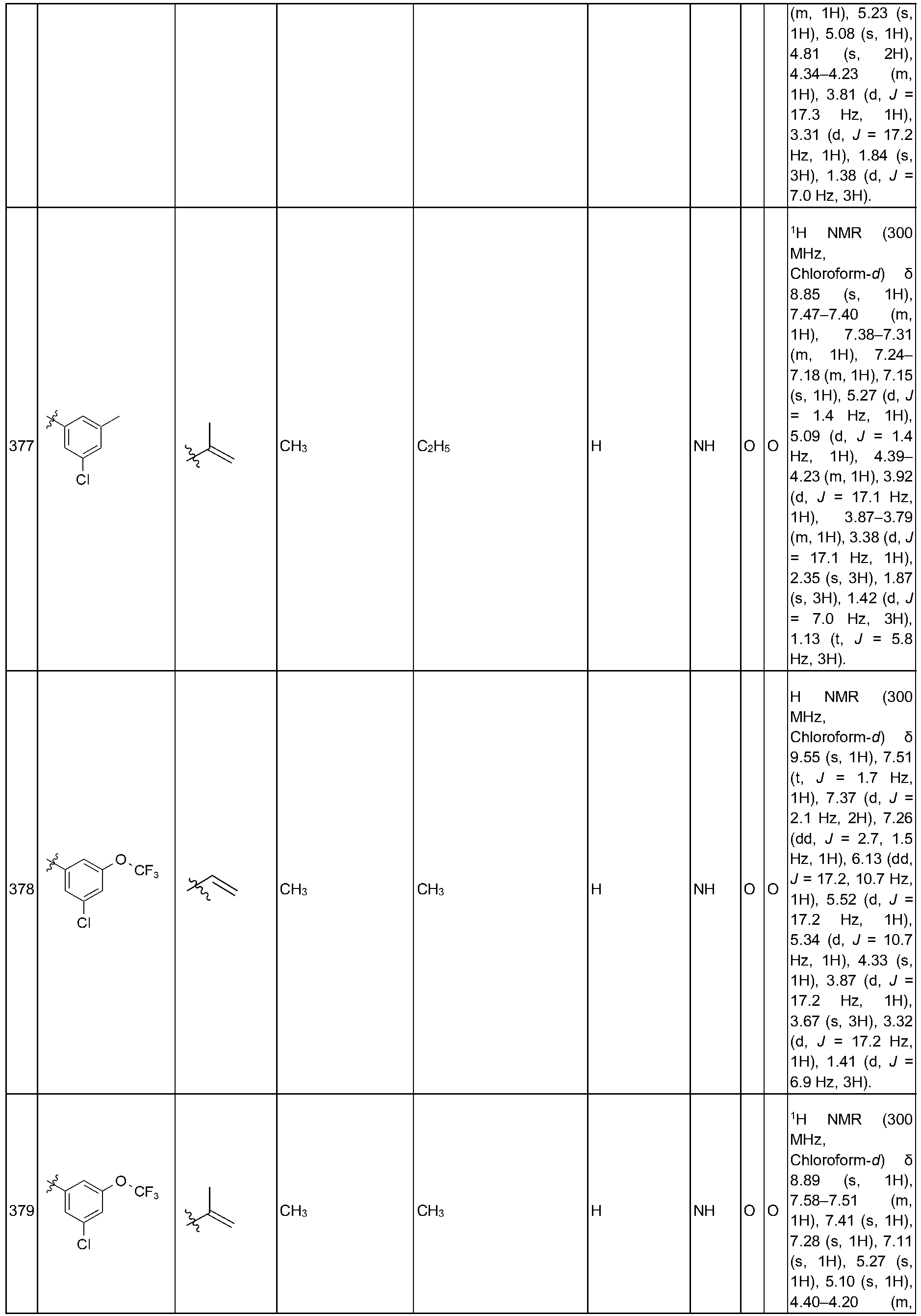

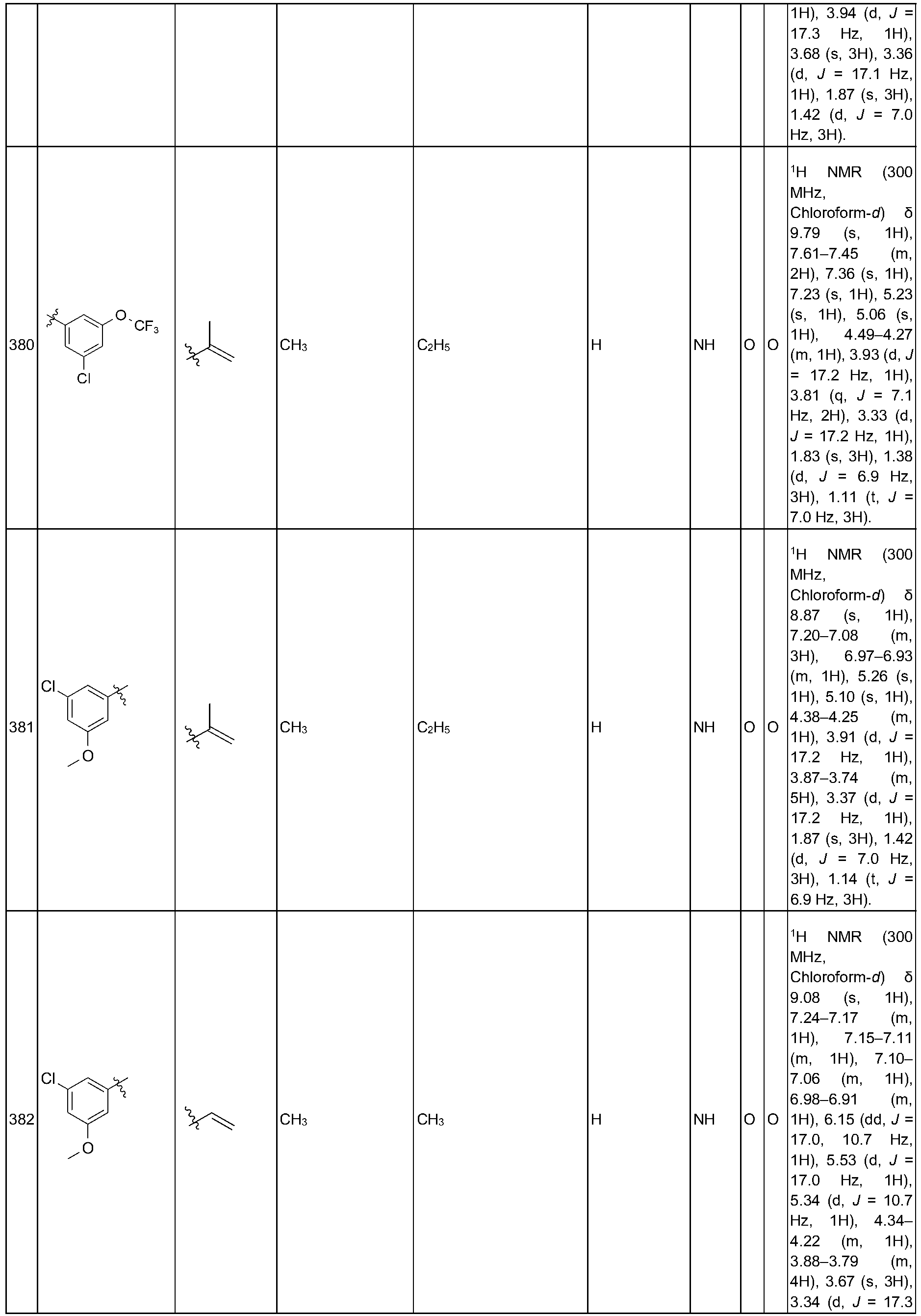

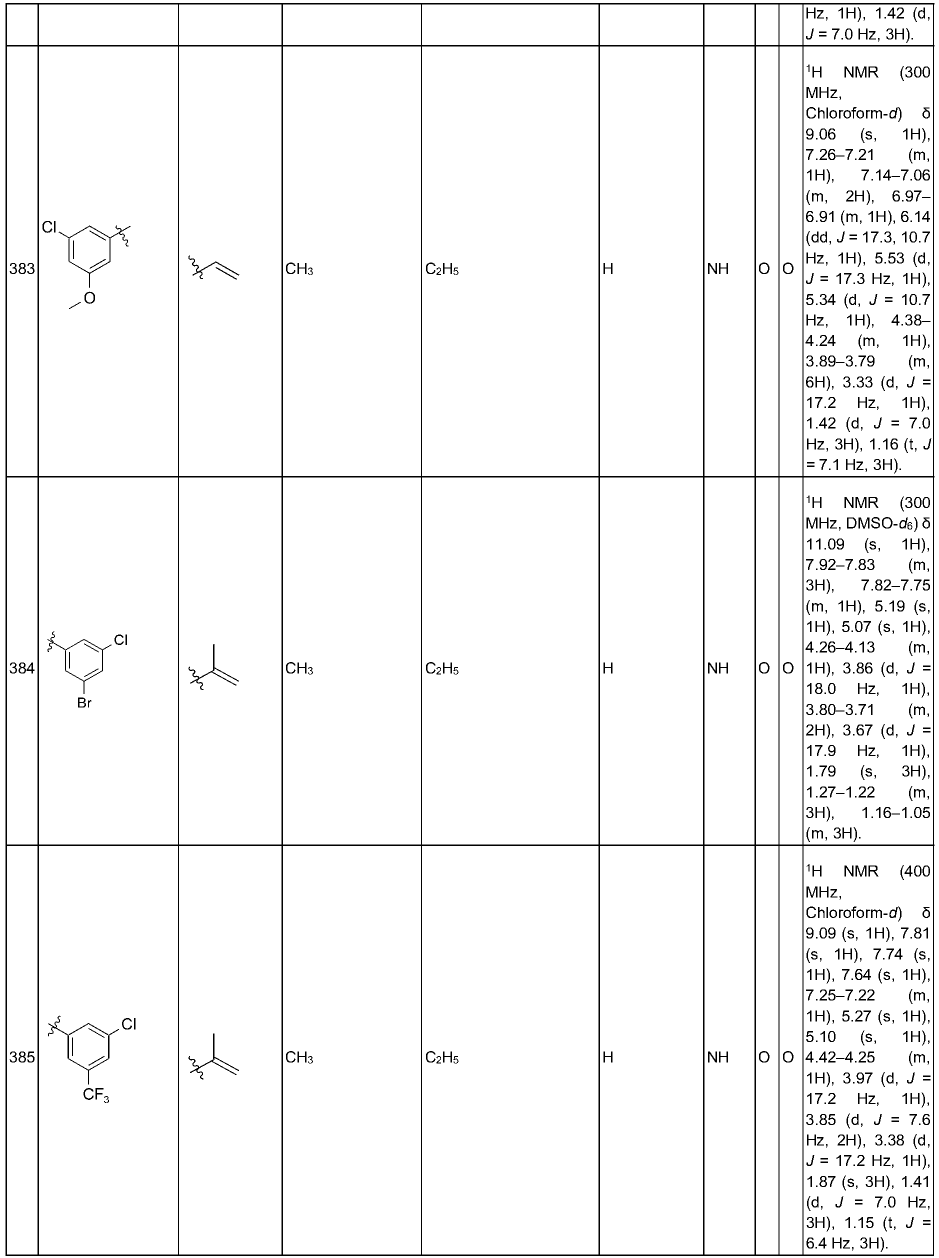

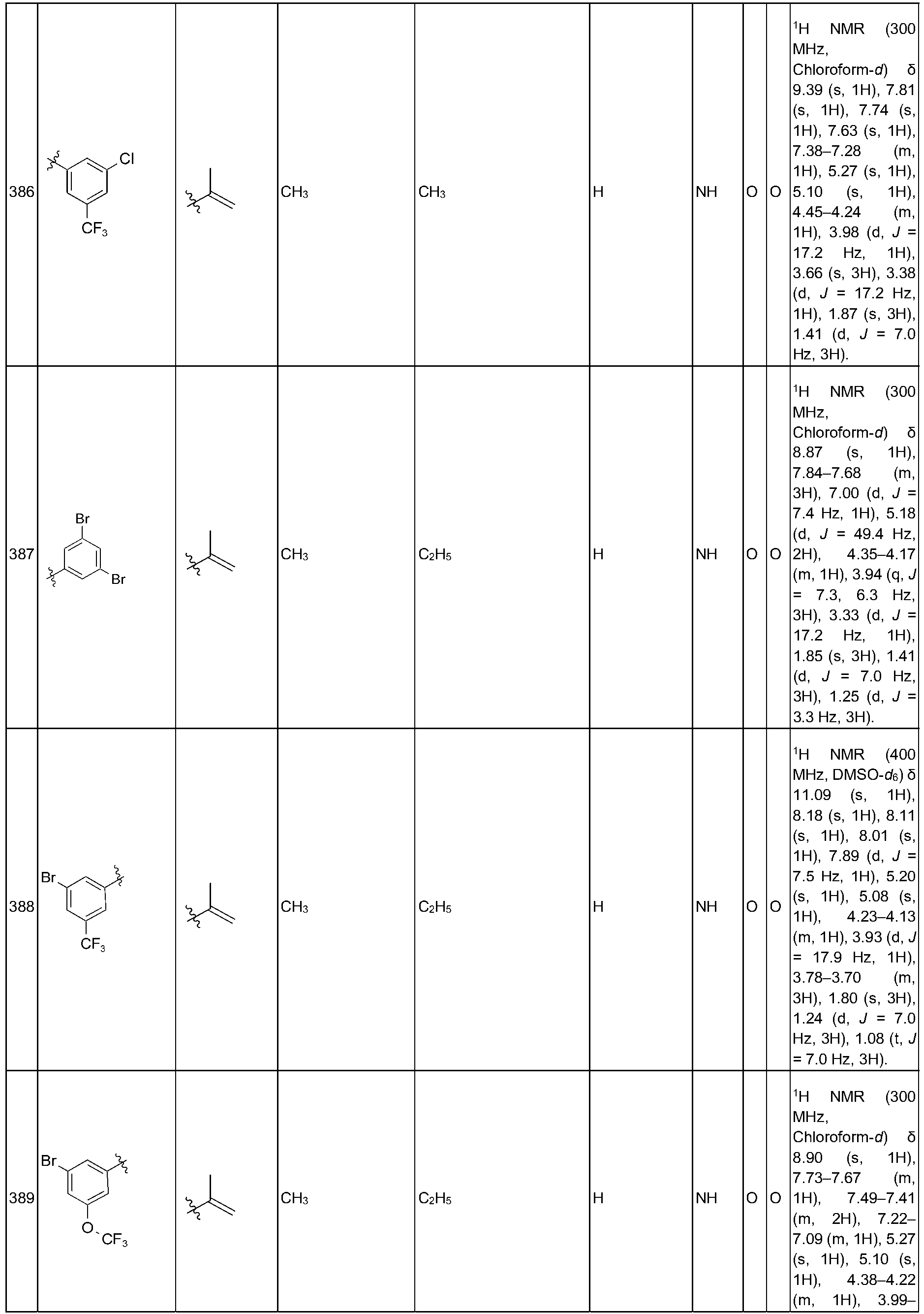

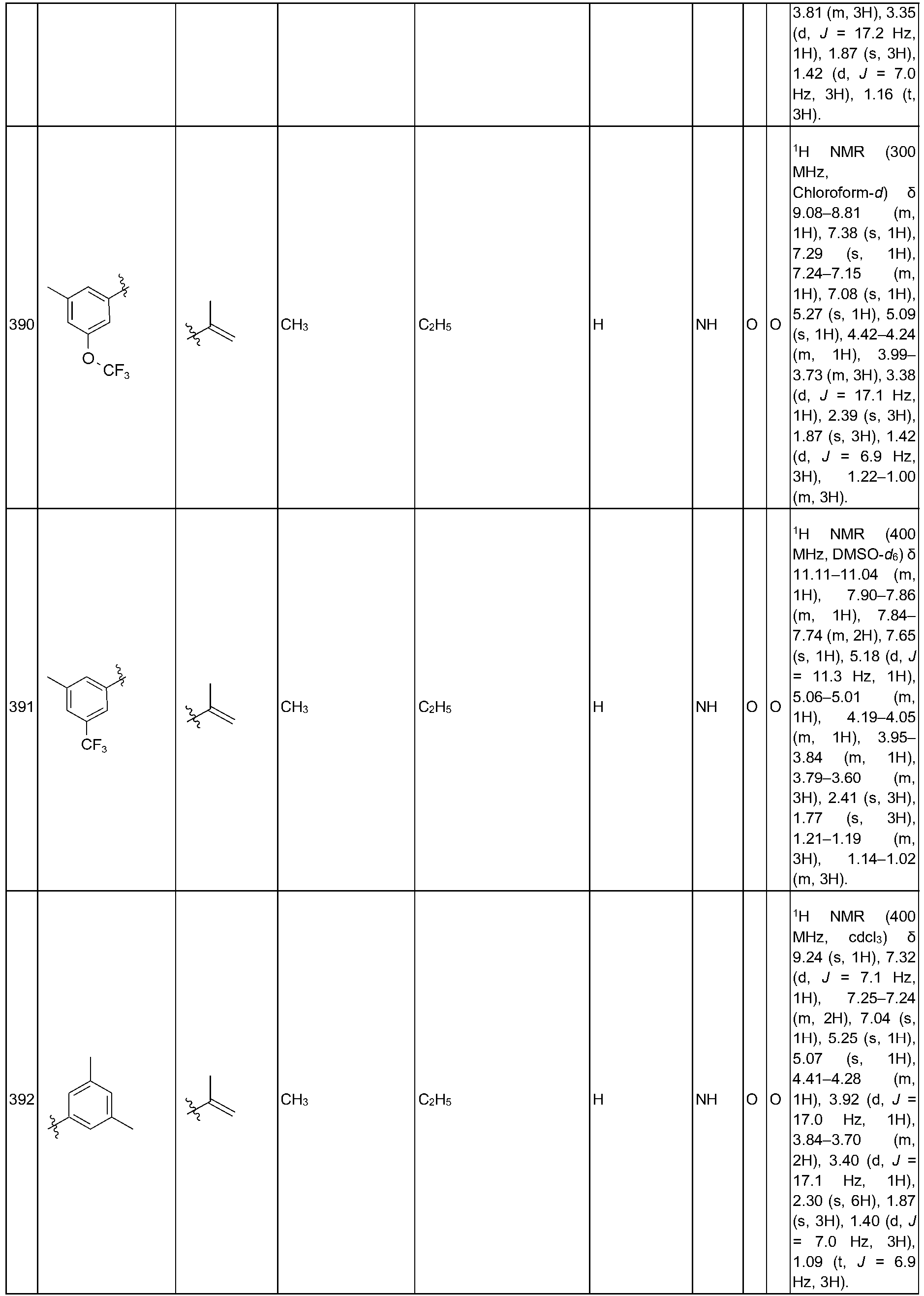

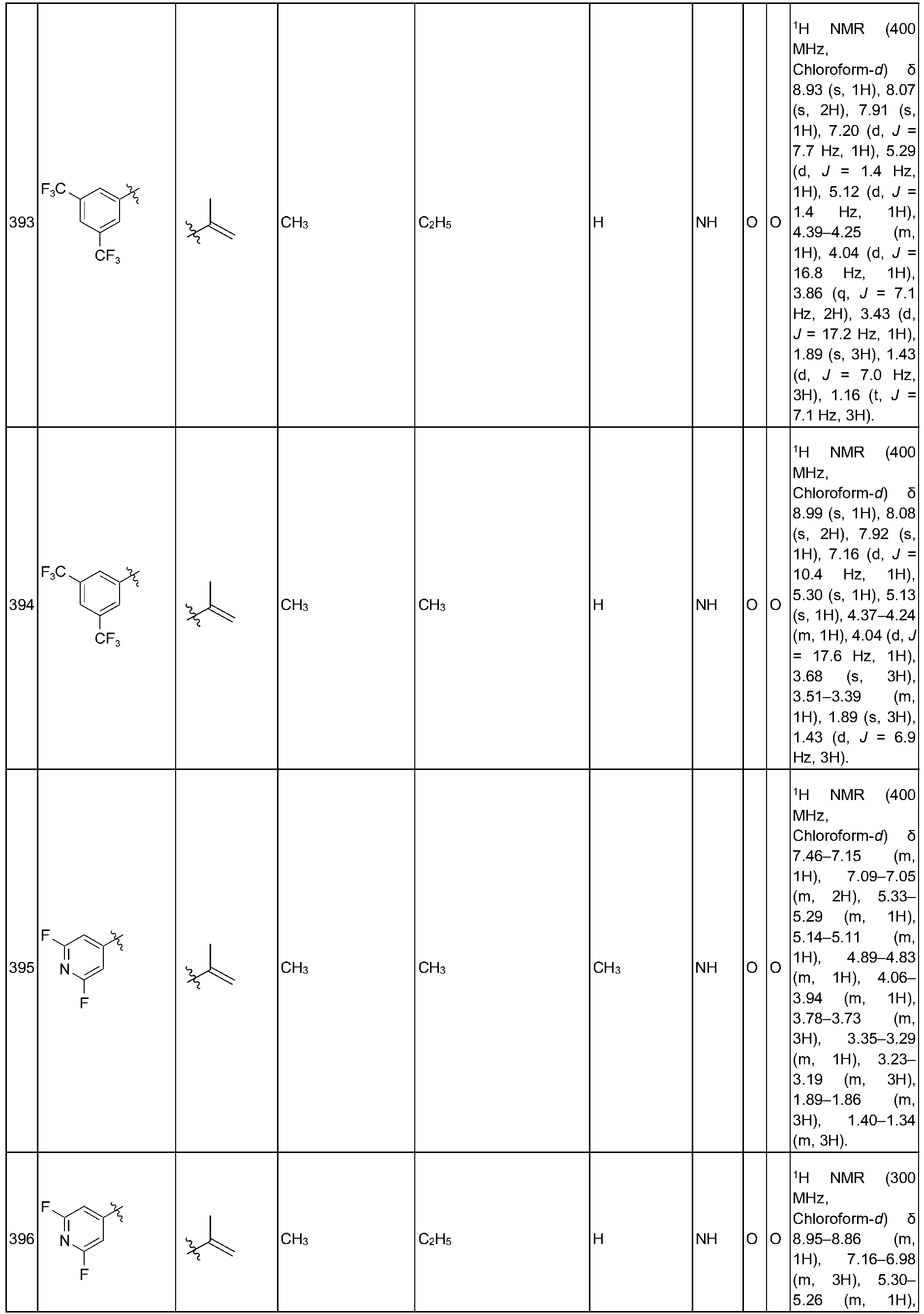

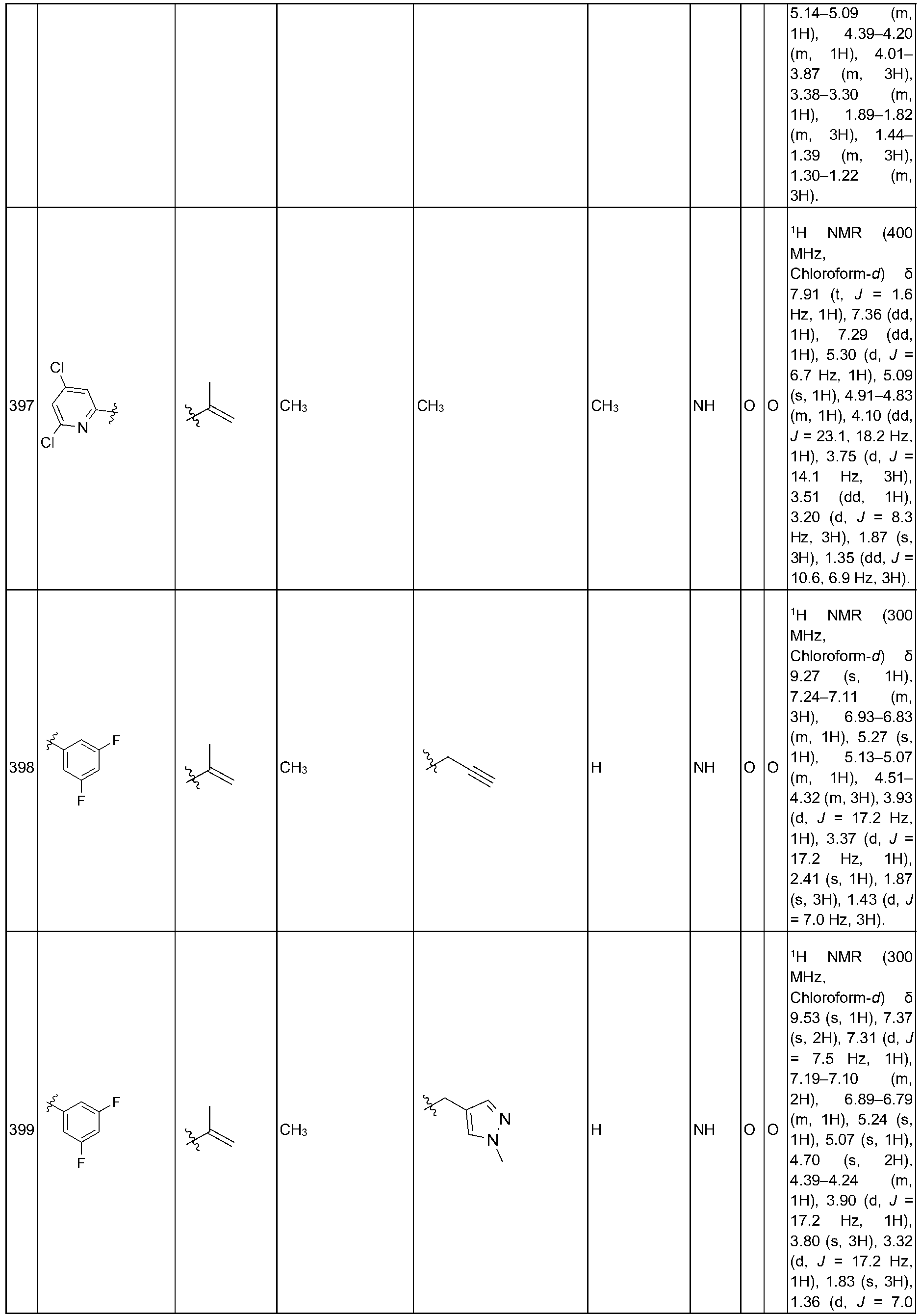

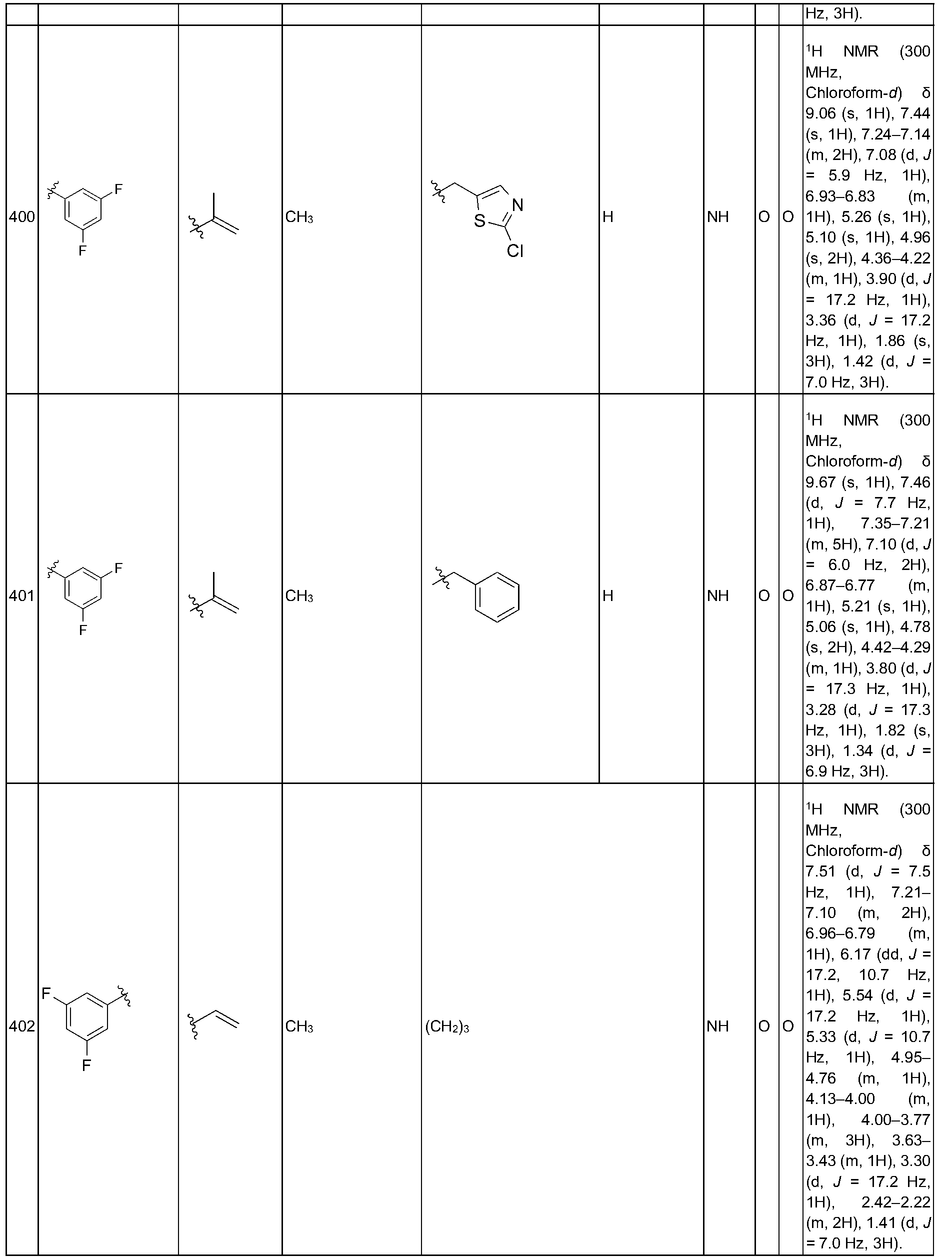

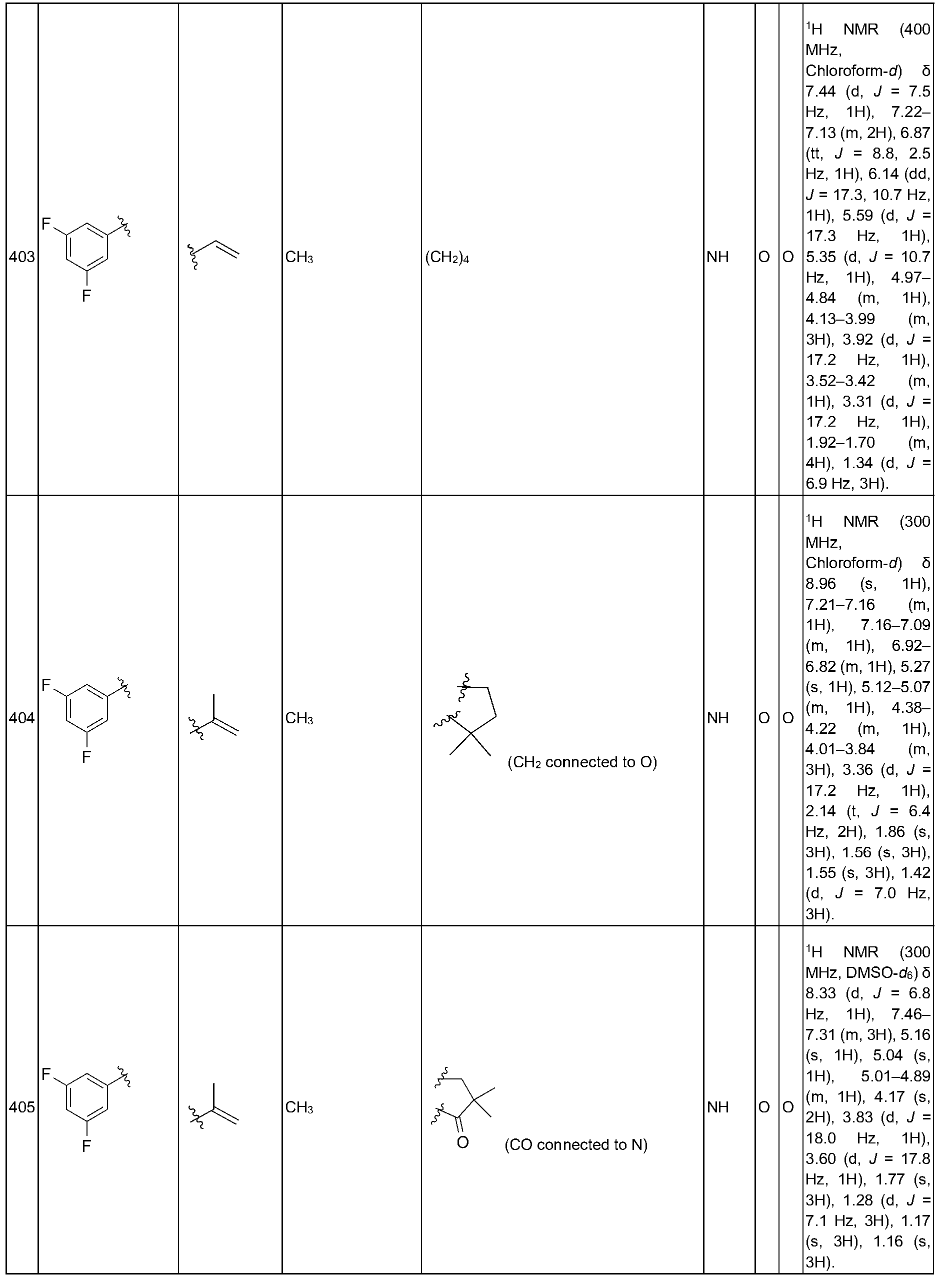

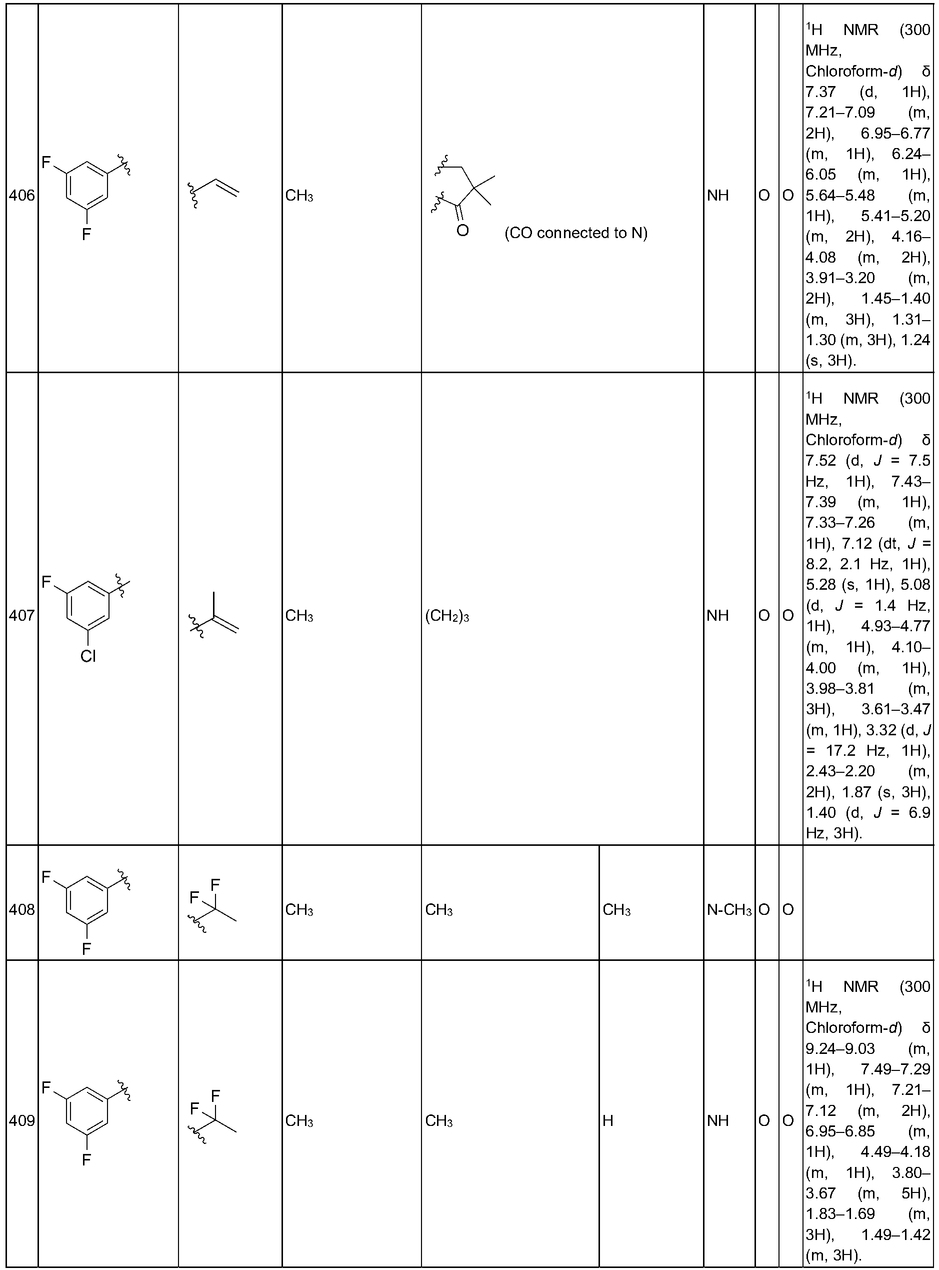

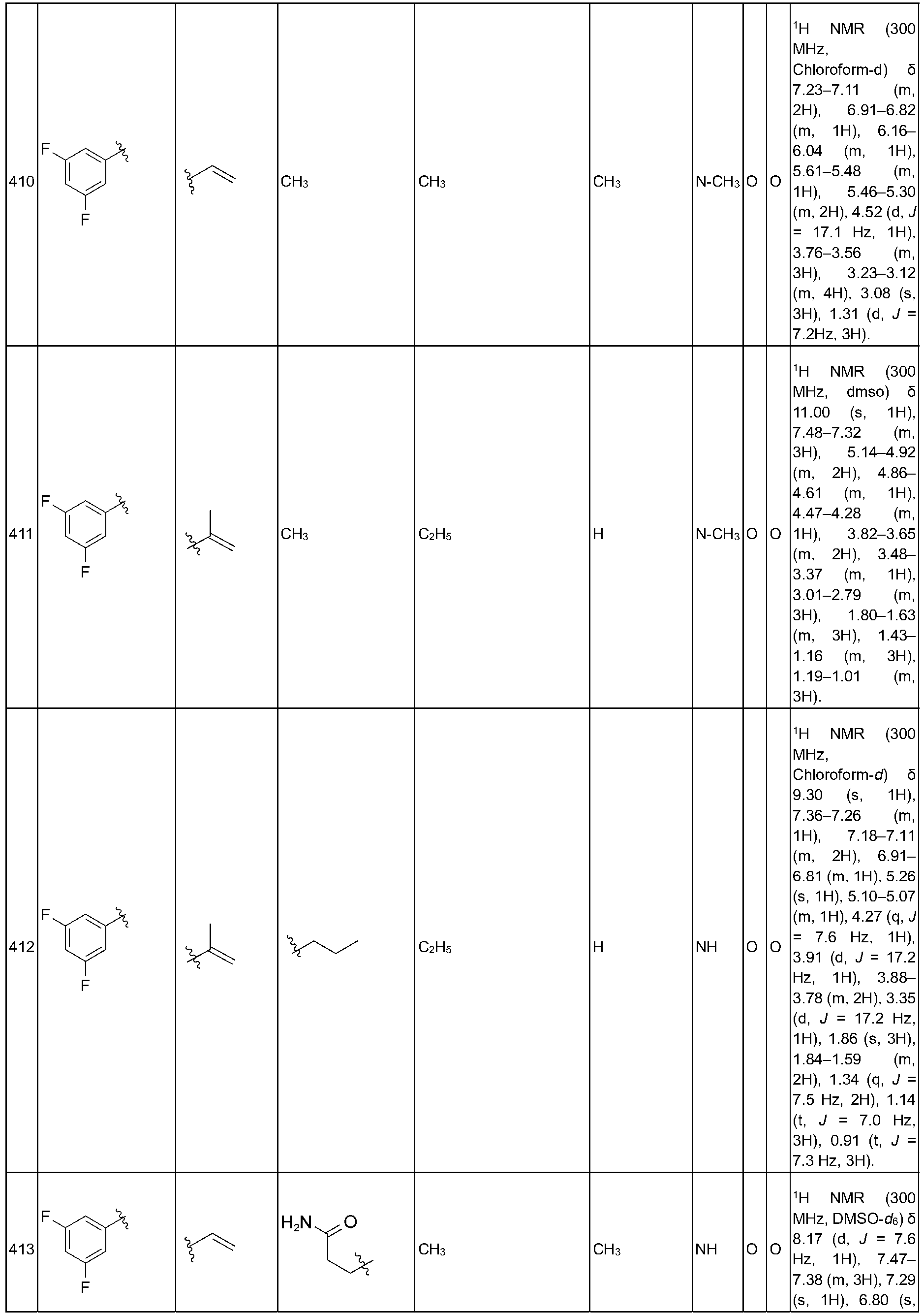

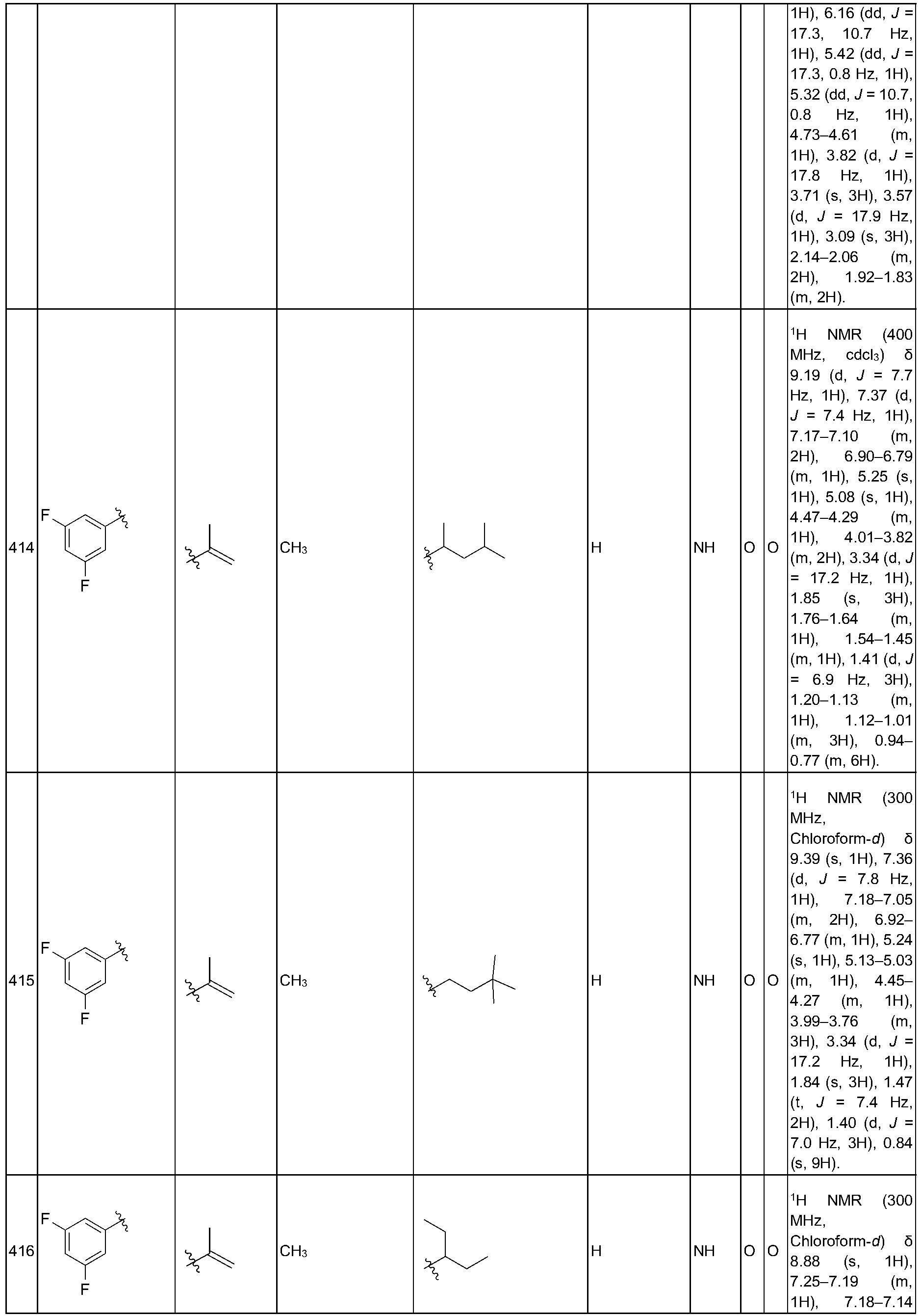

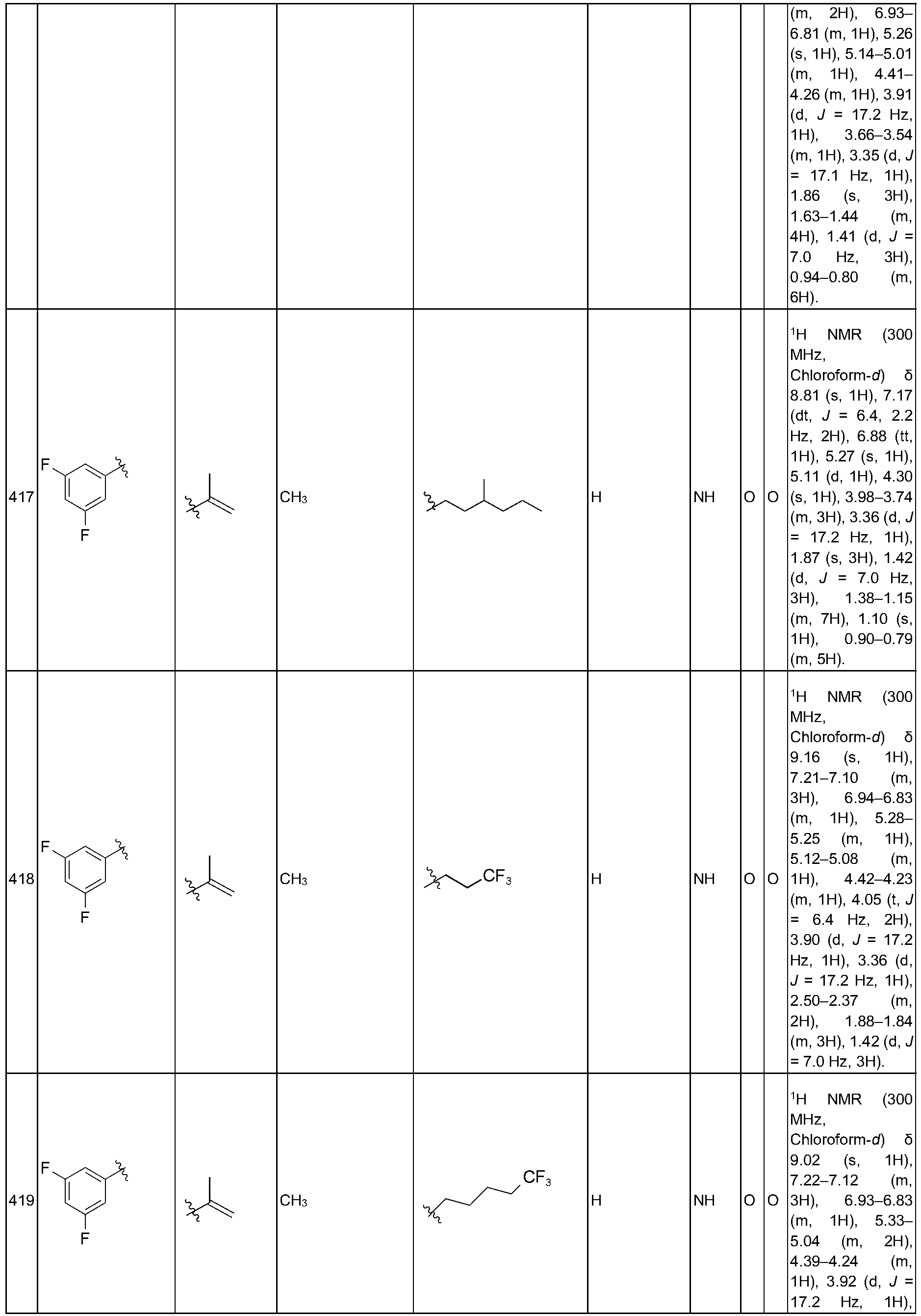

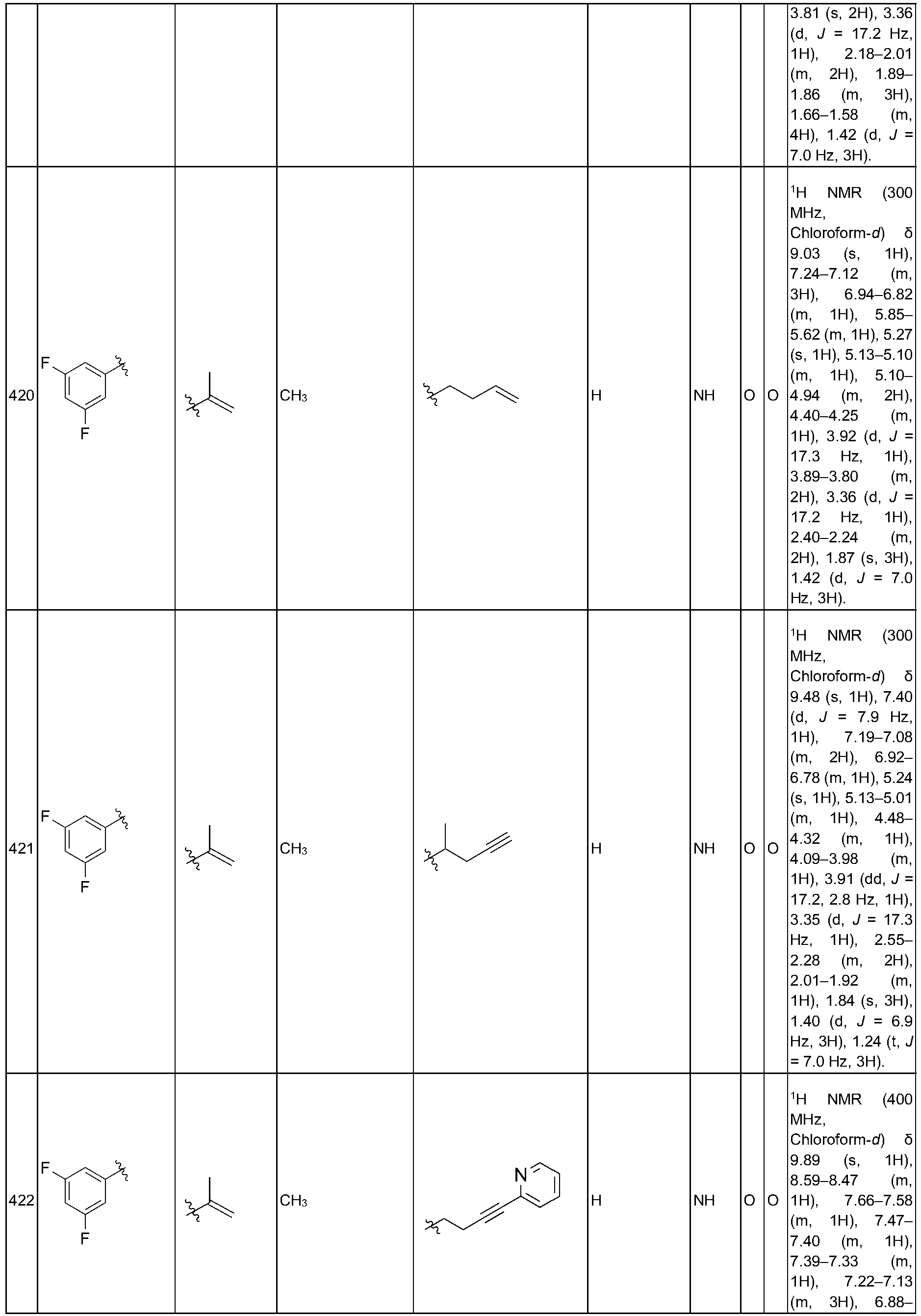

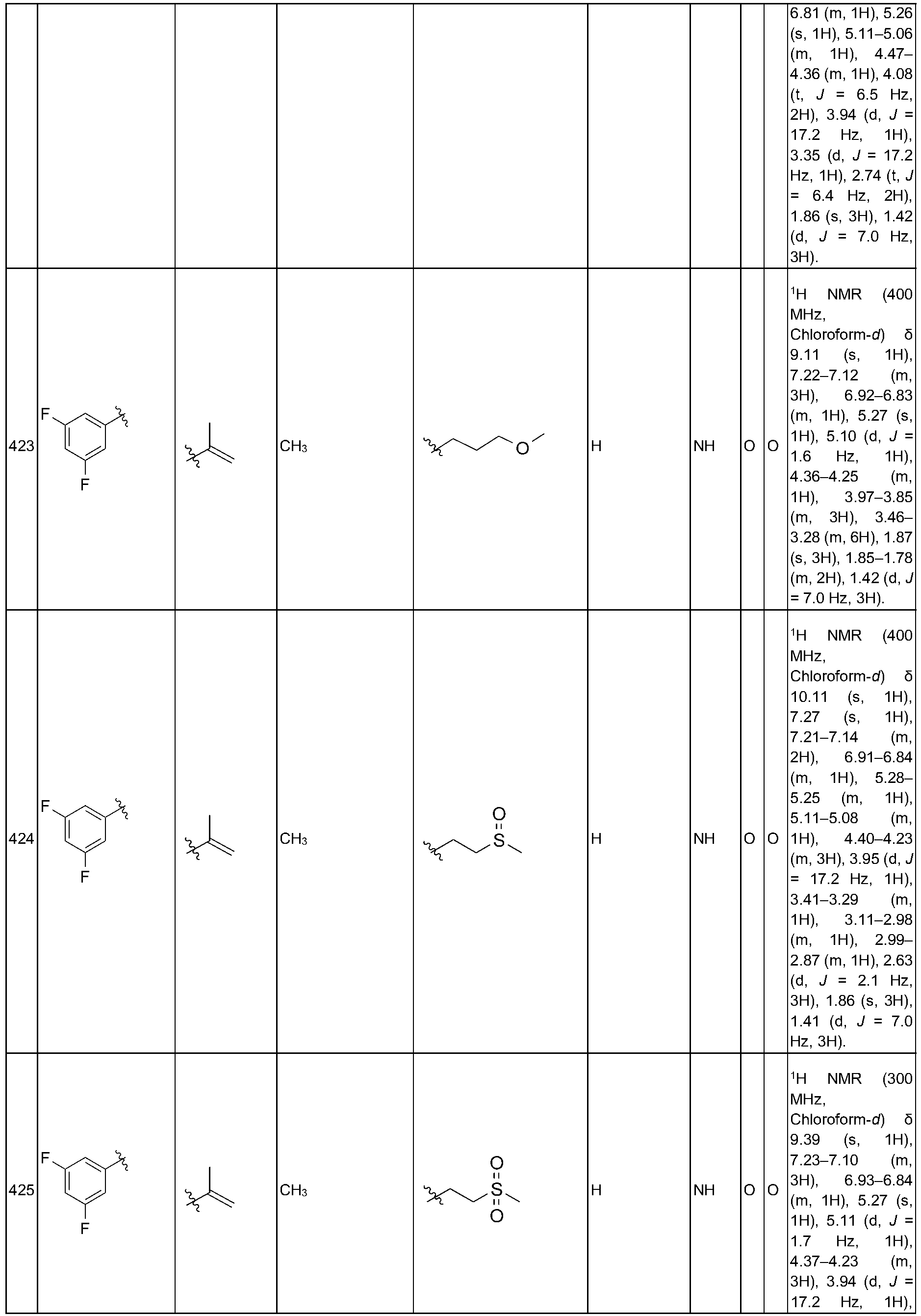

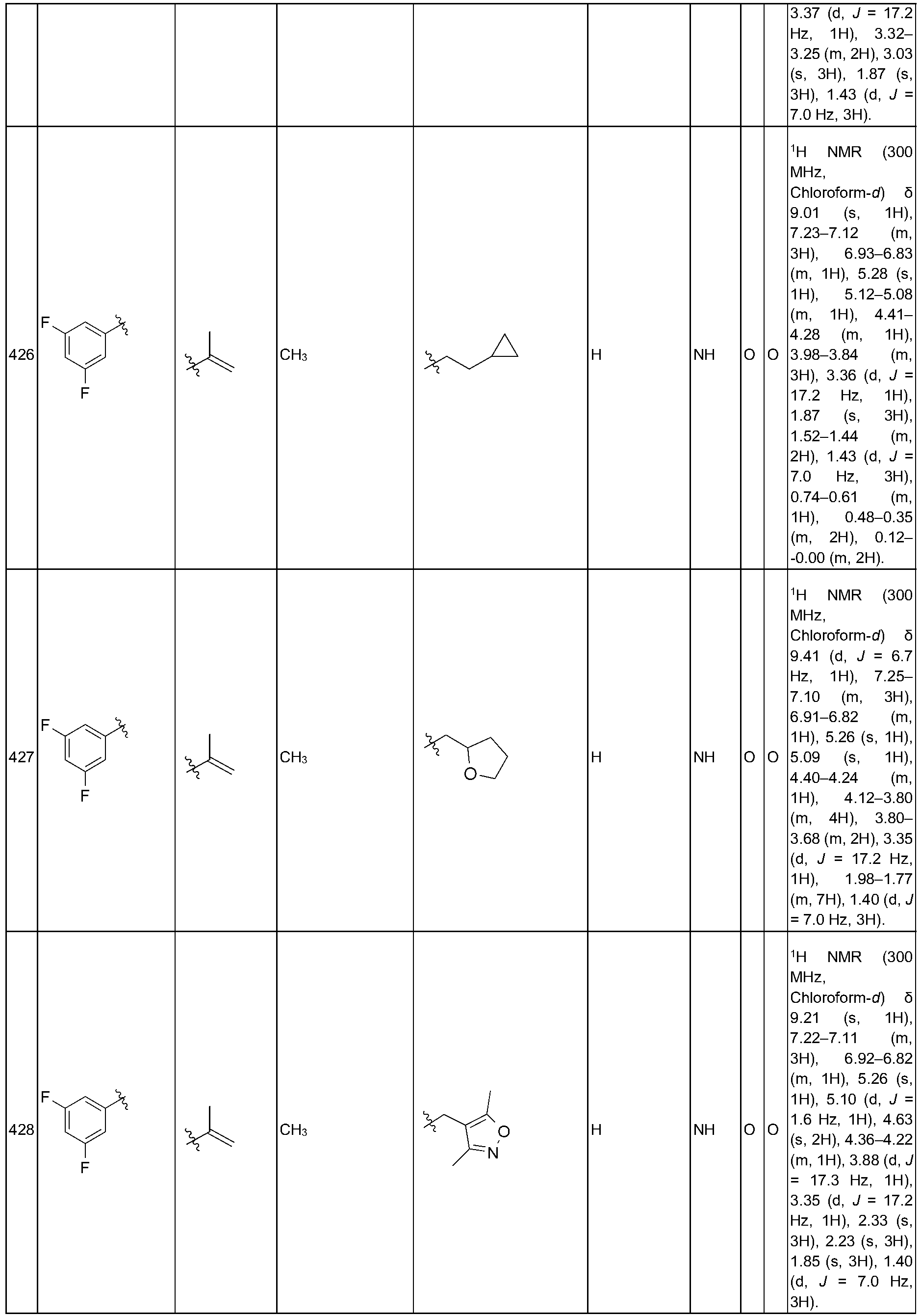

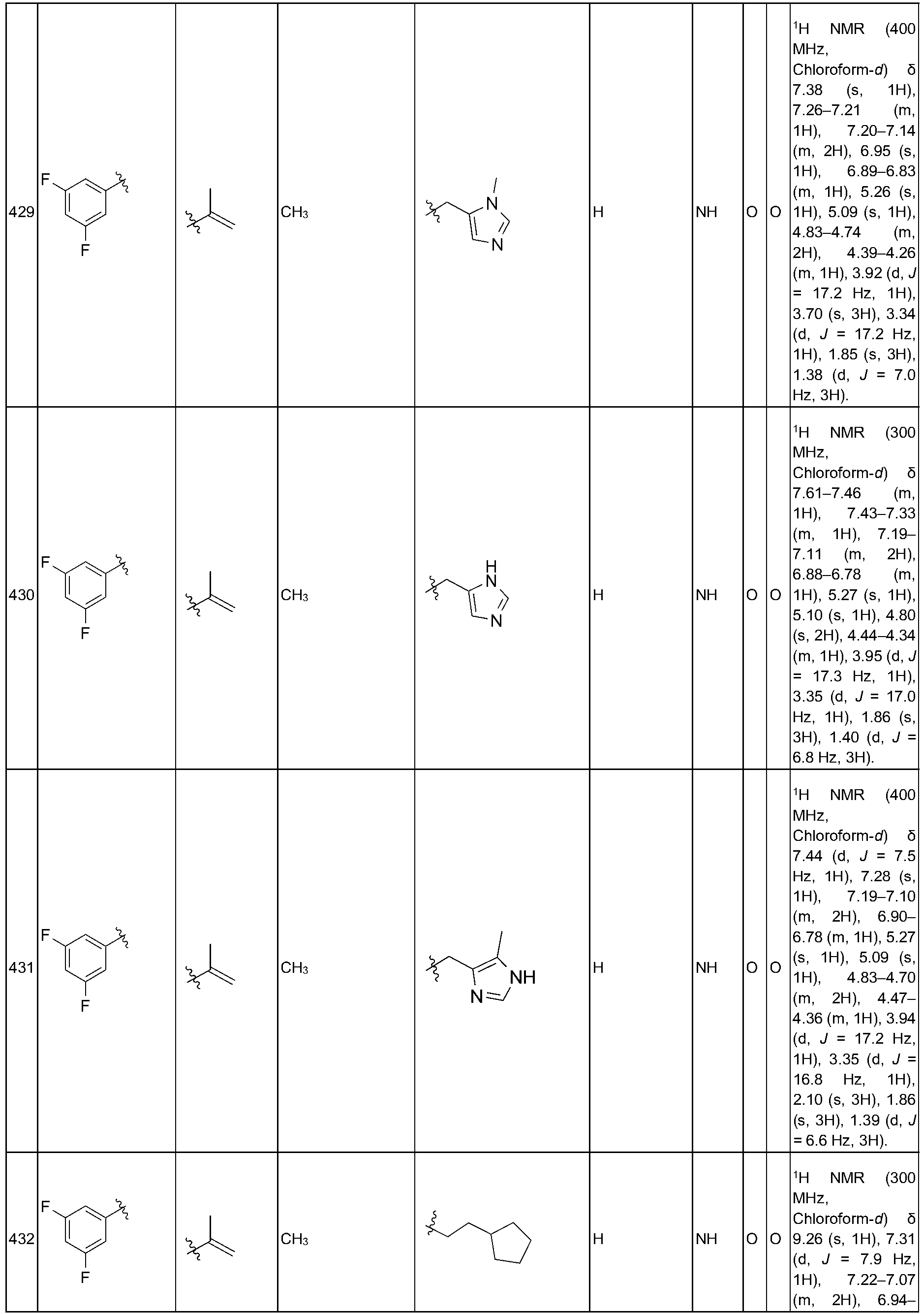

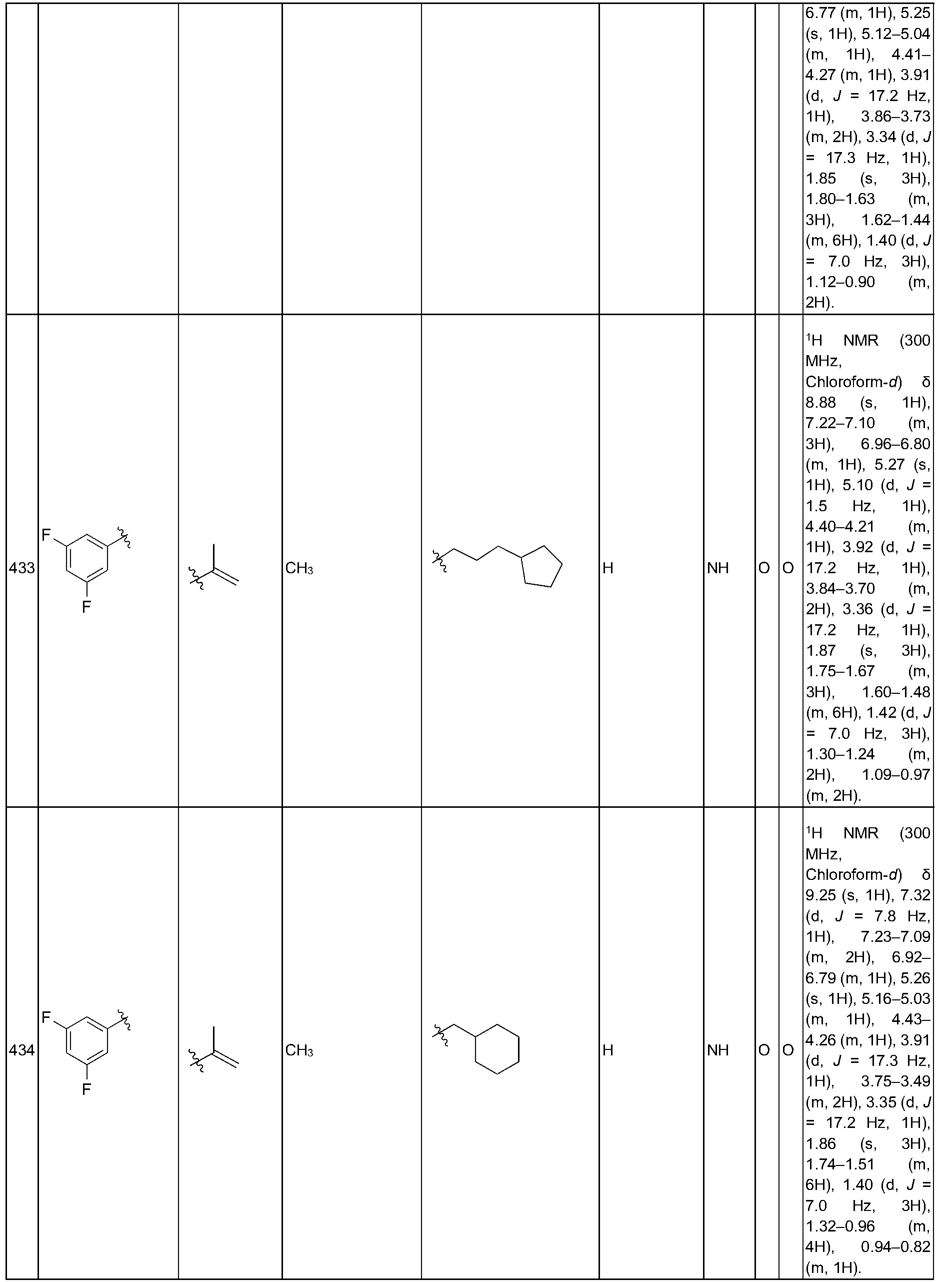

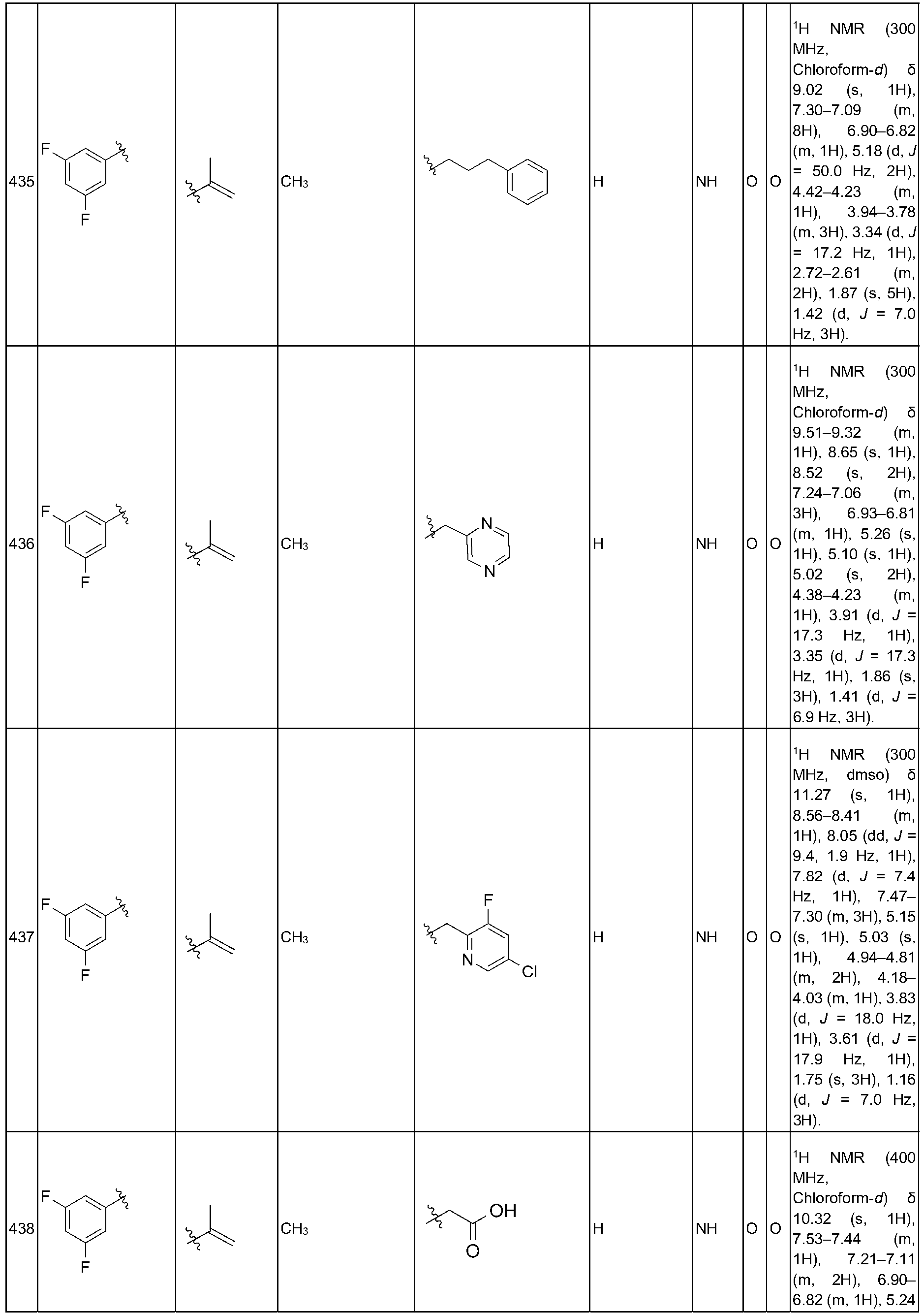

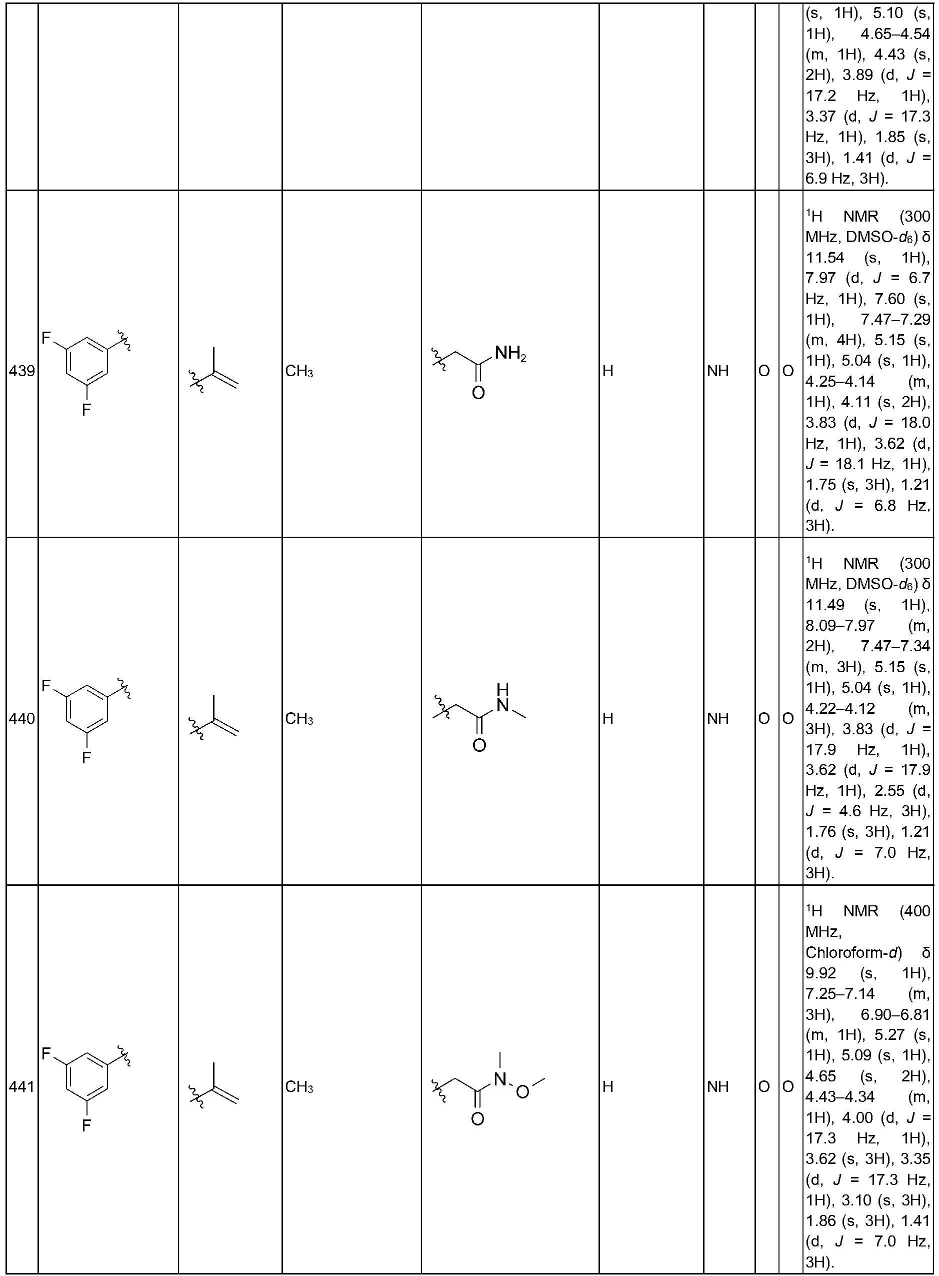

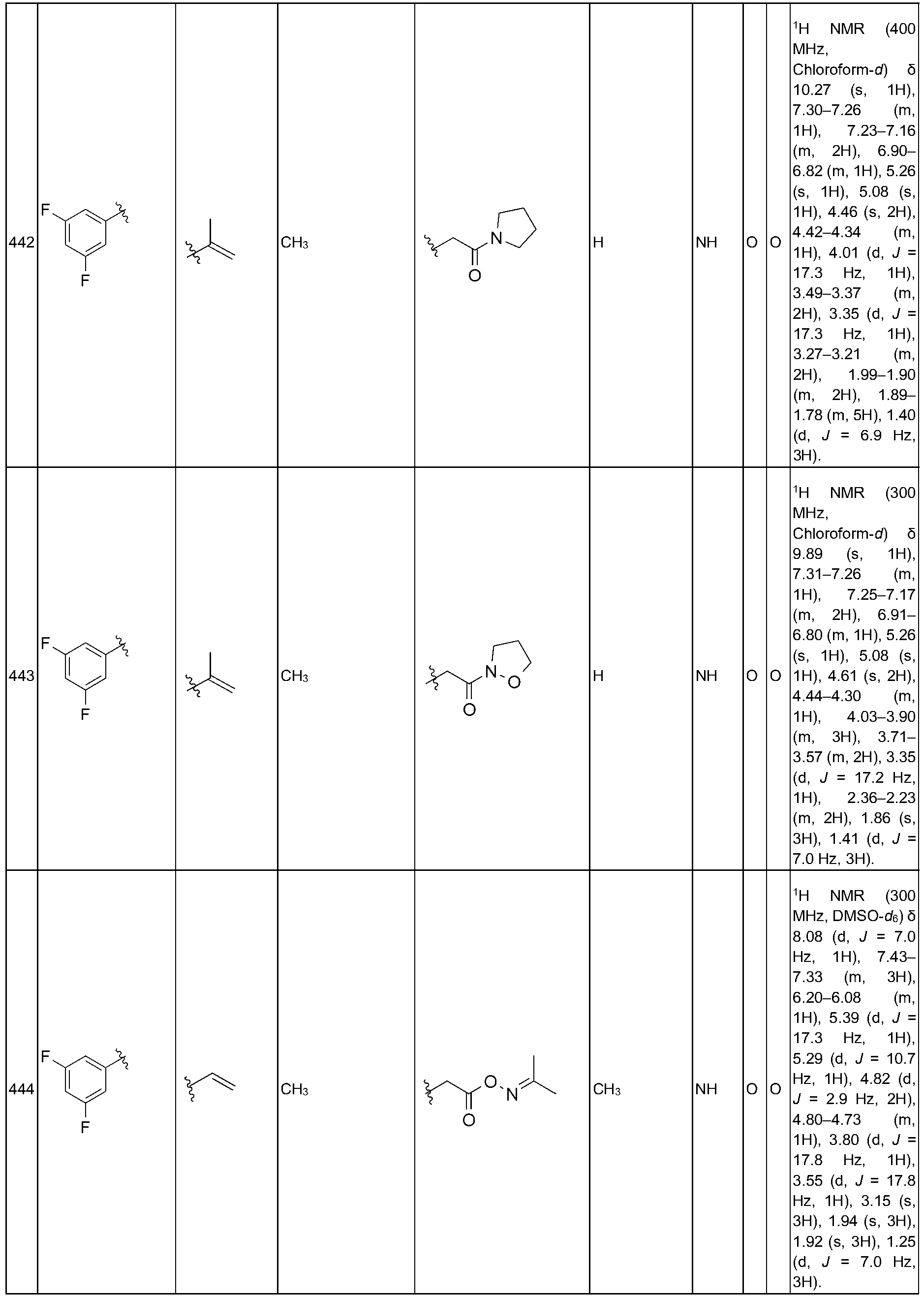

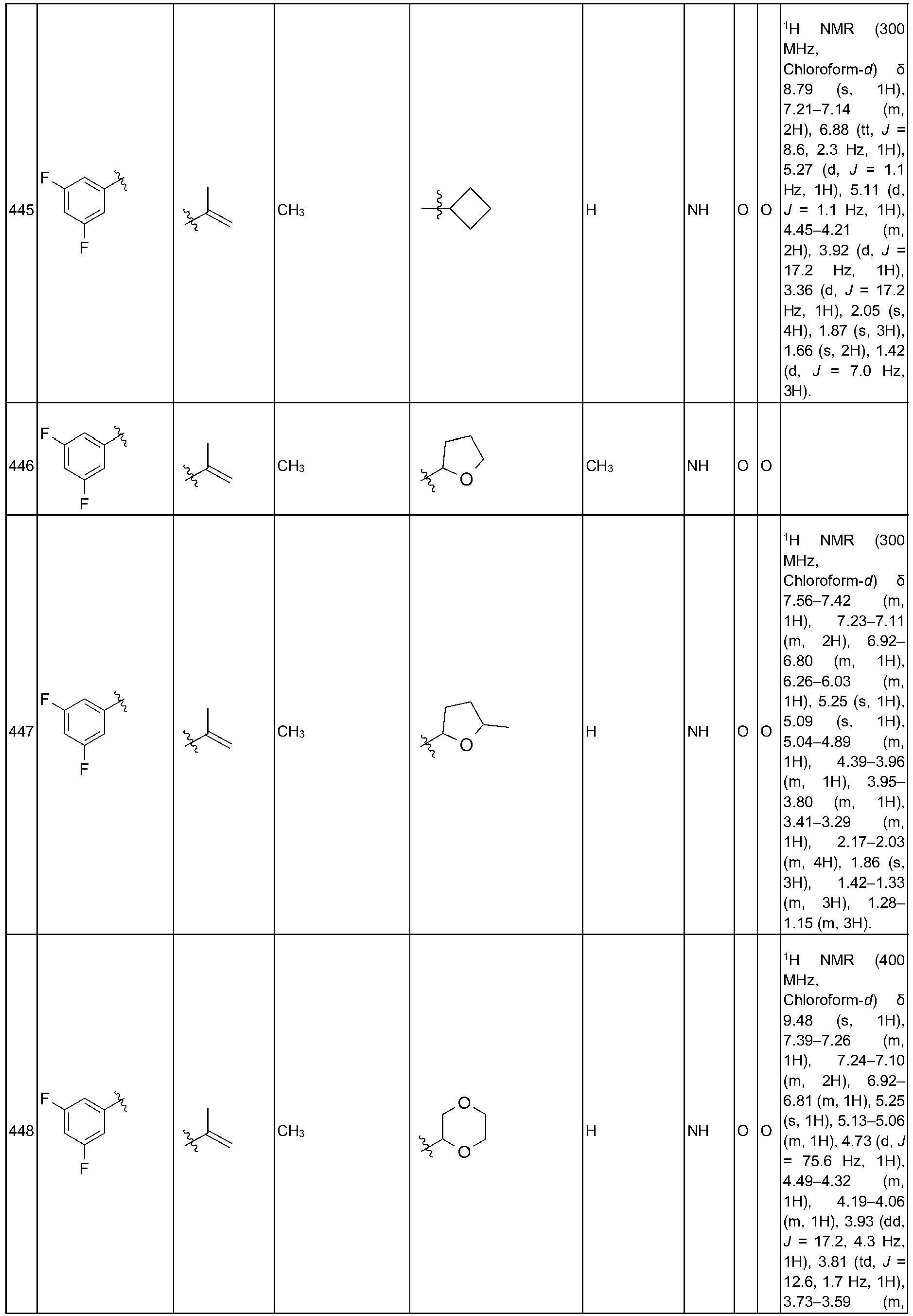

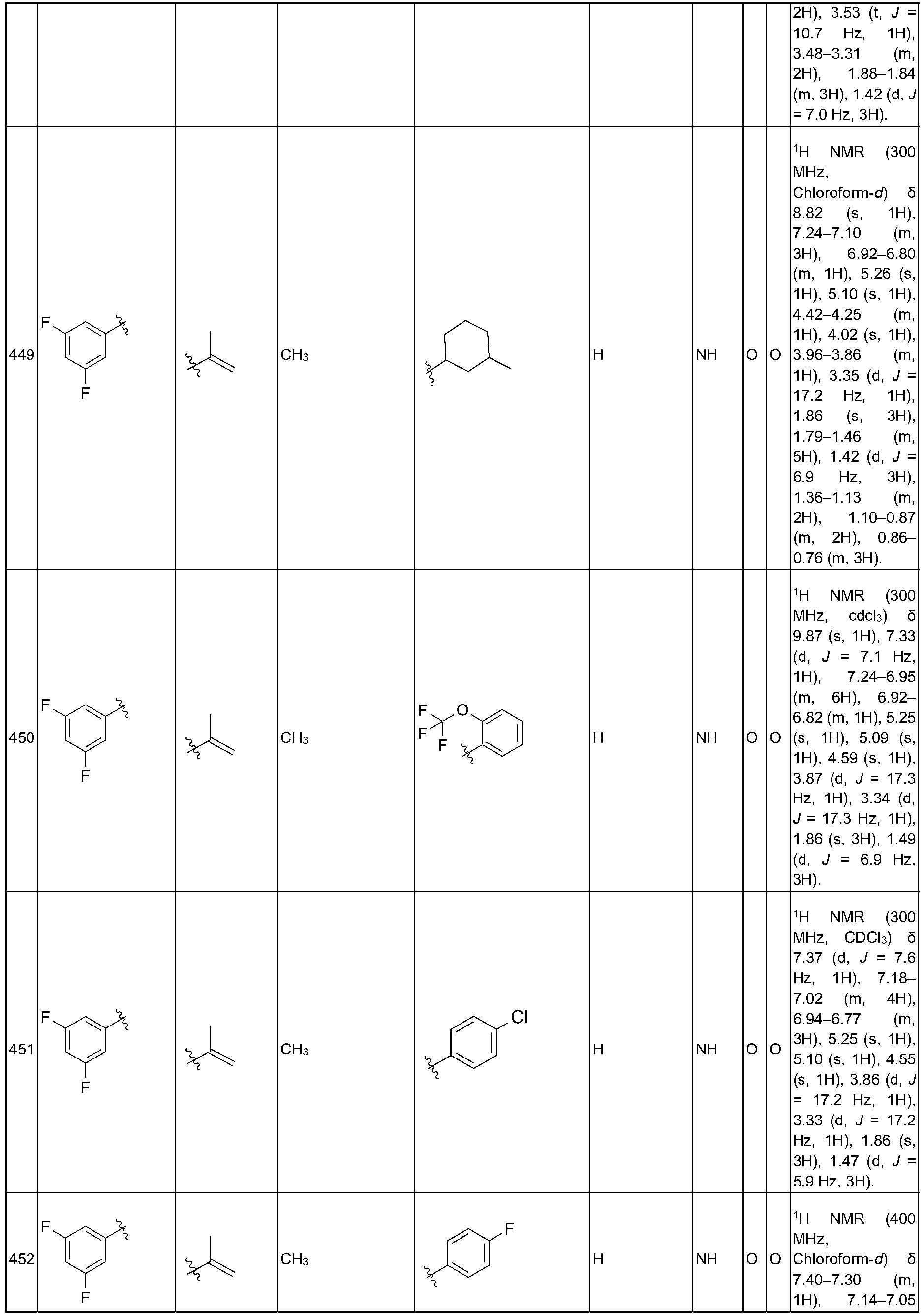

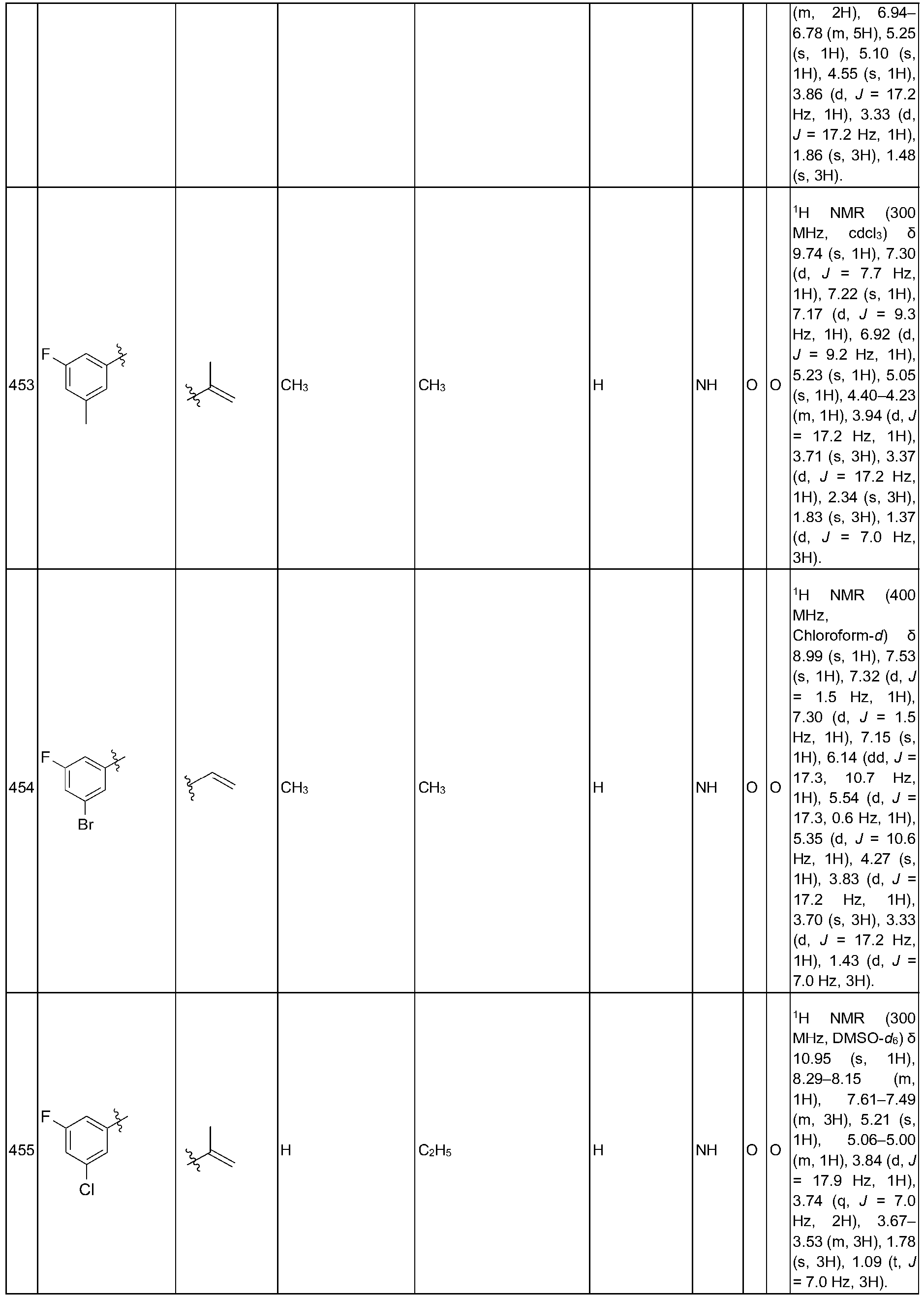

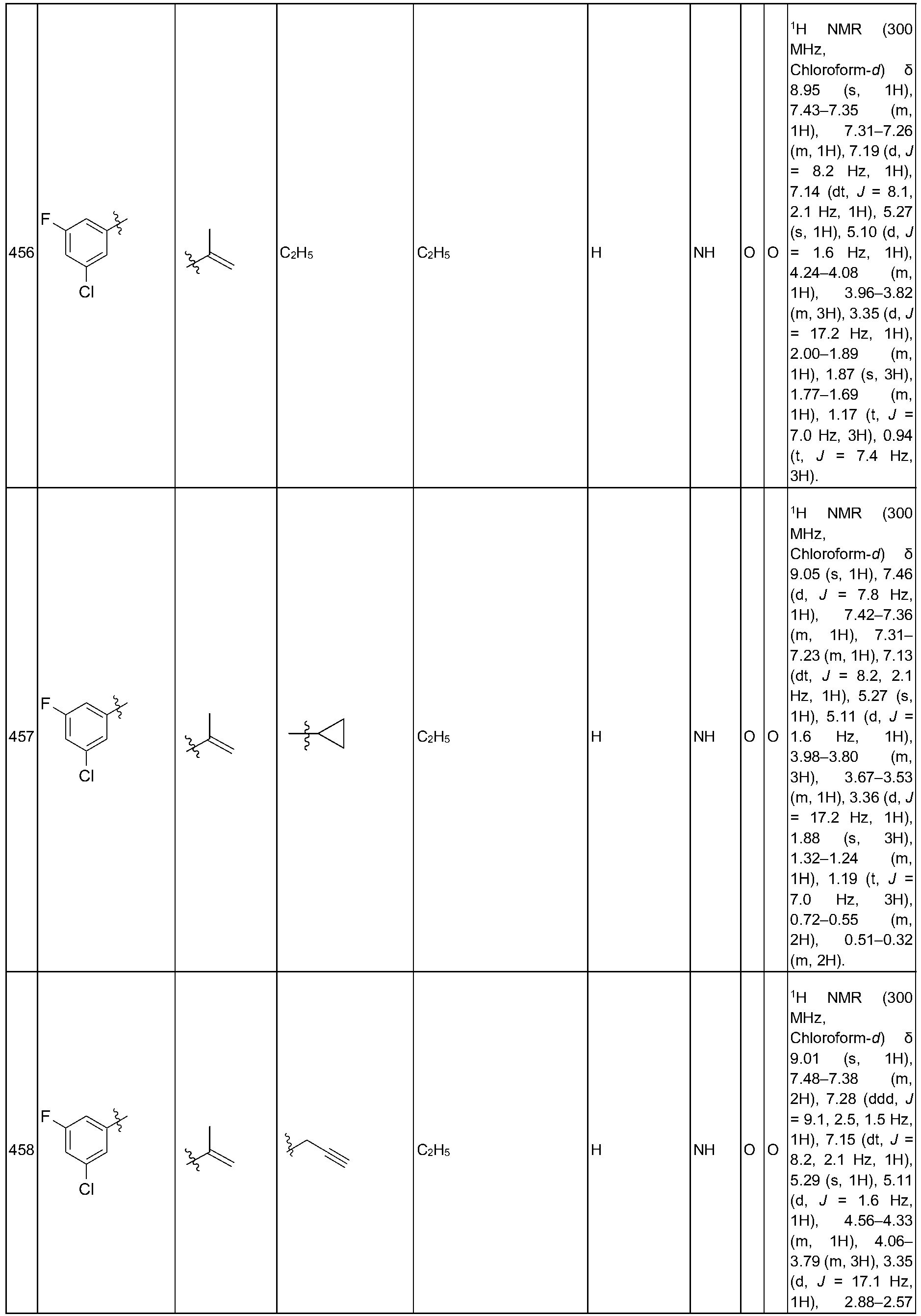

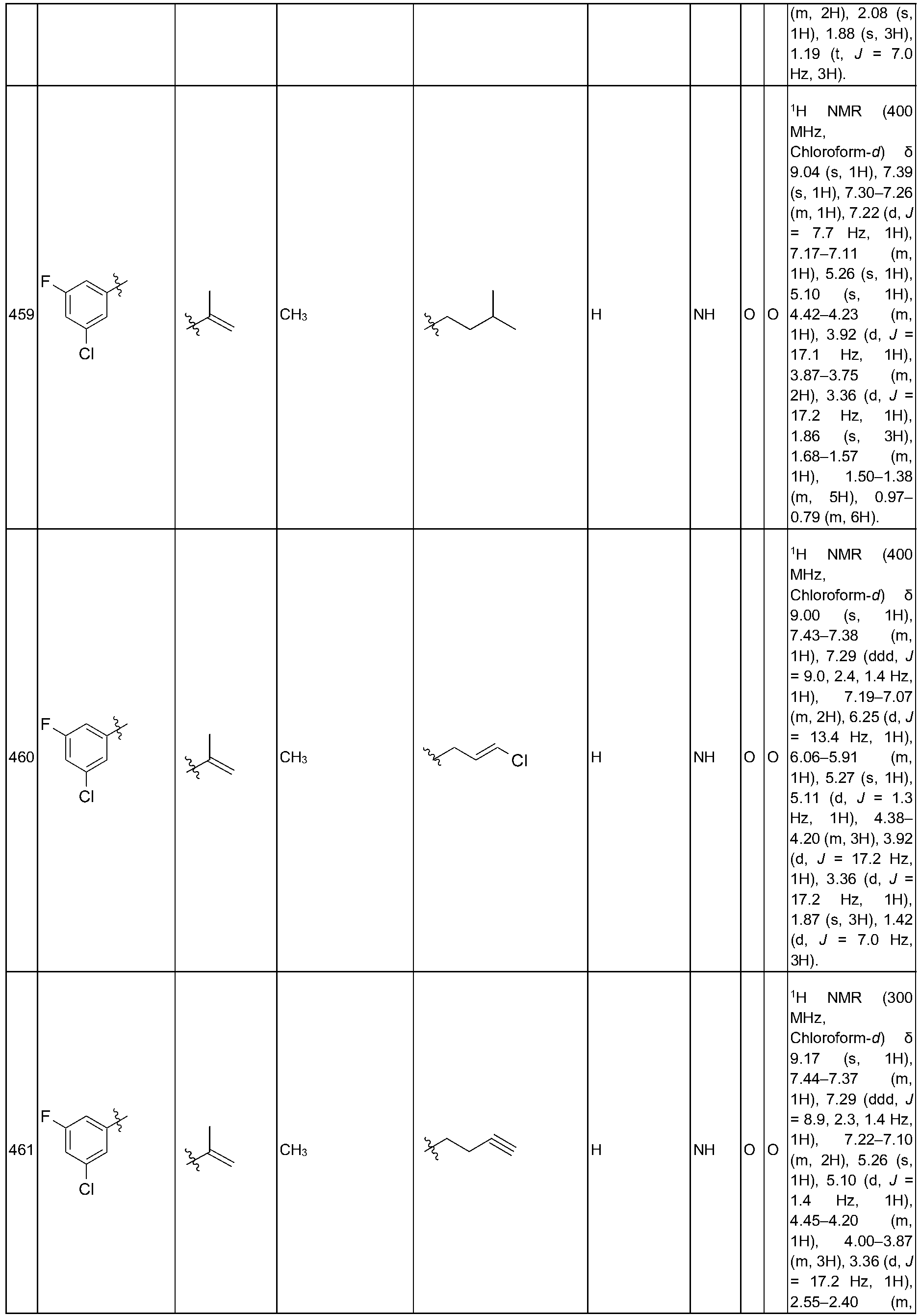

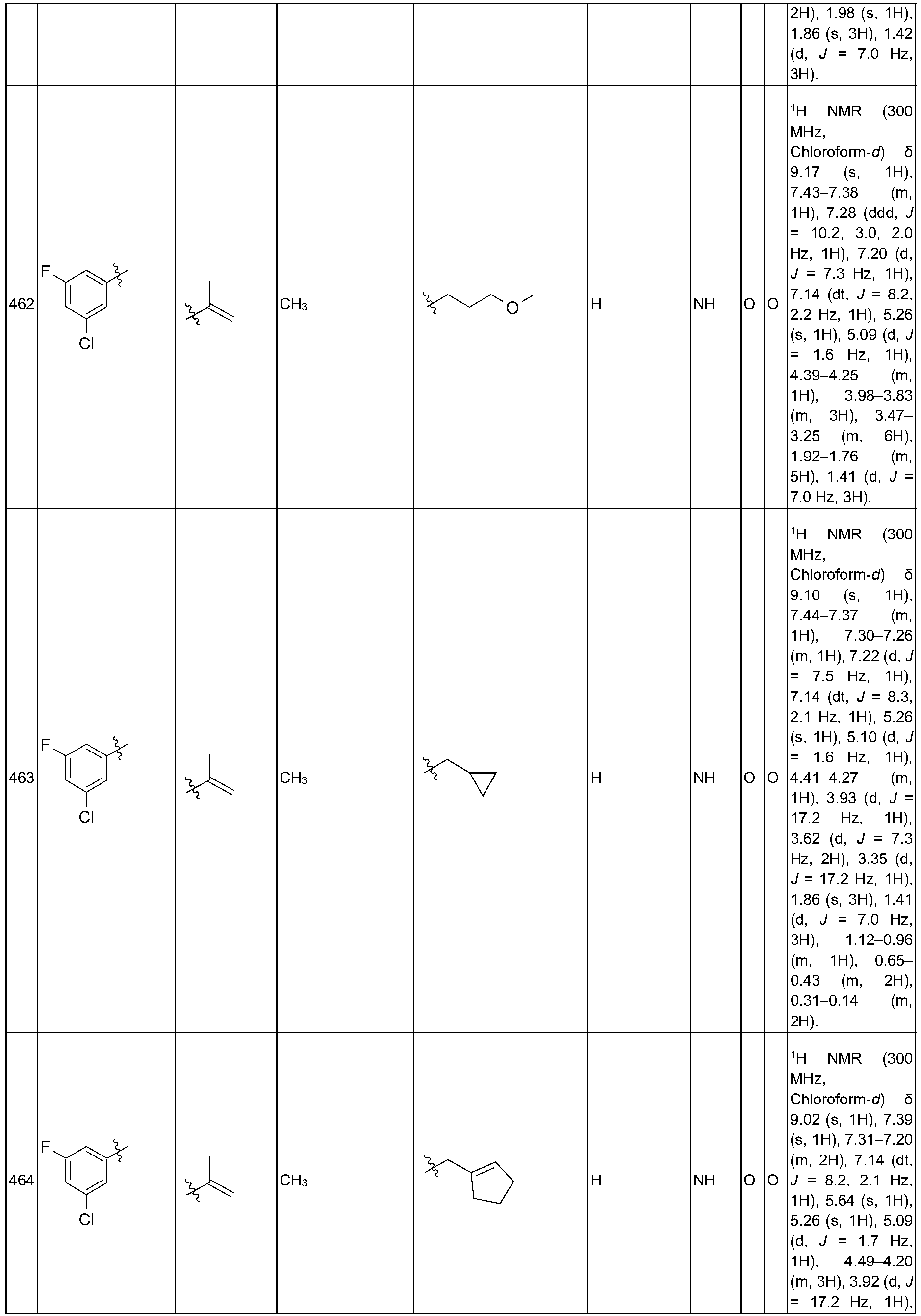

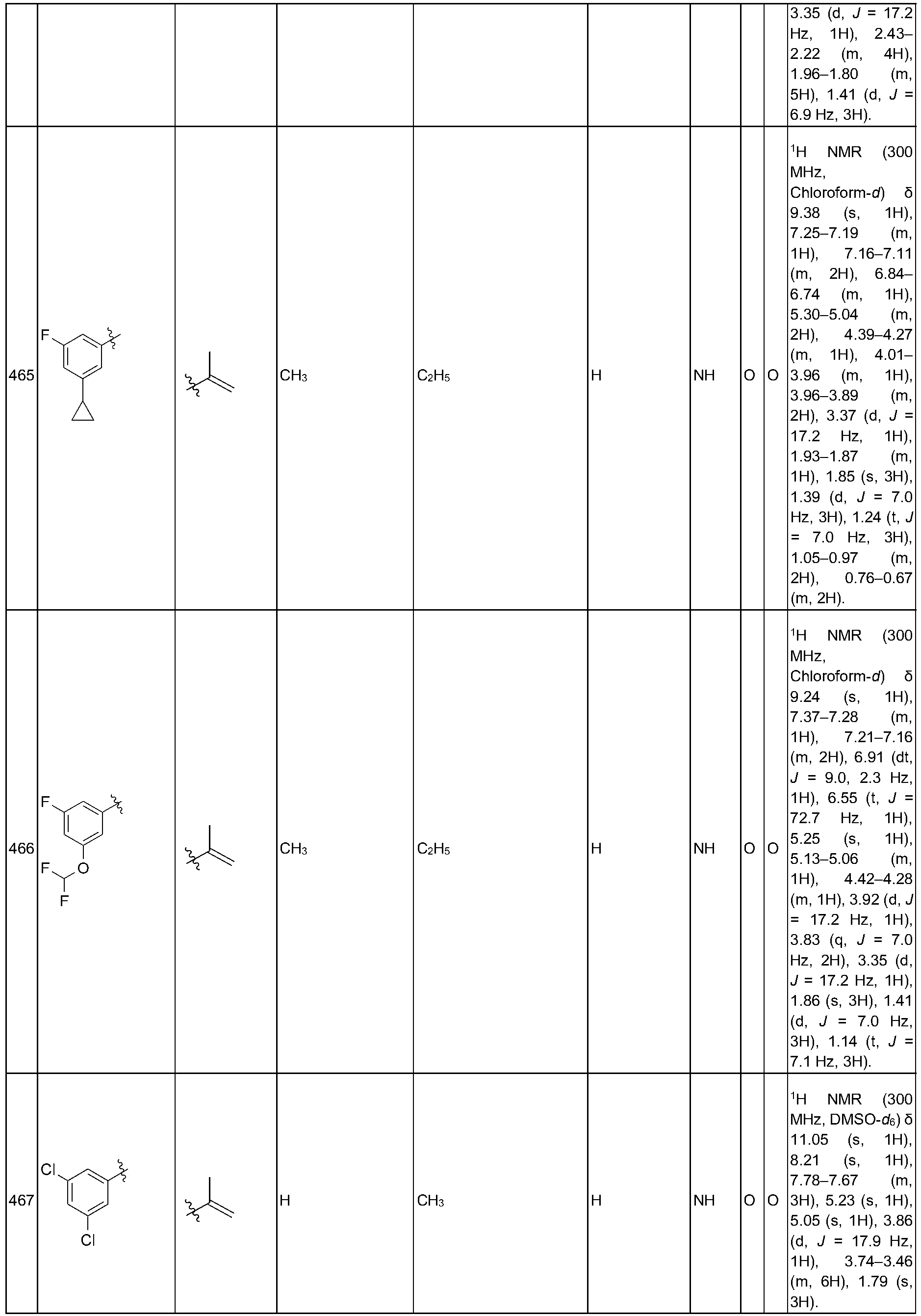

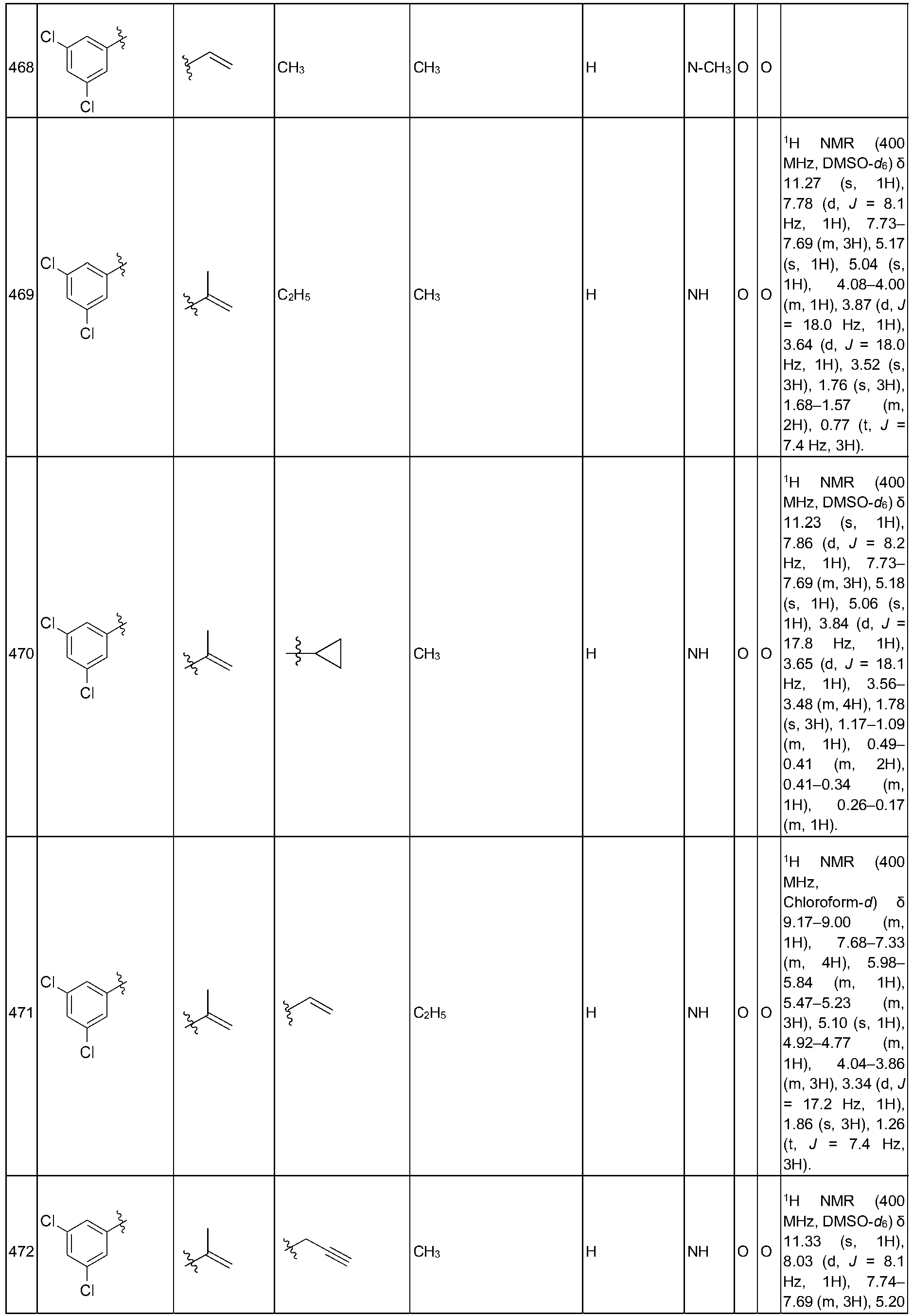

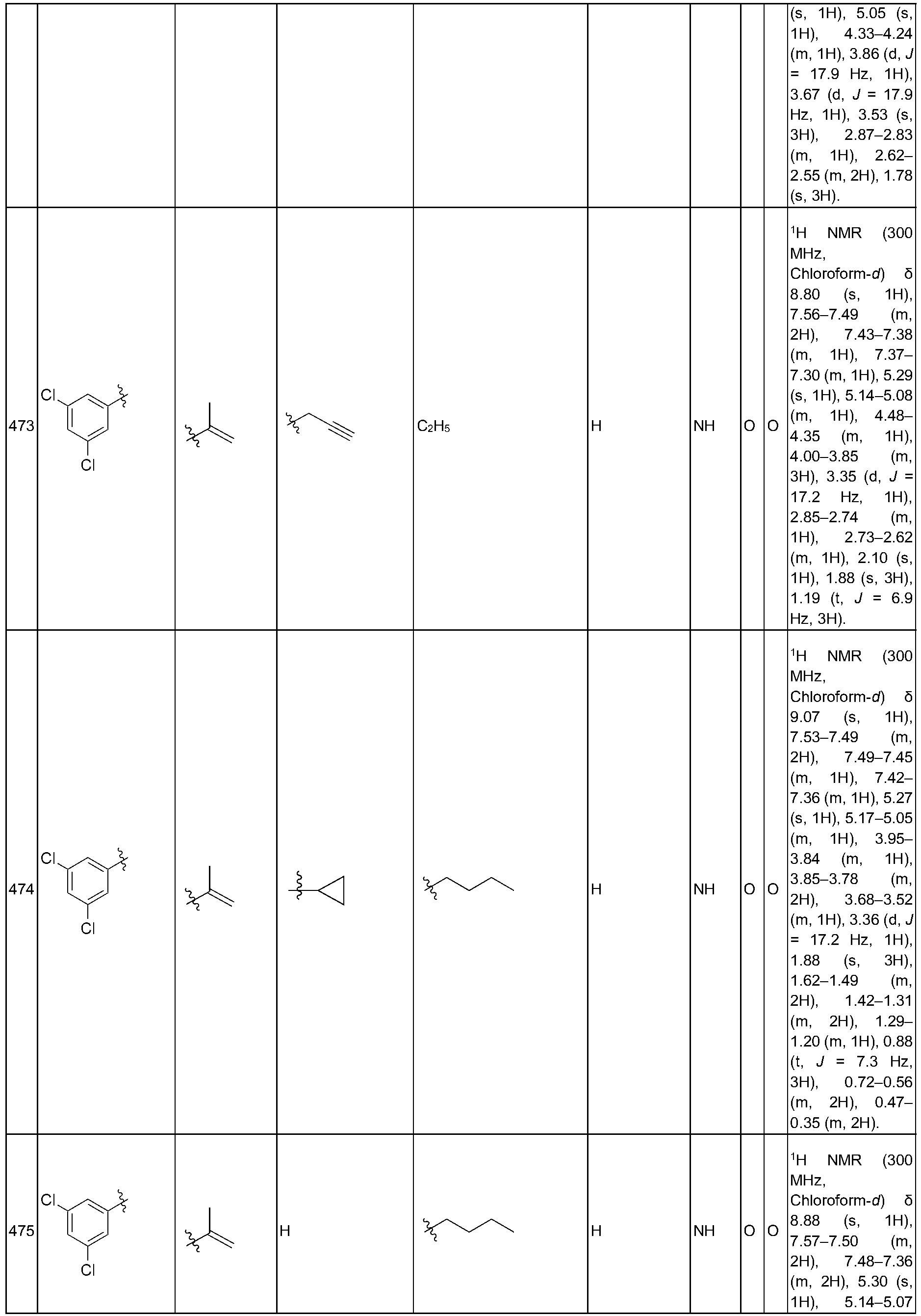

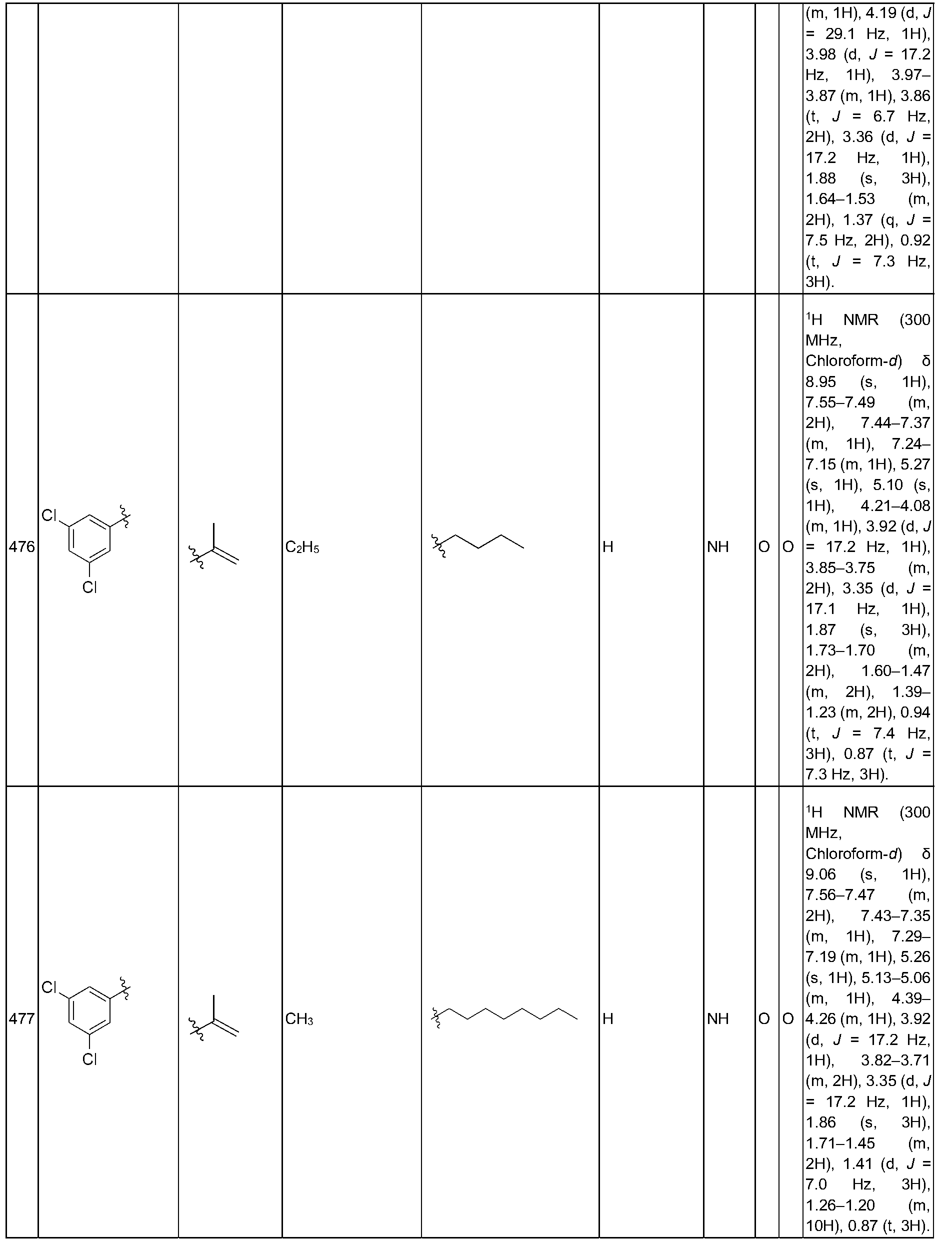

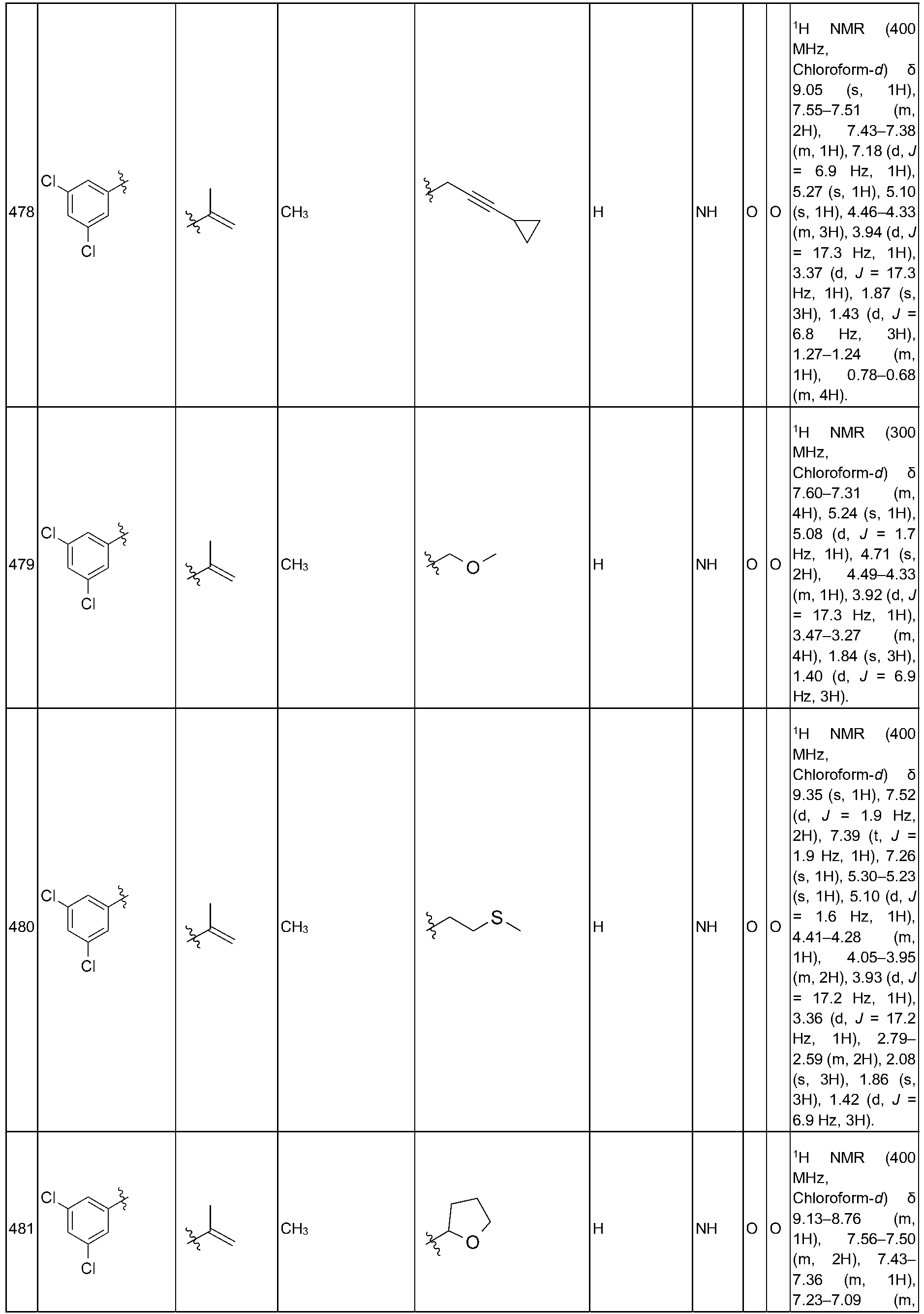

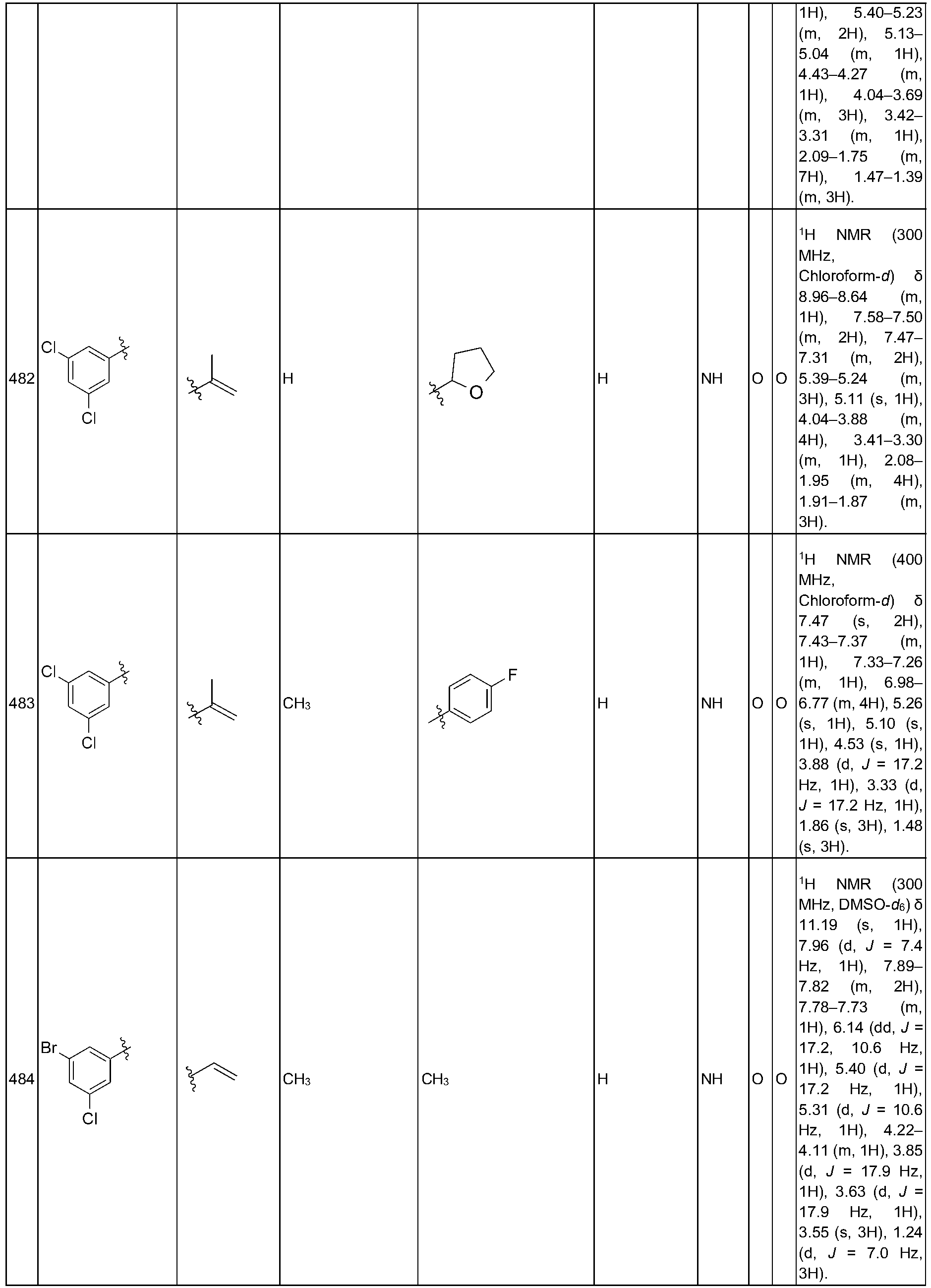

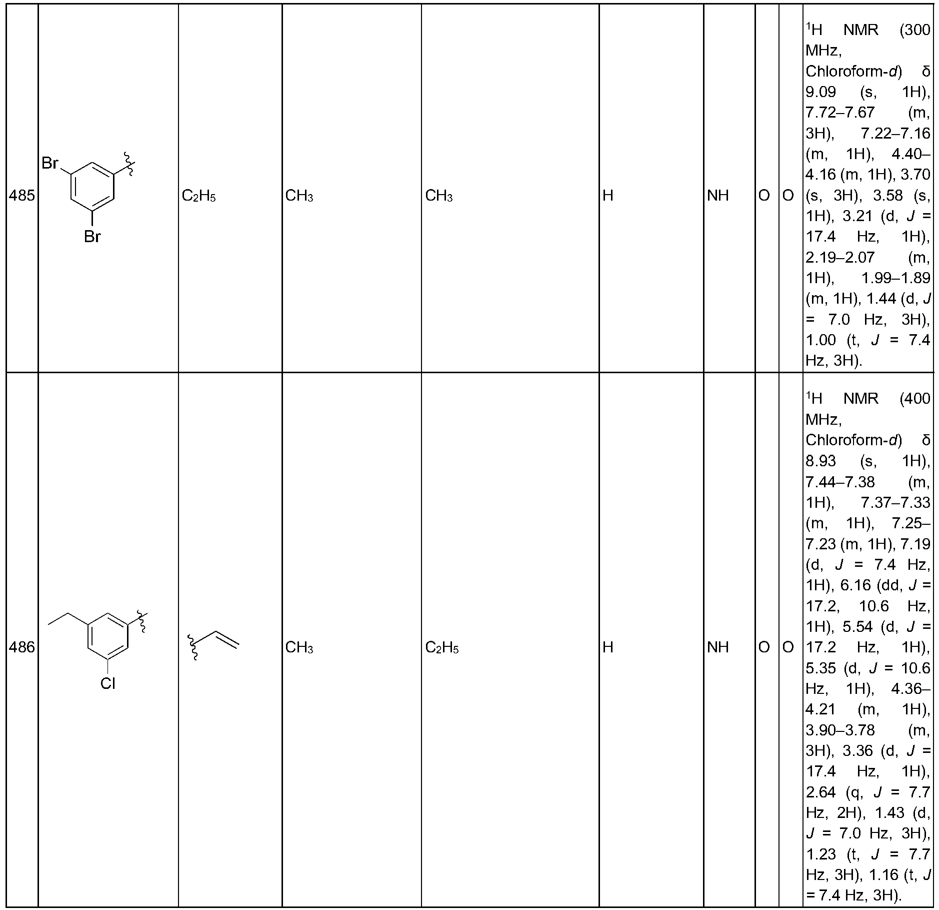

Tables A-F have the same structures as the above Table 1 does, except that the general formula I is replaced with the general formula I' 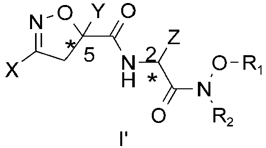 with chiral center(s). In Table A, the items below the column heading "No." are sequentially listed as 1(2R)-21(2R), 23(2R)-232(2R), 234(2R)-353(2R), 355(2R)-365(2R), 367(2R)-454(2R), 456(2R)-466(2R), 468(2R)-474(2R), 476(2R)-481(2R), 483(2R)-486(2R). For example, 1(2R) corresponds to a compound that is Compound 1 in Table 1 with R-configuration at position 2. In Table B, the items below the column heading "No." are sequentially listed as 1(2S)-21(2S), 23(2S)-232(2S), 234(2S)-353(2S), 355(2S)-365(2S), 367(2S)-454(2S), 456(2S)-466(2S), 468(2S)-474(2S), 476(2S)-481(2S), 483(2S)-486(2S). For example, 1(2S) corresponds to a compound that is Compound 1 in Table 1 with S-configuration at position 2. In Table C, the items below the column heading "No." are sequentially listed as 1(5S)-486(5S). For example, 1(5S) corresponds to a compound that is Compound 1 in Table 1 with S-configuration at position 5. In Table D, the items below the column heading "No." are sequentially listed as 1(5R)-486(5R). For example, 1(5R) corresponds to a compound that is Compound 1 in Table 1 with R-configuration at position 5. In Tables E-H, the items below the column heading "No." are sequentially listed as 1(2S,5S)-21(2S,5S), 23(2S,5S)-232(2S,5S), 234(2S,5S)-353(2S,5S), 355(2S,5S)-365(2S,5S), 367(2S,5S)-454(2S,5S), 456(2S,5S)-466(2S,5S), 468(2S,5S)-474(2S,5S), 476(2S,5S)-481(2S,5S), 483(2S,5S)-486(2S,5S), 1(2S,5R)-21(2S,5R), 23(2S,5R)-232(2S,5R), 234(2S,5R)-353(2S,5R), 355(2S,5R)-365(2S,5R), 367(2S,5R)-454(2S,5R), 456(2S,5R)-466(2S,5R), 468(2S,5R)-474(2S,5R), 476(2S,5R)-481(2S,5R), 483(2S,5R)-486(2S,5R), 1(2R,5S)-21(2R,5S), 23(2R,5S)-232(2R,5S), 234(2R,5S)-353(2R,5S), 355(2R,5S)-365(2R,5S), 367(2R,5S)-454(2R,5S), 456(2R,5S5)-466(2R,5S), 468(2R,5S)-474(2R,5S), 476(2R,5S)-481(2R,5S), 483(2R,5S)-486(2R,5S), 1(2R,5R)-21(2R,5R), 23(2R,5R)-232(2R,5R), 234(2R,5R)-353(2R,5R), 355(2R,5R)-365(2R,5R), 367(2R,5R)-454(2R,5R), 456(2R,5R)-466(2R,5R), 468(2R,5R)-474(2R,5R), 476(2R,5R)-481(2R,5R), 483(2R,5R)-486(2R,5R). For example, 1(2S,5S) corresponds to a compound that is Compound 1 in Table 1 with S-configuration at both positions 2 and 5; 1(2R,5S) corresponds to a compound that is Compound 1 in Table 1 with R-configuration at position 2 and S-configuration at position 5.

Several methods for preparing the compounds of the present invention are illustrated in detail in the following solutions and examples. Raw materials can be purchased on the market or can be prepared by methods known from literature or as shown in the detailed description. A person skilled in the art should understand that other synthetic routes may also be used to synthesize the compounds of the present invention. Although the specific raw materials and conditions in the synthetic routes have been illustrated below, they may be easily replaced with other similar raw materials and conditions. Various isomers and the like of the compounds resulted from such modifications to or variants of the preparation methods of the present invention are all included in the scope of the present invention. In addition, the preparation methods as described below can be further modified according to the disclosure of the present invention using conventional chemical methods well known to a person skilled in the art. For example, the protection for an appropriate group during the reaction, etc.

The examples of the methods provided below are used to enhance further understanding of the preparation method of the present invention. The specific substances, types and conditions used are determined to further illustrate the present invention rather than limit its reasonable scope. Reagents used in the synthesis of the compounds shown in the following table either are commercially available or can be easily prepared by a person skilled in the art.

Examples of representative compounds are as follows. The synthesis methods of other compounds are similar and will not be described in detail here.

### 1. Synthesis of Compound 19(2S)

(1) Compound 19-1 (0.2 g, 1.0 eq) was dissolved with dichloromethane in a round-bottom flask, added with Compound 19-2 (0.26 g, 1.0 eq), sequentially added with EDCI (0.75 g, 2.0 eq) and DMAP (0.11 g, 0.5 eq), reacted at room temperature overnight, and extracted with dichloromethane. The organic phase was dried over anhydrous sodium sulfate and spin-dried to obtain the crude product Compound 19-3 (0.3 g, 71% yield). 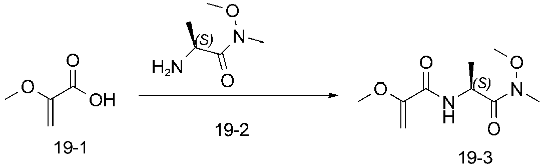
(2) Compound 19-3 (0.3 g, 1.0 eq) was dissolved with isopropanol in a round-bottom flask, sequentially added with Compound 19-4 (0.26 g, 1.0 eq) and NaHCO₃ (0.58 g, 5.0 eq), heated to 50°C, and reacted for two hours. After the reaction was completed, the reaction solution was spin-dried to remove isopropanol and extracted with ethyl acetate. The organic phase was dried over anhydrous sodium sulfate, purified through column chromatography, and spin-dried to obtain the white oily matter Compound 19(2S) (0.33 g, 94% purity, 64% yield).

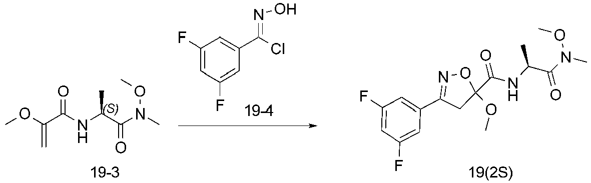

### 2. Synthesis of Compound 21(2S,5S)

(1) 400 mg of Compound 21-1 was dissolved with 10 mL of dichloromethane in a 50-mL round-bottom flask, sequentially added with triethylamine (3 eq), HOBT (1.2 eq), EDCI (1.2 eq), and Compound 21-2 (1.1 eq), and reacted at room temperature overnight. After the reaction was completed, the reaction solution was directly purified through column chromatography to obtain 480 mg of Compound 21-3 (90% yield). 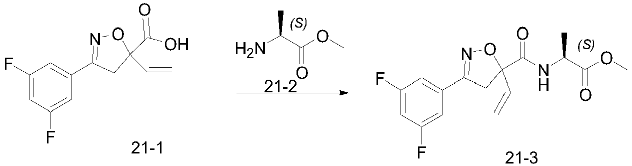
(2) 480 mg of Compound 21-3 was dissolved with 10 mL of tetrahydrofuran and 5 mL of water in a round-bottom flask, slowly added with lithium hydroxide (1.1 eq), and reacted at room temperature for 2 h. After the tetrahydrofuran was removed by spin-drying, the reaction solution was extracted with water and EA, and the organic phase was removed. The aqueous phase was adjusted to acidity (pH=2) and then extracted with DCM to obtain the product. The organic phase was washed with saturated saline solution, then dried over sodium sulfate and spin-dried to obtain 450 mg of the crude product Compound 21-4 (98% yield). 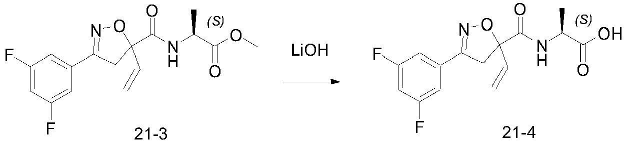
(3) 150 mg of Compound 21-4 was dissolved with 10 mL of dichloromethane in a round-bottom flask, then sequentially added with triethylamine (3 eq), HOBT (1.2 eq), EDCI (1.2 eq), and Compound 21-5 (1.1 eq), and reacted at room temperature overnight, and the reaction was completed. The reaction solution was directly purified through column chromatography to obtain 40 mg of Compound 21 (2S,5S) (25% yield).

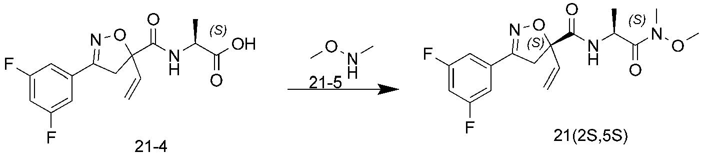

### 3. Synthesis of Compound 21(2R,5S)

(1) In a 100-mL round-bottom flask, Compound 21-1 (0.4 g, 1.0 eq), Compound 21-6 (1.5 eq), EDCI (1.5 eq), HOBT (1.5 eq), triethylamine (3 eq), and 10 mL of DCM were added, and reacted at room temperature overnight. Upon the completion of the reaction, the reaction solution was mixed with silica gel powder and purified through column chromatography, and spin-dried to obtain a white solid. Chiral resolution was then performed on the white solid to obtain Compound 21-7 (0.21 g, 97% purity, 40% yield). 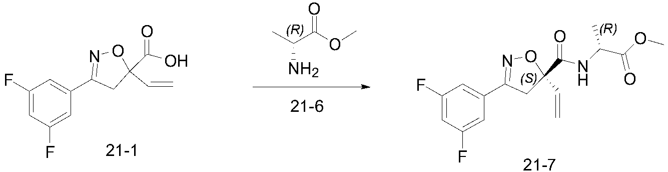
(2) In a 100-mL round-bottom flask, Compound 21-7 (0.210 g, 1.0 eq), LiOH (0.044 g, 3.0 eq), 10 mL of THF and 10 mL of water were added and reacted at room temperature for 1 h. Upon the completion of the reaction, the reaction solution was spin-dried to remove THF, adjusted to acidity with dilute hydrochloric acid, and extracted with ethyl acetate. The organic phase was dried over anhydrous sodium sulfate and spin-dried to obtain the white solid Compound 21-8 (0.15 g, 97% purity, 74% yield). 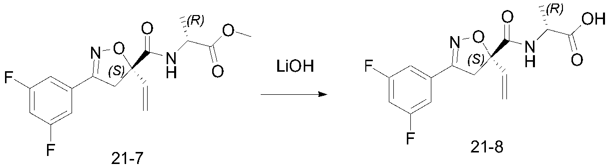
(3) In a 100-mL round-bottom flask, Compound 21-8 (0.150 g, 1.0 eq), Compound 21-5 (0.042 g, 1.5 eq), EDCI (0.132 g, 1.5 eq), HOBT (0.093 g, 1.5 eq), triethylamine (0.140 g, 3 eq), and 10 mL of DCM were added, and reacted at room temperature overnight. Upon the completion of the reaction, the reaction solution was mixed with silica gel powder and purified through column chromatography, and spin-dried to obtain the white solid Compound *21(2R,5S)* (0.090 g, 97% purity, 51% yield).

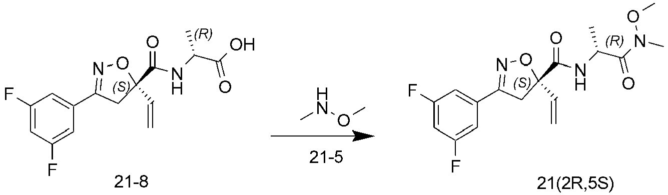

### 4. Synthesis of Compound 20(2R)

Compound 20-1 was prepared with reference to Compound 21-4.

In a 100-mL round-bottom flask, Compound 20-1 (0.120 g, 1.0 eq), Compound 20-2 (0.026 g, 1.5 eq), EDCI (0.106 g, 1.5 eq), HOBT (0.075 g, 1.5 eq), triethylamine (0.102 g, 3 eq), and 10 mL of DCM were added, and reacted at room temperature overnight. Upon the completion of the reaction, the reaction solution was mixed with silica gel powder and purified through column chromatography, and spin-dried to obtain the white oily Compound 20(2R) (0.084 g, 93% purity, 64% yield).

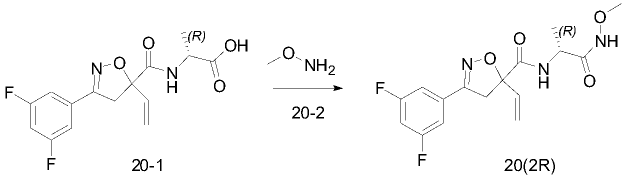

### 5. Synthesis of Compound 68(2R)

(1) In a 100-mL round-bottom flask, Compound 68-1 (0.400 g, 1.0 eq), Compound 21-6 (0.145 g, 1.0 eq), EDCI (0.325 g, 1.5 eq), HOBT (0.284 g, 1.5 eq), triethylamine (0.430 g, 3 eq), and 10 mL of DCM were added, and reacted at room temperature overnight. Upon the completion of the reaction, the reaction solution was mixed with silica gel powder and purified through column chromatography, and spin-dried to obtain the white solid Compound 68-2 (0.3 g, 97% purity, 56% yield). 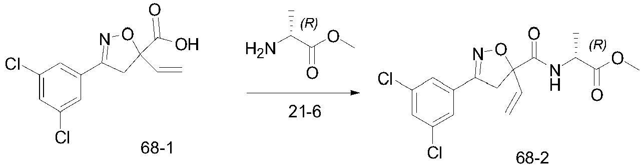
(2) In a 100-mL round-bottom flask, Compound 68-2 (0.240 g, 1.0 eq), LiOH (0.400 g, 2.0 eq), 10 mL of THF and 10 mL of water were added and reacted at room temperature for 1 h. Upon the completion of the reaction, the reaction solution was spin-dried to remove THF, adjusted to acidity with dilute hydrochloric acid, and extracted with ethyl acetate. The organic phase was dried over anhydrous sodium sulfate and spin-dried to obtain the white solid Compound 68-3 (0.18 g, 97% purity, 80% yield). 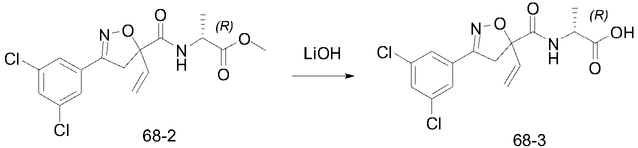
(3) In a 100-mL round-bottom flask, Compound 68-3 (0.180 g, 1.0 eq), Compound 21-5 (0.030 g, 1.0 eq), EDCI (0.145 g, 1.5 eq), HOBT (0.102 g, 1.5 eq), triethylamine (0.155 g, 3 eq), and 10 mL of DCM were added, and reacted at room temperature overnight. Upon the completion of the reaction, the reaction solution was mixed with silica gel powder and purified through column chromatography, and spin-dried to obtain the white solid Compound 68(2S) (0.034 g, 96% purity, 17% yield).

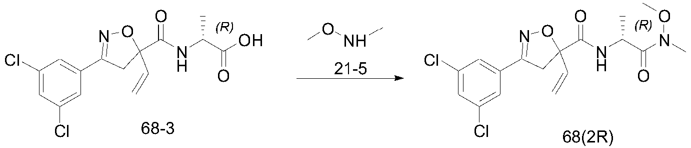

### 6. Synthesis of Compound 115(2S,5S)

(1) Compound 115-1 (1.0 g, 3.95 mmol) and Compound 115-2 (1.1 g, 5.13 mmol) were dissolved in 30 mL of dichloromethane, added with triethylamine (0.8 g, 7.90 mmol), slowly added with HOBT (0.8 g, 5.92 mmol) and EDCI (1.1 g, 5.92 mmol), and reacted at room temperature for 2 h. LCMS detected the completion of the reaction. The reaction solution was diluted with 30 mL of dichloromethane. The organic phase was washed with water and saturated saline solution, dried, and concentrated. The residue was purified through column chromatography to obtain Compound 115-3 (315 mg, 19% yield). 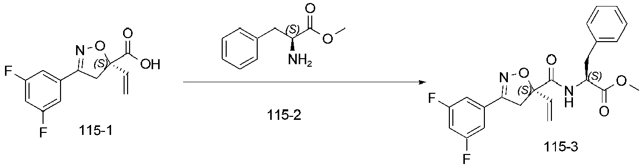
(2) Compound 115-3 (315 mg, 0.76 mmol) was dissolved in 5 mL of THF, and at room temperature added with 5 mL of aqueous solution of lithium hydroxide (64 mg, 1.52 mmol). LCMS detected the completion of the reaction. The reaction solution was adjusted to pH=5 using dilute hydrochloric acid and extracted with 20 mL of water and 20 mL of EA. The organic phase was dried and concentrated to obtain the crude Compound 115-4 (280 mg, 92% yield). 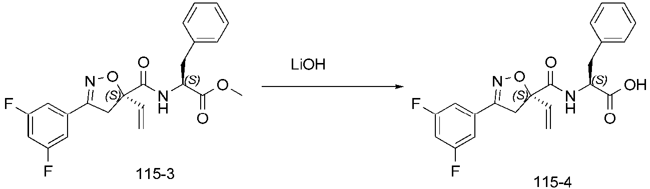
(3) Compound 115-4 (280 mg, 0.7 mmol) was dissolved in 10 mL of DMF, added with Compound 21-5 (68 mg, 1.4 mmol), added with DIPEA (450 mg, 3.5 mmol) and HATU (532 mg, 1.4 mmol), and at room temperature stirred for 2. LCMS detected the completion of the reaction. The reaction solution was diluted with EA. The organic phase was washed with water and saturated saline solution, dried, and concentrated. The residue was purified through column chromatography to obtain Compound 115(2S,5S) (70 mg, 23% yield).

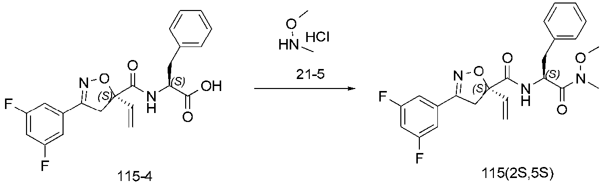

### 7. Synthesis of Compound 125(2S,5S)

Compound 125-2 was prepared with reference to the aforementioned Compound 21-8. Compound 125-2 (200 mg, 0.62 mmol) was dissolved in 5 mL of dichloromethane, added with triethylamine (354 mg, 1.85 mmol), EDCI (177.35 mg, 0.93 mmol), HOBT (354 mg, 0.93 mmol) and Compound 125-1 (63.63 mg, 0.62 mmol), and reacted at room temperature for 4 h. LCMS detected the completion of the reaction. The reaction solution was diluted with water. The aqueous phase was extracted with dichloromethane. The organic phase was dried and concentrated. The residue was purified through column chromatography (EA:PE = 1:2) to obtain Compound 125(2S,5S) (160 mg, 63% yield, pale yellow oily matter).

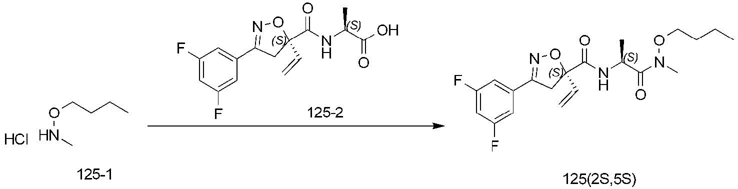

### 8. Synthesis of Compound 164(2S,5S)

(1) Compound 164-1 (7.00 g, 26.21 mmol) was dissolved in 50 mL of dichloromethane, at room temperature added with oxalyl chloride (3.30 mL, 39.31 mmol) and a catalytic amount of N,N-dimethylformamide, and at room temperature reacted for 2 h. LCMS detected the completion of the reaction. The reaction solution was concentrated for later use. Compound 164-2 (4.39 g, 34.07 mmol) was dissolved in 40 mL of tetrahydrofuran, at -78°C and under nitrogen gas protection, added dropwise with *n*-butyl lithium solution (12.58 mL, 31.45 mmol, 2.5 mol/L in THF), and reacted at -78°C for 1 h. The prepared acyl chloride was dissolved in 20 mL of tetrahydrofuran, at -78°C added dropwise to the above-mentioned system and reacted for 2 h. LCMS detected the completion of the reaction. The reaction solution was added with aqueous solution of ammonium chloride to quench the reaction and extracted with ethyl acetate. The organic phase was washed using saturated saline solution, dried and concentrated. The residue was purified through column chromatography (EA:PE = 1:5) to obtain Compound 164-3 (2.30 g, 46% yield, white solid). 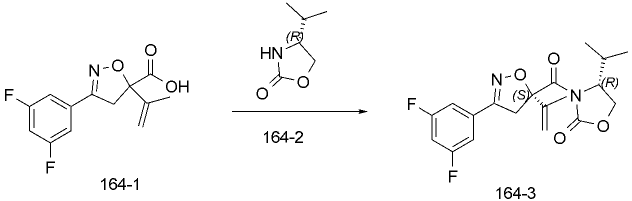
(2) Compound 164-3 (2.30 g, 6.08 mmol) was dissolved in 15 mL of tetrahydrofuran, in ice bath added with 30% hydrogen peroxide (2.75 g, 24.30 mmol) and lithium hydroxide (0.14 g, 6.08 mmol), and reacted for 30 min at room temperature. LCMS detected the completion of the reaction. The reaction solution was diluted with water. The aqueous phase was adjusted to acidity and extracted with ethyl acetate. The organic phase was washed with saturated saline solution, dried and concentrated to obtain Compound 164-4 (1.6 g, 98% yield, white solid). 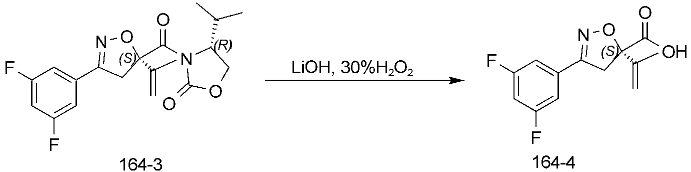
(3) Compound 164-4 (200 mg, 0.75 mmol) was dissolved in 2 mL of dichloromethane, at room temperature sequentially added with triethylamine (251 mg, 1.50 mmol), hydrochloride of Compound 19-2 (189 mg, 1.13 mmol), HOBT (152 mg, 1.13 mmol), and EDCI (216 mg, 1.13 mmol), and reacted at room temperature for 3 h. LCMS detected the completion of the reaction. The reaction solution was diluted with water and ethyl acetate. The organic phase was washed with saturated aqueous solution of ammonium chloride and saturated saline solution, dried, and concentrated. The residue was purified through column chromatography (EA:PE = 1:3) to obtain Compound 164(2S,5S) (210 mg, 73 % yield, white solid).

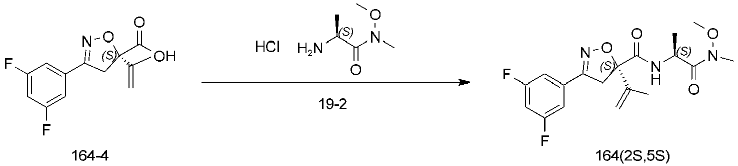

### 9. Synthesis of Compound 203(2S,5R)

(1) Compound 115-1 (160 mg, 0.63 mmol) was dissolved in 5 mL of methanol, and under nitrogen gas protection, added with 10% Pd/C (13 mg, 0.12 mmol). The air in the system was replaced with hydrogen gas, and the system reacted at room temperature for 2 h. The completion of the reaction was detected by LCMS. The reaction solution was filtered and concentrated to obtain the crude Compound 203-1 (150 mg, 90% yield, white oily matter) which was directly used in the next step. 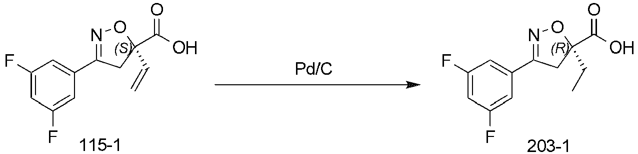
(2) Compound 203-1 (150 mg, 0.59 mmol) was dissolved in 2 mL of dichloromethane, sequentially added with triethylamine (178 mg, 1.76 mmol), Compound 203-2 (83 mg, 0.71 mmol), HOBT (119 mg, 0.88 mmol), and EDCI (169 mg, 0.88 mmol), and reacted at room temperature for 2 h. The completion of the reaction was detected by LCMS. The reaction solution was diluted with water and dichloromethane, and extracted with dichloromethane. The organic phase was washed with saturated saline solution, dried over anhydrous sodium sulfate and concentrated. The residue was purified through column chromatography (EA:PE = 1:1) to obtain Compound 203*(2S,5R)* (74 mg, 32% yield, pale yellow oily matter).

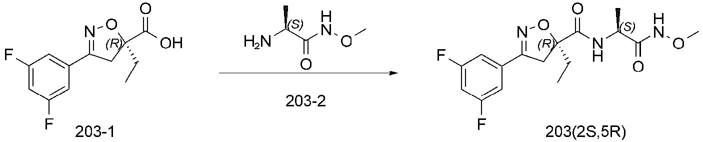

### 10. Synthesis of Compound 227(2S)

(1) Compound 19-4 (12.70 g, 66.30 mmol), Compound 227-1 (12.43 g, 86.19 mmol), NaHCO₃ (27.85 g, 331.49 mmol) and 70 mL of isopropanol were added in a 500-mL single-necked flask, heated to 50°C and stirred, and reacted for 2 h. LC-MS detected a main peak which indicated the product. The reaction solution was spin-dried to remove the solvent, extracted with ethyl acetate and water, mixed with silica gel powder and purified through column chromatography (PE/EA = 23%) to obtain Compound 227-2 (17 g, 85.6% yield). 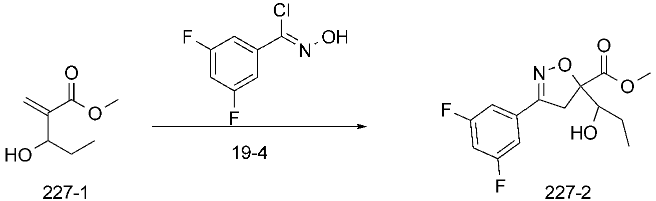
(2) Compound 227-2 (2.00 g, 6.68 mmol), 50 mL of DCM and pyridine (1.06 g, 13.37 mmol) were added in a 250-mL single-necked flask, in ice bath stirred for 15 min, then slowly added with Compound 227-3 (2.26 g, 8.02 mmol), and reacted at room temperature for 2 h. LC-MS detected a main product peak. The reaction solution was extracted with DCM and water and spin-dried to obtain 2 g of the crude Compound 227-4 with a yield of 69.4%. 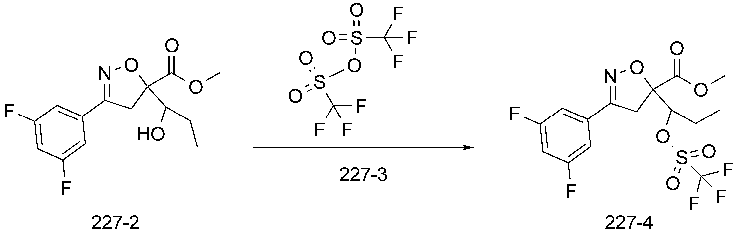
(3) Compound 227-4 (2.00 g, 4.64 mmol), 40 mL of dichloromethane and DBU (1.41 g, 9.27 mmol) were added in a 250-mL single-necked flask, and reacted at room temperature for 1 h. LC-MS detected a main product peak. The reaction solution was extracted with DCM and water, mixed with silica gel powder and purified through column chromatography (PE/EA = 45%) to obtain the product Compound 227-5 (1.13 g, 86.9% yield). 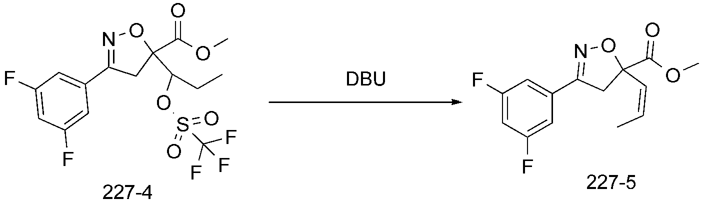
(4) Compound 227-5 (1.13 g, 4.02 mmol) and 30 mL of THF were added in a 250-mL single-necked flask, at room temperature stirred for 10 min, added with a solution obtained by dissolving lithium hydroxide (288.63 mg, 12.05 mmol) in 10 mL of water, and reacted for 2.5 h. LC-MS detected a main product peak. The reaction solution was adjusted to pH of 3-5 using hydrochloric acid, extracted with EA and water, mixed with silica gel powder, and purified through column chromatography (PE/EA = 50%) to obtain Compound 227-6 (900 mg, 84.1% yield). 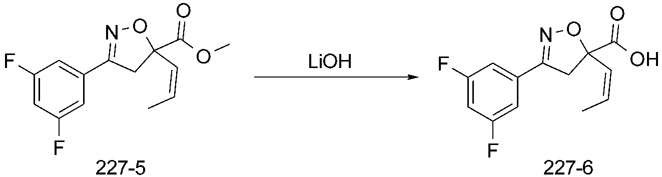
(5) Compound 227-6 (200 mg, 748.41 µmol), Compound 227-7 (148.37 mg, 1.12 mmol), triethylamine (227.20 mg, 2.25 mmol), HOBT (202.26 mg, 1.50 mmol) and 20 mL of DCM were added in a 100-mL single-necked flask, stirred at room temperature for 10 min, added with EDCI (232.38 mg, 1.50 mmol), and reacted overnight. LC-MS detected a main product peak. The reaction solution was mixed with silica gel powder and purified through column chromatography (PE/EA = 40%) to obtain Compound 227*(2S)* (127 mg, 44.5% yield).

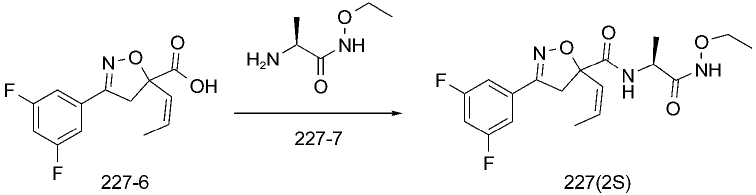

### 11. Synthesis of Compound 274(2S,5S)

(1) At 0°C, Compound 274-1 (0.5 g, 2.64 mmol) was dissolved in 10 mL of DCM, added with triethylamine (0.534 g, 5.29 mmol), Compound 274-2 (0.459 g, 3.16 mmol), EDCI (0.759 g, 3.96 mmol), and HOBT (0.534 g, 3.96 mmol), and reacted at room temperature for 4 h. The completion of the reaction was detected by LCMS. The reaction solution was quenched by saturated aqueous solution of ammonium chloride and extracted with DCM. The organic phase was purified through column chromatography (THF/PE = 1/3) to obtain pale yellow oily Compound 274-3 (700 mg, 95.8% yield). 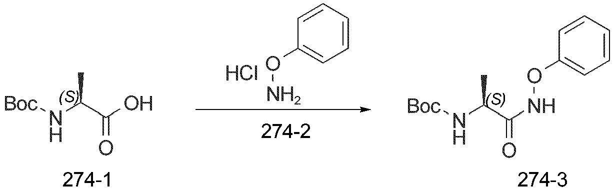
(2) At 0°C, Compound 274-3 (700 mg, 2.5 mmol) was dissolved in 5 mL of DCM, added with 1 mL of HCl/1,4-dioxane solution, slowly heated to room temperature, and reacted for 2 h. LCMS detected the completion of the reaction. The reaction solution was dried to obtain the pale yellow oily crude Compound 274-4. 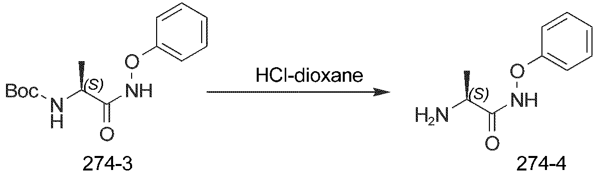
(3) At 0°C, Compound 164-4 (150 mg, 0.564 mmol) was dissolved in 5 mL of DCM, added with triethylamine (114 mg, 1.13 mmol), Compound 274-4 (121 mg, 0.677 mmol), EDCI (161 mg, 0.846 mmol), and HOBT (114 mg, 0.846 mmol), and reacted at room temperature for 4 h. The completion of the reaction was detected by LCMS. The reaction solution was quenched by saturated aqueous solution of ammonium chloride and extracted with DCM. The organic phase was purified through column chromatography (THF/PE = 1/3) to obtain pale yellow oily Compound 274*(2S,5S)* (120 mg, 50% yield).

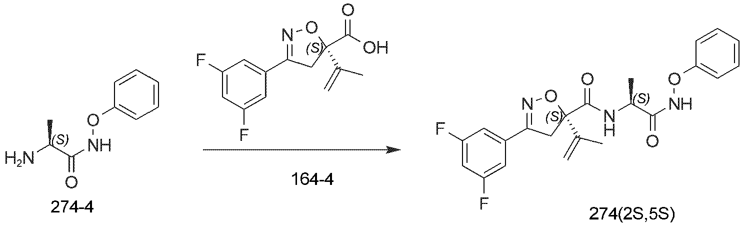

### 12. Synthesis of Compound 278(2S,5S)

Compound 278-1 was prepared with reference to Compound 21-4.

Compound 278-1 (100 mg, 0.29 mmol) was dissolved with 1.5 mL of dichloromethane in a 50-mL single-necked flask, added with triethylamine (59 mg, 0.58 mmol), Compound 278-2 (42 mg, 0.58 mmol), 1-hydroxybenzotriazole (HOBT, 59 mg, 0.44 mmol) and 1-(3-dimethylaminopropyl)-3-ethylcarbodiimide hydrochloride (EDCI, 84 mg, 0.44 mmol), and reacted at room temperature for 5 h. The completion of the reaction was detected by LCMS. The reaction solution was quenched with water and extracted with dichloromethane. The organic phase was washed with water, saturated ammonium chloride solution and saturated saline solution, dried and concentrated. The residue was purified through column chromatography (EA/PE = 1/10) to obtain Compound 278(2S,5S) (81 mg, 71.07% yield, pale yellow oily matter).

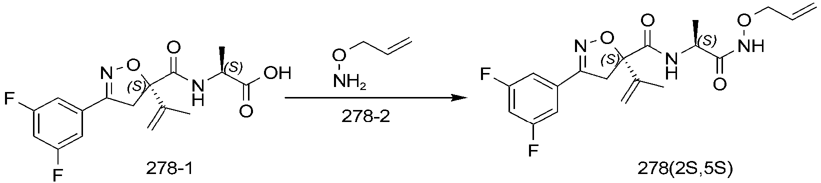

### 13. Synthesis of Compound 328(2S)

(1) Compound 328-1 (1000 mg, 5.18 mmol) was dissolved in 10 mL of tetrahydrofuran in a 100-mL three-necked flask and added with Compound 328-2 (2199 mg, 7.77 mmol). The air in the system was replaced three times with nitrogen gas. At -78 °C, lithium bis(trimethylsilyl)amide (1300 mg, 7.77 mmol) was slowly added dropwise to the reaction solution and reacted at 2 h at the constant temperature. The completion of the reaction was detected by LCMS. The reaction solution was quenched by saturated ammonium chloride solution and suction-filtered with a layer of diatomaceous earth on the bottom of the filter flask to remove insolubles. The filtrate was extracted with ethyl acetate. The organic phase was washed with water and saturated saline solution, dried and concentrated. The residue was purified through column chromatography (EA/PE = 1/10) to obtain Compound 328-3 (1500 mg, 60.84% yield, pale yellow solid). 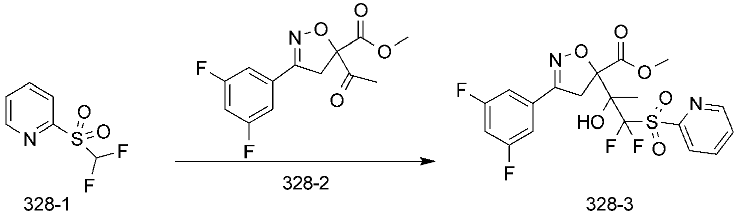
(2) Compound 328-3 (1500 mg, 3.15 mmol) was dissolved in 8 mL of N,N-dimethylformamide in a 50-mL single-necked flask, added with 6 M hydrochloric acid (345 mg, 9.45 mmol) and saturated ammonium chloride solution (505 mg, 9.45 mmol), and reacted at 120°C for 20 h. The completion of the reaction was detected by LCMS. The reaction solution was quenched by saturated sodium bicarbonate solution and extracted with ethyl acetate. The organic phase was washed with water and saturated saline solution, dried and concentrated. The residue was purified through column chromatography (EA/PE = 1/5) to obtain Compound 328-4 (200 mg, 20.03% yield, pale yellow solid). 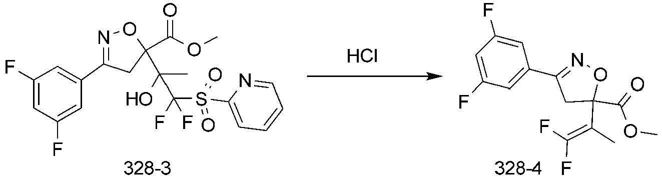
(3) Compound 328-4 (200 mg, 0.63 mmol) was dissolved in 2 mL of tetrahydrofuran in a 50-mL single-necked flask, added with aqueous solution (2 mL) of lithium hydroxide (23 mg, 0.95 mmol), and reacted at room temperature for 1 h. The completion of the reaction was detected by LCMS. The reaction solution was adjusted to pH ≈ 2 with 1 M hydrochloric acid and extracted with ethyl acetate. The organic phase was washed with water and saturated saline solution, dried and concentrated to obtain Compound 328-5 (150 mg, 78.58% yield, pale yellow crude product). 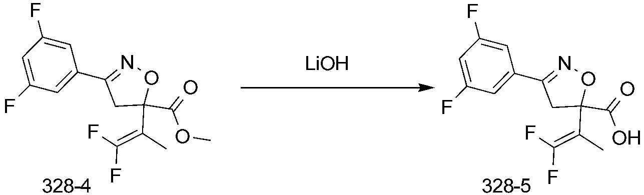
(4) Compound 328-5 (150 mg, 0.49 mmol) was dissolved with 2 mL of dichloromethane in a 50-mL single-necked flask, added with triethylamine (99 mg, 0.98 mmol), Compound 227-7 (129 mg, 0.98 mmol), 1-hydroxybenzotriazole (99 mg, 0.74 mmol), and 1-(3-dimethylaminopropyl)-3-ethylcarbodiimide hydrochloride (142 mg, 0.74 mmol), and reacted at room temperature for 5 h. The completion of the reaction was detected by LCMS. The reaction solution was quenched with water and extracted with dichloromethane. The organic phase was washed with water, saturated ammonium chloride solution and saturated saline solution, dried and concentrated. The residue was purified through column chromatography (EA/PE = 1/10) to obtain Compound 328(*2S*) (186 mg, 91.03% yield, pale yellow oily matter).

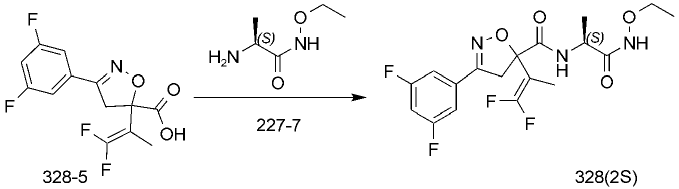

### 14. Synthesis of Compound 330

(1) Compound 330-1 (11.76 g, 31.80 mmol) was dissolved in 30 mL of tetrahydrofuran, in ice-water bath and under nitrogen gas atmosphere, added dropwise with NaHMDS (8.48 mL, 21.20 mmol), and reacted at room temperature for 1 h; Compound 328-2 (3.00 g, 10.60 mmol) was dissolved in 5 mL of tetrahydrofuran, in ice-water bath, added dropwise into the aforementioned reaction system, and reacted at room temperature for 12 h. The completion of the reaction was detected by LCMS. The reaction solution was quenched by aqueous solution of ammonium chloride. The aqueous phase was extracted with ethyl acetate. The organic phase was washed with saturated saline solution, dried and concentrated. The residue was purified through column chromatography (EA/PE = 1/10) to obtain Compound 330-2 (170 mg, 5.4% yield, yellow oily matter). 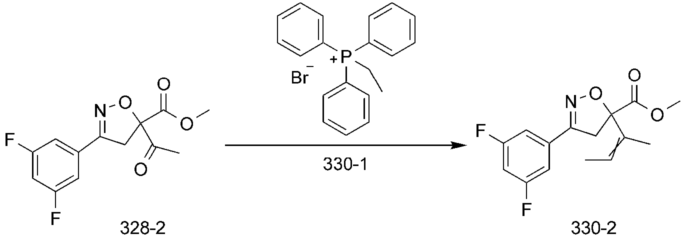
(2) Compound 330-2 (170 mg, 0.57 mmol) was dissolved in 3 mL of tetrahydrofuran and 1 mL of water, in ice-water bath, added with lithium hydroxide (21 mg, 0.86 mmol), and reacted at room temperature for 2 h. The completion of the reaction was detected by LCMS. The reaction solution was diluted with water and adjusted to acidity. The aqueous phase was extracted with ethyl acetate. The organic phase was washed with saturated saline solution, dried and concentrated to obtain the crude product Compound 330-3 (160 mg, yellow oily matter). 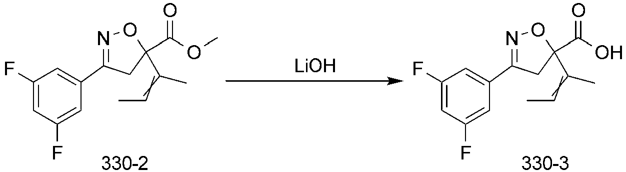
(3) Compound 330-3 (160 mg, 0.57 mmol) was dissolved in 2 mL of dichloromethane, sequentially added with triethylamine (172 mg, 1.70 mmol), Compound 227-7 (90 mg, 0.68 mmol), HOBT (115 mg, 0.85 mmol) and EDCI (163 mg, 0.85 mmol), and reacted at room temperature for 2 h. The completion of the reaction was detected by LCMS. The reaction solution was diluted with water and dichloromethane, and extracted with dichloromethane. The organic phase was washed with saturated ammonium chloride and saturated saline solution, dried over anhydrous sodium sulfate and concentrated. The residue was purified through column chromatography (EA/PE = 1/3) to obtain Compound 330 (140 mg, 62.5% yield, pale yellow oily matter).

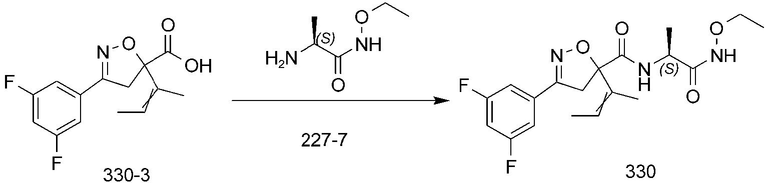

### Evaluation of Biological Activity:

The criteria for activity levels of plant damage (i.e., growth control rate) are as follows:
Level 9: completely dead;
Level 8: growth control rate greater than or equal to 90% and less than 100%;
Level 7: growth control rate greater than or equal to 80% and less than 90%;
Level 6: growth control rate greater than or equal to 70% and less than 80%;
Level 5: growth control rate greater than or equal to 50% and less than 70%;
Level 4: growth control rate greater than or equal to 30% and less than 50%;
Level 3: growth control rate greater than or equal to 20% and less than 30%;
Level 2: growth control rate greater than or equal to 10% and less than 20%;
Level 1: growth control rate less than 10%;
Level 0: no effect.

The above growth control rates are fresh weight control rates.

### Post-emergence test experiment:

Monocotyledonous and dicotyledonous weed seeds as well as major crop seeds were placed in plastic pots filled with soil and then covered with 0.5-2 cm of soil to allow them grow in a favorable greenhouse environment. Two weeks after sowing, the test plants were treated. The test compounds of the present invention were individually dissolved with acetone, then added with Tween^{®} 80 and the emulsifiable concentrate of methyl oleate as a synergist, diluted with certain amount of water to obtain solutions of certain concentrations, and sprayed to the plants using a spray tower. After drug application, the experimental results on weeds were collected after 3 weeks of cultivation in a greenhouse. The representative results are listed in Table 2.

**Table 2 Results of post-emergence test on weeds**

| **No.** | ***Echinochloa crus-galli*** | ***Digitaria sanguinalis*** | ***Avena fatua*** | ***Amaranthus retroflexus*** | **Dosage (g a.i./ha)** |
|---|---|---|---|---|---|
| 20(2S,5S) | 8 | 9 | 9 | N | 240 |
| 20(2S,5R) | N | N | 9 | N | 240 |
| 21(2S,5S) | N | N | 9 | N | 240 |
| 21(2R,5S) | N | N | 9 | N | 240 |
| 25(2S,5R) | N | N | 9 | N | 240 |
| 29(2S) | N | N | N | 6 | 60 |
| 47(2S,5S) | N | 5 | 9 | N | 240 |
| 68(2R) | N | N | 5 | N | 240 |
| 68(2S,5S) | N | N | 9 | N | 120 |
| 69(2R,5S) | N | N | 9 | N | 240 |
| 69(2R,5R) | N | N | 9 | N | 240 |
| 69(2S,5S) | N | 8 | N | 5 | 240 |
| 70(2S,5S) | N | N | 9 | N | 240 |
| 70(2S) | N | N | 9 | N | 240 |
| 73(2S,5S) | N | N | 9 | N | 240 |
| 74(2S,5S) | N | N | 9 | N | 240 |
| 75(2S,5S) | N | N | 6 | N | 240 |
| 104(2S,5S) | N | N | 9 | N | 240 |
| 126(2S,5S) | 5 | N | N | N | 240 |
| 138(2S,5S) | N | 6 | N | N | 240 |
| 139(2S,5S) | N | N | 9 | N | 240 |
| 148(2S,5S) | N | N | 9 | N | 240 |
| 154(2S,5S) | N | N | 9 | N | 120 |
| 155(2S,5S) | N | N | 9 | N | 120 |
| 164(2S,5S) | 9 | 8 | 8 | N | 240 |
| 165(2S,5S) | 8 | 9 | N | N | 240 |
| 166(2S,5S) | 8 | 9 | 9 | N | 240 |
| 167(2S) | 9 | 8 | 9 | N | 240 |
| 167(2R,5S) | N | N | 6 | N | 240 |
| 167(2S,5S) | 9 | 9 | 9 | N | 240 |
| 168(2S,5S) | N | 9 | N | N | 120 |
| 169(5S) | 9 | 8 | N | N | 60 |
| 169(2S,5S) | 8 | 6 | N | 6 | 60 |
| 171(2S,5R) | 5 | 6 | N | N | 240 |
| 173(2S,5R) | N | 5 | 9 | N | 240 |
| 208(2S,5S) | N | N | 8 | N | 240 |
| 226(2S,5S) | N | N | N | 7 | 60 |
| 228(2S,5S) | 8 | 8 | N | N | 60 |
| 229(2S,5S) | 6 | 8 | N | N | 60 |
| 230(2S,5S) | 8 | 9 | N | 8 | 60 |
| 231(2S,5S) | 8 | 8 | N | 6 | 60 |
| 232(2S,5S) | 6 | 9 | N | N | 60 |
| 233(5S) | 9 | 8 | N | N | 60 |
| 234(2S) | 5 | 6 | N | 5 | 60 |
| 236(2S,5S) | 6 | 8 | N | 8 | 60 |
| 237(2S,5S) | N | N | N | 5 | 60 |
| 238(2S,5S) | 9 | 8 | N | N | 60 |
| 239(2S,5S) | 6 | 8 | N | N | 60 |
| 240(2S,5S) | 9 | 8 | N | N | 60 |
| 241(2S,5S) | 8 | 9 | N | 5 | 60 |
| 242(2S,5S) | 6 | 8 | N | N | 60 |
| 243(2S,5S) | 8 | 8 | N | N | 60 |
| 244(2S,5S) | 8 | 8 | N | 8 | 60 |
| 245(2S,5S) | 8 | 9 | N | N | 60 |
| 246(2S,5S) | 9 | 9 | N | N | 60 |
| 247(2S,5S) | 8 | 8 | N | 6 | 60 |
| 248(2S,5S) | 8 | 8 | N | 5 | 60 |
| 249(2S,5S) | 6 | 8 | N | 6 | 60 |
| 250(2S,5S) | 6 | 8 | N | 8 | 60 |
| 251(2S,5S) | 8 | 8 | N | 5 | 60 |
| 252(2S,5S) | 8 | 6 | N | 6 | 60 |
| 253(2S,5S) | 8 | 9 | N | 6 | 60 |
| 254(2S,5S) | 9 | 8 | N | N | 60 |
| 255(2S,5S) | 8 | 8 | N | 6 | 60 |
| 256(2S,5S) | 8 | 8 | N | 5 | 60 |
| 257(2S,5S) | 6 | 8 | N | N | 60 |
| 258(2S,5S) | 8 | 8 | N | 6 | 60 |
| 259(2S,5S) | 8 | 9 | N | 5 | 60 |
| 260(2S,5S) | 9 | 8 | N | N | 60 |
| 261(2S,5S) | 9 | 8 | N | N | 60 |
| 262(2S,5S) | 8 | 6 | N | 6 | 60 |
| 263(2S,5S) | 8 | 8 | N | 8 | 60 |
| 264(2S,5S) | 8 | 8 | N | 5 | 60 |
| 265(2S,5S) | 8 | 8 | N | 8 | 60 |
| 266(2S,5S) | 8 | 8 | N | 5 | 60 |
| 267(2S,5S) | 9 | 8 | N | N | 60 |
| 268(2S,5S) | N | 5 | N | 5 | 60 |
| 269(2S,5S) | 8 | 9 | N | 5 | 60 |
| 270(2S,5S) | 8 | 8 | N | 6 | 60 |
| 271(2S,5S) | 8 | 6 | N | 6 | 60 |
| 272(2S,5S) | 9 | 8 | N | 6 | 60 |
| 273(2S,5S) | 8 | 6 | N | 5 | 60 |
| 274(2S,5S) | 5 | 8 | N | N | 60 |
| 275(2S,5S) | 8 | 8 | N | 6 | 60 |
| 276(2S,5S) | 8 | 8 | N | 6 | 60 |
| 277(2S,5S) | 8 | 8 | N | 5 | 60 |
| 278(2S,5S) | 8 | 8 | N | N | 60 |
| 279(2S,5S) | 8 | 8 | N | 6 | 60 |
| 280(2S,5S) | 8 | 8 | N | 5 | 60 |
| 282(2S,5S) | 8 | 9 | N | 6 | 60 |
| 283(2S,5S) | 8 | 8 | N | 6 | 60 |
| 284(2S,5S) | 9 | 9 | N | N | 60 |
| 285(2S,5S) | 8 | 8 | N | 8 | 60 |
| 286(2S,5S) | 8 | 8 | N | 6 | 60 |
| 287(2S,5S) | 8 | 8 | N | 8 | 60 |
| 288(2S,5S) | 8 | 8 | N | 6 | 60 |
| 289(2S,5S) | 8 | 8 | N | 5 | 60 |
| 290(2S,5S) | 9 | 9 | N | N | 60 |
| 291(2S,5S) | 9 | 8 | N | N | 60 |
| 292(2S,5S) | 8 | 8 | N | 5 | 60 |
| 293(2S,5S) | 9 | 9 | N | 8 | 60 |
| 294(2S,5S) | 9 | 9 | N | 8 | 60 |
| 295(2S,5S) | 6 | 6 | N | 8 | 60 |
| 296(2S,5S) | 6 | 8 | N | 6 | 60 |
| 297(2S,5S) | 8 | 8 | N | 8 | 60 |
| 298(2S,5S) | 6 | 8 | N | 5 | 60 |
| 299(2S,5S) | 8 | 9 | N | N | 60 |
| 300(2S,5S) | 8 | 8 | N | 5 | 60 |
| 301(2S,5S) | 6 | 8 | N | 8 | 60 |
| 302(2S,5S) | 8 | 9 | N | 8 | 60 |
| 303(2S,5S) | 8 | 8 | N | 6 | 60 |
| 304(2S,5S) | 9 | 9 | N | N | 60 |
| 305(2S,5S) | 8 | 6 | N | 5 | 60 |
| 306(2S,5S) | 8 | 8 | N | 6 | 60 |
| 307(2S,5S) | 6 | 8 | N | 6 | 60 |
| 308(2S,5S) | 8 | 8 | N | 5 | 60 |
| 309(5S) | N | N | N | 5 | 60 |
| 310(2S,5S) | 6 | 9 | N | N | 60 |
| 311 (2S,5S) | 6 | 8 | N | 5 | 60 |
| 312(2S,5S) | N | 6 | N | 5 | 60 |
| 313(2S,5S) | 8 | 9 | N | N | 60 |
| 314(2S,5S) | 9 | 9 | N | N | 60 |
| 315(2S,5S) | 6 | 9 | N | N | 60 |
| 316(2S,5S) | 9 | 9 | N | N | 60 |
| 317(2S,5S) | 6 | 8 | N | 6 | 60 |
| 318(2S,5S) | 8 | 9 | N | 8 | 60 |
| 319(2S,5S) | 6 | 9 | N | N | 60 |
| 320(2S,5S) | N | 6 | N | 5 | 60 |
| 321(2S,5S) | 6 | 8 | N | 8 | 60 |
| 322(2S,5S) | 6 | 8 | N | 5 | 60 |
| 323(2S,5S) | 8 | 8 | N | 5 | 60 |
| 324(2S,5S) | 8 | 8 | N | 5 | 60 |
| 325(2S,5S) | 8 | 8 | N | 6 | 60 |
| 326(2S,5S) | 6 | 8 | N | 6 | 60 |
| 327(2S,5S) | 8 | 8 | N | 6 | 60 |
| 332(2S,5S) | 8 | 8 | N | 6 | 60 |
| 334(2S,5S) | 9 | 9 | N | 8 | 60 |
| 335(2S,5S) | 9 | 9 | N | N | 60 |
| 336(2S,5S) | 8 | 8 | N | 8 | 60 |
| 337(2S,5S) | 8 | 8 | N | 8 | 60 |
| 338(2S,5S) | 8 | 8 | N | 6 | 60 |
| 339(2S,5S) | 9 | 9 | N | 8 | 60 |
| 340(2S,5S) | 8 | 8 | N | 8 | 60 |
| 341(2S,5S) | 9 | 9 | N | 8 | 60 |
| 342(2S,5S) | 8 | 6 | N | N | 60 |
| 345(2S,5S) | 5 | 5 | N | N | 60 |
| 347(2S,5S) | N | N | N | 5 | 60 |
| 348(2S,5S) | N | N | N | 5 | 60 |
| 349(2S,5S) | N | N | N | 6 | 60 |
| 352(2S,5S) | 5 | 5 | N | N | 60 |
| 353(2S,5S) | 6 | N | N | N | 60 |
| 354(5S) | 8 | 8 | N | 8 | 60 |
| 355(2S,5S) | 8 | 8 | N | 6 | 60 |
| 356(2S,5S) | 9 | 8 | N | N | 60 |
| 357(2S,5S) | 9 | 9 | N | 6 | 60 |
| 358(2S,5S) | N | N | N | 6 | 60 |
| 359(2S,5S) | 8 | 8 | N | 8 | 60 |
| 360(2S,5S) | 8 | 8 | N | 6 | 60 |
| 361(2S,5S) | 8 | 8 | N | 8 | 60 |
| 362(2S,5S) | 8 | 8 | N | 5 | 60 |
| 363(2S,5S) | 8 | 8 | N | 8 | 60 |
| 364(2S,5S) | 8 | 8 | N | 8 | 60 |
| 365(2S,5S) | 9 | 8 | N | 8 | 60 |
| 366(5S) | 6 | 8 | N | 8 | 60 |
| 367(2S,5S) | 6 | 8 | N | 8 | 60 |
| 368(2S,5S) | 8 | 8 | N | 6 | 60 |
| 369(2S,5S) | 8 | 8 | N | 5 | 60 |
| 370(2S,5S) | 8 | 8 | N | 8 | 60 |
| 371(2S,5S) | 8 | 6 | N | 8 | 60 |
| 372(2S,5S) | 8 | 8 | N | 8 | 60 |
| 373(2S,5S) | 8 | 8 | N | 8 | 60 |
| 374(2S,5S) | 8 | 8 | N | 6 | 60 |
| 375(2S,5S) | 6 | 6 | N | 6 | 60 |
| 376(2S,5S) | 8 | 8 | N | 6 | 60 |
| 378(2S,5S) | N | N | N | 6 | 60 |
| 381(2S,5S) | 6 | 5 | N | 5 | 60 |
| 384(2S,5S) | 9 | 8 | N | N | 60 |
| 385(2S,5S) | N | 5 | N | 6 | 60 |
| 386(2S,5S) | 5 | 6 | N | N | 60 |
| 387(2S,5S) | N | 5 | N | 8 | 60 |
| 388(2S,5S) | N | N | N | 8 | 60 |
| 389(2S,5S) | N | N | N | 6 | 60 |
| 393(2S,5S) | N | N | N | 5 | 60 |
| 394(2S,5S) | 8 | 5 | N | 6 | 60 |
| 396(2S) | 5 | N | N | N | 60 |
| 398(2S,5S) | 8 | 9 | N | 5 | 240 |
| 399(2S,5S) | 6 | 9 | N | N | 60 |
| 400(2S,5S) | 8 | 9 | N | 5 | 240 |
| 401 (2S,5S) | 8 | 9 | N | N | 240 |
| 402(2S,5S) | 6 | 6 | 9 | 6 | 240 |
| 404(2S,5S) | 8 | 8 | N | N | 60 |
| 405(2S,5S) | 8 | 8 | N | N | 240 |
| 406(2S,5S) | 5 | N | N | N | 240 |
| 407(2S,5S) | 8 | 8 | N | N | 60 |
| 409(2S) | N | N | N | 6 | 60 |
| 412(2S,5S) | 6 | 6 | N | N | 60 |
| 414(2S,5S) | 9 | 9 | N | 8 | 60 |
| 415(2S,5S) | 8 | 8 | N | 6 | 60 |
| 416(2S,5S) | 8 | 8 | N | N | 60 |
| 417(2S,5S) | 8 | 8 | N | N | 60 |
| 418(2S,5S) | 8 | 8 | N | N | 60 |
| 420(2S,5S) | 8 | 8 | N | N | 60 |
| 421(2S,5S) | 9 | 9 | N | 7 | 60 |
| 422(2S,5S) | 8 | 8 | N | N | 60 |
| 423(2S,5S) | 8 | 8 | N | N | 60 |
| 424(2S,5S) | 8 | 8 | N | N | 60 |
| 425(2S,5S) | 8 | 8 | N | N | 60 |
| 426(2S,5S) | 6 | 8 | N | N | 60 |
| 427(2S,5S) | 8 | 9 | N | 6 | 240 |
| 428(2S,5S) | 6 | 8 | N | N | 60 |
| 432(2S,5S) | 8 | 8 | N | 6 | 60 |
| 433(2S,5S) | 8 | 8 | N | 6 | 60 |
| 434(2S,5S) | 8 | 8 | N | N | 60 |
| 435(2S,5S) | 8 | 8 | N | 6 | 60 |
| 436(2S,5S) | 6 | 8 | N | N | 60 |
| 437(2S,5S) | 8 | 8 | N | N | 60 |
| 438(2S,5S) | 8 | 8 | N | N | 60 |
| 439(2S,5S) | 8 | 8 | N | 6 | 60 |
| 440(2S,5S) | 8 | 8 | N | N | 60 |
| 441(2S,5S) | 8 | 8 | N | N | 60 |
| 442(2S,5S) | 8 | 8 | N | N | 60 |
| 443(2S,5S) | 8 | 9 | N | N | 60 |
| 444(2S,5S) | 6 | 5 | N | N | 240 |
| 445(2S,5S) | 8 | 8 | N | N | 60 |
| 450(2S,5S) | 8 | 8 | N | N | 60 |
| 451(2S,5S) | 8 | 8 | N | N | 60 |
| 455(5S) | 8 | 8 | N | 8 | 60 |
| 456(2S,5S) | 8 | 8 | N | 6 | 60 |
| 457(2S,5S) | 8 | 8 | N | 6 | 60 |
| 458(2S,5S) | 8 | 8 | N | N | 60 |
| 459(2S,5S) | 8 | 8 | N | 6 | 60 |
| 460(2S,5S) | 8 | 8 | N | 6 | 60 |
| 461(2S,5S) | 8 | 8 | N | N | 60 |
| 462(2S,5S) | 8 | 8 | N | N | 60 |
| 463(2S,5S) | 8 | 8 | N | N | 60 |
| 464(2S,5S) | 6 | 8 | N | N | 60 |
| 467(5S) | N | 6 | N | N | 60 |
| 469(2S,5S) | 6 | 8 | N | N | 60 |
| 470(2S,5S) | 6 | 8 | N | N | 60 |
| 472(2S,5S) | 6 | 6 | N | N | 60 |
| 473(2S,5S) | 6 | 8 | N | 6 | 60 |
| 477(2S,5S) | 8 | 8 | N | 8 | 60 |
| 478(2S,5S) | 8 | 8 | N | 6 | 60 |
| 480(2S,5S) | 8 | 8 | N | N | 60 |
| 484(2S,5S) | N | N | N | 8 | 240 |
| Control Compound A | 0 | 0 | 0 | 0 | 60 |

| | | | | | |
|---|---|---|---|---|---|
| Note: N represents no data; Control compound A: 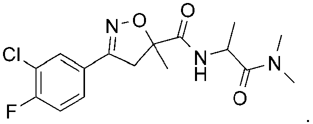 | | | | | |

### Pre-emergence test experiment:

Monocotyledonous and dicotyledonous weed seeds as well as major crop seeds were placed in plastic pots filled with soil and then covered with 0.5-2 cm of soil. The test compounds of the present invention were individually dissolved with acetone, then added with Tween^{®} 80, diluted with certain amount of water to obtain solutions of certain concentrations, and sprayed immediately after sowing. After drug application, the experimental results on weeds were observed after 4 weeks of cultivation in a greenhouse. The representative results are listed in Table 3.

**Table 3 Results of pre-emergence test on weeds**

| **No.** | ***Setaria viridis*** | ***Digitaria sanguinalis*** | ***Descurainia sophia*** | ***Lolium multiflorum*** | ***Zea mays*** | ***Triticum aestivum*** | **Dosage (g a.i./ha)** |
|---|---|---|---|---|---|---|---|
| 7(2S) | N | N | N | 9 | 2 | 2 | 240 |
| 17(2R) | N | N | 5 | N | 0 | 0 | 250 |
| 17(2S) | N | 8 | N | 9 | 2 | N | 240 |
| 18(2R) | 9 | 9 | N | 6 | 2 | N | 240 |
| 18(2S) | 9 | 9 | N | 9 | N | N | 240 |
| 18(2S,5R) | 8 | 8 | N | N | 2 | N | 240 |
| 19(2S) | N | 8 | N | N | 0 | N | 240 |
| 20(2R) | N | N | N | 8 | 0 | N | 240 |
| 20(2S,5S) | 5 | 5 | N | 8 | 1 | N | 240 |
| 20(2R,5S) | N | N | N | 8 | 1 | N | 240 |
| 21(2S,5S) | 9 | 9 | 9 | 9 | N | N | 250 |
| 21(2R,5S) | 9 | 9 | 6 | 9 | N | N | 250 |
| 21(2R) | 9 | 9 | N | N | 0 | N | 250 |
| 22 | 9 | 9 | N | 9 | N | N | 250 |
| 22(5S) | 6 | 6 | 9 | 9 | N | N | 240 |
| 25(2S,5R) | 9 | 9 | N | 9 | N | N | 240 |
| 27(2S,5S) | N | 5 | 9 | 9 | 2 | N | 240 |
| 27(2S,5R) | N | N | 6 | N | 0 | 0 | 240 |
| 29(2S) | 9 | 9 | 9 | 9 | 2 | N | 240 |
| 36(2S,5S) | 9 | 8 | N | 9 | 0 | N | 240 |
| 38(5S) | 8 | 5 | N | 8 | 2 | N | 240 |
| 39(2S,5S) | N | 5 | N | N | 2 | 0 | 240 |
| 40(2S,5R) | N | N | 5 | N | 0 | 1 | 240 |
| 44(2S,5S) | 9 | 9 | 9 | 9 | 1 | N | 240 |
| 45(2S,5S) | 9 | 9 | 8 | 9 | N | N | 240 |
| 46(2S,5S) | 5 | 5 | N | 7 | 2 | N | 240 |
| 47(2S,5S) | 9 | 9 | 7 | 9 | N | N | 240 |
| 47(2S,5R) | 5 | N | 5 | N | 0 | N | 240 |
| 49(2S,5S) | 9 | 9 | 8 | 9 | 2 | N | 240 |
| 68(2R) | 7 | 7 | N | 6 | 2 | 1 | 240 |
| 68(2S,5S) | 9 | 9 | 8 | 9 | 0 | N | 240 |
| 68(2R,5R) | N | N | 6 | N | 0 | 2 | 240 |
| 69(2R,5R) | N | N | 8 | N | 0 | 0 | 240 |
| 69(2R,5S) | N | 7 | 9 | 9 | 1 | N | 240 |
| 69(2S,5S) | N | N | 5 | 9 | 0 | N | 240 |
| 70(2S,5S) | 9 | 9 | 9 | 9 | 2 | N | 240 |
| 70(2S) | 7 | 7 | N | 7 | N | N | 240 |
| 73(2S,5S) | 9 | 9 | 6 | 9 | N | N | 240 |
| 74(2S,5R) | 5 | N | N | N | N | N | 240 |
| 75(2S,5S) | 9 | 9 | 8 | 9 | N | 2 | 240 |
| 75(2S,5R) | 5 | 5 | N | 5 | 0 | 2 | 240 |
| 76(2S,5S) | 8 | 8 | 7 | N | 2 | 2 | 240 |
| 82(2S,5S) | 9 | 9 | 6 | 9 | 1 | N | 240 |
| 84(2S) | 5 | N | 6 | N | 0 | N | 240 |
| 89(2S,5S) | N | N | N | 9 | 2 | N | 240 |
| 90(2S,5S) | N | 7 | N | N | 0 | 0 | 240 |
| 93(2R) | 8 | 8 | N | 9 | N | N | 240 |
| 94(2R) | N | N | 5 | N | 1 | 0 | 240 |
| 97(2S) | N | 5 | N | N | 0 | 0 | 240 |
| 98(2S,5S) | 9 | 9 | N | 8 | 2 | N | 240 |
| 100(2S,5S) | N | N | N | 7 | 0 | N | 240 |
| 101(2S,5S) | 7 | 5 | N | 6 | 0 | N | 240 |
| 102(2S,5S) | N | N | N | 5 | 0 | 0 | 240 |
| 104(2S,5S) | 7 | 8 | 9 | 9 | 0 | N | 240 |
| 105(2R,5S) | N | N | 5 | N | 1 | 0 | 240 |
| 105(2S,5S) | 9 | 9 | 9 | 9 | 0 | N | 240 |
| 105(5S) | 6 | 6 | N | 8 | 0 | N | 240 |
| 112(2S,5S) | N | 5 | N | N | 1 | N | 240 |
| 112(2S,5R) | N | 5 | N | N | 1 | N | 240 |
| 113(2S,5S) | 9 | N | N | N | 0 | N | 240 |
| 113(2S,5R) | N | N | 5 | N | 0 | 0 | 240 |
| 115(2S,5S) | N | 5 | N | N | 0 | N | 240 |
| 116(2S,5S) | N | N | N | 7 | 0 | N | 240 |
| 117(2S,5S) | N | N | N | 5 | 0 | N | 240 |
| 121(2S,5S) | N | N | 6 | N | 0 | 0 | 240 |
| 122(2S) | N | N | 8 | 5 | 0 | N | 240 |
| 122(2S,5S) | N | N | 7 | 9 | 0 | N | 240 |
| 123(2S,5S) | 9 | 9 | 9 | 9 | 0 | N | 240 |
| 124(2S) | 8 | 6 | N | 9 | 0 | N | 240 |
| 126(2S,5S) | N | N | 8 | 5 | 0 | N | 240 |
| 127(2S,5S) | 8 | 8 | N | N | 0 | 2 | 240 |
| 128(2S,5S) | N | N | N | 7 | N | N | 240 |
| 129(2S,5S) | N | N | 7 | 9 | 0 | N | 240 |
| 130(2S,5S) | N | N | 9 | 9 | 0 | N | 240 |
| 131(2S,5S) | N | N | N | 5 | 1 | 2 | 240 |
| 132(2S,5S) | N | N | 7 | 9 | 0 | N | 240 |
| 133(2S,5S) | N | N | 9 | N | 0 | N | 240 |
| 135(2S,5S) | N | N | N | 9 | 0 | N | 240 |
| 136(2S,5S) | N | N | 7 | 5 | 1 | N | 240 |
| 137(2S,5S) | N | N | 9 | 9 | 0 | N | 240 |
| 138(2R,5R) | N | N | 9 | N | 0 | 0 | 240 |
| 138(2R,5S) | 8 | 8 | 9 | 9 | 0 | N | 240 |
| 138(2S,5S) | N | N | 9 | 9 | 0 | N | 240 |
| 139(2S,5S) | 8 | 9 | 6 | 9 | 2 | N | 240 |
| 141(2R,5S) | N | N | 5 | N | 0 | 0 | 240 |
| 141(2R,5R) | N | N | 9 | 5 | 0 | N | 240 |
| 141 (2S,5S) | N | N | 9 | 9 | 2 | N | 240 |
| 142(2S,5S) | N | N | 5 | N | 0 | 1 | 240 |
| 143(2S,5R) | N | N | 5 | 7 | 0 | N | 240 |
| 144(2S,5S) | 9 | 8 | 9 | 9 | 0 | N | 240 |
| 145(2S,5S) | 9 | 5 | N | 9 | N | N | 240 |
| 147(2S) | N | N | 5 | N | 0 | 0 | 240 |
| 148(2S,5S) | 9 | 9 | N | 9 | N | N | 240 |
| 150(2S) | 6 | 8 | N | N | 0 | 0 | 240 |
| 151(2S) | 5 | N | N | N | 0 | 0 | 240 |
| 152(2S,5S) | 9 | 9 | N | 9 | 0 | 2 | 240 |
| 153(2S,5S) | 9 | 9 | N | 9 | 1 | N | 240 |
| 154(2S,5S) | N | N | N | 9 | 1 | N | 240 |
| 154(2R,5R) | 9 | N | N | N | 0 | 0 | 240 |
| 155(2S,5S) | 9 | 9 | N | 8 | 0 | N | 240 |
| 155(2S,5R) | N | N | 5 | N | 1 | 0 | 240 |
| 157(2S) | 9 | 9 | N | N | 0 | 0 | 240 |
| 159(2R) | N | N | 6 | N | 0 | 2 | 240 |
| 160(2S) | 6 | N | 6 | 9 | 1 | 1 | 240 |
| 161(2S) | N | N | N | 5 | 0 | N | 240 |
| 164(2S,5S) | 9 | 9 | N | 9 | N | N | 240 |
| 165(2S,5S) | 9 | 9 | 9 | 7 | 2 | N | 240 |
| 165(2S,5R) | 9 | 9 | N | N | N | 2 | 240 |
| 167(2S) | 9 | 9 | 9 | 9 | 2 | N | 240 |
| 167(2S,5S) | 9 | 9 | N | 9 | N | N | 240 |
| 168(2S,5S) | 9 | 9 | N | N | N | N | 240 |
| 169(5S) | 9 | 9 | 6 | 8 | N | N | 240 |
| 169(2S,5S) | 6 | 8 | N | N | N | N | 240 |
| 171(2S,5S) | 9 | 9 | N | 8 | N | N | 240 |
| 171(2S,5R) | 9 | 9 | 9 | 8 | N | N | 240 |
| 172(2S,5S) | 9 | 9 | 9 | 9 | 0 | 0 | 240 |
| 172(2S,5R) | N | N | 8 | N | 0 | 0 | 240 |
| 173(2S,5R) | 5 | 9 | N | 5 | 0 | 1 | 240 |
| 174(2S,5S) | 9 | 9 | N | 9 | 2 | 2 | 240 |
| 175(2S,5S) | 9 | 9 | N | 9 | 0 | N | 240 |
| 176(2S,5S) | 8 | 9 | 8 | 5 | 2 | 1 | 240 |
| 176(2S,5R) | 8 | 9 | 8 | 7 | 2 | 1 | 240 |
| 177(2S,5R) | 9 | 9 | N | 5 | N | 0 | 240 |
| 178(2S,5S) | 9 | 9 | N | 9 | 1 | N | 240 |
| 184(2S,5R) | 9 | 9 | N | 9 | 1 | 1 | 240 |
| 185(2S,5R) | 9 | 8 | N | 8 | 0 | 1 | 240 |
| 186(2S,5R) | 9 | 9 | 5 | 9 | 0 | 1 | 240 |
| 186(2S,5S) | N | N | 9 | N | 0 | 0 | 240 |
| 187(2S,5R) | 9 | 9 | N | 5 | 0 | 0 | 240 |
| 188(2S,5R) | 9 | 9 | N | 9 | 0 | 2 | 240 |
| 189(2S,5R) | 9 | 8 | N | 7 | 2 | N | 240 |
| 190(2S,5R) | 9 | 5 | 9 | 6 | 0 | 2 | 240 |
| 191(2S,5R) | 9 | 9 | N | 9 | N | N | 240 |
| 192(2S,5R) | N | 7 | 9 | N | 0 | 1 | 240 |
| 193(2S,5R) | 5 | 9 | N | N | 1 | 0 | 240 |
| 197(2S,5S) | 5 | 7 | N | N | 0 | 0 | 240 |
| 206(2S,5R) | N | N | 6 | 6 | 2 | N | 240 |
| 207(2S,5R) | 9 | 9 | N | 9 | 0 | N | 240 |
| 207(2S,5S) | N | N | 9 | N | 0 | 0 | 240 |
| 208(2S,5S) | 9 | 9 | N | 6 | 2 | N | 240 |
| 212(2R) | 5 | N | N | 9 | 0 | N | 240 |
| 212(2S) | N | N | 8 | 9 | 0 | N | 240 |
| 214(2S,5S) | N | N | N | 8 | 0 | N | 240 |
| 215(2S,5S) | N | N | N | 9 | 2 | N | 240 |
| 216(2S,5S) | N | N | 9 | N | 1 | 2 | 240 |
| 219(2S,5S) | N | N | N | 9 | 0 | N | 240 |
| 219(2S,5R) | N | N | 5 | N | 0 | 1 | 240 |
| 221(2S,5S) | N | N | N | 9 | 1 | N | 240 |
| 222(2S,5S) | N | 6 | N | 9 | 0 | N | 240 |
| 223(2S,5S) | N | N | N | 5 | 0 | 2 | 240 |
| 225(2S,5S) | N | N | N | 9 | 1 | N | 240 |
| 226(2S,5R) | N | N | 9 | 6 | 1 | 1 | 240 |
| 226(2S,5S) | N | N | 9 | 7 | 1 | N | 240 |
| 227(2S) | N | 6 | 9 | N | 1 | 1 | 240 |
| 228(2S,5S) | 9 | 9 | 9 | 9 | N | N | 240 |
| 229(2S,5S) | 9 | 9 | 9 | 9 | N | N | 240 |
| 230(2S,5S) | 9 | 9 | N | N | N | N | 240 |
| 231(2S,5S) | 9 | 9 | N | N | N | N | 240 |
| 232(2S,5S) | 9 | 9 | 6 | 9 | N | N | 240 |
| 233(5S) | 9 | 9 | N | 9 | N | N | 240 |
| 234(2S) | 6 | 9 | 5 | N | 1 | N | 240 |
| 236(2S,5S) | 8 | 9 | N | N | 1 | N | 240 |
| 237(2S,5S) | 6 | 9 | N | N | 1 | N | 240 |
| 238(2S,5S) | 9 | 9 | 9 | 9 | N | N | 240 |
| 239(2S,5S) | 8 | 9 | N | 9 | N | N | 240 |
| 240(2S,5S) | 9 | 9 | 9 | 9 | N | N | 240 |
| 242(2S,5S) | 9 | 9 | 9 | 9 | N | N | 240 |
| 243(2S,5S) | 9 | 9 | 5 | 9 | 2 | N | 240 |
| 244(2S,5S) | 9 | 9 | N | N | 2 | N | 240 |
| 245(2S,5S) | 9 | 9 | 6 | 9 | N | N | 240 |
| 246(2S,5S) | 9 | 9 | 5 | 9 | N | N | 240 |
| 247(2S,5S) | N | 6 | N | N | 1 | N | 240 |
| 248(2S,5S) | 9 | 9 | 7 | 7 | N | N | 240 |
| 249(2S,5S) | 9 | 8 | N | N | N | N | 240 |
| 250(2S,5S) | 9 | 9 | 6 | 5 | N | N | 240 |
| 251(2S,5S) | 9 | 9 | 8 | 6 | N | N | 240 |
| 252(2S,5S) | 9 | 9 | 9 | N | N | N | 240 |
| 254(2S,5S) | 8 | 9 | N | 9 | N | N | 240 |
| 255(2S,5S) | 9 | 9 | N | N | 1 | N | 240 |
| 256(2S,5S) | 9 | 9 | 6 | 6 | N | N | 240 |
| 257(2S,5S) | 8 | 9 | 9 | 9 | N | N | 240 |
| 258(2S,5S) | 9 | 9 | 9 | N | N | N | 240 |
| 259(2S,5S) | 9 | 8 | N | N | N | N | 240 |
| 260(2S,5S) | 8 | 8 | 9 | 9 | N | N | 240 |
| 261(2S,5S) | 6 | 5 | 9 | 9 | N | N | 240 |
| 262(2S,5S) | 6 | 8 | 9 | N | N | N | 240 |
| 263(2S,5S) | 9 | 8 | N | N | 1 | N | 240 |
| 264(2S,5S) | 9 | 9 | 6 | N | N | N | 240 |
| 265(2S,5S) | 9 | 9 | N | N | 1 | N | 240 |
| 266(2S,5S) | N | N | N | 9 | 1 | N | 240 |
| 267(2S,5S) | 6 | 6 | N | N | 2 | N | 240 |
| 268(2S,5S) | N | 6 | N | N | 1 | 1 | 240 |
| 269(2S,5S) | 6 | 8 | N | 9 | N | N | 240 |
| 270(2S,5S) | 8 | 9 | N | N | N | N | 240 |
| 271(2S,5S) | 8 | 9 | 9 | 5 | N | N | 240 |
| 272(2S,5S) | 9 | 8 | N | N | 1 | N | 240 |
| 273(2S,5S) | 9 | 9 | 9 | 5 | N | N | 240 |
| 274(2S,5S) | 9 | 9 | N | 9 | N | N | 240 |
| 275(2S,5S) | 9 | 9 | 8 | 8 | N | N | 240 |
| 276(2S,5S) | 9 | 9 | 9 | 6 | N | N | 240 |
| 277(2S,5S) | 9 | 9 | 6 | 6 | 1 | N | 240 |
| 278(2S,5S) | 9 | 9 | 9 | 9 | N | N | 240 |
| 279(2S,5S) | 9 | 9 | 8 | N | 1 | N | 240 |
| 280(2S,5S) | 9 | 9 | N | N | 1 | N | 240 |
| 281(2S,5S) | 9 | 9 | N | 9 | 1 | N | 240 |
| 282(2S,5S) | 9 | 9 | 6 | 5 | 1 | N | 240 |
| 283(2S,5S) | 9 | 9 | N | N | N | N | 240 |
| 284(2S,5S) | 6 | 9 | N | N | 2 | N | 240 |
| 285(2S,5S) | 9 | 9 | N | N | N | N | 240 |
| 286(2S,5S) | 9 | 9 | N | N | N | N | 240 |
| 287(2S,5S) | 9 | 9 | N | N | N | N | 240 |
| 288(2S,5S) | 9 | 9 | N | N | N | N | 240 |
| 289(2S,5S) | 9 | 9 | N | N | N | N | 240 |
| 290(2S,5S) | 8 | 8 | N | 9 | 1 | N | 240 |
| 291(2S,5S) | 9 | 9 | 5 | 9 | N | N | 240 |
| 292(2S,5S) | 9 | 9 | 8 | 5 | N | N | 240 |
| 293(2S,5S) | 9 | 9 | N | N | N | N | 240 |
| 294(2S,5S) | 9 | 9 | N | N | N | N | 240 |
| 295(2S,5S) | 7 | 9 | N | N | 1 | N | 240 |
| 296(2S,5S) | 6 | 8 | N | 6 | 1 | N | 240 |
| 297(2S,5S) | 8 | 9 | N | N | N | N | 240 |
| 298(2S,5S) | 8 | 9 | N | N | 1 | N | 240 |
| 299(2S,5S) | 8 | 8 | N | 9 | N | N | 240 |
| 300(2S,5S) | 9 | 9 | N | 5 | N | N | 240 |
| 301(2S,5S) | 8 | 8 | N | N | N | N | 240 |
| 302(2S,5S) | 8 | 9 | 8 | 6 | 1 | N | 240 |
| 303(2S,5S) | 8 | 9 | 8 | 5 | N | N | 240 |
| 305(2S,5S) | 9 | 9 | N | 5 | N | N | 240 |
| 306(2S,5S) | 8 | 8 | N | N | 1 | N | 240 |
| 307(2S,5S) | N | 5 | N | N | 1 | 1 | 240 |
| 308(2S,5S) | 8 | 9 | N | N | 1 | N | 240 |
| 310(2S,5S) | 6 | 8 | N | 5 | N | N | 240 |
| 311(2S,5S) | 6 | 6 | N | N | 2 | N | 240 |
| 312(2S,5S) | 6 | 8 | N | N | N | N | 240 |
| 314(2S,5S) | 6 | N | N | N | 0 | N | 240 |
| 317(2S,5S) | 8 | 8 | N | N | 1 | N | 240 |
| 319(2S,5S) | 6 | 8 | N | N | 0 | N | 240 |
| 320(2S,5S) | N | 6 | N | N | 1 | N | 240 |
| 321(2S,5S) | 9 | 9 | N | N | 1 | N | 240 |
| 322(2S,5S) | 6 | 8 | N | N | 1 | N | 240 |
| 323(2S,5S) | 9 | 9 | 9 | N | 1 | N | 240 |
| 324(2S,5S) | 9 | 9 | N | N | N | N | 240 |
| 325(2S,5S) | N | 8 | N | N | N | N | 240 |
| 326(2S,5S) | 6 | 8 | N | N | 1 | N | 240 |
| 327(2S,5S) | 6 | 9 | N | N | 1 | N | 240 |
| 331(2S,5R) | N | N | N | 6 | 1 | 1 | 240 |
| 332(2S,5S) | 8 | 9 | N | N | 1 | N | 240 |
| 334(2S,5S) | 9 | 9 | N | N | N | N | 240 |
| 335(2S,5S) | 9 | 9 | N | N | 2 | N | 240 |
| 336(2S,5S) | 8 | 9 | N | N | 1 | N | 240 |
| 337(2S,5S) | 7 | 8 | N | N | 1 | N | 240 |
| 338(2S,5S) | 9 | 9 | N | N | N | N | 240 |
| 339(2S,5S) | 9 | 9 | N | N | N | N | 240 |
| 340(2S,5S) | 7 | 9 | N | N | 1 | N | 240 |
| 341(2S,5S) | 9 | 9 | N | N | N | N | 240 |
| 342(2S,5S) | 8 | 9 | N | N | 1 | N | 240 |
| 343(2S,5S) | 6 | 8 | N | 8 | 0 | 1 | 240 |
| 344(2S,5S) | N | N | N | 6 | 0 | 0 | 240 |
| 345(2S,5S) | 8 | 6 | 8 | 9 | 2 | N | 240 |
| 345(2S,5R) | N | N | 9 | N | 1 | 1 | 240 |
| 352(2S,5S) | 7 | 8 | N | N | 1 | N | 240 |
| 354(5S) | 9 | 9 | N | N | 1 | N | 240 |
| 355(2S,5S) | 9 | 9 | N | N | N | N | 240 |
| 356(2S,5S) | 8 | 9 | 9 | 9 | N | N | 240 |
| 357(2S,5S) | 9 | 9 | N | 9 | N | N | 240 |
| 358(2S,5S) | 6 | 5 | N | N | 1 | N | 240 |
| 359(2S,5S) | 9 | 9 | N | N | 1 | N | 240 |
| 360(2S,5S) | 9 | 9 | N | N | N | N | 240 |
| 361(2S,5S) | 9 | 9 | N | N | N | N | 240 |
| 362(2S,5S) | 9 | 9 | N | N | 1 | N | 240 |
| 363(2S,5S) | 9 | 9 | N | N | N | N | 240 |
| 364(2S,5S) | 8 | 9 | N | N | 1 | 2 | 240 |
| 365(2S,5S) | 9 | 9 | N | 5 | N | 1 | 240 |
| 366(5S) | 9 | 9 | N | N | 1 | N | 240 |
| 367(2S,5S) | 8 | 9 | N | N | 1 | N | 240 |
| 368(2S,5S) | N | 9 | N | N | 1 | 1 | 240 |
| 369(2S,5S) | 9 | 9 | N | N | 1 | N | 240 |
| 370(2S,5S) | 9 | 9 | N | N | N | N | 240 |
| 371(2S,5S) | 9 | 9 | N | N | 1 | N | 240 |
| 372(2S,5S) | 8 | 9 | N | N | 1 | N | 240 |
| 373(2S,5S) | 9 | 9 | N | N | 1 | N | 240 |
| 374(2S,5S) | 8 | 8 | N | N | 1 | N | 240 |
| 375(2S,5S) | 8 | 9 | N | N | 1 | N | 240 |
| 376(2S,5S) | 9 | 9 | N | N | 1 | N | 240 |
| 377(2S,5S) | N | 5 | N | 9 | 0 | 2 | 240 |
| 378(2S,5S) | N | N | N | 9 | 1 | N | 240 |
| 381(2S,5S) | 6 | 8 | 9 | N | 1 | 1 | 240 |
| 386(2S,5S) | N | N | N | 9 | 2 | N | 240 |
| 387(2S,5S) | N | N | N | 9 | 1 | N | 240 |
| 388(2S,5S) | N | N | N | 6 | 2 | 1 | 240 |
| 389(2S,5R) | N | N | 9 | N | 1 | 1 | 240 |
| 390(2S,5R) | N | N | 8 | N | 1 | 1 | 240 |
| 391(2S) | N | N | N | 9 | 1 | N | 240 |
| 393(2S,5S) | 8 | 6 | 6 | N | 1 | 1 | 240 |
| 395(2S) | N | 5 | N | N | 1 | N | 240 |
| 402(2S,5S) | 8 | 8 | N | 9 | N | N | 240 |
| 403(2S,5S) | 9 | 9 | 9 | 8 | 0 | N | 240 |
| 404(2S,5S) | 9 | 9 | N | N | N | N | 240 |
| 405(2S,5S) | 8 | 6 | 5 | 9 | N | N | 240 |
| 406(2S,5S) | N | N | 9 | N | 1 | N | 240 |
| 407(2S,5S) | 9 | 9 | N | N | N | N | 240 |
| 409(2S) | 8 | 8 | 8 | 9 | 0 | N | 240 |
| 412(2S,5S) | 6 | 8 | N | N | N | N | 240 |
| 414(2S,5S) | 9 | 9 | N | N | N | N | 240 |
| 415(2S,5S) | 9 | 9 | N | N | N | N | 240 |
| 416(2S,5S) | 9 | 9 | N | N | N | N | 240 |
| 417(2S,5S) | 9 | 9 | N | N | N | N | 240 |
| 418(2S,5S) | 9 | 9 | N | N | N | N | 240 |
| 420(2S,5S) | 9 | 9 | N | N | N | N | 240 |
| 421(2S,5S) | 9 | 9 | N | N | N | N | 240 |
| 422(2S,5S) | 9 | 9 | N | N | 2 | N | 240 |
| 423(2S,5S) | 9 | 9 | N | N | N | N | 240 |
| 424(2S,5S) | 9 | 9 | N | N | N | N | 240 |
| 425(2S,5S) | 8 | 9 | N | N | N | N | 240 |
| 426(2S,5S) | 9 | 9 | N | N | N | N | 240 |
| 427(2S,5S) | 9 | 9 | N | 5 | N | N | 240 |
| 428(2S,5S) | 9 | 9 | N | N | N | N | 240 |
| 432(2S,5S) | 9 | 9 | N | N | N | N | 240 |
| 433(2S,5S) | 9 | 9 | N | N | N | N | 240 |
| 434(2S,5S) | 9 | 9 | N | N | N | N | 240 |
| 435(2S,5S) | 8 | 8 | N | N | N | N | 240 |
| 436(2S,5S) | 9 | 8 | N | N | N | 0 | 240 |
| 437(2S,5S) | 9 | 9 | N | N | N | N | 240 |
| 438(2S,5S) | 7 | 8 | N | N | N | N | 240 |
| 439(2S,5S) | 9 | 9 | N | N | N | N | 240 |
| 440(2S,5S) | 9 | 9 | N | N | N | N | 240 |
| 441(2S,5S) | 9 | 9 | N | N | N | N | 240 |
| 442(2S,5S) | 9 | 9 | N | N | N | N | 240 |
| 443(2S,5S) | 9 | 8 | N | N | 0 | N | 240 |
| 444(2S,5S) | N | N | 9 | 9 | 1 | N | 240 |
| 445(2S,5S) | 9 | 9 | N | N | N | N | 240 |
| 450(2S,5S) | 8 | 9 | N | N | N | N | 240 |
| 451(2S,5S) | 9 | 9 | N | N | N | N | 240 |
| 454(2S,5S) | N | N | 7 | 9 | 0 | N | 240 |
| 455(5S) | 9 | 9 | N | N | N | N | 240 |
| 456(2S,5S) | 9 | 9 | N | N | N | N | 240 |
| 457(2S,5S) | 6 | 9 | N | N | N | N | 240 |
| 458(2S,5S) | 9 | 9 | N | N | N | N | 240 |
| 459(2S,5S) | 9 | 9 | N | N | N | N | 240 |
| 460(2S,5S) | 9 | 9 | N | N | N | N | 240 |
| 461(2S,5S) | 9 | 9 | N | N | N | N | 240 |
| 462(2S,5S) | 9 | 9 | N | N | N | N | 240 |
| 463(2S,5S) | 9 | 9 | N | N | N | N | 240 |
| 464(2S,5S) | 9 | 9 | N | N | N | N | 240 |
| 467(5S) | 9 | 9 | N | N | N | 0 | 240 |
| 469(2S,5S) | 9 | 9 | N | N | N | N | 240 |
| 470(2S,5S) | 9 | 9 | N | N | 0 | N | 240 |
| 472(2S,5S) | 8 | 9 | N | N | 1 | 0 | 240 |
| 473(2S,5S) | 9 | 9 | N | N | 0 | 0 | 240 |
| 477(2S,5S) | 8 | 8 | N | N | 1 | 0 | 240 |
| 478(2S,5S) | 9 | 9 | N | N | 0 | N | 240 |
| 480(2S,5S) | 6 | 6 | N | N | 0 | N | 240 |
| 485(2S,5R) | N | N | N | 9 | 0 | N | 240 |
| Control Compound A | 1 | 1 | N | 2 | 0 | 0 | 240 |

| | | | | | | | |
|---|---|---|---|---|---|---|---|
| Note: N represents no data; Control compound A: 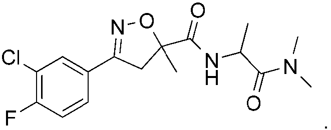 | | | | | | | |

Meanwhile, it has been found by experimentations on major weeds in wheat fields and paddy rice fields that the compounds of the present invention generally have good weed control effects; in particular, it has been noticed that the compounds have extremely high activity on broad-leaf weeds and *Cyperaceae* that are resistant to ALS inhibitors, for example, *Sagittaria trifolia, Schoenoplectiella juncoides, Cyperus difformis, Descurainia sophia, Capsella bursa-pastoris, Lithospermum arvense, Galium spurium, Cyperus rotundus,* etc., which have excellent commercial values.

### Safety evaluation on transplanted paddy rice and control effect evaluation on weeds in paddy fields:

After rice field soil was loaded into a 1/1,000,000 ha pot, the seeds of *Echinochloa crus-galli, Schoenoplectiella juncoides,* and *Bidens tripartita* were sowed and gently covered with soil. then left to stand in a greenhouse with water storage of 0.5-1 cm deep. The tuber of *Sagittaria trifolia* was planted on the next day or 2 days later. The water storage was kept at a depth of 3-4 cm thereafter. When the *Echinochloa crus-galli, Schoenoplectiella juncoides* and *Bidens tripartita* reached their 0.5-leaf stages and the *Sagittaria trifolia* reached the primary leaf stage, WP or SC aqueous dilutions of the compounds of the present invention formulated according to customary formulation methods were homogeneously dripped for treatment to the specified effective amount using pipettes.

In addition, after being loaded into the 1/1,000,000 ha pot, the paddy field soil was leveled to keep the water storage at a depth of 3-4 cm. The paddy rice (japonica rice) at 3-leaf stage was transplanted at a transplanting depth of 3 cm on the next day. The compound of the present invention was treated in the same manner as above on the 5^{th} day after the transplantation.

The developmental conditions of *Echinochloa crus-galli, Schoenoplectiella juncoides, Bidens tripartita,* and *Sagittaria trifolia* on the 14^{th} day after the drug treatment and the developmental conditions of rice on the 21^{st} day after the drug treatment were observed with the naked eye, respectively. The effects were evaluated according to the above-mentioned activity level criteria. Many compounds exhibited excellent activity and selectivity.

Note: *Echinochloa crus-galli, Schoenoplectiella juncoides* and *Bidens tripartita* seeds were collected from Heilongjiang, China and tested to be resistant to conventional doses of pyrazosulfuron-ethyl.

Meanwhile, it has been found by extensive experimentations that many of the compounds of the present invention and compositions thereof have good selectivity to gramineae grasses such as zoysia grass, bermuda grass, tall fescue, bluegrass, ryegrass, seashore paspalum, etc., and can prevent or eliminate many key gramineous weeds and broad-leaf weeds. The experiments on sugarcane, soybean, cotton, oil sunflower, potato, fruit trees, vegetables and the like using different drug application methods also demonstrated excellent selectivity and commercial values.

Finally, it should be noted that the above embodiments are only to illustrate the technical solutions of the present invention buy not to set limitations. Although the present invention has been described in detail with reference to preferred embodiments, it should be understood by one of ordinary skill in the art that the technical solutions of the present invention can be modified or equivalently replaced without departing from the spirit and the scope of the technical solutions of the present invention.

## Claims

1. An isoxazoline compound as shown in the general formula I or a salt thereof: 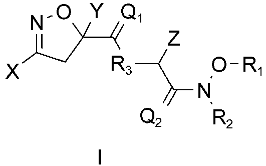
wherein, X is aryl or heterocyclyl;
Y is halogen, cyano, alkyl, alkenyl, alkynyl, cycloalkyl, cycloalkylalkyl, cycloalkenyl, cycloalkenylalkyl, haloalkyl, haloalkenyl, haloalkynyl, -OR, -SR, -alkylene-OR, -alkylene-O(CO)R, -alkylene-SR, -(CO)OR, or -(CO)R;
Z is hydrogen, halogen, alkyl, alkenyl, alkynyl, cycloalkyl, cycloalkylalkyl, cycloalkenyl, cycloalkenylalkyl, haloalkyl, haloalkenyl, haloalkynyl, cyanoalkyl, -OR, -SR, -N(R)₂, -alkylene-OR, -alkylene-SR, -alkylene-N(R)₂, -(CO)OR, -alkylene-(CO)OR, -alkylene-(CO)N(R)₂, -(CO)N(R)₂, aryl, heterocyclyl, arylalkyl, or heterocyclylalkyl;
R₁ is hydrogen, alkyl, alkenyl, alkynyl, cycloalkyl, cycloalkenyl, -alkylene-OR, -alkylene-SR, -alkylene-(SO)R, -alkylene-(SO)₂R, -alkylene-(CO)OR, -alkylene-(CO)N(R)₂, -alkylene-(CO)NR(OR), -alkylene-O-N=C(R)₂, -alkylene-(CO)O-N=C(R)₂, aryl, heterocyclyl; or alkyl, alkenyl, or alkynyl that are substituted by at least one group selected from halogen, cycloalkyl, cycloalkenyl, heterocyclyl, and aryl;
R₂ is hydrogen, alkyl, alkenyl, alkynyl, cycloalkyl, cycloalkylalkyl, cycloalkenyl, cycloalkenylalkyl, haloalkyl, haloalkenyl, haloalkynyl, aryl, heterocyclyl, arylalkyl, heterocyclylalkyl, -alkylene-OR, -alkylene-SR, cyanoalkyl, -(CO)OR, -SO₂R, or -SO₂N(R)₂;
or R₁ and R₂ together form -(CH₂)ₙ- that is unsubstituted or substituted by at least one group selected from alkyl, halogen, and oxo;
n is 3, 4, or 5;
R₃ is O, NH, or N-alkyl;
Q₁ and Q₂ each independently represent O or S;
the aforementioned cycloalkyl, cycloalkenyl, aryl, or heterocyclyl are optionally substituted by at least one group selected from oxo, halogen, cyano, nitro, alkyl, alkenyl, alkynyl, cycloalkyl, cycloalkylalkyl, haloalkyl, haloalkenyl, haloalkynyl, -OR, -SR, -alkylene-OR, -alkylene-SR, -O(CO)R, -(CO)R, -(CO)OR, -(CO)N(R)₂, -SO₂R, -OSO₂R, -N(R)₂, and -NR(OR);
R each independently represents hydrogen, alkyl, alkenyl, alkynyl; alkyl, alkenyl or alkynyl that are substituted by at least one group selected from halogen, hydroxy, alkoxy, cyano, trialkylsilyl, phenyl, and alkoxycarbonyl; cycloalkyl, cycloalkylalkyl, cycloalkenyl, cycloalkenylalkyl, phenyl, heterocyclyl; or phenyl or heterocyclyl that are substituted by at least one group selected from halogen, cyano, nitro, alkyl, haloalkyl, alkoxycarbonyl, alkylsulfanyl, alkylsulfonyl, alkoxy, haloalkoxy, phenyl, and halophenyl; or N(R)₂ together forms 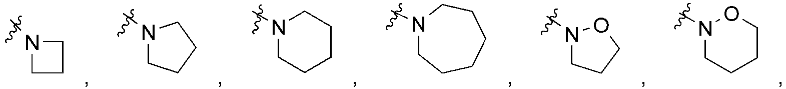 or

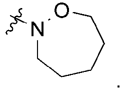

2. The isoxazoline compound or the salt thereof according to claim 1, wherein
Y is halogen, cyano, C1-C8 alkyl, C2-C8 alkenyl, C2-C8 alkynyl, C3-C8 cycloalkyl, C3-C8 cycloalkyl C1-C8 alkyl, C3-C8 cycloalkenyl, C3-C8 cycloalkenyl C1-C8 alkyl, halo C1-C8 alkyl, halo C2-C8 alkenyl, halo C2-C8 alkynyl, -OR, -SR, -(C1-C8 alkylene)-OR, -(C1-C8 alkylene)-O(CO)R, -(C1-C8 alkylene)-SR, -(CO)OR, or -(CO)R;
Z is hydrogen, halogen, C1-C8 alkyl, C2-C8 alkenyl, C2-C8 alkynyl, C3-C8 cycloalkyl, C3-C8 cycloalkyl C1-C8 alkyl, C3-C8 cycloalkenyl, C3-C8 cycloalkenyl C1-C8 alkyl, halo C1-C8 alkyl, halo C2-C8 alkenyl, halo C2-C8 alkynyl, cyano C1-C8 alkyl, -OR, -SR, -N(R)₂, -(C1-C8 alkylene)-OR, -(C1-C8 alkylene)-SR, -(C1-C8 alkylene)-N(R)₂, -(CO)OR, -(C1-C8 alkylene)-(CO)OR, -(C1-C8 alkylene)-(CO)N(R)₂, -(CO)N(R)₂, aryl, heterocyclyl, aryl C1-C8 alkyl, or heterocyclyl C1-C8 alkyl;
R₁ is hydrogen, C1-C8 alkyl, C2-C8 alkenyl, C2-C8 alkynyl, C3-C8 cycloalkyl, C3-C8 cycloalkenyl, -(C1-C8 alkylene)-OR, -(C1-C8 alkylene)-SR, -(C1-C8 alkylene)-(SO)R, -(C1-C8 alkylene)-(SO)₂R, -(C1-C8 alkylene)-(CO)OR, -(C1-C8 alkylene)-(CO)N(R)₂, -(C1-C8 alkylene)-(CO)NR(OR), -(C1-C8 alkylene)-O-N=C(R)₂, -(C1-C8 alkylene)-(CO)O-N=C(R)₂, aryl, heterocyclyl; or C1-C8 alkyl, C2-C8 alkenyl or C2-C8 alkynyl that are substituted by at least one group selected from halogen, C3-C8 cycloalkyl, C3-C8 cycloalkenyl, heterocyclyl, and aryl;
R₂ is hydrogen, C1-C8 alkyl, C2-C8 alkenyl, C2-C8 alkynyl, C3-C8 cycloalkyl, C3-C8 cycloalkyl C1-C8 alkyl, C3-C8 cycloalkenyl, C3-C8 cycloalkenyl C1-C8 alkyl, halo C1-C8 alkyl, halo C2-C8 alkenyl, halo C2-C8 alkynyl, aryl, heterocyclyl, aryl C1-C8 alkyl, heterocyclyl C1-C8 alkyl, -(C1-C8 alkylene)-OR, -(C1-C8 alkylene)-SR, cyano C1-C8 alkyl, -(CO)OR, -SO₂R, or -SO₂N(R)₂;
or R₁ and R₂ together form -(CH₂)ₙ- that is unsubstituted or substituted by at least one group selected from C1-C8 alkyl, halogen, and oxo;
n is 3, 4, or 5;
R₃ is O, NH, or N-(C1-C8 alkyl);
the aforementioned C3-C8 cycloalkyl, C3-C8 cycloalkenyl, aryl, or heterocyclyl are optionally substituted by at least one group selected from oxo, halogen, cyano, nitro, C1-C8 alkyl, C2-C8 alkenyl, C2-C8 alkynyl, C3-C8 cycloalkyl, C3-C8 cycloalkyl C1-C8 alkyl, halo C1-C8 alkyl, halo C2-C8 alkenyl, halo C2-C8 alkynyl, -OR, -SR, -(C1-C8 alkylene)-OR, -(C1-C8 alkylene)-SR, -O(CO)R, -(CO)R, -(CO)OR, -(CO)N(R)₂, -SO₂R, -OSO₂R, -N(R)₂, and -NR(OR);
R each independently represents hydrogen, C1-C8 alkyl, C2-C8 alkenyl, C2-C8 alkynyl; C1-C8 alkyl, C2-C8 alkenyl or C2-C8 alkynyl that are substituted by at least one group selected from halogen, hydroxy, C1-C8 alkoxy, cyano, tri C1-C8 alkylsilyl, phenyl, and C1-C8 alkoxycarbonyl; C3-C8 cycloalkyl, C3-C8 cycloalkyl C1-C8 alkyl, C3-C8 cycloalkenyl, C3-C8 cycloalkenyl C1-C8 alkyl, phenyl, heterocyclyl; or phenyl or heterocyclyl that are substituted by at least one group selected from halogen, cyano, nitro, C1-C8 alkyl, halo C1-C8 alkyl, C1-C8 alkoxycarbonyl, C1-C8 alkylsulfanyl, C1-C8 alkylsulfonyl, C1-C8 alkoxy, halo C1-C8 alkoxy, phenyl, and halophenyl; or N(R)₂ together forms

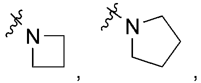

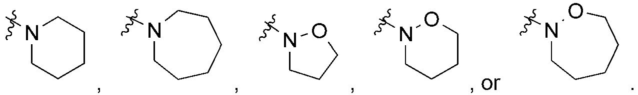

3. The isoxazoline compound or the salt thereof according claim 1 or 2, wherein
Y is halogen, cyano, C1-C6 alkyl, C2-C6 alkenyl, C2-C6 alkynyl, C3-C6 cycloalkyl, C3-C6 cycloalkyl C1-C6 alkyl, C3-C6 cycloalkenyl, C3-C6 cycloalkenyl C1-C6 alkyl, halo C1-C6 alkyl, halo C2-C6 alkenyl, halo C2-C6 alkynyl, -OR, -SR, -(C1-C6 alkylene)-OR, -(C1-C6 alkylene)-O(CO)R, -(C1-C6 alkylene)-SR, -(CO)OR, or -(CO)R;
Z is hydrogen, halogen, C1-C6 alkyl, C2-C6 alkenyl, C2-C6 alkynyl, C3-C6 cycloalkyl, C3-C6 cycloalkyl C1-C6 alkyl, C3-C6 cycloalkenyl, C3-C6 cycloalkenyl C1-C6 alkyl, halo C1-C6 alkyl, halo C2-C6 alkenyl, halo C2-C6 alkynyl, cyano C1-C6 alkyl, -OR, -SR, -N(R)₂, -(C1-C6 alkylene)-OR, -(C1-C6 alkylene)-SR, -(C1-C6 alkylene)-N(R)₂, -(CO)OR, -(C1-C6 alkylene)-(CO)OR, -(C1-C6 alkylene)-(CO)N(R)₂, -(CO)N(R)₂, aryl, heterocyclyl, aryl C1-C6 alkyl, or heterocyclyl C1-C6 alkyl;
R₁ is hydrogen, C1-C6 alkyl, C2-C6 alkenyl, C2-C6 alkynyl, C3-C6 cycloalkyl, C3-C6 cycloalkenyl, -(C1-C6 alkylene)-OR, -(C1-C6 alkylene)-SR, -(C1-C6 alkylene)-(SO)R, -(C1-C6 alkylene)-(SO)₂R, -(C1-C6 alkylene)-(CO)OR, -(C1-C6 alkylene)-(CO)N(R)₂, -(C1-C6 alkylene)-(CO)NR(OR), -(C1-C6 alkylene)-O-N=C(R)₂, -(C1-C6 alkylene)-(CO)O-N=C(R)₂, aryl, heterocyclyl; or C1-C6 alkyl, C2-C6 alkenyl or C2-C6 alkynyl that are substituted by at least one group selected from halogen, C3-C6 cycloalkyl, C3-C6 cycloalkenyl, heterocyclyl, and aryl;
R₂ is hydrogen, C1-C6 alkyl, C2-C6 alkenyl, C2-C6 alkynyl, C3-C6 cycloalkyl, C3-C6 cycloalkyl C1-C6 alkyl, C3-C6 cycloalkenyl, C3-C6 cycloalkenyl C1-C6 alkyl, halo C1-C6 alkyl, halo C2-C6 alkenyl, halo C2-C6 alkynyl, aryl, heterocyclyl, aryl C1-C6 alkyl, heterocyclyl C1-C6 alkyl, -(C1-C6 alkylene)-OR, -(C1-C6 alkylene)-SR, cyano C1-C6 alkyl, -(CO)OR, -SO₂R, or -SO₂N(R)₂;
or R₁ and R₂ together form -(CH₂)ₙ- that is unsubstituted or substituted by at least one group selected from C1-C6 alkyl, halogen, and oxo;
n is 3, 4, or 5;
R₃ is O, NH, or N-(C1-C6 alkyl);
the aforementioned C3-C6 cycloalkyl, C3-C6 cycloalkenyl, aryl, or heterocyclyl are optionally substituted by at least one group selected from oxo, halogen, cyano, nitro, C1-C6 alkyl, C2-C6 alkenyl, C2-C6 alkynyl, C3-C6 cycloalkyl, C3-C6 cycloalkyl C1-C6 alkyl, halo C1-C6 alkyl, halo C2-C6 alkenyl, halo C2-C6 alkynyl, -OR, -SR, -(C1-C6 alkylene)-OR, -(C1-C6 alkylene)-SR, -O(CO)R, -(CO)R, -(CO)OR, -(CO)N(R)₂, -SO₂R, -OSO₂R, -N(R)₂, and -NR(OR);
R each independently represents hydrogen, C1-C6 alkyl, C2-C6 alkenyl, C2-C6 alkynyl; C1-C6 alkyl, C2-C6 alkenyl or C2-C6 alkynyl that are substituted by at least one group selected from halogen, hydroxy, C1-C6 alkoxy, cyano, tri C1-C6 alkylsilyl, phenyl, and C1-C6 alkoxycarbonyl; C3-C6 cycloalkyl, C3-C6 cycloalkyl C1-C6 alkyl, C3-C6 cycloalkenyl, C3-C6 cycloalkenyl C1-C6 alkyl, phenyl, heterocyclyl; or phenyl or heterocyclyl that are substituted by at least one group selected from halogen, cyano, nitro, C1-C6 alkyl, halo C1-C6 alkyl, C1-C6 alkoxycarbonyl, C1-C6 alkylsulfanyl, C1-C6 alkylsulfonyl, C1-C6 alkoxy, halo C1-C6 alkoxy, phenyl, and halophenyl; or N(R)₂ together forms 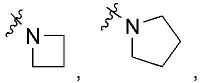 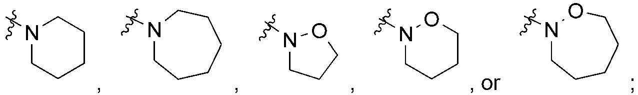
preferably, the compound is selected from any one in Table 1 and Tables A-H in the Specification.

4. An isoxazoline compound with a chiral center as shown in Formula I' or a salt thereof: 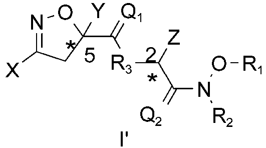
wherein, the carbon atom(s) (C*) at position 2 and/or 5 is a chiral center, and the definitions of substituents R₁, R₂, R₃, Q₁, Q₂, X, Y, and Z are as described in any one of claims 1 to 3 and Z is not hydrogen;
based on the contents of stereoisomers that have R- and S-configuration at position 2, it has a stereochemical purity of 60%-100% (S), preferably 70%-100% (S), more preferably 80%-100% (S), further preferably 90%-100% (S), even further preferably 95%-100% (S); or it has a stereochemical purity of 60%-100% (R), preferably 70%-100% (R), more preferably 80%-100% (R), further preferably 90%-100% (R), even further preferably 95%-100% (R);
and/or on an occasion of the carbon atom at position 5 being a chiral center, based on the contents of stereoisomers that have R- and S-configuration at this position, it has a stereochemical purity of 60%-100% (S), preferably 70%-100% (S), more preferably 80%-100% (S), further preferably 90%-100% (S), even further preferably 95%-100% (S); or it has a stereochemical purity of 60%-100% (R), preferably 70%-100% (R), more preferably 80%-*100%* (R), further preferably 90%-100% (R), even further preferably 95%-100% (R).

5. A method for preparing the isoxazoline compound or the salt thereof according to any one of claims 1 to 4, wherein it comprises the following steps:
(1) subjecting the compound shown in the general formula II and the compound shown in the general formula III or its salt to reaction to produce the compound as shown in the general formula I, and the reaction equation is as follows: 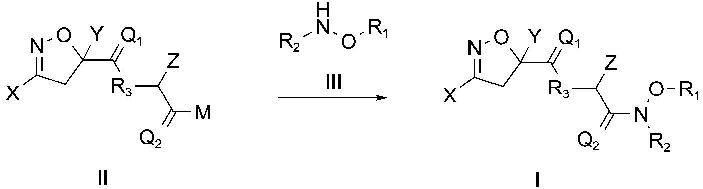
or (2) subjecting the compound shown in the general formula IV and the compound shown in the general formula V or its salt to reaction to produce the compound as shown in the general formula I, and the reaction equation is as follows: 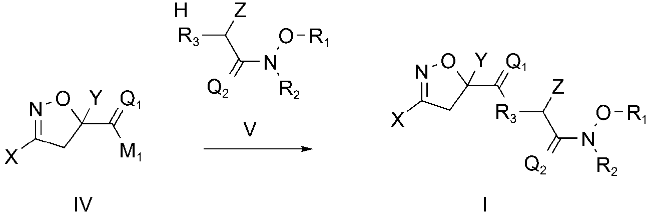
or (3) subjecting the compound shown in the general formula VI and the compound shown in the general formula VII to reaction to produce the compound as shown in the general formula I, and the reaction equation is as follows: 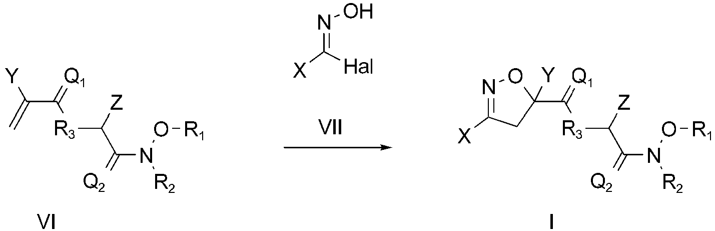
wherein, M and M₁ each independently represent OH or halogen; Hal represents halogen; the definitions of substituents R₁, R₂, R₃, Q₁, Q₂, X, Y, and Z are as described in any one of claims 1 to 4;
preferably, the reactions (1) and (2) are carried out in the presence of a solvent, respectively; more preferably, a base and/or a condensing agent is added during the reactions; further preferably, the base is selected from at least one of inorganic bases and organic bases; the condensing agent is selected from at least one of Py-BOP, Py-AOP, EDCI, HOBT, DCC, HBTU, and HATU; the solvent is selected from at least one of DMF, DMA, acetonitrile, dichloroethane, DMSO, dioxane, dichloromethane, tetrahydrofuran, and ethyl acetate;
preferably, the reaction (3) is carried out in the presence of a base and a solvent; more preferably, the base is selected from at least one of inorganic bases and organic bases; the solvent is selected from at least one of methanol, ethanol, isopropanol, DMF, DMA, acetonitrile, dichloroethane, DMSO, dioxane, dichloromethane, tetrahydrofuran, and ethyl acetate.

6. An intermediate, as shown in Formula II, III, V, or VI according to claim 5.

7. A herbicidal composition, wherein it comprises a herbicidally effective amount of at least one of the isoxazoline compound or the salt thereof according to any one of claims 1 to 4; preferably, also comprising a formulation auxiliary; more preferably, also comprising an additional active ingredient.

8. A method for controlling a weed, wherein it comprises applying a herbicidally effective amount of at least one of the isoxazoline compound or the salt thereof according to any one of claims 1 to 4 or the herbicidal composition according to claim 7 to a plant or a weedy area.

9. Use of at least one of the isoxazoline compound or the salt thereof according to any one of claims 1 to 4 or the herbicidal composition according to claim 7 in weed control; preferably, the isoxazoline compound or the salt thereof is used to control a weed among a useful crop, and the useful crop is a transgenic crop or a crop treated by genome editing techniques.
